# EUROPEAN PATENT APPLICATION

(11) **EP 2 065 383 A1**
(43) Date of publication of application: **03.06.2009**
(21) Application number: 08022584.0
(22) Date of filing: 19.11.2004
(51) Int. Cl.: C07D 403/04, C07D 401/14, C07D 403/14, C07D 403/12, A61K 31/416, A61K 31/4196

(54) **Indazole compounds and methods of use thereof as protein kinase inhibitors**

(30) Priority: 19.11.2003 US 608929 P
(62) Divisional of application: 04811774.1
(71) Applicant: SIGNAL PHARMACEUTICALS, INC., San Diego, CA 92121 (US)
(72) Inventor: Bhagwat, Shripad, S., San Diego, CA 92130 (US); Satoh, Yoshitaka, San Diego, CA 92131 (US); Sakata, Steven, T., San Diego, CA 92130 (US); Buhr, Chris, A., Redwood City, CA 94061 (US); Albers, Ronald, San Diego, ca 94111 (US); Sapienza, John, Chula Vista, CA 91913 (US); Plantevin, Veronique, San Diego, CA 92126 (US); Chao, Qi, San Diego, CA 92130 (US); Sahasrabudhe, Kiran, San Diego, CA 92130-3002 (US); Stengone, Rachel, San Diego, CA 92129 (US); Narla, Rama, K., San Diego, CA 92130 (US)
(74) Representative: Ritter, Thomas Kurt

(57) **Abstract**

This invention is directed to Indazole Compounds or pharmaceutically acceptable salts, solvates and hydrates thereof. The Indazole Compounds have utility in the treatment or prevention of a wide range of diseases and disorders that are responsive to the inhibition, modulation or regulation of kinases, such as inflammatory diseases, abnormal angiogenesis and diseases related thereto, cancer, atherosclerosis, a cardiovascular disease, a renal disease, an autoimmune condition, macular degeneration, disease-related wasting, an asbestos-related condition, pulmonary hypertension, diabetes, obesity, pain and others. Thus, methods of treating or preventing such diseases and disorders are also disclosed, as are pharmaceutical compositions comprising one or more of the Indazole Compounds. This invention is based, in part, upon the discovery of a novel class of 5-triazolyl substituted indazole molecules that have potent activity with respect to the modulation of protein kinases. Thus, the invention encompasses orally active molecules as well as parenterally active molecules which can be used at lower doses or serum concentrations for treating diseses or disorders associated with protein kinase signal transduction.

## Description

This application claims the benefit of U.S. provisional application no. 60/608,929, filed November 19, 2003, which is incorporated by reference herein in its entirety.

### 1. FIELD OF THE INVENTION

This invention is generally directed to novel compounds and their use in methods for treating or preventing diseases associated with protein kinases, including tyrosine kinases, such as inflammatory diseases, abnormal angiogenesis and diseases related thereto, cancer, atherosclerosis, macular degeneration, diabetes, obesity, pain and others. The methods comprise the administration to a patient in need thereof of a therapeutically effective amount of an indazole compound that inhibits, modulates or regulates one or more protein kinases. Novel indazole compounds or pharmaceutically acceptable salts thereof are presented herein.

### 2. BACKGROUND OF THE INVENTION

The protein kinases are a family of enzymes that catalyze protein phosphorylation and play a critical role in cellular signaling. Protein kinases may exert positive or negative regulatory effects, depending upon their target protein. Protein kinases can be divided into broad groups based upon the identity of the amino acid that they target (serine/threonine, tyrosine, lysine, and histidine). There are also dual-specific protein kinases that target both tyrosine and serine/threonine.

Any particular cell contains many protein kinases - some phosphorylate other protein kinases - some phosphorylate many different proteins, others only a single protein. Not surprisingly, there are several classes of protein kinases.

Protein kinases regulate nearly every cellular process, including metabolism, cell proliferation, cell differentiation, and cell survival, and are attractive targets for therapeutic intervention for certain disease states. For example, cell-cycle control and angiogenesis, in which protein kinases play a pivotal role are cellular processes associated with numerous disease conditions such as cancer, inflammatory diseases, abnormal angiogenesis and diseases related thereto, atherosclerosis, macular degeneration, diabetes, obesity, pain and others.

The tyrosine kinases can be of the receptor type (having extracellular, transmembrane and intracellular domains) or the non-receptor type (being wholly intracellular). For example, the non-receptor protein tyrosine kinase, LCK, is believed to mediate the transduction in T-cells of a signal from the interaction of a cell-surface protein (Cd4) with a cross-linked anti-Cd4 antibody. A detailed discussion of non-receptor tyrosine kinases is provided in Bolen, Oncogene, 8, 2025-2031 (1993).

The non-receptor tyrosine kinases represent a group of intracellular enzymes which lack extracellular and transmembrane sequences. Currently over 32 families of non-receptor tyrosine kinases have been identified. Oncogene 19:5548-5557 (2000). Examples are Src, Btk, Csk, ZAP70, Kak families. In particular the Src family of non-receptor tyrosine kinase family is the largest consisting of Src, Yes, Fyn, Lyn, Lck, Blk, Hck, Fgr and Yrk protein tyrosine kinases. The Src family of kinases have been linked to oncogenesis, cell proliferation and tumor progression. Detailed discussion of non-receptor protein tyrosine kinases is available in Oncogene 8:2025-2031 (1993). Many of these protein tyrosine kinases have been found to be involved in cellular signaling pathways involved in various pathological conditions including but not limited to cancer and hyperproliferative disorders and immune disorders. Small molecule inhibitors that modulate the activity of protein tyrosine kinases are useful for the prevention and treatment of above mentioned disease conditions have been identified. As such the identification of small molecule inhibitors which specifically inhibit signal transduction by modulating the activity of receptor and non-receptor tyrosine kinases and serine/threonine kinases to regulate abnormal or inappropriate cell proliferation, differentiation, and angiogenesis process and processes leading to the development and promotion of cancer associated disorders would be beneficial.

Protein kinases such as CHK1, which belongs to a family of serine/threonine protein kinases, play an important role as checkpoints in cell cycle progression. Checkpoints are control systems that coordinate cell cycle progression by influencing the formation, activation and subsequent inactivation of the cyclin-dependent kinases. Checkpoints prevent cell cycle progression at inappropriate times, maintain the metabolic balance of cells while the cell is arrested, and in some instances can induce apoptosis (programmed cell death) when the requirements of the checkpoint have not been met. *See, e.g.,* O'Connor, Cancer Surveys, 29, 151-182 (1997); Nurse, Cell, 91, 865-867 (1997); Hartwell et al., Science, 266, 1821-1828 (1994); Hartwell et al., Science, 246, 629-634 (1989). CDKs constitute a class of enzymes that play critical roles in regulating the transitions between different phases of the cell cycle, such as the progression from a quiescent stage in G₁ (the gap between mitosis and the onset of DNA replication for a new round of cell division) to S (the period of active DNA synthesis), or the progression from G₂ to M phase, in which active mitosis and cell-division occur. *See, e.g.,* the articles compiled in Science, vol. 274 (1996), pp. 1643-1677; and Ann. Rev. Cell Dev Biol, vol. 13 (1997), pp. 261-291. CDK complexes are formed through association of a regulatory cyclin subunit (e.g., cyclin A, B1, B2, D1, D2, D3, and E) and a catalytic kinase subunit (e.g., cdc2 (CDK1), CDK2, CDK4, CDK5, and CDK6). As the name implies, the CDKs display an absolute dependence on the cyclin subunit in order to phosphorylate their target substrates, and different kinase/cyclin pairs function to regulate progression through specific portions of the cell cycle.

Emerging data provide strong validation for the use of compounds inhibiting CDKs, and CDK4 and CDK2 in particular, as anti-proliferative therapeutic agents and several small molecules have been identified as CDK inhibitors (for recent reviews, *see* Webster, "The Therapeutic Potential of Targeting the Cell Cycle," Exp. Opin. Invest. Drugs, vol. 7 (1998), pp. 865-887, and Stover, et al., "Recent advances in protein kinase inhibition: current molecular scaffolds used for inhibitor synthesis," Current Opinion in Drug Discovery and Development, Vol. 2 (1999), pp. 274-285).

The p90 ribosomal S6 kinases (RSK) are serine/threonine kinases. The RSK family members have a role in mitogen-activated cell growth and proliferation, differentiation, and cell survival. The RSK family members are activated by extracellular signal-related kinases 1/2 and phosphoinositide-dependent protein kinase 1 (Frodin, M., and Gammeltoft, S. (1999) Mol. Cell. Endocrinol. 151, 65-77). Under basal conditions, RSK are localized in cytoplasm of cells and upon stimulation by mitogens, the activated (phosphorylated by extracellular-related kinase) RSK transiently traslocates to plasma membrane and become fully activated. The fully actiated RSK phosphorylates its substrates that are involved in cell growth and proliferation, differentiation, and cell survival (Richards, S. A., Fu, J., Romanelli, A., Shimamura, A., and Blenis, J. (1999) Curr. Biol. 9, 810-820; Richards, S. A., Dreisbach, V. C., Murphy, L. O., and Blenis, J. (2001) Mol. Cell. Biol. 21, 7470-7480). RSK signaling pathways have also been associated either modulation of cell cycle (Gross et al., J. Biol. Chem. 276(49): 46099-46103, 2001). Current data suggests that small molecules inhibiting RSK may be useful therapeutic agents for the prevention and treatment of cancer and inflammatory diseases.Other kinases such as AURORA, ROCK-II, Blk, GSK3α and β, p70S6K, PKCµ, PKD2, PRAK, and PRK2 have also been implicated in cellular processes.

Aurora kinases are a family of multigene mitotic serine-threonine kinases that functions as a class of novel oncogenes. These kinase comprise auroa-A, aurora-B and aurora-B members. These are hyperactivated and/or over-expressed in several solid tumors including but not limited to breast, ovary, prostate, pancrease, and colorectal cancers. In particular aurora-A is centrosome kinase and its localization depends on the cell cycle and plays an important role cell cycle progression and cell proliferation. Aurora-A is located in the 20q13 chromosome region that is frequently amplified in several different types of malignant tumors such as colorectal, breast and bladder cancers. There is a high correlation between aurora-A, high histo-prognostic grade, aneuploidy makes the kinase a potential prognostic factor. Inhibition of aurora kinase activity could help to reduce cell proliferation, tumor growth and potentially tumorigenesis. The detailed description of aurora kinase function is reviewed in Oncogene 21:6175-6183 (2002).

The Rho-associated coiled-coil-containing protein serine/threonine kinases ROCK-I and ROCK-II are thought to play a major role in cytoskeletal dynamics by serving as downstream effectors of the Rho/Rac family of cytokine- and growth factor-activated small GTPases. ROCKs phosphorylate various substrates, including myosin light chain phosphatase, myosin light chain, ezrin-radixin-moesin proteins and LIM (for Lin11, Isl1 and Mec3) kinases, and mediate the formation of actin stress fibres and focal adhesions in various cell types. ROCKs are known to have an important role in cell migration by enhancing cell contractility. For instance, they are required for tail retraction of monocytes and cancer cells. By way of example, a ROCK inhibitor has been used to reduce tumour-cell dissemination *in vivo.* Moreover, recent experiments have defined new functions of ROCKs in cells, including centrosome positioning and cell-size regulation, which might contribute to various physiological and pathological states. A detailed review can be found in Nature Reviews Molecular Cell Biology 4, 446 -456 (2003). As such, the ROCK family members are attractive intervention targets for a variety of pathologies, including cancer and cardiovascular disease. A pharmaceutical agent containing Rho kinase inhibitory activity is a good therapeutic agent for hypertension, angina pectoris, a suppressive agent of cerebrovascular contraction, a therapeutic agent of asthma, a therapeutic agent of peripheral circulation disorder, a therapeutic agent of arteriosclerosis, an anti-cancer drug, an anti-inflammatory agent, an immunosuppressant, a therapeutic agent of autoimmune disease, an anti-AIDS drug, a therapeutic agent of osteoporosis, a therapeutic agent of retinopathy, a brain function improving drug, a prophylactic agent of immature birth, a contraceptive and a prophylactic agent of digestive tract infection.

The 70 kDa ribosomal S6 kinase (p70S6K) is activated by numerous mitogens, growth factors and hormones. Activation of p70S6K occurs through phosphorylation at a number of sites and the primary target of the activated kinase is the 40S ribosomal protein S6, a major component of the machinery involved in protein synthesis in mammalian cells. In addition to its involvement in regulating translation, p70S6K activation has been implicated in cell cycle control, neuronal cell differentiation, regulation of cell motility and a cellular response that is important in tumour metastases, the immune response and tissue repair. Modulation of p70S6 kinase activity may have therapeutic implications for above mentioned deseases such as cancer, inflammation and immune and neuronal disorders. A detailed discussion can be found in Prog Cell Cycle Res. 1:21-32 (1995), Immunol Cell Biol. 78(4):447-51 (2000).

Glycogen synthase kinase 3 (GSK-3) are ubiquitously expressed constitutively active serine/threonine kinase that phosphorylates cellular substrates and thereby regulates a wide variety of cellular functions, including development, metabolism, gene transcription, protein translation, cytoskeletal organization, cell cycle regulation, and apoptosis. GSK-3 was initially described as a key enzyme involved in glycogen metabolism, but is now known to regulate a diverse array of cell functions. Two forms of the enzyme, GSK-3alpha and GSK-3beta, have been previously identified. The activity of GSK-3beta is negatively regulated by protein kinase B/Akt and by the Wnt signaling pathway. Small molecules inhibitors of GSK-3 may, therefore, have several therapeutic uses, including the treatment of neurodegenerative diseases, diabetes type II, bipolar disorders, stroke, cancer, and chronic inflammatory disease. See e.g., "Role of glycogen synthase kinase-3 in cancer: regulation by Wnts and other signaling pathways," Adv Cancer Res. 2002;84:203-29. GSK-3 inhibitors have been identified as new promising drugs for diabetes, neurodegeneration, cancer, and inflammation (Med Res Rev. 2002 Jul;22(4):373-84); Role of glycogen synthase kinase-3 in the phosphatidylinositol 3-Kinase/Akt cell survival pathway. (J Biol Chem. 1998, 273(32):19929-32).

The protein kinase D family of enzymes consists of three isoforms: PKD1/PKCmu PKD2 and PKD3/PKCnu. They all share a similar architecture with regulatory sub-domains that play specific roles in the activation, translocation and function of the enzymes. The PKD enzymes have recently been implicated in very diverse cellular functions, including Golgi organization and plasma membrane directed transport, metastasis, immune responses, apoptosis and cell proliferation.

Mitogen-activated protein (MAP) kinases are proline-directed serine/threonine kinases that are activated by dual phosphorylation on threonine and tyrosine residues in response to a wide array of extracellular stimuli. Three distinct groups of MAP kinases have been identified in mammalian cells: ERK, JNK, and P38. These three pathways are activated by phosphorylation in theonine and tyrosine by dual-specificity protein kinases, including tyrosine kinases such as growth factors. Moreover, such pathways have also been associated with modulation of cell-cycle progression.

Targets of the Jun N-terminal kinase (JNK) pathway include the transcription factors c-jun and ATF2 (Whitmarsh A.J., and Davis R.J.J. Mol. Med. 74:589-607, 1996). Activation of the JNK pathway has been documented in a number of disease settings, providing the rationale for targeting this pathway for drug discovery. In addition, molecular genetic approaches have validated the pathogenic role of this pathway in several diseases. For example, autoimmune and inflammatory diseases arise from the over-activation of the immune system. Activated immune cells express many genes encoding inflammatory molecules, including cytokines, growth factors, cell surface receptors, cell adhesion molecules and degradative enzymes. Many of these genes are regulated by the JNK pathway, through activation of the transcription factors AP-1 and ATF-2, including TNFα, IL-2, E-selectin and matrix metalloproteinases such as collagenase-1 (Manning et al., Exp. Opin Invest. Drugs 6: 555-567, 1997). Matrix metalloproteinases (MMPs) promote cartilage and bone erosion in rheumatoid arthritis, and generalized tissue destruction in other autoimmune diseases. Inducible expression of MMPs, including MMP-3 and MMP-9, type II and IV collagenases, are regulated via activation of the JNK pathway and AP-1 (Gum et al., Oncogene 14:1481-1493, 1997). The JNK pathway therefore regulates MMP expression in cells involved in rheumatoid arthritis.

The JNK pathway leading to AP-1 also appears to play a critical role in cancer. For example, expression of c-jun is altered in early lung cancer and may mediate growth factor signaling in non-small cell lung cancer (Yin et al., J. Biol. Chem. 272:19943-19950, 1997). In addition to regulating c-jun production and activity, JNK activation can regulate phosphorylation of p53, and thus can modulate cell cycle progression (Chen et al., Mol. Carcinogenesis 15:215-226, 1996). Selective inhibition of JNK activation by a naturally occurring JNK inhibitory protein, called JIP- 1, blocks cellular transformation caused by BCR-ABL expression (Raitano et al., Proc. Nat. Acad. Sci USA 92:11746-11750, 1995). Thus, JNK inhibitors may block transformation and tumor cell growth.

The JNK pathway is activated by atherogenic stimuli and regulates local cytokine and growth factor production in vascular cells (Yang et al, Immunity, 9:575, 1998). In addition, alterations in blood flow, hemodynamic forces and blood volume lead to JNK activation in vascular endothelium, leading to AP-1 activation and proatherosclerotic gene expression (Aspenstrom et al., Curr. Biol. 6:70-77, 1996). Vascular disorders such as atherosclerosis and restenosis which result from dysregulated growth of the vessel wall, restricting blood flow to vital organs have also been associated with JNK deregulation.

The involvement of JNK in insulin mediated diseases such as Type II diabetes and obesity has also been confirmed (Hirosumi, J. et al. Nature 420:333-336, 2002; International Publication No. WO 02/085396). Without being limited by theory, it is thought that phosphorylation at Ser 307 of insulin receptor substrate ("IRS-1") is responsible for TNF-α-induced and FFA-induced insulin resistance (Hotamisigil, G.H. Science 271:665-668, 1996).

In addition to their role in cell-cycle control, protein kinases also play a crucial role in angiogenesis. When required, the vascular system has the potential to generate new capillary networks in order to maintain the proper functioning of tissues and organs, including the process of wound healing and neovascularization of the endometrium during menstruation. See Merenmies et al., Cell Growth & Differentiation, 8, 3-10 (1997). However, angiogenesis is also associated with numerous diseases, such as retinopathies, psoriasis, rheumatoid arthritis, age-related macular degeneneration, and cancer (solid tumors). Folkman, Nature Med., 1, 27-31 (1995).

Protein kinases which have been shown to be involved in the angiogenic process include VEGF-R2 (vascular endothelial growth factor receptor 2, also know as KDR (kinase insert domain receptor) and as FLK-1); FGF-R (fibroblast growth factor receptor); and TEK (also known as Tie-2), all of which are members of the growth factor receptor tyrosine kinase family. VEGF-R2 binds the potent angiogenic growth factor VEGF and mediates the subsequent signal transduction through activation of its intracellular kinase activity. Inhibition of the kinase activity of VEGF-R2 results in the reduction of angiogenesis even in the presence of exogenous VEGF (see Strawn et al., Cancer Research, 56, 3540-3545 (1996)), as has been shown with mutants of VEGF-R2 which fail to mediate signal transduction. Millauer et al., Cancer Research, 56, 1615-1620 (1996).

Similarly, FGF-R binds the angiogenic growth factors aFGF and bFGF and mediates subsequent intracellular signal transduction. Growth factors such as bFGF may play a critical role in inducing angiogenesis in solid tumors that have reached a certain size. Yoshiji et al., Cancer Research, 57, 3924-3928 (1997). Systemic administration of a small molecule inhibitor of the kinase activity of FGF-R has been reported to block hFGF-induced angiogenesis in mice without apparent toxicity. Mohammad et al., EMBO Journal, 17, 5996-5904 (1998).

TEK (also known as Tie-2) has been shown to play a role in angiogenesis. TEK interaction with factor angiopoietin-1 results a signal transduction process that facilitates the maturation of newly formed blood vessels. The TEK interaction with factor angiopoietin-2, on the other hand, disrupts angiogenesis. Maisonpierre et al., Science, 277, 55-60 (1997).

As such, VEGF-R2, FGF-R, and/or TEK are considered therapeutic targets for treatment of various disease states. For example, WIPO International Publication No. WO 97/34876 discloses certain cinnoline derivatives that are inhibitors of VEGF-R2, which may be used for the treatment of disease states associated with abnormal angiogenesis and/or increased vascular permeability such as cancer, diabetes, psoriosis, rheumatoid arthritis, Kaposi's sarcoma, haemangioma, acute and chronic nephropathics, atheroma, arterial restinosis, autoimmune diseases, acute inflammation and ocular diseases with retinal vessel proliferation. In addition to the protein kinases identified above, many other protein kinases have been considered to be therapeutic targets, and numerous publications disclose inhibitors of kinase activity, as reviewed in the following: McMahon et al., Current Opinion in Drug Discovery & Development, 1, 131-146 (1998); Strawn et al., Exp. Opin. Invest. Drugs, 7, 553-573 (1998).

In general, the class of compounds known as "indazoles" is well known. More specifically, an "indazole" is a compound containing a fused, bicyclic ring system having the following structure: 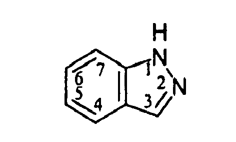 Compounds of the above structure are typically referred to as "1*H-*indazole" due to the presence of the hydrogen atom at the 1-position.

EP Patent Application 0 494 774 A1 discloses compounds of the following structure: 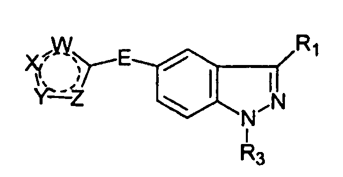 for use as agonists of the 5-hydroxytryptamine (5-HT) receptors. Such receptors exhibit selective vasoconstrictor activity, and the agonists of this published application are purported to have utility in the treatment of migraine, cluster headache, chronic paraxysmal hemicrania and headaches associated with vascular disorders. 1H-indazoles have also been made for synthetic and mechanistic studies, and as intermediates in the synthesis of other potential therapeutics. For example, the following references disclose 3-phenyl-5-methyl-1H-indazole: Pharmazie 54(2):99-101, 1999; Dopov. Akad. Nauk Ukr. 8:126-31, 1994; Pokl. Akad. Nauk SSSR 305(6):1378-81, 1989*;* Yakugaku Zasshi 106(11):1002-7, 1986 (also reports 5-Ph-3-CHO derivative); Yakugaku Zasshi 106(11):995-1001, 1986; Heterocycles 24(10):2771-5, 1986; JP 60/004184; JP 60/004185; EP 23633; J. Org. Chem. 43(10):2037-41, 1978 (also reports 3-(4-Me-Ph)-5-Me derivative); JP 60/004824; JP 59/036627; US 3,994,890; JP 58/030313; JP 60/003063. Additional 3-phenyl indazoles with the indicated 5-substituents are disclosed in the following references: EP 55450 (CHO); U.S. 5,760,028 and WO 97/23480 (CO₂Et; also disclose 3-C ≡CPh-5-CO₂Et derivative); DE 1266763 and Justus Liebigs Ann. Chem. 697:17-41, 1966 (OMe). EP 470039 discloses the 3-(4-fluorophenyl)-5-trifluoromethyl indazole, and Heterocycles (36(11) :2489-95, 1993) discloses the 3-(6,7-dimethoxyisoquinolin-1-yl)-5-hydroxy derivative.

3-Substituted indazoles, where the substituents include aryl groups and heterocyclic groups, and their alleged utility for treating proliferative disorders is disclosed in U.S. Patent No. 6,555,539 to Reich, et al*.* However, this patent focuses on 3-heterocycle substituents, such as imidazoles and benzimidazoles. 3-Aryl and 3-heterocycle substituted indazoles and their alleged utility as selective inhibitors of JNK are disclosed in International Publication No. WO 02/10137 to Bhagwat, et al*.*

There remains a need for other small-molecule compounds that may be readily synthesized and can act as protein kinase modulators, regulators or inhibitors tha have beneficial activity on multiple kinases as well as selective kinase inhibitors; each presents a beneficial albeit distinct approach to disease treatment. In addition, there is a need for pharmaceutical compositions comprising one or more of such protein kinase modulators, regulators or inhibitors, as well as for methods for treating diseases in animals which are responsive to such compounds.

### 3. SUMMARY OF THE INVENTION

In brief, the present invention relates to methods for treating or preventing diseases or disorders associated with protein kinase signal transduction, comprising administering to a patient in need thereof an amount of an Indazole Compound, or a pharmaceutically acceptable salt or solvate thereof.

The compounds of the invention include compounds having Formulas (I), (Ia), (Ib), (Ic), (Id), (Ie) and (II): 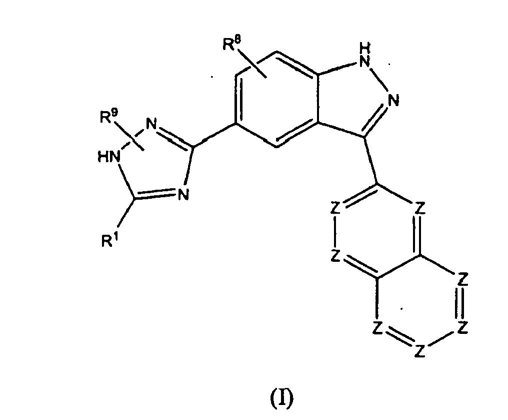 wherein Z, R¹, R⁸, and R⁹ are as defined below for the compounds of Formula (I), including isomers, prodrugs and pharmaceutically acceptable salts, solvates or hydrates thereof. 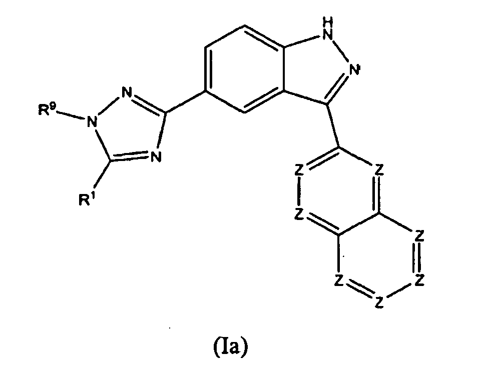 wherein Z, R¹, and R⁹ are as defined below for the compounds of Formula (Ia), including isomers, prodrugs and pharmaceutically acceptable salts, solvates or hydrates thereof. 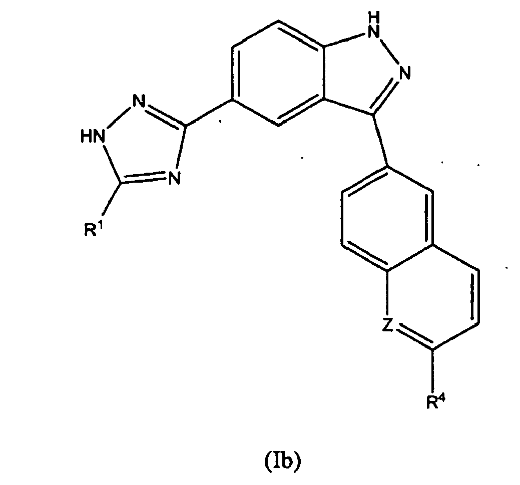 wherein Z, R¹ and R⁴ are as defined below for the compounds of Formula (Ib), including isomers, prodrugs and pharmaceutically acceptable salts, solvates or hydrates thereof. 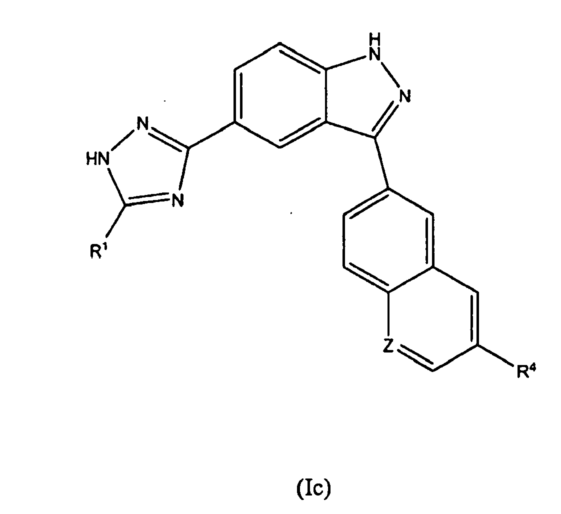 wherein Z, R¹ and R⁴ are as defined below for the compounds of Formula (Ic), including isomers, prodrugs and pharmaceutically acceptable salts, solvates or hydrates thereof. 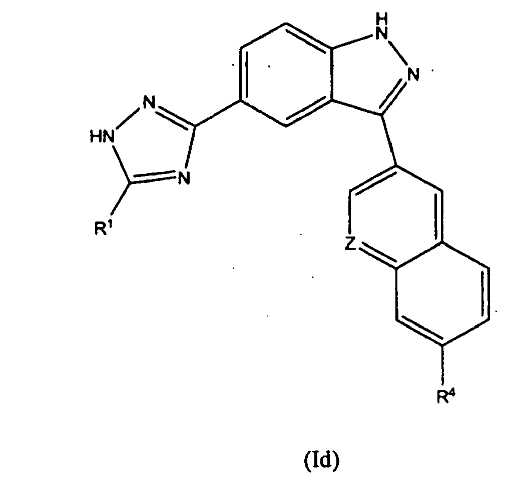 wherein Z, R¹ and R⁴ are as defined below for the compounds of Formula (Id), including isomers, prodrugs and pharmaceutically acceptable salts, solvates or hydrates thereof. 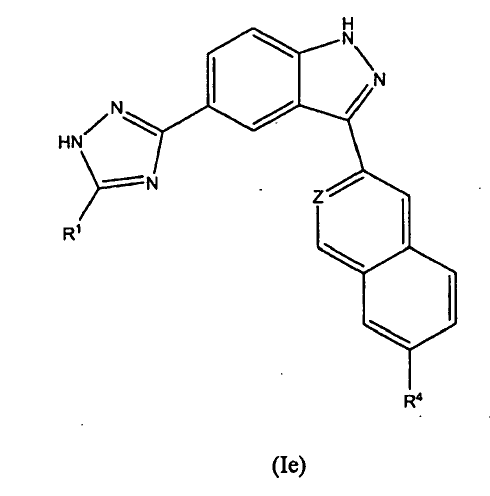 wherein Z, R¹ and R⁴ are as defined below for the compounds of Formula (Ie), including isomers, prodrugs and pharmaceutically acceptable salts, solvates or hydrates thereof. 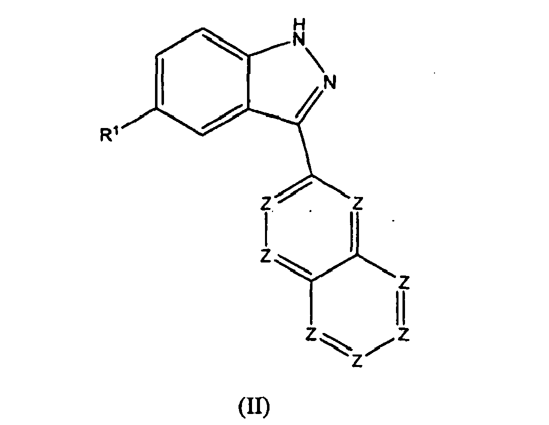 wherein Z and R¹ are as defined below for the compounds of Formula (II), including isomers, prodrugs and pharmaceutically acceptable salts, solvates or hydrates thereof.

A compound of formula (I), (Ia), (Ib), (Ic), (Id), (Ie) or (II), or a pharmaceutically acceptable salt thereof, is hereinafter referred to as an "Indazole Compound."

The present invention is also directed to methods for treating a variety of diseases, conditions, or disorders by administering a therapeutically effective amount of an Indazole Compound to a patient, typically a warm-blooded animal (including a human). In particular, the invention contemplates the use of an Indazole Compound for treating or preventing diseases, conditions, or disorders associated with protein kinases. In one embodiment, the Indazole Compound modulates, regulates or inhibits multiple protein kinases. In an alternative embodiment, the Indazole Compound selectively modulates, regulates or inhibits a specific protein kinase.

In certain embodiments, the Indazole Compounds are useful for treating or preventing cancer, a cardiovascular disease, a renal disease, an autoimmune condition, an inflammatory condition, macular degeneration, pain and related syndromes, disease-related wasting, an asbestos-related condition or pulmonary hypertension.

In a particular embodiment, the invention relates to stents containing or coated with an amount of an Indazole Compound effective for treating or preventing a cardiovascular disease or renal disease.

Prior to administration, one or more Indazole Compounds are typically formulated as a pharmaceutical composition which contains a therapeutically effective amount of one or more such Indazole Compounds in combination with one (or more) pharmaceutically acceptable carrier(s). Conditions that may be treated by the administration of an Indazole Compound, or a pharmaceutical composition containing an Indazole Compound, include any condition which may benefit from administration of a protein kinase modulator, regulator or inhibitor, and are particularly useful for the prevention and/or treatment of various diseases such as an inflammatory condition including, but not limited to, diabetes (such as Type II diabetes, Type I diabetes, diabetes insipidus, diabetes mellitus, maturity-onset diabetes, juvenile diabetes, insulin-dependant diabetes, non-insulin dependant diabetes, malnutrition-related diabetes, ketosis-prone diabetes or ketosis-resistant diabetes); nephropathy (such as glomerulonephritis or acute/chronic kidney failure); obesity (such as hereditary obesity, dietary obesity, hormone related obesity or obesity related to the administration of medication); hearing loss (such as that from otitis externa or acute otitis media); fibrosis related diseases (such as pulmonary interstitial fibrosis, renal fibrosis, cystic fibrosis, liver fibrosis, wound-healing or bum-healing, wherein the burn is a first- , second- or third-degree burn and/or a thermal, chemical or electrical bum); arthritis (such as rheumatoid arthritis, rheumatoid spondylitis, osteoarthritis or gout); an allergy; allergic rhinitis; acute respiratory distress syndrome; asthma; bronchitis; an inflammatory bowel disease (such as irritable bowel syndrome, mucous colitis, ulcerative colitis, Crohn's disease, gastritis, esophagitis, pancreatitis or peritonitis); or an autoimmune disease (such as scleroderma, systemic lupus erythematosus, myasthenia gravis, transplant rejection, endotoxin shock, sepsis, psoriasis, eczema, dermatitis or multiple sclerosis).

Indazole Compounds are also useful for treating or preventing a liver disease (such as hepatitis, alcohol-induced liver disease, toxin-induced liver disease, steatosis or sclerosis); a cardiovascular disease (such as atherosclerosis, restenosis following angioplasty, left ventricular hypertrophy, myocardial infarction, chronic obstructive pulmonary disease or stroke); ischemic damage (such as to the heart, kidney, liver or brain); ischemia-reperfusion injury (such as that caused by transplant, surgical trauma, hypotension, thrombosis or trauma injury); neurodegenerative disease (such as epilepsy, Alzheimer's disease, Huntington's disease, Amyotrophic lateral sclerosis, peripheral neuropathies, spinal cord damage, AIDS dementia complex or Parkinson's disease); cancer (such as cancer of the head, neck, eye, mouth, throat, esophagus, chest, bone, lung, colon, rectum, stomach, prostate, breast, ovaries, testicles or other reproductive organs, skin, thyroid, blood, lymph nodes, kidney, liver, pancreas, and brain or central nervous system); other diseases characterized by abnormal cellular proliferation (such as benign prostatic hyperplasia, familial adenomatosis polyposis, neuro-fibromatosis, atherosclerosis, pulmonary fibrosis, arthritis, psoriasis, glomerulonephritis, restenosis following angioplasty or vascular surgery, hypertrophic scar formation, inflammatory bowel disease, transplantation rejection, endotoxic shock, and fungal infections; and defective apoptosis-associated conditions, such as cancers (including but not limited to those types mentioned hereinabove); viral infections (including but not limited to herpesvirus, poxvirns, Epstein-Barr virus, Sindbis virus and adenovirus); AIDS development in HIV infected individuals; autoimmune diseases (including but not limited to systemic lupus erythematosus, rheumatoid arthritis, psoriasis, autoimmune mediated glomerulonephritis, inflammatory bowel disease and autoimmunc diabetes mcllitus); neurodegenerative disorders (including but not limited to Alzheimer's disease, amyotrophic lateral sclerosis, retinitis pigmentosa, Parkinson's disease, AIDS-related dementia, spinal muscular atrophy and cerebellar degeneration); myelodysplastic syndromes; aplastic anemia; ischemic injury associated with myocardial infarctions; stroke and reperfusion injury; arrhythmia; atherosclerosis; toxin-induced or alcohol related liver diseases; hematological diseases (including but not limited to chronic anemia and aplastic anemia); degenerative diseases of the musculoskeletal system (including but not limited to osteroporosis and arthritis); aspirin-sensitive rhinosinusitis; cystic fibrosis; multiple sclerosis; kidney diseases; and cancer pain.

The Indazole Compounds are also useful in the inhibition of the development of cancer, tumor angiogenesis and metastasis.

Moreover, the Indazole Compounds can modulate the level of cellular RNA and DNA synthesis and therefore are expected to be useful in the treatment of viral infections such as HIV, human papilloma virus, herpes virus, Epstein-Barr virus, adenovirus, Sindbis virus, pox virus and the like.

In one embodiment, the present methods for treating or preventing further comprise the administration of a therapeutically effective amount of another therapeutic agent useful for treating or preventing the diseases or disorders disclosed herein. In this embodiment, the time that the therapeutic effect of the other therapeutic agent is exerted overlaps with the time that the therapeutic effect of the Indazole Compound is exerted.

Indazole Compounds described herein are also be useful as an adjunct to existing and/or experimental therapies.

These and other aspects of this invention will be evident upon reference to the following detailed description. To that end, certain patent and other documents are cited herein to more specifically set forth various aspects of this invention. Each of these documents are hereby incorporated by reference in their entirety.

### 4. DETAILED DESCRIPTION OF THE INVENTION

As mentioned above, this invention is generally directed to novel compounds and their use in methods for treating or preventing diseases associated with protein kinases, including tyrosine kinases, such as inflammatory diseases, abnormal angiogenesis and diseases related thereto, cancer, atherosclerosis, macular degeneration, diabetes, obesity, stroke, ischemia, trauma, pain and others. In addition, some of these compounds can be used as active agents against solid tumors, malignant ascites, hematopoitic cancers, hyperproliferative disorders such as thyroid hyperplasia, the cysts (such as hypervascularity of ovrian stroma characteristic of polycystic ovarian syndrome) since such diseases require proliferation of blood vessels cells and associated cells for growth and metastasis.

It is envisaged that the disorders listed above are mediated to a significant extent by protein tyrosine kinase activity involving enzymes listed above. By inhibiting the activity of one or more protein tyrosine kinases simultaneously, the progression of the above disorders can be inhibited because these diseases require the activity of these protein kinases. Furthermore several of these diseases are dependent on the active proliferation of cells and angiogenesis. By inhibiting the related protein tyrosine kinases more than one simultaneously provides a more effective method of treating the disease than inhibiting one specific kinase because rarely any disease state is solely dependent on one specific kinase.

The compounds in this invention have inhibitory activity against a variety of protein kinases. These compounds modulate signal transduction of protein kinases. Compounds of this invention inhibit a variety of families of protein kinases. In particular, these compounds are capable of targeting kinases include but not limited to Aurora-A, Blk, CDK1, CDK2, CDK3, CDK5, CDK6, CHK1, CHK2, the Src family of kinases, cSrc, Yes, Fyn, Lck, Fes, , Lyn, Syk, , FGF-R3, GSK3a, GSK3b, MAPK family including JNK, MEK, p70S6K, PKCmu, PKD2, PRAK, PRK2, ROCK-II, RSK1, RSK2, RSK3.

### 4.1 DEFINITIONS AND ABBREVIATIONS

The terms used herein having following meaning:

The term "C₁-C₆ alkyl" as used herein refers to a straight or branched chain, saturated hydrocarbon having from 1 to 6 carbon atoms. Representative C₁-C₆ alkyl groups include, but are not limited to methyl, ethyl, propyl, isopropyl, butyl, *sec-*butyl, *tert*-butyl, pentyl, isopentyl, neopentyl, hexyl, isohexyl, and neohexyl. A C₁-C₆ alkyl group can be unsubstituted or optionally substituted with one or more of the following groups: -halo, -C₁-C₆ alkyl, -OH, -CN, -COOR', -OC(O)R', NHR', N(R')₂, - NHC(O)R' or -C(O)N(R')₂ groups wherein each occurrence of R' is independently -H or unsubstituted -C₁-C₆ alkyl.

The term "C₂-C₆ alkenyl" as used herein refers to a straight or branched chain unsaturated hydrocarbon containing 2-6 carbon atoms and at least one double bond. Examples of a C₂-C₆ alkenyl group include, but are not limited to, ethylene, propylene, 1-butylene, 2-butylene, isobutylene, sec-butylene, 1-pentene, 2-pentene, isopentene, 1-hexene, 2-hexene, 3-hexene, and isohexene.

The term "C₂-C₆ alkynyl" as used herein refers to a straight or branched chain unsaturated hydrocarbon containing 2-6 carbon atoms and at least one triple bond. Examples of a C₂-C₆ alkynyl group include, but are not limited to, acetylene, propyne, 1-butyne, 2-butyne, isobutyne, sec-butyne, 1-pentyne, 2-pentyne, isopentyne, 1-hexyne, 2-hexyne, 3-hexyne, and isohexyne.

The term "C₁-C₆ alkylene" as used herein refers to a C₁-C₆ alkyl group in which one of the C₁-C₆ alkyl group's hydrogen atoms has been replaced with a bond. Examples of a C₁-C₆ alkylene include -CH₂-, -CH₂CH₂-, -CH(CH₃)-, -CH₂CH₂CH₂-, -CH₂CH₂CH₂CH₂-, -CH₂CH₂CH₂CH₂CH₂-, and -CH₂CH₂CH₂ CH₂CH₂CH₂-.

The term "C₁-C₆ alkoxy" as used herein refers to a group having the formula -O-(C₁-C₆ alkyl). Examples of a C₁-C₆ alkoxy group include -O-methyl, -O-ethyl, -O-propyl, -O-isopropyl, -O-butyl, -O-sec-butyl, -O-tert-butyl, -O-pentyl, -O-isopentyl, -O-neopentyl, -O-hexyl, -O-isohexyl, and -O-neohexyl.

The term "aryl" as used herein refers to a 6- to 14-membered monocyclic, bicyclic or tricyclic aromatic hydrocarbon ring system. Examples of an aryl group include phenyl and naphthyl. An aryl group can be unsubstituted or optionally substituted with one or more of the following groups: -halo, -C₁-C₆ alkyl, -O-(C₁-C₆ alkyl), -OH, -CN, -COOR', -OC(O)R', NHR', N(R')₂, -NHC(O)R' or -C(O)N(R')₂ groups wherein each occurrence of R' is independently -H or unsubstituted -C₁-C₆ alkyl.

The term "cycloalkyl" as used herein refers to a 3- to 14-membered saturated or unsaturated non-aromatic monocyclic, bicyclic or tricyclic hydrocarbon ring system. Included in this class are cycloalkyl groups which are fused to a benzene ring. Representative cycloalkyl groups include, but are not limited to, cyclopropyl, cyclobutyl, cyclobutenyl, cyclopentyl, cyclopentenyl, cyclopentadienyl, cyclohexyl, cyclohexenyl, 1,3-cyclohexadienyl, cycloheptyl, cycloheptenyl, 1,3-cycloheptadienyl, 1,4-cycloheptadienyl, -1,3,5-cycloheptatrienyl, cyclooctyl, cyclooctenyl, 1,3-cyclooctadienyl, 1,4-cyclooctadienyl, -1,3,5-cyclooctatrienyl, decahydronaphthalene, octahydronaphthalene, hexahydronaphthalene, octahydroindene, hexahydroindene, tetrahydroinden, decahydrobenzocycloheptene, octahydrobenzocycloheptene, hexahydrobenzocycloheptene, tetrahydrobenzocyclopheptene, dodecahydroheptalene, decahydroheptalene, octahydroheptalene, hexahydroheptalene, and tetrahydroheptalene. A cycloalkyl group can be unsubstituted or optionally substituted with one or more of the following groups: -halo, -C₁-C₆ alkyl, -O-(C₁-C₆ alkyl), -OH, -CN, -COOR', - OC(O)R', NHR', N(R')₂, -NHC(O)R' or -C(O)N(R')₂ groups wherein each occurrence of R' is independently -H or unsubstituted -C₁-C₆ alkyl.

The term "therapeutically effective amount" as used herein refers to an amount of an Indazole Compound or other active ingredient sufficient to provide a therapeutic benefit in the treatment or prevention of a disease or disorder disclosed or to delay or minimize symptoms associated with a disease or disorder. Further, a therapeutically effective amount with respect to an Indazole Compound means that amount of therapeutic agent alone, or in combination with other therapies, that provides a therapeutic benefit in the treatment or prevention of a disease or disorder. Used in connection with an Indazole Compound, the term can encompass an amount that improves overall therapy, reduces or avoids symptoms or causes of a disease or disorder, or enhances the therapeutic efficacy of or synergies with another therapeutic agent.

The term "halo" as used herein refers to -F, -Cl, -Br or -I.

"Heteroaryl" refers to an aromatic heterocycle ring of 5 to 14 members and having at least one heteroatom selected from nitrogen, oxygen and sulfur, and containing at least 1 carbon atom, including monocyclic, bicyclic, and tricyclic ring systems. Representative heteroaryls are triazolyl, tetrazolyl, oxadiazolyl, pyridyl, furyl, benzofuranyl, thiophenyl, benzothiophenyl, quinolinyl, pyrrolyl, indolyl, oxazolyl, benzoxazolyl, imidazolyl, benzimidazolyl, thiazolyl, benzothiazolyl, isoxazolyl, pyrazolyl, isothiazolyl, pyridazinyl, pyrimidinyl, pyrazinyl, triazinyl, cinnolinyl, phthalazinyl, quinazolinyl, pyrimidyl, oxetanyl, azepinyl, piperazinyl, morpholinyl, dioxanyl, thietanyl and oxazolyl. A heteroaryl group can be unsubstituted or optionally substituted with one or more of the following groups: -halo, -C₁-C₆ alkyl, -O-(C₁-C₆ alkyl), -OH, -CN, -COOR', -OC(O)R', NHR', N(R')₂, -NHC(O)R' or -C(O)N(R')₂ groups wherein each occurrence of R' is independently -H or unsubstituted -C₁-C₆ alkyl.

As used herein, the term "heterocycle" as used herein refers to 5- to 14-membered ring systems which are either saturated, unsaturated, or aromatic, and which contains from 1 to 4 heteroatoms independently selected from nitrogen, oxygen and sulfur, and wherein the nitrogen and sulfur heteroatoms may be optionally oxidized, and the nitrogen heteroatom may be optionally quaternized, including, including monocyclic, bicyclic, and tricyclic ring systems. The bicyclic and tricyclic ring systems may encompass a heterocycle or heteroaryl fused to a benzene ring. The heterocycle may be attached via any heteroatom or carbon atom. Heterocycles include heteroaryls as defined above. Representative examples of heterocycles include, but are not limited to, aziridinyl, oxiranyl, thiiranyl, triazolyl, tetrazolyl, azirinyl, diaziridinyl, diazirinyl, oxaziridinyl, azetidinyl, azetidinonyl, oxetanyl, thietanyl, piperidinyl, piperazinyl, morpholinyl, thiomorpholinyl, pyrrolyl, oxazinyl, thiazinyl, diazinyl, triazinyl, tetrazinyl, imidazolyl, imidazolidin-2-one, tetrazolyl, pyrrolidinyl, isoxazolyl, furanyl, furazanyl, pyridinyl, oxazolyl, benzoxazolyl, benzisoxazolyl, thiazolyl, benzthiazolyl, thiophenyl, pyrazolyl, triazolyl, pyrimidinyl, benzimidazolyl, isoindolyl, indazolyl, benzodiazolyl, benzotriazolyl, benzoxazolyl, benzisoxazolyl, purinyl, indolyl, isoquinolinyl, quinolinyl, and quinazolinyl. A heterocycle group can be unsubstituted or optionally substituted with one or more of the following groups: -halo, -C₁-C₆alkyl, -O-(C₁-C₆ alkyl), -OH, - CN, -COOR', -OC(O)R', NHR', N(R')₂, -NHC(O)R' or -C(O)N(R')₂ groups wherein each occurrence of R' is independently -H or unsubstituted -C₁-C₆ alkyl.

A "patient" includes an animal (e.g., cow, horse, sheep, pig, chicken, turkey, quail, cat, dog, mouse, rat, rabbit or guinea pig), in one embodiment a mammal such as a non-primate or a primate (e.g., monkey and human), and in another embodiment a human. In certain embodiments, the patient is an infant, child, adolescent or adult.

As used herein, the term "macular degeneration" encompasses all forms of macular degenerative diseases regardless of a patient's age, although some macular degenerative diseases are more common in certain age groups. These include, but are not limited to, Best's disease or vitelliform (most common in patients under about seven years of age); Stargardt's disease, juvenile macular dystrophy or fundus flavimaculatus (most common in patients between about five and about 20 years of age); Behr's disease, Sorsby's disease, Doyne's disease or honeycomb dystrophy (most common in patients between about 30 and about 50 years of age); and age-related macular degeneration (most common in patients of about 60 years of age or older). In one embodiment, the cause of the macular degenerative disease is genetic. In another embodiment, the cause of the macular degenerative disease is physical trauma. In another embodiment, the cause of the macular degenerative disease is diabetes. In another embodiment, the cause of the macular degenerative disease is malnutrition. In another embodiment, the cause of the macular degenerative disease is infection.

As used herein, the phrase "pain and related syndromes" includes nociceptive pain, such as that resulting from physical trauma (e.g., a cut or contusion of the skin; or a chemical or thermal bum), osteoarthritis, rheumatoid arthritis or tendonitis; myofascial pain; neuropathic pain, such as that associated with stroke, diabetic neuropathy, luetic neuropathy, postherpetic neuralgia, trigeminal neuralgia, fibromyalgia, or painful neuropathy induced iatrogenically by drugs such as vincristine, velcade or thalidomide; or mixed pain (i.e., pain with both nociceptive and neuropathic components). Further types of pain that can be treated or prevented by administering an effective amount of an Aminopurine Compound to a patient in need thereof include, but are not limited to, visceral pain; headache pain (e.g., migraine headache pain); CRPS; CRPS type I; CRPS type II; RSD; reflex neurovascular dystrophy; reflex dystrophy; sympathetically maintained pain syndrome; causalgia; Sudeck atrophy of bone; algoneurodystrophy; shoulder hand syndrome; post-traumatic dystrophy; autonomic dysfunction; cancer-related pain; phantom limb pain; chronic fatigue syndrome; post-operative pain; spinal cord injury pain; central post-stroke pain; radiculopathy; sensitivity to temperature, light touch or color change to the skin (allodynia); pain from hyperthermic or hypothermic conditions; and other painful conditions (e.g., diabetic neuropathy, luetic neuropathy, postherpetic neuralgia, trigeminal neuralgia).

The term "disease-related wasting" means wasting (e.g, a loss of physical bulk through the breakdown of bodily tissue) associated with a disease such as HIV, AIDS, cancer, end-stage renal disease, kidney failure, chronic heart disease, obstructive pulmonary disease, tuberculosis, rheumatoid arthritis, a chronic inflammatory disease (e.g., scleroderma or mixed connective tissue disease) or a chronic infectious disease (e.g., osteoarthritis or bacterial endocarditis).

The term "asbestos-related disease" includes diseases and disorders such as malignant mesothelioma, asbestosis, malignant pleural effusion, benign pleural effusion, pleural plaque, pleural calcification, diffuse pleural thickening, round atelectasis, and bronchogenic carcinoma, as well as symptoms of asbestos-related diseases and disorders such as dyspnea, obliteration of the diaphragm, radiolucent sheet-like encasement of the pleura, pleural effusion, pleural thickening, decreased size of the chest, chest discomfort, chest pain, easy fatigability, fever, sweats and weight loss.

The term "pulmonary hypertension" includes diseases characterized by sustained elevations of pulmonary artery pressure as well as symptoms associated with pulmonary hypertension such as dyspnea, fatigue, weakness, chest pain, recurrent syncope, seizures, light-headedness, neurologic deficits, leg edema and palpitations.

An Indazole Compound can be in the form of a pharmaceutically acceptable salt. The phrase "pharmaceutically acceptable salt," as used herein, refers to a pharmaceutically acceptable organic or inorganic acid or base salt of an Indazole Compound. Representative pharmaceutically acceptable salts include, e.g., alkali metal salts, alkali earth salts, ammonium salts, water-soluble and water-insoluble salts, such as the acetate, amsonate (4,4-diaminostilbene-2, 2 -disulfonate), benzenesulfonate, benzonate, bicarbonate, bisulfate, bitartrate, borate, bromide, butyrate, calcium, calcium edetate, camsylate, carbonate, chloride, citrate, clavulariate, dihydrochloride, edetate, edisylate, estolate, esylate, fiunarate, gluceptate, gluconate, glutamate, glycollylarsanilate, hexafluorophosphate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, 3-hydroxy-2-naphthoate, oleate, oxalate, palpitate, pamoate (1,1-methene-bis-2-hydroxy-3-naphthoate, einbonate), pantothenate, phosphate/diphosphate, picrate, polygalacturonate, propionate, p-toluenesulfonate, salicylate, stearate, subacetate, succinate, sulfate, sulfosaliculate, suramate, tannate, tartrate, teoclate, tosylate, triethiodide, and valerate salts. Furthermore, a pharmaceutically acceptable salt can have more than one charged atom in its structure. In this instance the pharmaceutically acceptable salt can have multiple counterions. Hence, a pharmaceutically acceptable salt can have one or more charged atoms and/or one or more counterions.

As used herein, the term "isolated and purified form" means that when isolated (e.g., from other components of a synthetic organic chemical reaction mixture), the isolate contains at least 30%, at least 35%, at least 40%, at least 45%, at least 50%, at least 55%, at least 60%, at least 65%, at least 70%, at least 75%, at least 80%, at least 85%, at least 90%, at least 95% or at least 98% of an Indazole Compound by weight of the isolate. In one embodiment, the isolate contains at least 95% of an Indazole Compound by weight of the isolate.

As used herein, the terms "prevent", "preventing" and "prevention" refer to the prevention of the onset, recurrence or spread of the disease in a patient resulting from the administration of a prophylactic or therapeutic agent.

As used herein, the term "prodrug" means a derivative of a compound that can hydrolyze, oxidize, or otherwise react under biological conditions (*in vitro* or *in vivo*) to provide an active compound, particularly an Indazole Compound. Examples of prodrugs include, but are not limited to, derivatives and metabolites of an Indazole Compound that include biohydrolyzable groups such as biohydrolyzable amides, biohydrolyzable esters, biohydrolyzable carbamates, biohydrolyzable carbonates, biohydrolyzable ureides, and biohydrolyzable phosphate analogues (e.g., monophosphate, diphosphate or triphosphate). Preferably, prodrugs of compounds with carboxyl functional groups are the lower alkyl esters of the carboxylic acid. The carboxylate esters are conveniently formed by esterifying any of the carboxylic acid moieties present on the molecule. Prodrugs can typically be prepared using well-known methods, such as those described by Burger's Medicinal Chemistry and Drug Discovery 6th ed. (Donald J. Abraham ed., 2001, Wiley) and Design and Application of Prodrugs (H. Bundgaard ed., 1985, Harwood Academic Publishers Gmfh).

As used herein, the terms "treat", "treating" and "treatment" refer to the eradication or amelioration of the disease or symptoms associated with the disease. In certain embodiments, such terms refer to minimizing the spread or worsening of the disease resulting from the administration of one or more prophylactic or therapeutic agents to a patient with such a disease.

The Indazole Compound can also exist in various isomeric forms, including configurational, geometric and conformational isomers, as well as existing in various tautomeric forms, particularly those that differ in the point of attachment of a hydrogen atom. As used herein, the term "isomer" is intended to encompass all isomeric forms of an Indazole Compound, including tautomeric forms of the compound.

Certain Indazole Compounds may have asymmetric centers and therefore exist in different enantiomeric and diastereomeric forms. An Indazole Compound can be in the form of an optical isomer or a diastereomer. Accordingly, the invention encompasses Indazole Compounds and their uses as described herein in the form of their optical isomers, diasteriomers and mixtures thereof, including a racemic mixture. Optical isomers of the Indazole Compounds can be obtained by known techniques such as asymmetric synthesis, chiral chromatography, simulated moving bed technology or via chemical separation of stereoisomers through the employment of optically active resolving agents.

As used herein and unless otherwise indicated, the term "stereoisomer" or "stereomerically pure" means one stereoisomer of a compound that is substantially free of other stereoisomers of that compound. For example, a stereomerically pure compound having one chiral center will be substantially free of the opposite enantiomer of the compound. A stereomerically pure a compound having two chiral centers will be substantially free of other diastereomers of the compound. A typical stereomerically pure compound comprises greater than about 80% by weight of one stereoisomer of the compound and less than about 20% by weight of other stereoisomers of the compound, more preferably greater than about 90% by weight of one stereoisomer of the compound and less than about 10% by weight of the other stereoisomers of the compound, even more preferably greater than about 95% by weight of one stereoisomer of the compound and less than about 5% by weight of the other stereoisomers of the compound, and most preferably greater than about 97% by weight of one stereoisomer of the compound and less than about 3% by weight of the other stereoisomers of the compound.

It should be noted that if there is a discrepancy between a depicted structure and a name given that structure, the depicted structure controls. In addition, if the stereochemistry of a structure or a portion of a structure is not indicated with, for example, bold or dashed lines, the structure or portion of the structure is to be interpreted as encompassing all stereoisomers of it.

The following abbreviations are used herein and have the indicated definitions: DHP is dihydroypyran; DIAD is diethylazodicarboxylate; Et₃N is triethylamine; EtOH is ethanol; MeOH is methanol; MS is mass spectrometry; NMR is nuclear magnetic resonance; PPh₃ is triphenylphosphine; THF is tetrahydrofuran; THP is tetrahydropyranyl; and p-TsOH is para-toluene sulfonic acid.

### 4.2 THE INDAZOLE COMPOUNDS OF THE INVENTION

### 4.2.1 THE INDAZOLE COMPOUNDS OF FORMULA (I)

In one embodiment, the invention provides Indazole Compounds having the Formula (I): 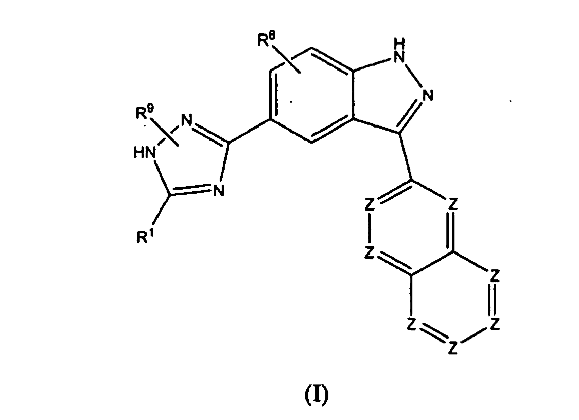 and isomers, prodrugs and pharmaceutically acceptable salts, solvates and hydrates thereof,
wherein,
R¹ is -H, -halo, -CN, -C₁-C₆ alkyl, -C₁-C₆ alkenyl, -C₁C₆ alkynyl, -OR³, -N(R⁴)₂, -CN, -NO₂, -C(O)R⁵, -OC(O)R⁵, -NHC(O)R⁵, -SO₂R⁶, -aryl, -heterocycle, -heteroaryl, -cycloalkyl, -(C₁-C₆ alkylene)-R² or -O-(C₁-C₆ alkylene)-R²;
R² is -H, -halo, -C₁-C₆ alkyl, -C₁-C₆ alkenyl, -C₁-C₆ alkynyl, -OR³, - N(R⁴)₂, -CN, -NO₂, -C(O)R⁵, -OC(O)R⁵, -NHC(O)R⁵, -SO₂R⁶, -aryl, -heterocycle, - heteroaryl, or -cycloalkyl;
R³ is independently -H, -C₁-C₆ alkyl, -C₁-C₆ alkenyl, -C₁-C₆ alkynyl, -C₁-C₆ haloalkyl, -cycloalkyl, -aryl, or -heterocycle;
each occurrence of R⁴ is independently -H, -C₁-C₆ alkyl, -C₁-C₆ alkenyl, - C₁-C₆ alkynyl, -cycloalkyl, -aryl, -heterocycle, or -(C₁-C₆ alkylene)-OR³;
R⁵ is -H, -C₁-C₆ alkyl, -C₁-C₆ alkenyl, -C₁-C₆ alkynyl, -cycloalkyl, -aryl, -heterocycle, -OR³, -N(R⁴)₂,
R⁶ is -H, -C₁-C₆ alkyl, -C₁-C₆ alkenyl, -C₁-C₆ alkynyl, -N(R⁴)₂, - cycloalkyl, -aryl, or -heterocycle;
each occurrence of Z is -C(R⁷)- or -N-, wherein up to 3 occurences of Z can be -N-;
R⁷ is -H, -halo, -C₁-C₆ alkyl, -C₁-C₆ haloalkyl, -O-(C₁-C₆ haloalkyl), -C₁-C₆ alkenyl, -C₁-C₆ alkynyl, -OR³, -N(R⁴)₂, -CN, -NO₂, -C(O)R⁵, -OC(O)R⁵, -NHC(O)R⁵,
-SO₂R⁶, -aryl, -heterocycle, -cycloalkyl, -C(O)NH-(C₁-C₆ alkylene)ₙ-cycloalkyl, - C(O)NH-(C₁-C₆ alkylene)ₙ-aryl, -C(O)NH-(C₁-C₆ alkylene )ₙ-heterocycle, -(C₁-C₆ alkylene)-cycloalkyl, -(C₁-C₆ alkylene)-aryl, -(C₁-C₆ alkylene)-heterocycle, -O-(C₁-C₆ alkylene)-C(O)R⁵, -O-(C₁-C₆ alkylene)-N(R⁴)₂, -O-(C₁-C₆ alkylene)-cycloalkyl, -O-(C₁-C₆ alkylene)-aryl, or -O-(C₁-C₆ alkylene)-heterocycle, wherein R⁷ is attached to the bicyclic ring system via a carbon atom and n is 0 or 1;
R⁸ is -H, -halo, -C₁-C₆ alkyl, -C₁-C₆ haloalkyl, -O-(C₁-C₆ haloalkyl), -C₁-C₆ alkenyl, -C₁-C₆ alkynyl, -OR³, -N(R⁴)₂, -CN, -NO₂, -C(O)R⁵, -OC(O)R⁵, -NHC(O)R⁵, -SO₂R⁶, -aryl, -heterocycle, or -cycloalkyl; and
R⁹ is -H, -C₁-C₆ alkyl, or cycloalkyl.

In one embodiment, all occurrences of -Z are - C(R⁷)-.

In another embodiment, R₁ is -C₁-C₆ alkyl.

In still another embodiment, R₁ is -(alkylene)-heterocycle.

In yet another embodiment, R₁ is -CH₂-heterocycle.

In a further embodiment, R₁ is -(alkylene)-cycloalkyl.

In another embodiment, R⁸ is -H.

In another embodiment, R⁹ is -H.

In another embodiment, R⁸ is -H and R⁹ is -H.

The present invention also provides compositions comprising a therapeutically effective amount of a Indazole Compound of Formula (I) and a pharmaceutically acceptable vehicle.

The invention further provides Indazole Compounds of Formula (I) that are in isolated and purified form.

### 4.2.2 THE INDAZOLE COMPOUNDS OF FORMULA (Ia)

In one embodiment, the invention provides Indazole Compounds having the Formula (Ia): 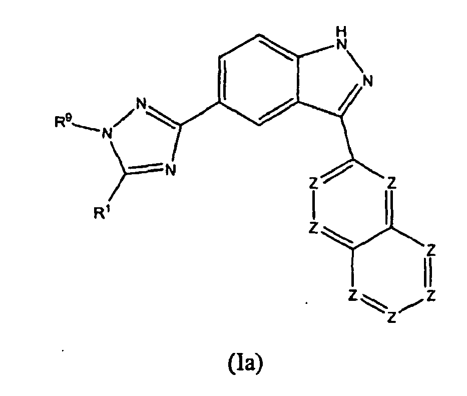 and isomers, prodrugs and pharmaceutically acceptable salts, solvates and hydrates thereof,
wherein,
R¹ is -H, -halo, -C₁-C₆ alkyl, -C₁-C₆ alkenyl, -C₁-C₆ alkynyl, -OR³, - N(R⁴)₂, -CN, -NO₂, -C(O)R⁵, -OC(O)R⁵, -NHC(O)R⁵, -SO₂R⁶, -aryl, -heterocycle, - heteroaryl, -cycloalkyl, -(C₁-C₆ alkylene)-R² or -O-(C₁-C₆ alkylene)-R²;
R² is -H, -halo, -C₁-C₆ alkyl, -C₁-C₆ alkenyl, -C₁-C₆ alkynyl. -OR³, - N(R⁴)₂, -CN, -NO₂, -C(O)R⁵, -OC(O)R⁵, -NHC(O)R⁵, -SO₂R⁶, -aryl, -heterocycle, - heteroaryl, or -cycloalkyl;
R³ is independently -H, -C₁-C₆ alkyl, -C₁-C₆ alkenyl, -C₁-C₆ alkynyl, -C₁-C₆ haloalkyl, -cycloalkyl, -aryl, or -heterocycle;
each occurrence of R⁴ is independently -H, -C₁-C₆ alkyl, -C₁-C₆ alkenyl, - C₁-C₆ alkynyl, -cycloalkyl, -aryl, -heterocycle, or -(C₁-C₆ alkylene)-OR³;
R⁵ is -H, -C₁-C₆ alkyl, -C₁-C₆ alkenyl, -C₁-C₆ alkynyl, -cycloalkyl, -aryl, -heterocycle, -OR³, -N(R⁴)₂,
R⁶ is -H, -C₁-C₆ alkyl, -C₁-C₆ alkenyl, -C₁-C₆ alkynyl, -N(R⁴)₂, - cycloalkyl, -aryl, or -heterocycle;
each occurrence of Z is -C(R⁷)- or -N-, wherein up to 3 occurences of Z can be -N-;
R⁷ is -H, -halo, -C₁-C₆ alkyl, -C₁-C₆ haloalkyl, -O-(C₁-C₆ haloalkyl), -C₁-C₆ alkenyl, -C₁-C₆ alkynyl, -OR³, -N(R⁴)₂, -CN, -NO₂, -C(O)R⁵, -OC(O)R⁵, -NHC(O)R⁵, -SO₂R⁶, -aryl, -heterocycle, -cycloalkyl, -C(O)NH-(C₁-C₆ alkylene)ₙ-cycloalkyl, - C(O)NH-(C₁-C₆ alkylene)ₙ-aryl, -C(O)NH-(C₁-C₆ alkylene)ₙ-heterocycle, -(C₁-C₆ alkylene)-cycloalkyl, -(C₁-C₆ alkylene)-aryl, -(C₁-C₆ alkylene)-heterocycle, -O-(C₁-C₆ alkylene)-C(O)R⁵, -O-(C₁-C₆ alkylene)-N(R⁴)₂, -O-(C₁-C₆ alkylene)-cycloalkyl, -O-(C₁-C₆ alkylene)-aryl, or -O-(C₁-C₆ alkylene)-heterocycle, wherein R⁷ is attached to the bicyclic ring system via a carbon atom and n is 0 or 1; and
R⁹ is -H, -C₁-C₆ alkyl, or cycloalkyl.

In one embodiment, each occurrence of -Z is -C(R⁷)-.

In another embodiment, R¹ is -H.

In still another embodiment, R¹ is -C₁-C₆ alkyl.

In another embodiment, R¹ is -(C₁-C₆ alkylene)-heterocycle.

In yet another embodiment, R¹ is -CH₂-heterocycle.

In a further embodiment, R¹ is -O-(C₁-C₆ alkylene)-heterocycle.

In another embodiment, R¹ is -O-CH₂-heterocycle.

In another embodiment, R¹ is -(C₁-C₆ alkylene)-N(R⁴)₂, wherein each occurrence of R⁴ is independently -H or C₁-C₆ alkyl.

In another embodiment, R¹ is -(C₁-C₆ alkylene)-NH(C₁-C₆ alkyl).

In another embodiment, R¹ is -(C₁-C₆ alkylene)-N(R⁴)₂, wherein both R⁴ groups are -(C₁-C₆ alkylene)-(O-C₁-C₆ alkyl).

In a embodiment, R¹ is -CH₂-N(R⁴)₂, wherein each occurrence of R⁴ is independently -H or C₁-C₆ alkyl.

In one embodiment, R⁷ is -H.

In one embodiment, R⁷ is -halo.

In one embodiment, R⁷ is -O-(C₁-C₆ alkyl).

In another embodiment, R⁷ is -O-(C₁-C₆ alkylene)-heterocycle.

In still another embodiment, R⁷ is -C(O)-(C₁-C₆ alkyl).

In another embodiment, R⁷ is -O-(C₁-C₆ haloalkyl).

In one embodiment, R⁸ is -H.

In another embodiment, R⁹ is -H.

In still another embodiment, R⁸ is -H and R⁹ is -H.

In a preferred embodiment, R₁ is -(C₁-C₆ alkylene)-heterocycle and R⁷ is - O-(C₁-C₆ alkyl).

In a further preferred embodiment, R₁ is -(C₁-C₆ alkylene)-heterocycle and R⁷ is -O-(C₁-C₆ alkyl), R⁸ is -H and R⁹ is -H.

In another preferred embodiment, R¹ is -(C₁-C₆ alkylene)-N(R⁴)₂, R⁷ is - O-(C₁-C₆ alkyl), R⁸ is -H and R⁹ is -H.

Illustrative Indazole Compounds of Formula (Ia) include the following:

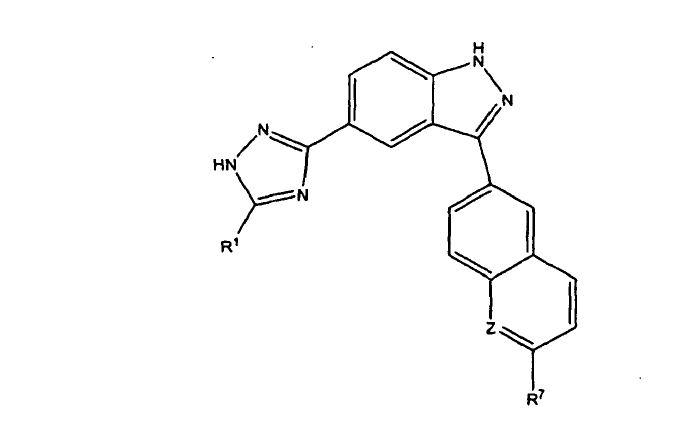

| Compound | R¹ | R⁷ | Z |
|---|---|---|---|
| 1 | -H | -OCH₃ | -CH- |
| 2 | -H | -OCH₂CH₃ | -CH- |
| 3 | -H | -O-n-butyl | -CH- |
| 4 | -H | -H | -CH- |
| 5 | -Me | -OCH₃ | -CH- |
| 6 | -isobutyl | -OCH₃ | -CH- |
| 7 | -isobutyl | -H | -CH- |
| 8 | -CH₂-pyrrolidin-1-yl | -H | -CH- |
| 9 | -CH₂-pyrrolidin-1-yl | -OCH₂CH₃ | -CH- |
| 10 | -CH₂-pynolidin-1-yl | -OH | -CH- |
| 11 | -CH₂-pyrrolidin-1-yl | -C(O)OCH₂CH₃ | -CH- |
| 12 | -CH₂-pyrrolidin-1-yl | -OCHF₂ | -CH- |
| 13 | -CH₂-pyrrolidin-1-yl | -F | -CH- |
| 14 | -CH₂-pyrrolidin-1-yl | -C(O)NHCH₂CH₃ | -CH- |
| 15 | -CH₂-pyrrolidin-1-yl | -O(CH₂)₂-CH(CH₃)₂ | -CH- |
| 16 | -CH₂-pyrrolidin-1-yl | -OH | -CH- |
| 17 | -CH₂-pyrrolidin-1-yl | -OCH₃ | -CH- |
| 18 | -CH₂-pyrrolidin-1-yl | -C(O)NH₂ | -CH- |
| 19 | -CH₂-piperidin-1-yl | -OCH₃ | -CH- |
| 20 | -CH₂-morpholin-1-yl | -OCH₃ | -CH- |
| 21 | -OCH₂-(2-methyl-pyrrolidin-1-yl) | -OCH₃ | -CH- |
| 22 | -cyclopentyl | -OCH₃ | -CH- |
| 23 | -(CH₂)₂-piperidin-1-yl | -OCH₃ | -CH- |
| 24 | -CH₂-(1-methyl-piperidin-4-yl) | -OCH₃ | -CH- |
| 25 | -CH₂OH | -OCH₃ | -CH- |
| 26 | -CH(CH₃)-(pyn-olidin-1-yl) | -OCH₃ | -CH- |
| 27 | -CH₂-(2-methyl-piperidin-1-yl | -OCH₃ | -CH- |
| 28 | -CH₂-(2,6-dimethyl-piperidin-1-yl | -OCH₃ | -CH- |
| 29 | -CH₂-(azepan-1-yl) | -OCH₃ | -CH- |
| 30 | -CH₂-(piperazin-l-yl) | -OCH₃ | -CH- |
| 31 | -CH₂-(1-acetyl-piperazin-4-yl) | -OCH₃ | -CH- |
| 32 | -CH₂NH₂ | -OCH₃ | -CH- |
| 33 | -CH₂N(CH₃)₂ | -OCH₃ | -CH- |
| 34 | -(CH₂)₂NH₂ | -OCH₃ | -CH- |
| 35 | -CH₂NH-(t-butyl) | -OCH₃ | -CH- |
| 36 | CH₂N(CH₂CH₂OCH₃)₂ | -OCH₃ | -CH- |
| 37 | -CH₂-piperidin-1-yl | -OCH₃ | -N- |
| 38 | -CH₂-morpholin-1-yl | -H | -N- |
| 39 | -CH₂-morpholin-1-yl | -OCH₃ | -N- |
| 40 | -CH₂-morpholin-1-yl | -N(CH₃)₂ | -N- |
| 41 | -CH₂-morpholin-1-yl | -N(H)(CH₃) | -N- |
| 42 | -CH₂-pyrrolidin-1-yl | -OCH₃ | -N- |

and isomers, prodrugs and pharmaceutically acceptable salts, solvates and hydrates thereof.

The present invention also provides compositions comprising a therapeutically effective amount of a Indazole Compound of Formula (Ia) and a pharmaceutically acceptable vehicle.

The invention further provides Indazole Compounds of Formula (Ia) that are in isolated and purified form.

### 4.2.3 THE INDAZOLE COMPOUNDS OF FORMULA (Ib)

In one embodiment, the invention provides Indazole Compounds having the Formula (Ib): 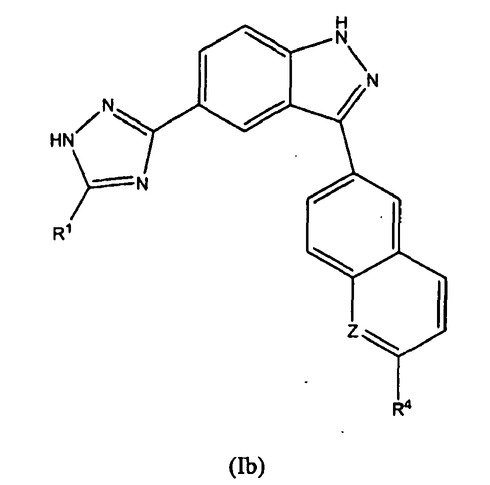 and isomers, prodrugs and pharmaceutically acceptable salts, solvates and hydrates thereof,
wherein
R¹ is -H, -C₁-C₆ alkyl, -(C₁-C₆ alkylene)-R² or -O-(C₁-C₆ alkylene)-R²;
R² is -C₁-C₆ alkyl, -C₁-C₆ alkoxy, -OH, -N(R³)₂, -aryl, -heteroaryl, -heterocycle, or -cycloalkyl;
each occurrence of R³ is independently -H, -C₁-C₆ alkyl, or -C₁-C₆ alkylene-(C₁-C₆ alkoxy);
R⁴ is -N(R⁵)₂, -O-C₁-C₆ alkyl, -C(O)NH-(C₁-C₆ alkylene)ₘ-heterocycle, - C(O)-heterocycle, -C(O)NH-(C₁-C₆ alkylene)ₘ-heteroaryl, -C(O)-heteroaryl, -(C₁-C₆ alkylene)-cycloalkyl, -O-(C₁-C₆ alkylene)-N(R⁵)₂, -O-(C₁-C₆ alkylene)ₘ-heterocycle, -O-(C₁-C₆ alkylene)ₘ-heteroaryl,
   -O-(C₁-C₆ alkylene)ₘ-cycloalkyl or -O-(C₁-C₆ alkylene)ₘ-C(O)R⁵;
Z is -CH- or -N-;
each occurrence of R⁵ is independently -H or -C₁-C₆ alkyl; and
m is 0 or 1.

In one embodiment, -Z is -CH-.

In another embodiment, R¹ is -H.

In still another embodiment, R¹ is -C₁-C₆ alkyl.

In another embodiment, R¹ is -(C₁-C₆ alkylene)-heterocycle.

In yet another embodiment, R¹ is -CH₂-heterocycle.

In a further embodiment, R⁴ is -N(R⁵)₂.

In a further embodiment, R⁴ is -O-(C₁-C₆ alkylene)-heterocycle.

In a further embodiment, R⁴ is -O-C₁-C₆ alkyl, preferable -OCH₃.

In another embodiment, R⁴ is -O-CH₂-heterocycle.

In another embodiment, R⁴ is -O-(CH₂)₂-heterocycle.

In another embodiment, R⁴ is -O-(CH₂)₃-heterocycle.

In another embodiment, R⁴ is -O-(C₁-C₆ alkylene)-heterocycle.

In a preferred embodiment, R¹ is -H and R⁴ is -O-(C₁-C₆ alkylene)-heterocycle.

In another preferred embodiment, R₁ is -CH₂-heterocycle and R⁴ is -O-(C₁-C₆ alkylene)-heterocycle.

In still another preferred embodiment, R¹ is -C₁-C₆ alkyl and R⁴ is -O-(C₁-C₆ alkylene)-heterocycle.

In still another preferred embodiment, Z is -CH-, R¹ is -(C₁-C₆ alkylene)-R², R² is -N(R³)₂ and R⁴ is -OCH₃.

In still another preferred embodiment, Z is -CH-, R¹ is -C₁-C₆ alkyl and R⁴ is -O-(C₂ alkylene)-N(R⁵)₂.

Illustrative Indazole Compounds of Formula (Ib) include the following:

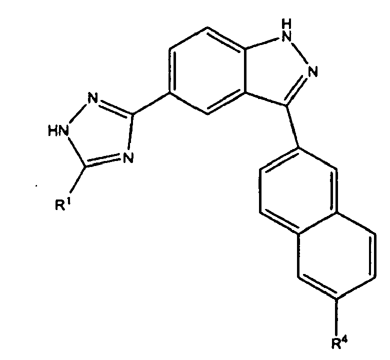

| Compound | R¹ | R⁴ |
|---|---|---|
| 43 | -H | -O(CH₂)₂-(pyrrolidin-1-yl) |
| 44 | -H | -O(CH₂)₂-(piperidin-1-yl) |
| 45 | -Me | -O(CH₂)₂-(azepan-1-yl) |
| 46 | -Me | -O(CH₂)₂-(pyrrolidin-1-yl) |
| 47 | -Me | -O(CH₂)₂-(2,6-dimethyl-piperidin-1-yl) |
| 48 | -CN | -OH |
| 49 | -isopropyl | -O(CH₂)₂-(2(S),6(R)-dimethyl-piperidin-1-yl) |
| 50 | -isobutyl | -O(CH₂)₂-(piperidin-1-yl) |
| 51 | -isobutyl | -O(CH₂)₃-(piperidin-1-yl) |
| 52 | -isobutyl | -O(CH₂)₂-(Pyrrolidin-1-yl) |
| 53 | -isobutyl | -O(CH₂)₂-(azepan-1-yl) |
| 54 | -isobutyl | -O(CH₂)₂-(2,6-dimethyl-piperidin-1-yl) |
| 55 | -isobutyl | -O(CH₂)₂-(1-methyl-pyrrolidin-2(R)-yl) |
| 56 | -isobutyl | -O(CH₂)₂-(2-methyl-piperidin-1-yl) |
| 57 | -isobutyl | -O-CH₂-(pyridin-2-yl) |
| 58 | -isobutyl | -O(CH₂)₂-(2(S),6(R)-dimethyl-piperidin-1-yl) |
| 59 | -isobutyl | -O(CH₂)₃-(2(S),6(R)-dimethyl-piperidin-1-yl) |
| 60 | -isobutyl | -OCH₂-(1,4-dimethyl-piperazin-2-yl) |
| 61 | -isobutyl | -O(CH₂)₂-(2,2,6,6-tetramethyl-piperidin-1-yl) |
| 62 | -isobutyl | -O(CH₂)₂-(2-methyl-1-piperidyl) |
| 63 | -isobutyl | -O(CH₂)₂-(2(S)-methyl-1-piperidyl) |
| 64 | -isobutyl | -O(CH₂)₂-(2(R)-methyl-1-piperidyl) |
| 65 | -isobutyl | -O(CH₂)₂-(2(S)-methyl-pyrrolidin-1-yl) |
| 66 | -isobutyl | -O(CH₂)₂-(2(R)-methyl-pyrrolidin-1-yl) |
| 67 | -isobutyl | -OCH₂-(1-methyl-pyrrolidin-2(S)-yl) |
| 68 | -isobutyl | -OCH₂-(1-methyl-pyrrolidin-2(R)-yl) |
| 69 | -isobutyl | -OCH₂-(1-ethyl-pyrrolidin-2(R)-yl) |
| 70 | -isobutyl | -OCH₂-(1-ethyl-pyrrolidin-2(S)-yl) |
| 71 | -isobutyl | -OCH₂-(2-methyl-pyrrolidin-1-yl-2-one) |
| 72 | -isobutyl | -OCH₂-(3-methyl-3*H-*imidazol-4-yl) |
| 73 | -isobutyl | -O(CH₂)₂N(CH₃)₂ |
| 74 | -isobutyl | -O(CH₂)₂N(isopropyl)₂ |
| 75 | -isobutyl | -O(CH₂)₂-(2(R),6(S)-dimethyl-piperidin-1-yl) |
| 76 | -isobutyl | -O(CH₂)₃-(2(R),6(S)-dimethyl-piperidin-1-yl) |
| 77 | -isobutyl | -O(CH₂)₂-(2,5-dimethyl-pyrrolidin-1-yl) |
| 78 | -isobutyl | -O(CH₂)₂-(2(R),5(S)-dimethyl-pyrrolidin-1-yl) |
| 79 | -isobutyl | -O(CH₂)₂-(2(S),5(R)-dimethyl-pyrrolidin-1-yl) |
| 80 | -isobutyl | -O(CH₂)-C(O)-(2(S),6(R)-dimethyl-piperidin-1-yl) |
| 81 | -isobutyl | -O(CH₂)-C(O)-(2(R),6(S)-dimethyl-piperidin-1-yl) |
| 82 | -t-butyl | -O(CH₂)₂-(pyrrolidin-1-yl) |
| 83 | -t-butyl | -O(CH₂)₂-(azepan-1-yl) |
| 84 | -t-butyl | -O(CH₂)₂-(piperidin-1-yl) |
| 85 | -t-butyl | -O(CH₂)₂-(2(S),6(R)-dimethyl-piperidin-1-yl) |
| 86 | -t-butyl | -OCH₂-(1-methyl-pyrrolidin-2(S)-yl) |
| 87 | -t-butyl | -OCH₂-(1-methyl-pyrrolidin-2(R)-yl) |
| 88 | -t-butyl | -OCH₂-(1,4-dimethyl-piperazin-2-yl) |
| 89 | -t-butyl | -O(CH₂)₂-imidazol-1-yl |
| 90 | -neopentyl | -F |
| 91 | -neopentyl | -O-(pyridin-2-yl) |
| 92 | -neopentyl | -OCH₂-(pyridin-2-yl) |
| 93 | -neopentyl | -O(CH₂)₂-(pyridin-3-yl) |
| 94 | -neopentyl | -OCH₂-(1,4-dimethyl-piperazin-2-yl) |
| 95 | -neopentyl | -O(CH₂)₂-(pyrrolidin-1-yl) |
| 96 | -neopentyl | -C(O)NH(CH₂)₂-(pyrrolidin-1-yl) |
| 97 | -neopentyl | -OCH₂-(1-methyl-piperidin-2-yl) |
| 98 | -neopentyl | -O(CH₂)₂-(2(S),6(R)-dimethyl-piperidin-1-yl) |
| 99 | -neopentyl | -O(CH₂)₂-(2(R),6(S)-dimethyl-piperidin-1-yl) |
| 100 | -neopentyl | -O(CH₂)-C(O)-(2(R),6(S)-dimethyl-piperidin-1-yl) |
| 101 | -neopentyl | -O(CH₂)-C(O)-(2(S),6(R)-dimethyl-piperidin-1-yl) |
| 102 | -neopentyl | -OCH₂-(1-methyl-pyrrolidin-2(S)-yl) |
| 103 | -neopentyl | -OCH₂-(1-methyl-pyrrolidin-2(R)-yl) |
| 104 | -neopentyl | -O(CH₂)₂-(pyrrolidin-1-yl) |
| 105 | -neopentyl | -O(CH₂)₂-(2,5-dimethyl-pyrrolidin-1-yl) |
| 106 | -neopentyl | -O(CH₂)₂-(2(R),5(S)-dimethyl-pyrrolidin-1-yl) |
| 107 | -neopentyl | -O(CH₂)₂-(2(S),5(R)-dimethyl-pyrrolidin-1-yl) |
| 108 | -neopentyl | -O-(CH₂)₂-N(ethyl)(isopropyl) |
| 109 | -neopentyl | -O(CH₂)₃-(2(S),6(R)-dimethyl-pipendin-1-yl) |
| 110 | -neopentyl | -O(CH₂)₃-(2(S),6(R)-dimethyl-piperidin-1-yl) |
| 111 | -neopentyl | -OCH₂-(1,4-dimethyl-piperazin-2-yl) |
| 112 | -neopentyl | -O(CH₂)₂-(pyrrolidin-1-yl-2-one) |
| 113 | -neopentyl | -OCH₂CH(isopropyl)(pyrrolidin-1-yl) |
| 114 | -neopentyl | -OCH₂-(1-methyl-piperazin-2-yl) |
| 115 | -neopentyl | -O(CH₂)₂-(imidazol-1-yl) |
| 116 | -neopentyl | -O(CH₂)₂-(2(S)-methyl-piperidin-1-yl) |
| 117 | -neopentyl | -OCH₂-(1-ethyl-pyrrolidin-2(S)-yl) |
| 118 | -neopentyl | -OCH₂-(1-ethyl-pyrrolidin-2(R)-yl) |
| 119 | -neopentyl | -O(CH₂)₂-(2,2,6,6-tetramethyl-piperidin-1-yl) |
| 120 | -neopentyl | -OCH₂-(1-methyl-pyrrolidin-2(R)-yl) |
| 121 | -neopentyl | -O(CH₂)₂N(isopropyl)₂ |
| 122 | -neopentyl | -O-(CH₂)₂-3-methyl-imidazolidin-2-one |
| 123 | -isopropyl | -O(CH₂)₂-(2(S),6(R)-dimethyl-piperidin-1-yl |
| 124 | -CH₂-pyrrolidin-1-yl | -Cl |
| 125 | -CH₂-pyrrolidin-1-yl | -O(CH₂)₂-(piperidin-1-yl) |
| 126 | -CH₂-pyrrolidin-1-yl | -O(CH₂)₂-(pyrrolidin-1-yl) |
| 127 | -CH₂-pyrrolidin-1-yl | -O(CH₂)₂-(azepan-1-yl) |
| 128 | -CH₂pyrrolidin-1-yl | -O(CH₂)₂-cyclopentyl |
| 129 | -CH₂-pyrrolidin-1-yl | -O(CH₂)₂-(2(S),6(R)-dimethyl-piperidin-1-yl |
| 130 | -CH₂-pyrrolidin-1-yl | -O(CH₂)₃-(2(S)-methyl-piperidin-1-yl) |
| 131 | -CH₂-pyrrolidin-1-yl | -OCH₂-(1-methyl-pyrrolidin-2(S)-yl) |
| 132 | -CH₂-pyrrolidin-1-yl | -OCH₂-(1-methyl-pyrrolidin-2(R)-yl) |
| 133 | -CH₂-pyrrolidin-1-yl | -OCH₂-(pyrrolidin-1-yl-2-one) |
| 134 | -CH₂-pyrrolidin-1-yl | -O(CH₂)₂-pyrrolidin-1-yl-2-one |
| 135 | -CH₂-pyrrolidin-1-yl | -OCH₂-(2-methyl-pyrrolidin-1-yl-2-one) |
| 136 | -CH₂-pyrrolidin-1-yl | C(O)-(pyrrolidin-1-yl) |
| 137 | -CH₂-pyrrolidin-1-yl | -OCH₂-(pyridin-2-yl) |
| 138 | -CH₂-pyrrolidin-1-yl | -O(CH₂)₂-(pyridin-2-yl) |
| 139 | -CH₂-pyrrolidin-1-yl | -O(CH₂)₂-(pyridin-3-yl) |
| 140 | -CH₂-pyrrolidin-1-yl | -C(O)-NH₂ |
| 141 | -CH₂-pyrrolidin-1-yl | -O(CH₂)₂N(CH₃)₂ |
| 142 | -CH₂-pyrrolidin-1-yl | -O(CH₂)₂-(2(S)-methyl-1-piperidyl) |
| 143 | -CH₂-pyrrolidin-1-yl | -O(CH₂)₂-(2(R)-methyl-1-piperidyl) |
| 144 | -CH₂-morpholin-1-yl | -OCHF₂ |
| 145 | -CH₂-morpholin-1-yl | -O(CH₂)₂-(pyrrolidin-1-yl) |
| 146 | -CH₂-morpholin-1-yl | -O(CH₂)₂-(piperidin-1-yl) |
| 147 | -CH₂-morpholin-1-yl | -OCH₂-(pyridin-2-yl) |
| 148 | -CH₂-morpholin-1-yl | -O(CH₂)₂-(pyridin-2-yl) |
| 149 | -CH₂-cyclopentyl | -O(CH₂)₂-(2(S),6(R)-dimethyl-piperidin-1-yl |
| 150 | -CH₂-cyclopropyl | -O(CH₂)₂-(piperidin-1-yl) |
| 151 | -CH₂-cyclopropyl | -O(CH₂)₂-(pyrrolidin-1-yl) |
| 152 | -CH₂-cyclopropyl | -O(CH₂)₂-(azepan-1-yl) |
| 153 | -CH₂-cyclopropyl | -O(CH₂)₂-(2(S),6(R)-dimethyl-piperidin-1-yl) |
| 154 | -CH₂-O-*t*-butyl | -O(CH₂)₂-(2(S),6(R)-dimethyl-piperidin-1-yl) |
| 155 | -CH₂-O-*t*-butyl | -O(CH₂)₂-pyrrolidin-1-yl-2-one |
| 156 | -CH₂-NH(*t*-butyl) | -OCH₃ |
| 157 | -CH₂-N((CH₂)₂-O-CH₃)₂ | -OCH₃ |
| 158 | 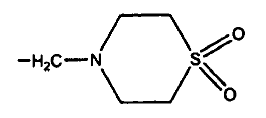 | -OCH₃ |

and isomers, prodrugs and pharmaceutically acceptable salts, solvates and hydrates thereof.

The present invention also provides compositions comprising a therapeutically effective amount of a Indazole Compound of Formula (Ib) and a pharmaceutically acceptable vehicle.

The invention further provides Indazole Compounds of Formula (Ib) that are in isolated and purified form.

### 4.2.4 THE INDAZOLE COMPOUNDS OF FORMULA Ic)

In one embodiment, the invention provides Indazole Compounds having the Formula (Ic): 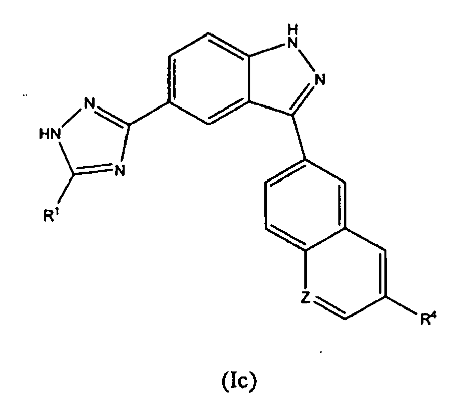 and isomers, prodrugs and pharmaceutically acceptable salts, solvates and hydrates thereof,
wherein
R¹ is -H, -(C₁-C₆ alkylene)-R² or -O-(C₁-C₆ alkylene)-R²;
R² is -C₁-C₆ alkyl, -C₁-C₆ alkoxy, -OH, -N(R³)₂, -aryl, -heteroaryl, - heterocycle, or -cycloalkyl;
each occurrence of R³ is independently -H, -C₁-C₆ alkyl, or -C₁-C₆ alkylene-(C₁-C₆ alkoxy);
R⁴ is -C(O)NH-(C₁-C₆ alkylene)ₘ-heterocycle, -C(O)-heterocycle, - C(O)NH-(C₁-C₆ alkylene)ₘ-heteroaryl, -C(O)-heteroaryl, -(C₁-C₆ alkylene)-cycloalkyl, - O-(C₁-C₆ alkylene)-N(R⁵)₂, -O-(C₁-C₆ alkylene)ₘ-heterocycle, -O-(C₁-C₆ alkylene)ₘ-heteroaryl,
   -O-(C₁-C₆ alkylene)ₘ-cycloalkyl or -O-(C₁-C₆ alkylene)ₘ-C(O)R⁵;
Z is -CH- or -N-;
each occurrence of R⁵ is independently -H or -C₁-C₆ alkyl; and
m is 0 or 1.

In one embodiment, -Z is -CH-.

In another embodiment, R¹ is -H.

In still another embodiment, R¹ is -C₁-C₆ alkyl.

In another embodiment, R¹ is -(C₁-C₆ alkylene)-heterocycle.

In yet another embodiment, R¹ is -CH₂-heterocycle.

In a further embodiment, R⁴ is -O-(C₁-C₆ alkylene)-heterocycle.

In another embodiment, R⁴ is -O-CH₂-heterocycle.

In another embodiment, R⁴ is -O-(CH₂)₂-heterocycle.

In another embodiment, R⁴ is -O-(CH₂)₃-heterocycle.

In another embodiment, R⁴ is -O-(C₁-C₆ alkylene)-heterocycle.

In a preferred embodiment, R₁ is -H and R⁴ is -O-(C₁-C₆ alkylene)-heterocycle.

In another preferred embodiment, R₁, is -CH₂-heterocycle and R⁴ is -O-(C₁-C₆ alkylene)-heterocycle.

In still another preferred embodiment, R₁, is -C₁-C₆ alkyl and R⁴ is -O-(C₁-C₆ alkylene)-heterocycle.

Illustrative Indazole Compounds of Formula (Ic) include the following:

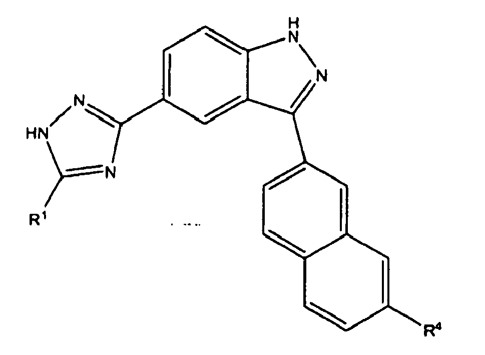

| Compound | R¹ | R⁴ |
|---|---|---|
| 159 | -isobutyl | -O(CH₂)₂-(2(S),6(R)-dimethyl-piperidin-1-yl) |
| 160 | -isobutyl | -O(CH₂)₂-(2(R),6(S)-dimethyl-piperidin-1-yl) |
| 161 | -isobutyl | -O(CH₂)₃-(2(S),6(R)-dimethyl-piperidin-1-yl) |
| 162 | -isobutyl | -O(CH₂)₃-(2(R),6(S)-dimethyl-piperidin-1-yl) |
| 163 | -isopentyl | -O(CH₂)₂-(2(S),6(R)-dimethyl-piperidin-1-yl) |
| 164 | -isopentyl | -O(CH₂)₂-(2(R),6(S)-dimethyl-piperidin-1-yl) |
| 165 | -isopentyl | -O(CH₂)₃-(2(S),6(R)-dimethyl-piperidin-1-yl) |
| 166 | -isopentyl | -O(CH₂)₃-(2(R),6(S)-dimethyl-piperidin-1-yl) |
| 167 | -isopentyl | -O(CH₂)₂-(pyrrolidin-1-yl) |
| 168 | -CH₂-piperidin-1-yl | -OCH₃ |
| 169 | -CH₂-morpholin-1-yl | -OCH₃ |
| 170 | -CH₂-morpholin-1-yl | -H |
| 171 | -CH₂-pyrrolidin-1-yl | -OCH₃ |

and isomers, prodrugs and pharmaceutically acceptable salts, solvates and hydrates thereof.

The present invention also provides compositions comprising a therapeutically effective amount of a Indazole Compound of Formula (Ic) and a pharmaceutically acceptable vehicle.

The invention further provides Indazole Compounds of Formula (Ic) that are in isolated and purified form.

### 4.2.5 THE INDAZOLE COMPOUNDS OF FORMULA (Id)

In one embodiment, the invention provides Indazole Compounds having the Formula (Id): 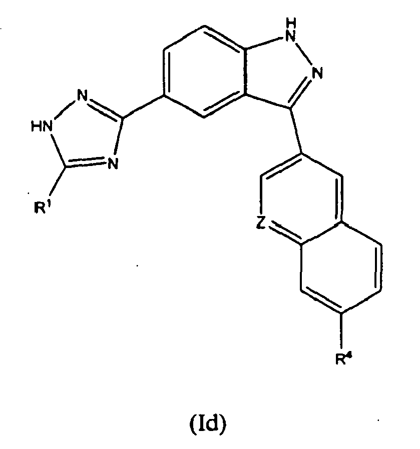 and isomers, prodrugs and pharmaceutically acceptable salts, solvates and hydrates thereof,
wherein
R¹ is -H, -C₁-C₆ alkyl, -(C₁-C₆ alkylene)-R² or -O-(C₁-C₆ alkylene)-R²;
R² is -C₁-C₆ alkyl, -C₁-C₆ alkoxy, -OH, -N(R³)₂, -aryl, -heteroaryl, - heterocycle, or -cycloalkyl;
each occurrence of R³ is independently -H, -C₁-C₆ alkyl, or -C₁-C₆ alkylene-(C₁-C₆ alkoxy);
R⁴ is H, -C(O)NH-(C₁-C₆ alkylene)ₘ-heterocycle, -C(O)-heterocycle, -C(O)NH-(C₁-C₆ alkylene)ₘ-heteroaryl, -C(O)-heteroaryl, -(C₁-C₆ alkylene)-cycloalkyl, -O-(C₁-C₆ alkylene)-N(R⁵)₂, -O-(C₁-C₆ alkylene)ₘ-heterocycle, -O-(C₁-C₆ alkylene)ₘ-heteroaryl,
   -O-(C₁-C₆ alkylene)ₘ-cycloalkyl or -O-(C₁-C₆ alkylene)ₘ-C(O)R⁵;
Z is -CH- or -N-;
each occurrence of R⁵ is independently -H or -C₁-C₆ alkyl; and
m is 0 or 1.

In one embodiment, -Z is -CH-.

In one embodiment, -Z is -N-.

In another embodiment, R¹ is -H.

In still another embodiment, R¹ is -C₁-C₆ alkyl.

In another embodiment, R¹ is -(C₁-C₆ alkylene)-heterocycle.

In yet another embodiment, R¹ is -CH₂-heterocycle.

In a further embodiment, R⁴ is H.

In a further embodiment, R⁴ is -CH₂-morpholine.

In a further embodiment, R⁴ is -CH₂-pyrrolidine.

In a further embodiment, R⁴ is -CH₂-N(CH₃)₂.

In a further embodiment, R⁴ is isobutyl.

In a further embodiment, R⁴ is -O-(C₁-C₆ alkylene)-heterocycle.

In another embodiment, R⁴ is -O-CH₂-heterocycle.

In another embodiment, R⁴ is -O-(CH₂)₂-heterocyde.

In another embodiment, R⁴ is -O-(CH₂)₃-heterocycle.

In another embodiment, R⁴ is -O-(C₁-C₆ alkylene)-heterocycle.

In a preferred embodiment, R₁, is -H and R⁴ is -O-(C₁-C₆ alkylene)-heterocycle.

In another preferred embodiment, R₁ is -CH₂-heterocycle and R⁴ is -O-(C₁-C₆ alkylene)-heterocycle.

In still another preferred embodiment, R₁ is -C₁-C₆ alkyl and R⁴ is -O-(C₁-C₆ alkylene)-heterocycle.

Illustrative Indazole Compounds of Formula (Id) include the following:

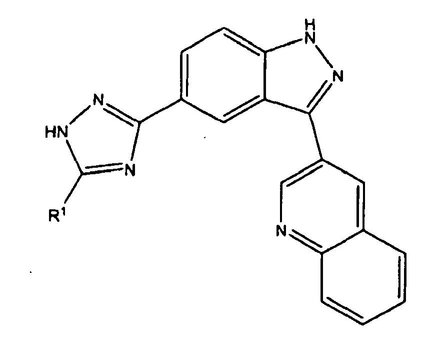

| Compound | R¹ |
|---|---|
| 172 | -CH₂-morpholin-1-yl |
| 173 | -CH₂-pyrrolidin-1-yl |
| 174 | -CH₂-N(CH₃)₂ |
| 175 | -isobutyl |

and isomers, prodrugs and pharmaceutically acceptable salts, solvates and hydrates thereof.

The present invention also provides compositions comprising a therapeutically effective amount of a Indazole Compound of Formula (Id) and a pharmaceutically acceptable vehicle.

The invention further provides Indazole Compounds of Formula (Id) that are in isolated and purified form.

### 4.2.6 THE INDAZOLE COMPOUNDS OF FORMULA (Ie)

In one embodiment, the invention provides Indazole Compounds having the Formula (Ie): 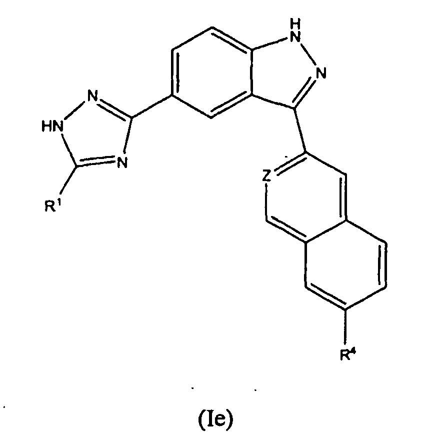 and isomers, prodrugs and pharmaceutically acceptable salts, solvates and hydrates thereof,
wherein
R¹ is -H, -C₁-C₆ alkyl, -(C₁-C₆ alkylene)-R² or -O-(C₁-C₆ alkylene)-R²;
R² is -C₁-C₆ alkyl, -C₁-C₆ alkoxy, -OH, -N(R³)₂, -aryl, -heteroaryl, - heterocycle, or -cycloalkyl;
each occurrence of R³ is independently -H, -C₁-C₆ alkyl, or -C₁-C₆ alkylene-(C₁-C₆ alkoxy);
R⁴ is H, -C(O)NH-(C₁-C₆ alkylene)ₘ-heterocycle, -C(O)-heterocycle, - C(O)NH-(C₁-C₆ alkylene)ₘ-heteroaryl, -C(O)-heteroaryl, -(C₁-C₆ alkylene)-cycloalkyl, - O-(C₁-C₆ alkylene)-N(R⁵)₂, -O-(C₁-C₆ alkylene)ₘ-heterocycle, -O-(C₁-C₆ alkylene)ₘ-heteroaryl,
   -O-(C₁-C₆ alkylene)ₘ-cycloalkyl or -O-(C₁-C₆ alkytene)ₘ-C(O)R⁵;
Z is -CH- or -N-;
each occurrence of R⁵ is independently -H or -C₁-C₆ alkyl; and
m is 0 or 1.

In one embodiment, -Z is -CH-.

In one embodiment, -Z is -N-.

In another embodiment, R¹ is -H.

In still another embodiment, R¹ is -C₁-C₆ alkyl.

In another embodiment, R¹ is -(C₁-C₆ alkylene)-heterocycle.

In yet another embodiment, R¹ is -CH₂-heterocycle.

In a further embodiment, R⁴ is H.

In a further embodiment, R⁴ is -CH₂-morpholine.

In a further embodiment, R⁴ is -CH₂-pyrrolidine.

In a further embodiment, R⁴ is -CH₂-N(CH₃)₂.

In a further embodiment, R⁴ is isobutyl.

In a further embodiment, R⁴ is -O-(C₁-C₆ alkylene)-heterocycle.

In another embodiment, R⁴ is -O-CH₂-heterocycle.

In another embodiment, R⁴ is -O-(CH₂)₂-heterocycle.

In another embodiment, R⁴ is -O-(CH₂)₃-heterocycle.

In another embodiment, R⁴ is -O-(C₁-C₆ alkylene)-heterocycle.

In a preferred embodiment, R₁ is -H and R⁴ is -O-(C₁-C₆ alkylene)-heterocycle.

In another preferred embodiment, R₁ is -CH₂-heterocycle and R⁴ is -O-(C₁-C₆ alkylene)-heterocycle.

In still another preferred embodiment, R₁ is -C₁-C₆ alkyl and R⁴ is -O-(C₁-C₆ alkylene)-heterocycle.

Illustrative Indazole Compounds of Formula (Id) include the following:

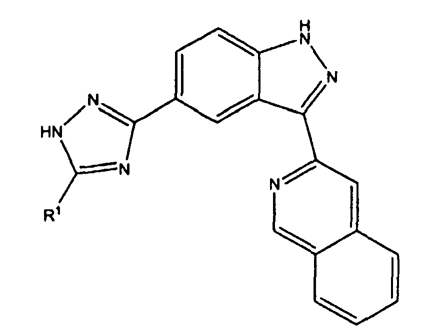

| Compound | R¹ |
|---|---|
| 176 | -CH₂-morpholin-1-yl |

and isomers, prodrugs and pharmaceutically acceptable salts, solvates and hydrates thereof.

The present invention also provides compositions comprising a therapeutically effective amount of a Indazole Compound of Formula (Ie) and a pharmaceutically acceptable vehicle.

The invention further provides Indazole Compounds of Formula (Ie) that are in isolated and purified form.

### 4.2.7 THE INDAZOLE COMPOUNDS OF FORMULA (II)

In another embodiment, the invention provides Indazole Compounds having the Formula (II): 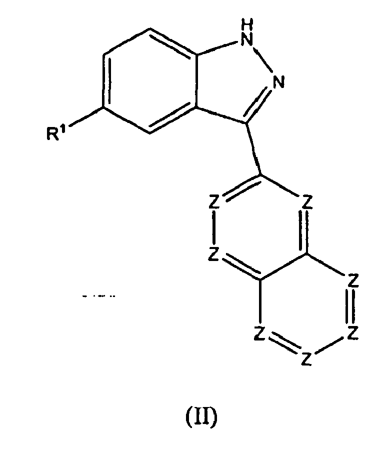 and isomers, prodrugs and pharmaceutically acceptable salts, solvates and hydrates thereof,
wherein
R¹ is -COOR²,-CN, -NO₂, -C(O)-heterocycle, -CH=CH-heterocycle, -C(O)N(R²)₂, or -1*H-*tetrazol-5-yl;
each occurrence of R² is independently -H, -C₁-C₆ alkyl, -hydroxyalkyl, -C₁-C₆ alkyl-N(R²)₂, -C₁-C₆ alkyl-O-C₁-C₆ alkyl, -(CH₂)ₙ-cycloalkyl, -(CH₂)ₙ-heteroaryl, -(CH₂)ₙ-heterocycle, or -(CH₂)ₙ-aryl;
each occurrence of Z is -C(R³)- or -N-, wherein up to 3 occurrences of Z can be -N-;
R³ is -COOR², -CN, -NO₂, -C(O)N(R²)₂, N(R²)₂, -C(O)O-(C₁-C₆ alkyl), -C(O)NH-(CH₂)ₙ-heterocycle, -C(O)NH-(CH₂)ₙ-heteroaryl, -C(O)-heterocycle, -C(O)-heteroaryl, -(CH₂)ₙ-heterocycle, -(CH₂)ₙ-heteroaryl, (CH₂)ₚ-cycloalkyl, -O-(CH₂)ₙ-N(R²)₂, -O-(CH₂)ₙ-heterocycle), -O-(CH₂)ₙ-heteroaryl, or -O-(CH₂)ₙ-cycloalkyl);
m is 0 or 1;
n is an integer ranging from 0 to 3; and
p is an integer ranging from 1 to 3.

In one embodiment, each occurrence of Z is -C(R³)-.

In another embodiment, R₁ is -CN.

In another embodiment, R₁ is -NO₂.

In still another preferred embodiment, R₁ is -C(O)NH₂.

In yet another preferred embodiment, R₁ is -1*H*-tetrazol-5-yl.

In a further embodiment, R₁ is -COOH.

In another embodiment, R₁ is -C(O)O-(C₁-C₆ alkyl).

In one embodiment, R₃ is -O-(C₁-C₆ alkylene)-heterocycle.

In a preferred embodiment, R₁ is -CN and R₃ is -O-(C₁-C₆ alkylene)-heterocycle.

In another preferred embodiment, R₁ is -C(O)NH₂ and R₃ is -O-(C₁-C₆ alkylene)-heterocycle.

In another preferred embodiment, R₁ is -C(O)N(R²)₂ wherein one R₂ is H and the other R₂ is C₁-C₆ alkyl.

In another preferred embodiment, R₁ is -C(O)N(R²)₂ wherein one R₂ is H and the other R₂ is -(CH₂)ₙ-heterocycle.

In another preferred embodiment, R₁ is -C(O)N(R²)₂ wherein one R₂ is H and the other R₂ is -(CH₂)ₙ-cycloalkyl.

In another preferred embodiment, R₁ is -C(O)N(R²)₂ wherein one R₂ is H and the other R₂ is -C₁-C₆ alkyl-O-C₁-C₆ alkyl.

Illustrative examples of Compounds of Formula (II), include the following:

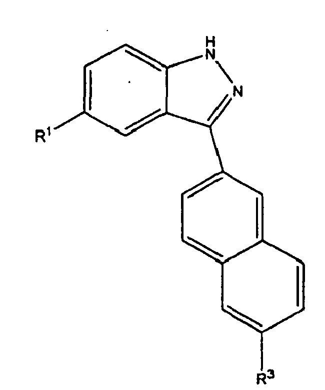

| Compound | R¹ | R³ |
|---|---|---|
| 177 | -CN | -O(CH₂)₂-(1-pyrrolidinyl) |
| 178 | -C(O)NH₂ | -OCH₂-(1-methyl-2-(R)-pyrrolidinyl) |
| 179 | -C(O)NH₂ | -OCH₂-(1-methyl-2-(S)-pyrrolidinyl) |
| 180 | -CH=CH-(1,2,4-triazol-3-yl-methylmorpholin-1-yl | -OCH₃ |
| 181 | -C(O)NH(3,3-dimethylbutane) | -O-(CH₂)₂-N(isopropyl)₂ |
| 182 | -C(O)NH-CH₂-cyclopropyl | -OCH₂-(1-ethyl-pyrrolidin-2(S)-yl) |
| 183 | -C(O)NH-CH₂-cyclopropyl | -OCH₂-(1-ethyl-pyrrolidin-2(R)-yl) |
| 184 | -C(O)NH(butyl) | -OCH₂-(1-ethyl-pyrrolidin-2(S)-yl) |
| 185 | -C(O)NH(butyl) | -OCH₂-(1-ethyl-pyrrolidin-2(R)-yl) |
| 186 | -C(O)NH(isobutyl) | -OCH₂-(1-ethyl-pyrrolidin-2(S)-yl) |
| 187 | -C(O)NH(isobutyl) | -OCH₂-(1-ethyl-pyrrolidin-2(R)-yl) |
| 188 | -C(O)NH(t-butyl) | -OCH₂-(1-ethyl-pyrrolidin-2(S)-yl) |
| 189 | -C(O)NH(t-butyl) | -OCH2-(1-ethyl-pyrrolidin-2(R)-yl) |
| 190 | -C(O)NH(sec-butyl) | -OCH₂-(1-ethyl-pyrrolidin-2(S)-yl) |
| 191 | -C(O)NH(sec-butyl) | -OCH₂-(1-ethyl-pyrrolidin-2(R)-yl) |
| 192 | -C(O)NH(isopentyl) | -O-(CH₂)₂-N(isopropyl)₂ |
| 193 | -C(O)NH(isopentyl) | -OCH₂-(1-ethyl-pyrrolidin-2(S)-yl) |
| 194 | -C(O)NH(isopentyl) | -OCH₂-(1-ethyl-pyrrolidin-2(R)-yl) |
| 195 | -C(O)NH(ethyl) | -OCH₃ |
| 196 | -C(O)NH((CH₂)₂-morpholin-1-yl) | -OCH₃ |
| 197 | -C(O)NH((CH₂)₂-pyrrolidin-1-yl) | -OCH₃ |
| 198 | -C(O)NH((CH₂)₂-Pyrrolidin-1-yl) | -OCH₂-(1-methyl-pyrrolidin-2(S)-yl) |
| 199 | -C(O)NH((CH₂)₂-pycrolidin-1-yl) | -OCH₂-(1-methyl-pyrrolidin-2(R)-yl) |
| 200 | -C(O)NH((CH₂)₂OCH₃) | -OCH₃ |
| 201 | -C(O)NH((CH₂)₂OCH₃) | -OCH₂-(1-methyl-pyrrolidin-2(S)-yl) |
| 202 | -C(O)NH((CH₂)₂OCH₃) | -OCH₂-(1-methyl-pyrrolidin-2(R)-yl) |
| 203 | -C(O)-purrolidin-1-yl | -OCH₂-(1-methyl-pyrrolidin-2(S)-yl) |
| 204 | -C(O)-pyrrolidin-1-yl | -OCH₂-(1-methyl-pyrrolidin-2(R)-yl) |
| 205 | -C(O)NH((CH₂)₂N(CH₃)₂) | -OCH₂-(1-methyl-pyrrolidin-2(S)-yl) |
| 206 | -C(O)NH((CH₂)₂OCH₃) | -OCH₂-(1-methyl-pyrrolidin-2(R)-yl) |

and isomers, prodrugs and pharmaceutically acceptable salts, solvates and hydrates thereof.

The present invention also provides compositions comprising a therapeutically effective amount of an Indazole Compound of Formula (II) and a pharmaceutically acceptable vehicle.

The invention further provides Indazole Compounds of Formula (II) that are in isolated and purified form.

In another embodiment, the present invention provides a method for treating a Condition, comprising administering a therapeutically effective amount of an Indazole Compound of Formula (II) to a patient in need thereof.

In another embodiment, the present invention provides a method for treating a Condition, comprising administering a therapeutically effective amount of an Indazole Compound of Formula (I), (Ia), (Ib), (Ic), (Id), (Ie) or (II) to a patient in need thereof.

### 4.3 METHODS FOR MAKING THE INDAZOLE COMPOUNDS

The Indazole Compounds can be made using techniques known to those skilled in the art of organic synthesis using known starting materials and reagents, as well as by the following general techniques and by the procedures set forth in the Examples. To that end, the Indazole Compounds can be made according to the following Schemes 1 through 5 (it should be noted that, in the following reaction schemes, hydrogen atoms are sometimes not depicted and that one skilled in the art of organic chemistry would appreciate such accepted shorthand notation).

Scheme 1 illustrates a method for making the 5-cyano indazole C, which is a useful intermediate for making the Indazole Compounds.

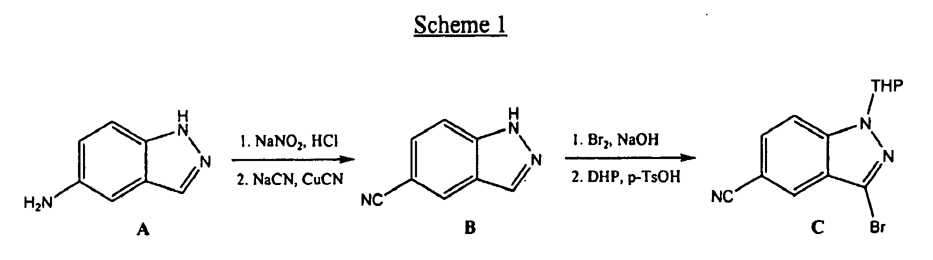

Commercially available 5-Aminoindazole (A) is converted to it's 5-nitro derivative using sodium nitrate in acidic media. The 5-nitro intermediate is then converted to the 5-CN intermediate **B** upon treatment with sodium cyanide in the presence of copper(II) cyanide. Compound **B** is then brominated in the 3-position and protected as its 1-N-THP derivative C using DHP and catalytic p-TsOH.

Scheme 2 shows a method useful for making Indazole Derviatives of Formula (II) wherein R³ is -O-(CH₂)ₙ- N(R⁴)₂, -O-(CH₂)ₙ-heterocycle, or -O-(CH₂)ₙ-cycloalkyl). 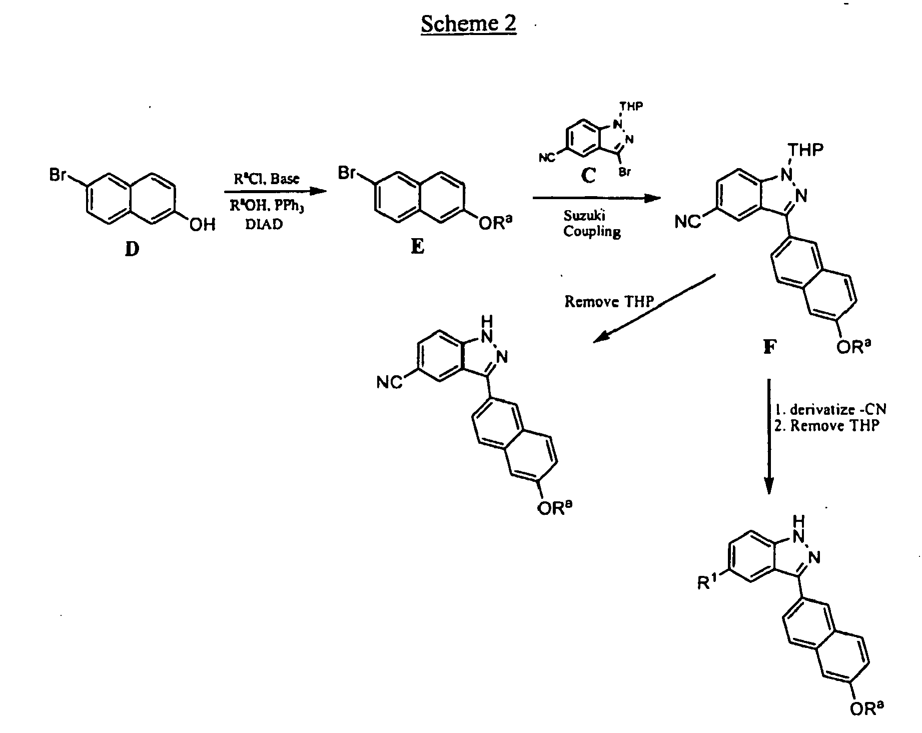 wherein R¹ is as defined above for the Indazole Compounds of Formula (II) and R^{a} is -(CH₂)ₙ-N(R⁴)₂, -(CH₂)ₙ-heterocycle, or -(CH₂)ₙ-cycloalkyl.

Commercially available 6-bromo-2-naphthol (**D**) is converted to the ether derivatives of formula **E** by either treating the alkoxide of **D** with an electrophile of formula R^{a}-Cl, or via a Mitsunobu coupling of D with a hydroxy compound of formula R^{a}OH in the presence of DIAD and triphenylphosphine. The compounds of formula **E** can be converted to their boronic acid or ester derviatives, then coupled with compound **C** using a Suzuki-type coupling (see Miyaura et al., Tetrahedron Letters, 1979, 3427; and Miyaura et al., Chem. Comm., 1979, 866) to provide the 3-substituted indazoles of formula **F**. The THP protecting group of the compounds of formula **F** can subsequently be removed via acid hydrolysis according to the procedure set forth in Robins et al., J. Am. Chem. Soc., 1961, 83:2574, to provide the Indazole Derviatives of Formula (II) wherein R¹ is -CN and R³ is -O-(CH₂)ₙ-N(R⁴)₂, -O-(CH₂)ₙ-heterocycle, -O-(CH₂)ₙ-cycloalkyl. Alternatively, the -CN group of the compounds of formula **F** can be derivatized using well-known methodology prior to THP removal to provide the Indazole Derviatives of Formula (II) wherein R¹ is other than -CN and R³ is -O-(CH₂)ₙ-N(R⁴)₂, - O-(CH₂)ₙ-heterocycle, or -O-(CH₂)ₙ cycloalkyl.

Scheme 3 shows a method useful for making the Indazole Derviatives of formula (I), (Ia) or (Ib) wherein R⁴ is -O-(CH₂)ₙ-N(R⁴)₂, -O-(CH₂)ₙ-heterocycle, or -O-(CH₂)ₙ-cycloalkyl. 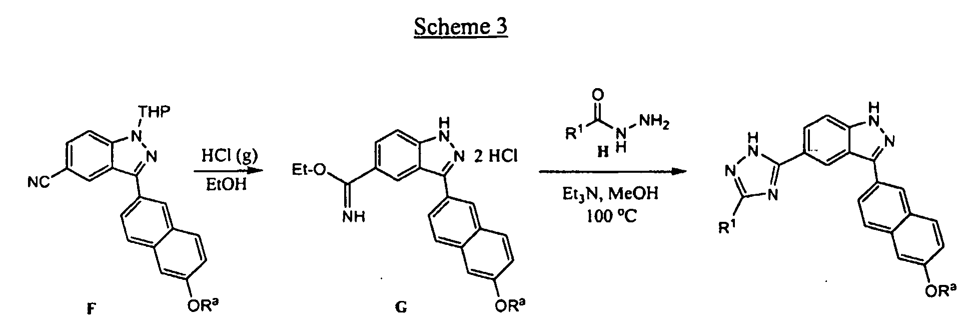 wherein R¹ is as defined above for the Indazole Compounds of Formula formula (I), (Ia) or (Ib) and R^{a} is -(CH₂)ₙ-N(R⁴)₂, -(CH₂)ₙ-heterocycle, or -(CH₂)ₙ-cycloalkyl.

The compounds of formula **F** can also be treated with HCl in ethanol to provide the imidate intermediates of formula **G** which are then reacted with a compound of formula H in the presence of triethylamine to provide the corresponding 5-triazolyl derivatives to provide the compounds of Formula (1), (Ia) or (Ib) wherein R¹ is as defined above for the compounds of Formula (I), (Ia) or (Ib) and R⁴ is -O-(CH₂)ₙ- N(R⁴)₂, -O-(CH₂)ₙ-heterocycle, or -O-(CH₂)ₙ-cycloalkyl.

Scheme 4 shows a method useful for making the Indazole Derviatives of formula (II) wherein R³ is other than -O-(CH₂)ₙ- N(R⁴)₂, -O-(CH₂)ₙ-heterocycle, or -O-(CH₂)ₙ-cycloalkyl. 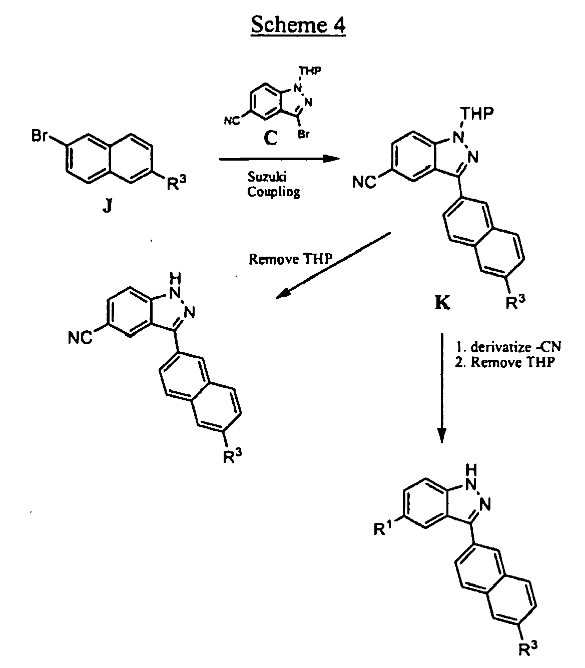 wherein R¹ is as defined above for the Indazole Compounds of Formula (II) and R³ is as defined above for the Indazole Compounds of Formula (II) other than -O-(CH₂)ₙ- N(R⁴)₂, -O-(CH₂)ₙ-heterocycle, -O-(CH₂)ₙ-cycloalkyl.

The 2-bromonaphtyl derivatives of formula **J** (which can be commerically available or if not commercially available, can be made from commercially available 2-bromonaphtyl derivatives using well-known synthetic organic chemistry methodology) can be converted to their boronic acid or ester derviatives, then coupled with compound C using a Suzuki-type coupling (see Miyaura et al., Tetrahedron Letters, 1979, 3427; and Miyaura et al., Chem. Comm., 1979, 866) to provide the 3-substituted indazoles of formula **K**. The THP protecting group of the compounds of formula **K** can subsequently be removed via acid hydrolysis according to the procedure set forth in Robins et al., J. Am. Chem. Soc., 1961, 83:2574, to provide the Indazole Derviatives of Formula (II) wherein R¹ is -CN and R³, is as defined above for the compounds of Formula (II), and is other than -O-(CH₂)ₙ- N(R⁴)₂, -O-(CH₂)ₙ-heterocycle, -O-(CH₂)ₙ-cycloalkyl. Alternatively, the -CN group of the compounds of formula **K** can be derivatized using well-known methodology prior to THP removal to provide the Indazole Derviatives of Formula (II) wherein R¹ is other than -CN and R³ is other than -O-(CH₂)ₙ- N(R⁴)₂, -O-(CH₂)ₙ- heterocycle, or -O-(CH₂)ₙ-cycloalkyl.

Scheme 5 shows a method useful for making the Indazole Derviatives of Formula (I), (Ia) or (Ib)wherein R⁴ is other than -O-(CH₂)ₙ- N(R⁴)₂, -O-(CH₂)ₙ-heterocycle, or -O-(CH₂)ₙ-cycloakyl. 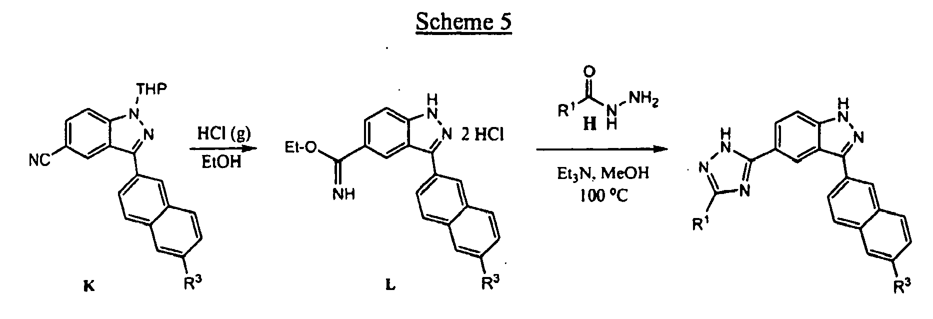 wherein R¹ is as defined above for the Indazole Compounds of Formula (I), (Ia) or (Ib) and R³ is as defined above for the Indazole Compounds of Formula (I), (Ia) or (Ib) other than -O-(CH₂)ₙ- N(R⁴)₂, -O-(CH₂)ₙ-heterocycle, or -O-(CH₂)ₙ-cycloalkyl.

The compounds of formula **K** can also be treated with HCl in ethanol to provide the imidate intermediates of formula **L** which are then reacted with a compound of formula H in the presence of triethylamine to provide the corresponding 5-triazolyl derivatives to provide the compounds of Formula (I), (Ia) or (Ib) wherein R² and R³ are as defined above for the compounds of Formula (I), (Ia) or (Ib) and R³ is other than -O-(CH₂)ₙ- N(R⁴)₂, -O-(CH₂)ₙ-heterocycle, or -O-(CH₂)ₙ-cycloalkyl.

It should be noted that the above synthetic schemes can be used to prepare Indazole Compounds wherein the naphthalenyl or quinolinyl ring is substituted at any available position (e.g., those of formula (Ic), (Id) or (Ie)) by using the appropriately substituted starting material.

An Indazole Compound can be in the form of a pharmaceutically acceptable salt or a free base. Pharmaceutically acceptable salts of the Indazole Compounds can be formed from organic and inorganic acids. Suitable non-toxic acids include, but are not limited to, inorganic and organic acids such as acetic, alginic, anthranilic, benzenesulfonic, benzoic, camphorsulfonic, citric, ethenesulfonic, formic, fumaric, furoic, galacturonic, gluconic, glucuronic, glutamic, glycolic, hydrobromic, hydrochloric, isethionic, lactic, maleic, malic, mandelic, methanesulfonic, mucic, nitric, pamoic, pantothenic, phenylacetic, phosphoric, propionic, salicylic, stearic, succinic, sulfanilic, sulfuric, tartaric acid, and p-toluenesulfonic acid. Specific non-toxic acids include hydrochloric, hydrobromic, phosphoric, sulfuric, and methanesulfonic acids. The Indazole Compounds can also be used in the form of base addition salts. Suitable pharmaceutically acceptable base addition salts for the Indazole Compounds include, but are not limited to metallic salts made from aluminum, calcium, lithium, magnesium, potassium, sodium and zinc or organic salts made from lysine, N,N'-dibenzylethylenediamine, chloroprocaine, choline, diethanolamine, ethylenediamine, meglumine (N-methylglucamine) and procaine. Examples of specific salts thus include hydrochloride and mesylate salts. Others are well-known in the art, see for example, Remington's Pharmaceutical Sciences, 18th eds., Mack Publishing, Easton PA (1990) or Remington: The Science and Practice of Pharmacy, 19th eds., Mack Publishing, Easton PA (1995). Thus, the term "pharmaceutically acceptable salt" of an Indazole Compound is intended to encompass any and all acceptable salt forms.

Pharmaceutically acceptable salts of this invention may be formed by conventional and known techniques, such as by reacting a compound of this invention with a suitable acid as disclosed above. Such salts are typically formed in high yields at moderate temperatures, and often are prepared by merely isolating the compound from a suitable acidic wash in the final step of the synthesis. The salt-forming acid may dissolved in an appropriate organic solvent, or aqueous organic solvent, such as an alkanol, ketone or ester. On the other hand, if the Indazole Compound is desired in the free base form, it can be isolated from a basic final wash step, according to known techniques. For example, a typical technique for preparing hydrochloride salt is to dissolve the free base in a suitable solvent, and dry the solution thoroughly, as over molecular sieves, before bubbling hydrogen chloride gas through it.

### 4.4 USES OF THE INDAZOLE COMPOUNDS

In one embodiment, the invention relates to methods for treating or preventing cancer, a cardiovascular disease, a renal disease, an autoimmune condition, an inflammatory condition, macular degeneration, pain and related syndromes, disease-related wasting, an asbestos-related condition, pulmonary hypertension or a condition treatable or preventable by inhibition of the JNK pathway, comprising administering an effective amount of an Indazole Compound to a patient in need of the treating or preventing.

Representative autoimmune conditions that the Indazole Compounds are useful for treating or preventing include, but are not limited to, rheumatoid arthritis, rheumatoid spondylitis, osteoarthritis, multiple sclerosis, lupus, inflammatory bowel disease, ulcerative colitis, Crohn's disease, myasthenia gravis, Grave's disease and diabetes (e.g., Type I diabetes).

Representative inflammatory conditions that the Indazole Compounds are useful for treating or preventing include, but are not limited to, asthma and allergic rhinitis, bronchitis, chronic obstructive pulmonary disease, cystic fibrosis, inflammatory bowel disease, irritable bowel syndrome, Crohn's disease, mucous colitis, ulcerative colitis and obesity.

Representative cardiovascular diseases that the Indazole Compounds are useful for treating or preventing include, but are not limited to, stroke, myocardial infarction or iscehmic damage to the heart, lung, gut, kidney, liver, pancreas, spleen or brain.

Representative cardiovascular and renal diseases that an Indazole Compound containing or coated stent is useful for treating or preventing include atherosclerosis and the treatment or prevention of restenosis after vascular intervention such as angioplasty.

An Indazole Compound containing or coated stent can further comprise an effective amount of another active agent useful for treating or preventing a cardiovascular or renal disease, including, but are not limited to, an anticoagulant agent, an antimetabolite agent, an anti-inflammatory agent, an antiplatelet agent, an antithrombin agent, an antimitotic agent, a cytostatic agent or an antiproliferative agent. Illustrative examples of other active agents that the Aminopurine Compound containing or coated stent can further comprise include, but are not limited to, IMiDs^{®} and SelCIDs^{®} (Celgene Corporation, New Jersey) (e.g., those disclosed in U.S. patent nos. 6,075,041; 5,877,200; 5,698,579; 5,703,098; 6,429,221; 5,736,570; 5,658,940; 5,728,845; 5,728,844; 6,262,101; 6,020,358; 5,929,117; 6,326,388; 6,281,230; 5,635,517; 5,798,368; 6,395,754; 5,955,476; 6,403,613; 6,380,239; and 6,458,810, each of which is incorporated herein by reference), PDE IV inhibitors (*e.g.,* cilomast, theophylline, zardaverine, rolipram, pentoxyfylline, enoximone), paclitaxel, docetaxel or a derivative thereof, an epothilone, a nitric oxide release agent, heparin, aspirin, coumadin, PPACK, hirudin, polypeptide from angiostatin and endostatin, methotrexate, 5-fluorouracil, estradiol, P-selectin Glycoprotein ligand-1 chimera, abciximab, exochelin, eleutherobin and sarcodictyin, fludarabine, sirolimus, tranilast, VEGF, transforming growth factor (TGF)-beta, Insulin-like growth factor (IGF), platelet derived growth factor (PDGF), fibroblast growth factor (FGF), RGD peptide, a beta or gamma ray emitter (radioactive) agent.

The Indazole Compounds are also useful for treating or preventing ischemia / reperfusion injury in general. Accordingly, the Aminopurine Compounds are useful for treating or preventing acute or chronic organ transplant rejection and for the preservation of tissue and organs.

### 4.4.1 TREATMENT OR PREVENTION OF PROLIFERATIVE DISORDERS

A proliferative disorder can be treated or prevented by administration of a therapeutically effective amount of an Indazole Compound.

Proliferative disorders that can be treated or prevented by administering a therapeutically effective amount of an Indazole Compound include, but are not limited to cancer, uterine fibroids, benign prostatic hyperplasia, familial adenomatosis polyposis, neuro-fibromatosis, atherosclerosis, pulmonary fibrosis, arthritis, psoriasis, glomerulonephritis, restenosis following angioplasty or vascular surgery, hypertrophic scar formation, inflammatory bowel disease, transplantation rejection, endotoxic shock, fungal infections, and defective apoptosis-associated conditions.

### 4.4.2 TREATMENT OR PREVENTION OF CANCER

Cancer can be treated or prevented by administration of a therapeutically effective amount of an Indazole Compound.

In a majority of cancers, overexpression and/or hyperactivation of a variety of protein kinases, such as receptor and non-receptor kinases, serine/threonine kinases, PI3 kinases and cell cycle-associated kinases is common. Several of these kinases, either alone or in conjunction with other kinases have been implicated in numerous processes important for cell survival, proliferation, growth and malignant transformation, motility and invasion leading to metastasis and angiogenesis. In some instances, inhibition of a single kinase or a single cell transduction pathway may not be sufficient to elicit a desirable therapeutic effect. Without being bound by theory, the simultaneous inhibition of various kinases may be useful for treating or preventing proliferative disorders, such as cancer. Compounds that simultaneously inhibit more than one kinase are commonly referred to as "mixed kinase inhibitors." As such, mixed kinase inhibitors may be useful for the simultaneous inhibition of various kinases which are responsible for a variety of cellular processes, including proliferation, growth, motility, and invasiveness. Therefore, the Indazole Compounds, which can act as mixed kinase inhibitors, are be useful for the treatment of cancer or other proliferative diseases.

The Indazole Compounds can also be administered to prevent progression to a neoplastic or malignant state, including but not limited to the cancers listed in Table 1. Such prophylactic use is indicated in conditions known or suspected of preceding progression to neoplasia or cancer, in particular, where non-neoplastic cell growth consisting of hyperplasia, metaplasia, or most particularly, dysplasia has occurred (for review of such abnormal growth conditions, see Robbins and Angell, 1976, Basic Pathology, 2d Ed., W.B. Saunders Co., Philadelphia, pp. 68-79). Hyperplasia is a form of controlled cell proliferation involving an increase in cell number in a tissue or organ, without significant alteration in structure or function. For example, endometrial hyperplasia often precedes endometrial cancer and precancerous colon polyps often transform into cancerous lesions. Metaplasia is a form of controlled cell growth in which one type of adult or fully differentiated cell substitutes for another type of adult cell. Metaplasia can occur in epithelial or connective tissue cells. A typical metaplasia involves a somewhat disorderly metaplastic epithelium. Dysplasia is frequently a forerunner of cancer, and is found mainly in the epithelia; it is the most disorderly form of non-neoplastic cell growth, involving a loss in individual cell uniformity and in the architectural orientation of cells. Dysplastic cells often have abnormally large, deeply stained nuclei, and exhibit pleomorphism. Dysplasia characteristically occurs where there exists chronic irritation or inflammation, and is often found in the cervix, respiratory passages, oral cavity, and gall bladder.

Alternatively or in addition to the presence of abnormal cell growth characterized as hyperplasia, metaplasia, or dysplasia, the presence of one or more characteristics of a transformed phenotype, or of a malignant phenotype, displayed in *vivo* or displayed *in vitro* by a cell sample from a patient, can indicate the desirability of prophylactic/therapeutic administration of the composition of the invention. Such characteristics of a transformed phenotype include morphology changes, looser substratum attachment, loss of contact inhibition, loss of anchorage dependence, protease release, increased sugar transport, decreased serum requirement, expression of fetal antigens, disappearance of the 250,000 dalton cell surface protein, etc. (see also *id.,* at pp. 84-90 for characteristics associated with a transformed or malignant phenotype).

In a specific embodiment, leukoplakia, a benign-appearing hyperplastic or dysplastic lesion of the epithelium, or Bowen's disease, a carcinoma *in situ,* are pre-neoplastic lesions indicative of the desirability of prophylactic intervention.

In another embodiment, fibrocystic disease (cystic hyperplasia, mammary dysplasia, particularly adenosis (benign epithelial hyperplasia)) is indicative of the desirability of prophylactic intervention.

The prophylactic use of the compounds and methods of the present invention are also indicated in some viral infections that may lead to cancer. For example, human papilloma virus can lead to cervical cancer (see, e.g., Hernandez-Avila et al., Archives of Medical Research (1997) 28:265-271), Epstein-Barr virus (EBV) can lead to lymphoma (see, *e.g.,* Herrmann et al., J Pathol (2003) 199(2):140-5), hepatitis B or C virus can lead to liver carcinoma (see, e.g., El-Serag, J Clin Gastroenterol (2002) 35(5 Suppl 2):S72-8), human T cell leukemia virus (HTLV)-I can lead to T-cell leukemia (see e.g., Mortreux et al., Leukemia (2003) 17(1):26-38), human herpesvirus-8 infection can lead to Kaposi's sarcoma (see, e.g., Kadow et al., Curr Opin Investig Drugs (2002) 3(11):1574-9), and Human Immune deficiency Virus (HIV) infection contribute to cancer development as a consequence of immunodeficiency (see, *e.g.,* Dal Maso et al., Lancet Oncol (2003) 4(2):110-9).

In other embodiments, a patient which exhibits one or more of the following predisposing factors for malignancy can treated by administration of the compounds or methods of the invention: a chromosomal translocation associated with a malignancy (e.g., the Philadelphia chromosome for chronic myelogenous leukemia, t(14;18) for follicular lymphoma, etc.), familial polyposis or Gardner's syndrome (possible forerunners of colon cancer), benign monoclonal gammopathy (a possible forerunner of multiple myeloma), a first degree kinship with persons having a cancer or precancerous disease showing a Mendelian (genetic) inheritance pattern (*e.g*., familial polyposis of the colon, Gardner's syndrome, hereditary exostosis, polyendocrine adenomatosis, medullary thyroid carcinoma with amyloid production and pheochromocytoma, Peutz-Jeghers syndrome, neurofibromatosis of Von Recklinghausen, retinoblastoma, carotid body tumor, cutaneous melanocarcinoma, intraocular melanocarcinoma, xeroderma pigmentosum, ataxia telangiectasia, Chediak-Higashi syndrome, albinism, Fanconi's aplastic anemia, and Bloom's syndrome; see Robbins and Angell, 1976, Basic Pathology, 2d Ed., W.B. Saunders Co., Philadelphia, pp. 112-113) etc.), and exposure to carcinogens (e.g., smoking, and inhalation of or contacting with certain chemicals).

In a preferred embodiment, the present invention provides methods for treating cancer, including but not limited to: killing a cancer cell or neoplastic cell; inhibiting the growth of a cancer cell or neoplastic cell; inhibiting the replication of a cancer cell or neoplastic cell; or ameliorating a symptom thereof, the methods comprising administering to a patient in need thereof an amount of the Indazole Compounds effective to treat cancer.

In one embodiment, the invention provides a method for treating cancer, said method comprising administering to a patient in need thereof an amount of an Indazole Compound or a pharmaceutically acceptable salt thereof, said amount sufficient to treat cancer.

In another embodiment, the invention provides a method for treating cancer, said method comprising administering to a patient in need thereof a pharmaceutical composition comprising an amount of an Indazole Compound effective to treat cancer.

In a specific embodiment, the patient in need of treatment has previously undergone treatment for cancer. Such previous treatments include, but are not limited to, prior chemotherapy, radiotherapy, surgery, or immunotherapy, such as cancer vaccines.

Cancers that can be treated with the Indazole Compounds and methods of the Invention include, but are not limited to, cancers disclosed below in Table 1 and metastases thereof.

**TABLE 1**

| | |
|---|---|
| Solid tumors, including but not limited to: | |
| | fibrosarcoma |
| | myxosarcoma |
| | liposarcoma |
| | chondrosarcoma |
| | osteogenic sarcoma |
| | chordoma |
| | angiosarcoma |
| | endotheliosarcoma |
| | lymphangiosarcoma |
| | lymphangioendotheliosarcoma |
| | synovioma |
| | mesothelioma |
| | Ewing's tumor |
| | leiomyosarcoma |
| | rhabdomyosarcoma |
| | colon cancer |
| | colorectal cancer |
| | kidney cancer |
| | pancreatic cancer |
| | bone cancer |
| | breast cancer |
| | ovarian cancer |
| | prostate cancer |
| | esophageal cancer |
| | stomach cancer |
| | oral cancer |
| | nasal cancer |
| | throat cancer |
| | squamous cell carcinoma |
| | basal cell carcinoma |
| | adenocarcinoma |
| | sweat gland carcinoma |
| | sebaceous gland carcinoma |
| | papillary carcinoma |
| | papillary adenocarcinomas |
| | cystadenocarcinoma |
| | medullary carcinoma |
| | bronchogenic carcinoma |
| | renal cell carcinoma |
| | hepatoma |
| | bile duct carcinoma |
| | choriocarcinoma |
| | seminoma |
| | embryonal carcinoma |
| | Wilms' tumor |
| | cervical cancer |
| | uterine cancer |
| | testicular cancer |
| | small cell lung carcinoma |
| | bladder carcinoma |
| | lung cancer |
| | epithelial carcinoma |
| | glioma |
| | glioblastoma multiforme |
| | astrocytoma |
| | medulloblastoma |
| | craniopharyngioma |
| | ependymoma |
| | pinealoma |
| | hemangioblastoma |
| | acoustic neuroma |
| | oligodendroglioma |
| | meningioma |
| | skin cancer |
| | melanoma |
| | neuroblastoma |
| | retinoblastoma |
| blood-borne cancers, including but not limited to: | |
| | acute lymphoblastic leukemia ("ALL") |
| | acute lymphoblastic B-cell leukemia |
| | acute lymphoblastic T-cell leukemia |
| | acute myeloblastic leukemia ("AML") |
| | acute promyelocytic leukemia ("APL") |
| | acute monoblastic leukemia |
| | acute erythroleukemic leukemia |
| | acute megakaryoblastic leukemia |
| | acute myelomonocytic leukemia |
| | acute nonlymphocyctic leukemia |
| | acute undifferentiated leukemia |
| | chronic myelocytic leukemia ("CML") |
| | chronic lymphocytic leukemia ("CLL") |
| | hairy cell leukemia |
| | multiple myeloma |
| acute and chronic leukemias: | |
| | lymphoblastic |
| | myelogenous |
| | lymphocytic |
| | myelocytic leukemias |
| Lymphomas: | |
| | Hodgkin's disease |
| | non-Hodgkin's Lymphoma |
| | Multiple myeloma |
| | Waldenström's macroglobulinemia |
| | Heavy chain disease |
| | Polycythemia vera |

In one embodiment, the cancer is lung cancer, breast cancer, colorectal cancer, prostate cancer, brain cancer, esophageal cancer, pancreatic cancer, stomach cancer, liver cancer, kidney cancer, adrenal cancer, testicular cancer, ovarian cancer, cervical cancer, leukemia, Hodgkin's disease, non-Hodgkin's lympoma, skin cancer, bone cancer, a cancer of the central nervous system, or a cancer of the blood or lymphatic system.

### 4.4.2.1 MULTI-MODALITY THERAPY FOR CANCER

The Indazole Compounds can be administered to a patient that has undergone or is currently undergoing one or more additional anticancer treatment modalities including, but not limited to, chemotherapy, radiotherapy, surgery or immunotherapy, such as cancer vaccines.

In one embodiment, the invention provides methods for treating cancer comprising (a) administering to a patient in need thereof a therapeutic amount of an Indazole Compound of the invention; and (b) administering to said patient one or more additional anticancer treatment modalities including, but not limited to, radiotherapy, chemotherapy, surgery or immunotherapy, such as a cancer vaccine. In one embodiment, the administering of step (a) is done prior to the administering of step (b). In another embodiement, the administering of step (a) is done subsequent to the administering of step (b). In still another embodiment, the administering of step (a) is done concurrently with the administering of step (b).

In one embodiment, the additional anticancer treatment modality is chemotherapy.

In another embodiment, the additional anticancer treatment modality is surgery.

In yet another embodiment, the additional anticancer treatment modality is radiation therapy.

In still another embodiment, the additional anticancer treatment modality is immunotherapy, such as cancer vaccines.

The Indazole Compound and the additional treament modalities of the combination therapies of the invention can act additively or synergistically (i.e., the combination of an Indazole Compound or a pharmaceutically acceptable salt thereof, and an additional anticancer treatment modality is more effective than their additive effects when each are administered alone). A synergistic combination permits the use of lower dosages of the Indazole Compound and/or the additional treatment modality and/or less frequent administration of the Indazole Compound and/or additional treatment modality to a patient with cancer. The ability to utilize lower dosages of an Indazole Compound and/or an additional treatment modality and/or to administer an Indazole Compound and said additional treament modality less frequently can reduce the toxicity associated with the administration of an Indazole Compound and/or the additional treatement modality to a patient without reducing the efficacy of an Indazole Compound and/or the additional treatement modality in the treatment of cancer. In addition, a synergistic effect can result in the improved efficacy of the treatment of cancer and/or the reduction of adverse or unwanted side effects associated with the administration of an Indazole Compound and/or an additional anticancer treatment modality as monotherapy.

When the Indazole Compound and additional anticancer treatment modality are administered to a patient concurrently, the term "concurrently" is not limited to the administration of an Indazole Compound and an additional anticancer treatment modality at exactly the same time, but rather it is meant that they are administered to a patient in a sequence and within a time interval such that they can act synergistically to provide an increased benefit than if they were administered otherwise. For example, the Indazole Compounds may be administered at the same time or sequentially in any order at different points in time as an additional anticancer treament modality; however, if not administered at the same time, they should be administered sufficiently close in time so as to provide the desired therapeutic effect, preferably in a synergistic fashion. The Indazole Compound and the additional anticancer treatment modality can be administered separately, in any appropriate form and by any suitable route. When the Indazole Compound and the additional anticancer treatment modality are not administered concurrently, it is understood that they can be administered in any order to a patient in need thereof. For example, an Indazole Compound can be administered prior to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to *(e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of an additional anticancer treatment modality (e.g., radiotherapy), to a patient in need thereof. In various embodiments the Indazole Compound and the additional anticancer treatment modality are administered 1 minute apart, 10 minutes apart, 30 minutes apart, less than 1 hour apart, 1 hour apart, 1 hour to 2 . hours apart, 2 hours to 3 hours apart, 3 hours to 4 hours apart, 4 hours to 5 hours apart, 5 hours to 6 hours apart, 6 hours to 7 hours apart, 7 hours to 8 hours apart, 8 hours to 9 hours apart, 9 hours to 10 hours apart, 10 hours to 11 hours apart, 11 hours to 12 hours apart, no more than 24 hours apart or no more than 48 hours apart. In one embodiment, the components of the combination therapies of the invention are administered within the same office or hospital visit. In another embodiment, the Indazole Compound and the additional anticancer treatment modality are administered at 1 minute to 24 hours apart.

In one embodiment, an Indazole Compound is administered prior or subsequent to an additional anticancer treatment modality, preferably at least an hour, five hours, 12 hours, a day, a week, a month, more preferably several months (e.g., up to three months), prior or subsequent to administration of an additional anticancer treatment modality.

When the combination theapy of the invention comprises administering an Indazole Compound are with one or more additional anticancer agents, the Indazole Compound and the additional anticancer agents can be administered concurrently or sequentially to a patient. The agents can also be cyclically administered. Cycling therapy involves the administration of one or more anticancer agents for a period of time, followed by the administration of one or more different anticancer agents for a period of time and repeating this sequential administration, i.e., the cycle, in order to reduce the development of resistance to one or more of the anticancer agents of being administered, to avoid or reduce the side effects of one or more of the anticancer agents being administered, and/or to improve the efficacy of the treatment.

An additional anticancer agent may be administered over a series of sessions; any one or a combination of the additional anticancer agents listed below may be administered.

The present invention includes methods for treating cancer, comprising administering to a patient in need thereof an Indazole Compound, and one or more additional anticancer agents or pharmaceutically acceptable salts thereof. The Indazole Compound and the additional anticancer agent(s) can act additively or synergistically. Suitable anticancer agents include, but are not limited to, gemcitabine, capecitabine, methotrexate, taxol, taxotere, mercaptopurine, thioguanine, hydroxyurea, cytarabine, cyclophosphamide, ifosfamide, nitrosoureas, cisplatin, carboplatin, mitomycin, dacarbazine, procarbizine, etoposide, teniposide, campathecins, bleomycin, doxorubicin, idarubicin, daunorubicin, dactinomycin, plicamycin, mitoxantrone, L-asparaginase, doxorubicin, epirubicin, 5-fluorouracil (5-FU), taxanes such as docetaxel and paclitaxel, leucovorin, levamisole, irinotecan, estramustine, etoposide, nitrogen mustards, BCNU, nitrosoureas such as carmustine and lomustine, vinca alkaloids such as vinblastine, vincristine and vinorelbine, platinum complexes such as cisplatin, carboplatin and oxaliplatin, imatinib mesylate, hexamethylmelamine, topotecan, tyrosine kinase inhibitors, tyrphostins herbimycin A, genistein, erbstatin, and lavendustin A.

In one embodiment, the anti-cancer agent can be, but is not limited to, a drug listed in Table 2.

**TABLE 2**

| Alkylating agents | |
|---|---|
| Nitrogen mustards: | Cyclophosphamide |
| | Ifosfamide |
| | Trofosfamide |
| | Chlorambucil |
| Nitrosoureas: | Carmustine (BCNU) |
| | Lomustine (CCNU) |
| Alkylsulphonates: | Busulfan |
| | Treosulfan |
| Triazenes: | Dacarbazine |
| Platinum complexes: | Cisplatin |
| | Carboplatin |
| | Oxaliplatin |
| Plant Alkaloids | |
| Vinca alkaloids: | Vincristine |
| | Vinblastine |
| | Vindesine |
| | Vinorelbine |
| Taxoids: | Paclitaxel |
| | Docetaxel |
| DNA Topoisomerase Inhibitors | |
| Epipodophyllins: | Etoposide |
| | Teniposide |
| | Topotecan |
| | 9-aminocamptothecin |
| | Camptothecin |
| | Crisnatol |
| Mitomycins: | Mitomycin C |
| | Anti-metabolites |
| Anti-folates: | |
| DHFR inhibitors: | Methotrexate |
| | Trimetrexate |
| | Pemetrexed (Alitma) |
| IMP dehydrogenase Inhibitors: | Mycophenolic acid |
| | Tiazofurin |
| | Ribavirin |
| | EICAR |
| Ribonuclotide reductase Inhibitors: | Hydroxyurea |
| | Deferoxamine |
| Pyrimidine analogs: | |
| Uracil analogs: | 5-Fluorouracil |
| | Floxuridine |
| | Doxifluridine |
| | Ratitrexed |
| Cytosine analogs: | Cytarabine (ara C) |
| | Cytosine arabinoside |
| | Fludarabine |
| | Gemcitabine |
| | Capecitabine |
| Purine analogs: | Mercaptopurine |
| | Thioguanine |
| DNA Antimetabolites: | 3-HP |
| | 2'-deoxy-5-fluorouridine |
| | 5-HP |
| | alpha-TGDR |
| | aphidicolin glycinate |
| | ara-C |
| | 5-aza-2'-deoxycytidine |
| | beta-TGDR |
| | cyclocytidine |
| | guanazole |
| | inosine glycodialdehyde |
| | macebecin II |
| | Pyrazoloimidazole |
| Hormonal therapies: | |
| Receptor antagonists: | |
| Anti-estrogen: | Tamoxifen |
| | Raloxifene |
| | Megestrol |
| LHRH agonists: | Goserelin |
| | Leuprolide acetate |
| Anti-androgens: | Flutamide |
| | Bicalutamide |
| Retinoids/Deltoids | |
| | Cis-retinoic acid |
| Vitamin A derivative: | All-trans retinoic acid (ATRA-IV) |
| Vitamin D3 analogs: | EB 1089 |
| | CB 1093 |
| | KH 1060 |
| Photodynamic therapies : | Vertoporfin (BPD-MA) |
| | Phthalocyanine |
| | Photosensitizer Pc4 |
| | Demethoxy-hypocrellin A |
| | (2BA-2-DMHA) |
| Cytokines: | Interferon-α |
| | Interferon-β |
| | Interferon-γ |
| | Tumor necrosis factor |
| Angiogenesis Inhibitors: | Angiostatin (plasminogen fragment) |
| | antiangiogenic antithrombin III |
| | Angiozyme |
| | ABT-627 |
| | Bay 12-9566 |
| | Benefin |
| | Bevacizumab |
| | BMS-275291 |
| | cartilage-derived inhibitor (CDI) |
| | CAI |
| | CD59 complement fragment |
| | CEP-7055 |
| | Col 3 |
| | Combretastatin A-4 |
| | Endostatin (collagen XVIII fragment) |
| | Fibronectin fragment |
| | Gro-beta |
| | Halofuginone |
| | Heparinases |
| | Heparin hexasaccharide fragment |
| | HMV833 |
| | Human chorionic gonadotropin (hCG) IM-862 |
| | Interferon alpha/beta/gamma |
| | Interferon inducible protein (IP-10) |
| | Interleukin-12 |
| | Kringle 5 (plasminogen fragment) Marimastat |
| | Metalloproteinase inhibitors (TIMPs) |
| | 2-Methoxyestradiol |
| | MMI 270 (CGS 27023A) |
| | MoAb IMC-1C11 |
| | Neovastat |
| | NM-3 |
| | Panzem |
| | PI-88 |
| | Placental ribonuclease inhibitor |
| | Plasminogen activator inhibitor |
| | Platelet factor-4 (PF4) |
| | Prinomastat |
| | Prolactin 16kD fragment |
| | Proliferin-related protein (PRP) |
| | PTK 787/ZK 222594 |
| | Retinoids |
| | Solimastat |
| | Squalamine |
| | SS 3304 |
| | SU 5416 |
| | SU6668 |
| | SU11248 |
| | Tetrahydrocortisol-S |
| | Tetrathiomolybdate |
| | Thalidomide |
| | Thrombospondin-1 (TSP-1) |
| | TNP-470 |
| | Transforming growth factor-beta (TGF-β) |
| | Vasculostatin |
| | Vasostatin (calreticulin fragment) |
| | ZD6126 |
| | ZD 6474 |
| | farnesyl transferase inhibitors (FTI) |
| | Bisphosphonates |
| Antimitotic agents : | Allocolchicine |
| | Halichondrin B |
| | Colchicine |
| | colchicine derivative |
| | dolstatin 10 |
| | Maytansine |
| | Rhizoxin |
| | Thiocolchicine |
| | trityl cysteine |
| Others: | |
| Isoprenylation inhibitors: | |
| Dopaminergic neurotoxins: | 1-methyl-4-phenylpyridinium ion |
| Cell cycle inhibitors: | Staurosporine |
| Actinomycins: | Actinomycin D |
| | Dactinomycin |
| Bleomycins: | Bleomycin A2 |
| | Bleomycin B2 |
| | Peplomycin |
| Anthracyclines: | Daunorubicin |
| | Doxorubicin (adriamycin) |
| | Idarubicin |
| | Epirubicin |
| | Pirarubicin |
| | Zorubicin |
| | Mitoxantrone |
| MDR inhibitors: | Verapamil |
| Ca²⁺ATPase inhibitors: | Thapsigargin |

In a preferred embodiment, the additional anticancer agent is premetrexed.

It is a further aspect of the invention the Indazole Compounds can be administered in conjunction with chemical agents that are understood to mimic the effects of radiotherapy and/or that function by direct contact with DNA. Preferred agents for use in combination with the Indazole Compounds for treating cancer include, but are not limited to cis-diamminedichloro platinum (II) (cisplatin), doxorubicin, 5-fluorouracil, taxol, and topoisomerase inhibitors such as etoposide, teniposide, irinotecan and topotecan.

Additionally, the invention provides methods of treatment of cancer using the Indazole Compounds as an alternative to chemotherapy alone or radiotherapy alone where the chemotherapy or the radiotherapy has proven or can prove too toxic, e.g., results in unacceptable or unbearable side effects, for the patient being treated. The patient being treated can, optionally, be treated with another anticancer treatment modality such as chemotherapy, surgery, or immunotherapy, depending on which treatment is found to be acceptable or bearable.

The Indazole Compounds can also be used in an in vitro or ex vivo fashion, such as for the treatment of certain cancers, including, but not limited to leukemias and lymphomas, such treatment involving autologous stem cell transplants. This can involve a multi-step process in which the patient's autologous hematopoietic stem cells are harvested and purged of all cancer cells, the patient is then administered an amount of an Indazole Compound effective to eradicate the patient's remaining bone-marrow cell population, then the stem cell graft is infused back into the patient. Supportive care is then provided while bone marrow function is restored and the patient recovers.

### 4.4.3 TREATMENT OR PREVENTION OF NEUROLOGICAL DISEASES

A neurological disease can be treated or prevented by administration of an effective amount of an Indazole Compound.

Neurological diseases that can be treated or prevented by administering an effective amount of an Indazole Compound include, but are not limited to, stroke, ischemia, trauma-induced cerebral edema, hypoxia-induced cerebral edema, ocular edema, macular edema, brain-tumor associated cerebral edema, Huntington's disease, epilepsy, lupus, schizophrenia, multiple sclerosis, muscular dystrophy, a drug-induced movement disorders, Creutzfeldt-Jakob disease, amyotrophic lateral sclerosis, Pick's disease, Alzheimer's disease, Lewy body dementia, cortico basal degeneration, dystonia, myoclonus, Tourette's Syndrome, tremor, chorea, restless leg syndrome, Parkinson's disease, and a Parkinsonian Syndrome, such as progressive supranuclear palsy, multiple system atrophy, Wilson's disease, mult-infarct state, spinal muscular atrophy, cerebellar degeneration peripheral neuropathies, spinal cord damage, or AIDS dementia.

In one embodiment, the neurological disease is stroke, ischemia, trauma-induced cerebral edema, hypoxia-induced cerebral edema, ocular edema, macular edema, or brain-tumor associated cerebral edema.

### 4.4.4 TREATMENT OR PREVENTION OF INFLAMMATORY DISEASES

Inflammatory diseases can be treated or prevented by administration of an effective amount of an Indazole Compound.

Inflammatory diseases that can be treated or prevented by administering an effective amount of an Indazole Compound include, but are not limited to, organ transplant rejection; reoxygenation injury resulting from organ transplantation (see Grupp et al. J. Mol. Cell Cardiol. 31:297-303 (1999)) including, but not limited to, transplantation of the following organs: heart, lung, liver and kidney; systemic inflammatory response syndrome; chronic inflammatory diseases of the joints, including arthritis, rheumatoid arthritis, osteoarthritis and bone diseases associated with increased bone resorption; inflammatory bowel diseases such as ileitis, ulcerative colitis, Barrett's syndrome, and Crohn's disease; inflammatory lung diseases such as asthma, adult respiratory distress syndrome, and chronic obstructive airway disease; inflammatory diseases of the eye including corneal dystrophy, trachoma, onchocerciasis, uveitis, sympathetic ophthalmitis and endophthalmitis; chronic inflammatory diseases of the gum, including gingivitis and periodontitis; inflammatory diseases of the joints including arthritis and osteoarthritis; inflammatory diseases of the kidney including uremic complications, glomerulonephritis and nephrosis; inflammatory diseases of the skin including sclerodermatitis, psoriasis and eczema; inflammatory diseases of the central nervous system, including chronic demyelinating diseases of the nervous system, multiple sclerosis, AIDS-related neurodegeneration and Alzheimer's disease, infectious meningitis, encephalomyelitis, Parkinson's disease, Huntington's disease, amyotrophic lateral sclerosis and viral or autoinimune encephalitis; diabetes, including Type I and Type II diabetes mellitus, diabetes insipidus, maturity-onset diabetes, juvenile diabetes, insulin-dependant diabetes, non-insulin dependant diabetes, malnutrition-related diabetes, ketosis-prone diabetes or ketosis-resistant diabetes; diabetic complications such as diabetic retinopathy, neovascular glaucoma, diabetic cataract, glaucoma, nephropathy (such as microaluminuria and progressive diabetic nephropathy), polyneuropathy, gangrene of the feet, atherosclerotic coronary arterial disease, peripheral arterial disease, retinitis pigmentosa, nonketotic hyperglycemic-hyperosmolar coma, mononeuropathies, autonomic neuropathy, foot ulcers, joint problems, and a skin or mucous membrane complication, such as an infection, a shin spot, a candidal infection or necrobiosis lipoidica diabeticorum; immune-complex vasculitis, systemic lupus erythematosus (SLE); inflammatory diseases of the heart such as cardiomyopathy, ischemic heart disease hypercholesterolemia, and atherosclerosis.

The Indazole Compounds are also useful for the treatment of prevention of various diseases that can have significant inflammatory components, including preeclampsia; chronic liver failure, and brain and spinal cord trauma.

The inflammatory disease can also be a systemic inflammation of the body, exemplified by gram-positive or gram negative shock, hemorrhagic or anaphylactic shock, or shock induced by cancer chemotherapy in response to pro-inflammatory cytokines, e.g., shock associated with pro-inflammatory cytokines. Such shock can be induced, e.g., by a chemotherapeutic agent that is adminstered as a treatment for cancer.

### 4.4.5 TREATMENT OR PREVENTION OF VIRAL DISEASES

Viral diseases can be treated or prevented by administration of an effective amount of an Indazole Compound.

Viral diseases that can be treated or prevented by administering an effective amount of an Indazole Compound include, but are not limited to, HIV, human papilloma virus, herpes virus, Epstein-Barr virus, adenovirus, Sindbis virus, and pox virus.

### 4.4.6 TREATMENT OR PREVENTION OF OTHER CONDITIONS

Other conditions that may be treated by the administration of a therapeutically effective amount of an Indazole Compound, or a pharmaceutical composition comprising an Indazole Compound, include any condition which is responsive to modulation, regulation or inhibition of one or more protein kinases, including modulation, reguation or inhibition of protein kinase signal transduction, and thereby benefit from administration of such a modulator.

Illustrative examples of additional condtions that may be treated via administering the Indazole Compounds include, but are not limited to; obesity (such as hereditary obesity, dietary obesity, hormone related obesity or obesity related to the administration of medication); hearing loss (such as that from otitis externa, acute otitis media or a chronic progressive disease resulting in sensory hair-cell death in the organ of Corti of the inner ear); fibrosis related diseases (such as pulmonary interstitial fibrosis, renal fibrosis, cystic fibrosis, liver fibrosis, wound-healing or burn-healing, wherein the burn is a first-, second- or third-degree burn and/or a thermal, chemical or electrical bum); arthritis (such as rheumatoid arthritis, rheumatoid spondylitis, osteoarthritis or gout); an allergy; allergic rhinitis; acute respiratory distress syndrome; asthma; bronchitis; or an autoimmune disease (such as scleroderma, systemic lupus erythematosus, myasthenia gravis, transplant rejection, endotoxin shock, sepsis, psoriasis, eczema, dermatitis or multiple sclerosis).

Indazole Compounds are also useful for treating or preventing a liver disease (such as hepatitis, alcohol-induced liver disease, toxin-induced liver disease, steatosis or sclerosis); a cardiovascular disease (such as atherosclerosis, restenosis following angioplasty, left ventricular hypertrophy, myocardial infarction, chronic obstructive pulmonary disease or stroke); ischemic damage (such as to the heart, kidney, liver or brain); ischemia-reperfusion injury (such as that caused by transplant, surgical trauma, hypotension, thrombosis or trauma injury);); viral infections (including but not limited to herpesvirus, poxvirns, Epstein-Barr virus, Sindbis virus and adenovirus), prevention of AIDS development in HIV infected individuals, autoimmune diseases (including but not limited to systemic lupus erythematosus, rheumatoid arthritis, psoriasis, autoimmune mediated glomerulonephritis, inflammatory bowel disease and autoimmune diabetes mellitus); myelodysplastic syndromes, aplastic anemia, ischemic injury associated with myocardial infarctions, stroke and reperfusion injury, arrhythmia, atherosclerosis, toxin-induced or alcohol related liver diseases, hematological diseases (including but not limited to chronic anemia and aplastic anemia), degenerative diseases of the musculoskeletal system (including but not limited to osteroporosis and arthritis), aspirin-sensitive rhinosinusitis, cystic fibrosis, multiple sclerosis, kidney diseases and cancer pain.

### 4.4.7 MODULATION OF PROTEIN KINASES

In one embodiment, the Indazole Compounds of the invention are protein kinase modulators, regulators or inhibitors that target multiple protein kinases. In an alternative embodiment, the Indazole Compounds of the invention are protein kinase modulators, regulators or inhibitors that selectively target a specific protein kinase.

In one embodiment, the invention provides a method for modulating protein kinase signal transduction in or between cells, comprising administering to a patient in need thereof a therapeutically effective amount of an Indazole Compound. In a preferred embodiment, the protein kinase whose signal transduction is being modulated is ABL, AKT1, AKT2, AMPK, Aurora-A, Aurora-B, Blk, CaMKII, CaMKIV, CDK1/B, CDK2/A, CDK2/E, CDK3/E, CDK5/p35, CDK6/D3, CDK7/H/MAT1, CHK1, CHK2, CK2, CSK, ERK, EGFR, Fes, FGFR3, Fyn, GSK3β, IGF-1R, IKKα, IKKβ, IKK1, IKK2EE, IR, IRTK, JNK1α1, JNK2α2, JNK3, Lck, Lyn, MAPK1, MAPK2, MAPKAP-K2, MEK1, MKK3, MKK4, MKK6, MKK7, MKK7β, MSK1, p38α, p38β, p70S6K, PAK2, PDGGRα, PDK1, PKA, PKBα, PKBβ, PKCα, PKC∈, PKCγ, PKCθ, PKCβII, PKA, PRAK, PRK2, c-RAF, ROCK-II, Rsk1, Rsk2, Rsk3, SAPK2a, SAPK2b, SAPK3, SAPK4, SGK, c-SRC, Syk, Yes, or ZAP-70.

In another embodiment, the invention provides methods for modulating the activity of one or more protein kinases, comprising administering to a patient in need thereof a therapeutically effective amount of an Indazole Compound. In a preferred embodiment, the protein kinase whose activity is being modulated is ABL, AKT1, AKT2, AMPK, Aurora-A, Aurora-B, B1k, CaMKII, CaMKIV, CDK1/B, CDK2/A, CDK2/E, CDK3/E, CDK5/p35, CDK6/D3, CDK7/H/MAT1, CHK1, CHK2, CK2, CSK, ERK, EGFR, Fes, FGFR3, Fyn, GSK3β, IGF-1R, IKKα, IKKβ, IKK1, IKK2EE, IR, IRTK, JNKIα1, JNK2α2, JNK3, Lck, Lyn, MAPK1, MAPK2, MAPKAP-K2, MEK1, MKK3, MKK4, MKK6, MKK7, MKK7β, MSK1, p38α, p38β, p70S6K, PAK2, PDGGRα, PDK1, PKA, PKBα, PKBβ, PKCα, PKC∈, PKCγ, PKCθ, PKCβII, PKA, PRAK, PRK2, c-RAF, ROCK-II, Rsk1, Rsk2, Rsk3, SAPK2a, SAPK2b, SAPK3, SAPK4, SGK, c-SRC, Syk, Yes, or ZAP-70.

In various embodiments, the Indazole Compounds of the invention are useful in the treatment of conditions, diseases, and disorders associated with protein kinases such as tyrosine kinases, serine/threonine kinases, lysine kinases, or histidine kinases, preferably tyrosine kinases or serine/threonine kinases. The invention contemplates methods for modulating, inhibiting or regulating such kinases, including methods for modulating, inhibiting or regulating kinase signal transduction pathways.

In a preferred embodiment, the Indazole Compounds of the invention are useful in the treatment of conditions, diseases, and disorders associated with tyrosine kinases. In another preferred embodiment, the Indazole Compounds of the invention are useful in the treatment of conditions, diseases, and disorders associated with serine/threonine kinases. The invention contemplates methods for modulating, inhibiting or regulating tyrosine kinases, including methods for modulating, inhibiting or regulating tyrosine kinase signal transduction pathways. The invention also contemplates methods for modulating, inhibiting or regulating serine/threonine kinases, including methods for modulating, inhibiting or regulating serine/threonine kinase signal transduction pathways. The kinases can be receptor of the receptor type or can be the non-receptor type.

The invention contemplates the use of the Indazole Compounds in treating diseases, disorders, or conditions associated with a MAP kinase, including diseases, disorders, or conditions associated with an ERK kinase or ERK pathway, a JNK kinase or JNK kinase, or a p38 kinase or a p38 pathway. In various embodiments, the Indazole Compounds are useful for modulating, inhibiting, or regulating a MAP kinase pathway. The one embodiment, Indazole Compounds are useful for modulating, inhibiting, or regulating the ERK pathway. In another embodiment, Indazole Compounds are useful for modulating, inhibiting, or regulating the p38 pathway. In yet another embodiment, the Indazole Compounds are useful for modulating, inhibiting, or regulating the JNK pathway. By way of example, in one embodiment, the present methods for treating or preventing an inflammatory condition, a liver disease, a cardiovascular disease, ischemic damage, a neurodegenerative disease or cancer comprise inhibiting JNK *in vivo.* In another embodiment, inhibiting JNK in *vivo* comprises inhibiting TNF-α in *vivo.* In a specific embodiment the JNK is JNK1. In another specific embodiment the JNK is JNK2. In yet another specific embodiment the JNK is JNK3.

The invention also contemplates using the Indazole Compounds for treating diseases, disorders, or conditions associated with cyclin dependent kinases or cell cycle checkpoint kinases. In certain embodiments, the Indazole Compounds are useful for modulating, inhibiting, or regulating a cyclin dependent kinase or cyclin dependent kinase pathway. In other embodiments, the Indazole Compounds are useful for modulating, inhibiting, or regulating a cell cycle kinase or a cell cycle kinase pathway. Such kinases include but are not limited to CDK1, CDK2, CDK4, CDK5, CDK6, and CHK1.

The invention also contemplates using the Indazole Compounds for treating diseases, disorders, or conditions associated with Src family of kinases. In certain embodiments, the Indazole Compounds are useful for modulating, inhibiting, or regulating one or members of Src family of kinase pathway. In other embodiments, the Indazole Compounds are useful for modulating, inhibiting, or regulating one or more members of Src family of kinases simultaneously. Such kinases include but are not limited to cSrc, Fyn, Lyn, and cYes.

The invention further contemplates using the Indazole Compounds for treating diseases, disorders, or conditions associated with the RSK family of kinases. In certain embodiments, the Indazole Compounds are useful for modulating, inhibiting, or regulating one or members of the RSK family of kinase pathway. In other embodiments, the Indazole Compounds are useful for modulating, inhibiting, or regulating one or more members of the RSK family of kinases simultaneously. Such kinases include but are not limited to RSK1, RSK2 and RSK3.

The invention further contemplates using the Indazole Compounds for treating diseases, disorders, or conditions associated with the MAPK family of kinases. In certain embodiments, the Indazole Compounds are useful for modulating, inhibiting, or regulating one or members of the MAPK family of kinase pathway. In other embodiments, the Indazole Compounds are useful for modulating, inhibiting, or regulating one or more members of the MAPK family of kinases simultaneously. Such kinases include but are not limited to the ERK, JNK and P38 classes.

The invention further contemplates using the Indazole Compounds for treating diseases, disorders, or conditions associated with the CDK family of kinases. In certain embodiments, the Indazole Compounds are useful for modulating, inhibiting, or regulating one or members of the CDK family of kinase pathway. In other embodiments, the Indazole Compounds are useful for modulating, inhibiting, or regulating one or more members of the CDK family of kinases simultaneously. Such kinases include but are not limited to CDK1, CDK2, CDK4, CDK5, and CDK6.

The invention further contemplates using the Indazole Compounds for treating diseases, disorders, or conditions associated with the Checkpoint kinases. In certain embodiments, the Indazole Compounds are useful for modulating, inhibiting, or regulating one or members of the Checkpoint kinase pathway. In other embodiments, the Indazole Compounds are useful for modulating, inhibiting, or regulating one or more members of the Checkpoint kinases simultaneously. Such kinases include but are not limited to CHK1.

The invention further contemplates using the Indazole Compounds for treating diseases, disorders, or conditions associated with the Aurora family of kinases. In certain embodiments, the Indazole Compounds are useful for modulating, inhibiting, or regulating one or members of the Aurora family of kinase pathway. In other embodiments, the Indazole Compounds are useful for modulating, inhibiting, or regulating one or more members of the Aurora family of kinases simultaneously. Such kinases include but are not limited to Aurora-A, Aurora-B and Aurora-B members.

The invention also contemplates using the Indazole Compounds for treating diseases, disorders, or conditions associated with the ROCK family of kinases. In certain embodiments, the Indazole Compounds are useful for modulating, inhibiting, or regulating one or members of the ROCK family of kinase pathway. In other embodiments, the Indazole Compounds are useful for modulating, inhibiting, or regulating one or more members of the ROCK family of kinases simultaneously. Such kinases include but are not limited to ROCK-I and ROCK-II.

The invention also contemplates using the Indazole Compounds for treating diseases, disorders, or conditions associated with the kinase B1k. In certain embodiments, the Indazole Compounds are useful for modulating, inhibiting, or regulating the Blk kinase pathway. In other embodiments, the Indazole Compounds are useful for modulating, inhibiting, or regulating Blk.

The invention further contemplates using the Indazole Compounds for treating diseases, disorders, or conditions associated with the kinases GSK3α and GSK3β. In certain embodiments, the Indazole Compounds are useful for modulating, inhibiting, or regulating one or both of the GSK3α and GSK3β pathways. In other embodiments, the Indazole Compounds are useful for modulating, inhibiting, or regulating one or both of the GSK3α and GSK3β kinases.

The invention also contemplates using the Indazole Compounds for treating diseases, disorders, or conditions associated with the kinase P70S6K. In certain embodiments, the Indazole Compounds are useful for modulating, inhibiting, or regulating the P70S6K kinase pathway. In other embodiments, the Indazole Compounds are useful for modulating, inhibiting, or regulating P70S6K.

The invention also contemplates using the Indazole Compounds for treating diseases, disorders, or conditions associated with the kinase PCKµ. In certain embodiments, the Indazole Compounds are useful for modulating, inhibiting, or regulating the PCKµ kinase pathway. In other embodiments, the Indazole Compounds are useful for modulating, inhibiting, or regulating PCKµ.

The invention also contemplates using the Indazole Compounds for treating diseases, disorders, or conditions associated with the kinase PKD2. In certain embodiments, the Indazole Compounds are useful for modulating, inhibiting, or regulating the PKD2 kinase pathway. In other embodiments, the Indazole Compounds are useful for modulating, inhibiting, or regulating PKD2.

The invention also contemplates using the Indazole Compounds for treating diseases, disorders, or conditions associated with the kinase PRAK. In certain embodiments, the Indazole Compounds are useful for modulating, inhibiting, or regulating the PRAK kinase pathway. In other embodiments, the Indazole Compounds are useful for modulating, inhibiting, or regulating PRAK.

The invention also contemplates using the Indazole Compounds for treating diseases, disorders, or conditions associated with the kinase PRK2. In certain embodiments, the Indazole Compounds are useful for modulating, inhibiting, or regulating the PRK2 kinase pathway. In other embodiments, the Indazole Compounds are useful for modulating, inhibiting, or regulating PRK2.

The invention also contemplates using the Indazole Compounds for treating diseases, disorders, or conditions associated with growth factor kinases or growth factor kinase pathways. In certain embodiments, the Indazole Compounds are useful for modulating, inhibiting, or regulating a growth factor kinase or growth factor kinase pathway. Such kinases include but are not limited to VEGF-R2, FGF-R, and TEK.

The compounds described herein could also be useful as an adjunct to existing and/or experimental therapies.In one embodiment, the Indazole Compounds of the invention are protein kinase modulators, regulators or inhibitors that target multiple protein kinases. In an alternative embodiment, the Indazole Compounds of the invention are protein kinase modulators, regulators or inhibitors that selectively target a specific protein kinase.

In various embodiments, the Indazole Compounds of the invention are useful in the treatment of conditions, diseases, and disorders associated with protein kinases such as tyrosine kinases, serine/threonine kinases, lysine kinases, or histidine kinases, preferably tyrosine kinases or serine/threonine kinases. The invention contemplates methods for modulating, inhibiting or regulating such kinases, including methods for modulating, inhibiting or regulating kinase signal transduction pathways.

In a preferred embodiment, the Indazole Compounds of the invention are useful in the treatment of conditions, diseases, and disorders associated with tyrosine kinases. In another preferred embodiment, the Indazole Compounds of the invention are useful in the treatment of conditions, diseases, and disorders associated with serine/threonine kinases. The invention contemplates methods for modulating, inhibiting or regulating tyrosine kinases, including methods for modulating, inhibiting or regulating tyrosine kinase signal transduction pathways. The invention also contemplates methods for modulating, inhibiting or regulating serine/threonine kinases, including methods for modulating, inhibiting or regulating serine/threonine kinase signal transduction pathways. The kinases can be receptor of the receptor type or can be the non-receptor type.

The invention contemplates the use of the Indazole Compounds in treating diseases, disorders, or conditions associated with a MAP kinase, including diseases, disorders, or conditions associated with an ERK kinase or ERK pathway, a JNK kinase or JNK kinase, or a p38 kinase or a p38 pathway. In various embodiments, the Indazole Compounds are useful for modulating, inhibiting, or regulating a MAP kinase pathway. The one embodiment, Indazole Compounds are useful for modulating, inhibiting, or regulating the ERK pathway. In another embodiment, Indazole Compounds are useful for modulating, inhibiting, or regulating the p38 pathway. In yet another embodiment, the Indazole Compounds are useful for modulating, inhibiting, or regulating the JNK pathway. By way of example, in one embodiment, the present methods for treating or preventing an inflammatory condition, a liver disease, a cardiovascular disease, ischemic damage, a neurodegenerative disease or cancer comprise inhibiting JNK in *vivo.* In another embodiment, inhibiting JNK *in vivo* comprises inhibiting TNF-α *in vivo.* In a specific embodiment the JNK is JNK1. In another specific embodiment the JNK is JNK2. In yet another specific embodiment the JNK is JNK3.

The invention also contemplates using the Indazole Compounds for treating diseases, disorders, or conditions associated with cyclin dependent kinases or cell cycle checkpoint kinases. In certain embodiments, the Indazole Compounds are useful for modulating, inhibiting, or regulating a cyclin dependent kinase or cyclin dependent kinase pathway. In other embodiments, the Indazole Compounds are useful for modulating, inhibiting, or regulating a cell cycle kinase or a cell cycle kinase pathway. Such kinases include but are not limited to CDK1, CDK2, CDK4, CDK5, CDK6, and CHK 1.

The invention also contemplates using the Indazole Compounds for treating diseases, disorders, or conditions associated with growth factor kinases or growth factor kinase pathways. In certain embodiments, the Indazole Compounds are useful for modulating, inhibiting, or regulating a growth factor kinase or growth factor kinase pathway. Such kinases include but are not limited to VEGF-R2, FGF-R, and TEK.

### 4.4.8 ADDITIONAL THERAPEUTIC AGENTS

The present methods for treating or preventing the diseases and disorders disclosed herein can further comprise the administration of an additional therapeutic agent or pharmaceutically acceptable salt, solvate or hydrate thereof. In this embodiment, the time that the therapeutic effect of the additional therapeutic agent is exerted overlaps with the time that the therapeutic effect of the Indazole Compound is exerted. In one embodiment, a composition comprising an Indazole Compound is administered concurrently with the administration of one or more additional therapeutic agent(s), which may be part of the same composition or in a different composition from that comprising the Indazole Compound. In another embodiment, an Indazole Compound is administered prior to or subsequent to administration of another therapeutic agent(s).

In the present methods for treating cancer the other therapeutic agent may be an antiemetic agent, an anxiolytic agent, a hematopoietic colony stimulating factor, or an anti-inflammatory agent.

In the present methods for treating inflammatory diseases, the other therapeutic agent includes, but is not limited to, steroids (e.g., cortisol, cortisone, fludrocortisone, prednisone, 6α-methylprednisone, triamcinolone, betamethasone or dexamethasone), nonsteroidal anti-inflammatory drugs ("NSAIDS", e.g., aspirin, acetaminophen, tolmetin, ibuprofen, mefenamic acid, piroxicam, nabumetone, rofecoxib, celecoxib, etodolac or nimesulide). In another embodiment, the other therapeutic agent is an antibiotic (e.g., vancomycin, penicillin, amoxicillin, ampicillin, cefotaxime, ceftriaxone, cefixime, rifampinmetronidazole, doxycycline or streptomycin). In still another embodiment, the other therapeutic agent is a PDE4 inhibitor (e.g., roflumilast or rolipram). In another embodiment, the other therapeutic agent is an antihistamine (e.g., cyclizine, hydroxyzine, promethazine or diphenhydramine). In yet another embodiment, the other therapeutic agent is an anti-malarial (e.g., artemisinin, artemether, artsunate, chloroquine phosphate, mefloquine hydrochloride, doxycycline hyclate, proguanil hydrochloride, atovaquone or halofantrine). In a further embodiment, the other therapeutic agent is drotrecogin alfa.

### 4.5 COMPOSITIONS AND METHODS OF ADMINISTRATION

Pharmaceutical compositions and single unit dosage forms comprising an Indazole Compound, or a pharmaceutically acceptable polymorph, prodrug, salt, solvate, hydrate, or clathrate thereof, are also encompassed by the invention. Individual dosage forms of the invention may be suitable for oral, mucosal (including sublingual, buccal, rectal, nasal, or vaginal), parenteral (including subcutaneous, intramuscular, bolus injection, intraarterial, or intravenous), transdermal, or topical administration.

Pharmaceutical compositions and dosage forms of the invention comprise an Indazole Compound, or a pharmaceutically acceptable prodrug, polymorph, salt, solvate, hydrate, or clathrate thereof. Pharmaceutical compositions and dosage forms of the invention typically also comprise one or more pharmaceutically acceptable excipients.

A particular pharmaceutical composition encompassed by this embodiment comprises an Indazole Compound, or a pharmaceutically acceptable polymorph, prodrug, salt, solvate, hydrate, or clathrate thereof, and at least one additional therapeutic agent. Examples of additional therapeutic agents include, but are not limited to: anti-cancer drugs and anti-inflammation therapies including, but not limited to, those listed above in Sections 4.4.2.1 and 4.4.8.

Single unit dosage forms of the invention are suitable for oral, mucosal (e.g., nasal, sublingual, vaginal, buccal, or rectal), parenteral (e.g., subcutaneous, intravenous, bolus injection, intramuscular, or intraarterial), or transdermal administration to a patient. Examples of dosage forms include, but are not limited to: tablets; caplets; capsules, such as soft elastic gelatin capsules; cachets; troches; lozenges; dispersions; suppositories; ointments; cataplasms (poultices); pastes; powders; dressings; creams; plasters; solutions; patches; aerosols (e.g., nasal sprays or inhalers); gels; liquid dosage forms suitable for oral or mucosal administration to a patient, including suspensions (*e*.*g*., aqueous or non-aqueous liquid suspensions, oil-in-water emulsions, or a water-in-oil liquid emulsions), solutions, and elixirs; liquid dosage forms suitable for parenteral administration to a patient; and sterile solids (e.g., crystalline or amorphous solids) that can be reconstituted to provide liquid dosage forms suitable for parenteral administration to a patient.

The composition, shape, and type of dosage forms of the invention will typically vary depending on their use. For example, a dosage form used in the acute treatment of inflammation or a related disease may contain larger amounts of one or more of the active ingredients it comprises than a dosage form used in the chronic treatment of the same disease. Similarly, a parenteral dosage form may contain smaller amounts of one or more of the active ingredients it comprises than an oral dosage form used to treat the same disease or disorder. These and other ways in which specific dosage forms encompassed by this invention will vary from one another will be readily apparent to those skilled in the art. See, e.g., Remington's Pharmaceutical Sciences, 18th ed., Mack Publishing, Easton PA (1990).

Typical pharmaceutical compositions and dosage forms comprise one or more carriers, excipients or diluents. Suitable excipients are well known to those skilled in the art of pharmacy, and non-limiting examples of suitable excipients are provided herein. Whether a particular excipient is suitable for incorporation into a pharmaceutical composition or dosage form depends on a variety of factors well known in the art including, but not limited to, the way in which the dosage form will be administered to a patient. For example, oral dosage forms such as tablets may contain excipients not suited for use in parenteral dosage forms. The suitability of a particular excipient may also depend on the specific active ingredients in the dosage form.

This invention further encompasses anhydrous (e.g., <1% water) pharmaceutical compositions and dosage forms comprising active ingredients, since water can facilitate the degradation of some compounds. For example, the addition of water (e.g., 5%) is widely accepted in the pharmaceutical arts as a means of simulating long-term storage in order to determine characteristics such as shelf-life or the stability of formulations over time. See, e.g., Jens T. Carstensen, Drug Stability: Principles & Practice, 2d. Ed., Marcel Dekker, NY, NY, 1995, pp. 379-80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms of the invention can be prepared using anhydrous or low moisture containing ingredients and low moisture or low humidity conditions. Pharmaceutical compositions and dosage forms that comprise lactose and at least one active ingredient that comprises a primary or secondary amine are preferably anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions are preferably packaged using materials known to prevent exposure to water such that they can be included in suitable formulary kits. Examples of suitable packaging include, but are not limited to, hermetically sealed foils, plastics, unit dose containers (e.g., vials), blister packs, and strip packs.

The invention further encompasses pharmaceutical compositions and dosage forms that comprise one or more compounds that reduce the rate by which an active ingredient will decompose. Such compounds, which are referred to herein as "stabilizers," include, but are not limited to, antioxidants such as ascorbic acid, pH buffers, or salt buffers.

Like the amounts and types of excipients, the amounts and specific types of active ingredients in a dosage form may differ depending on factors such as, but not limited to, the route by which it is to be administered to patients. However, typical dosage forms of the invention comprise an Indazole Compound, or a pharmaceutically acceptable salt, solvate, clathrate, hydrate, polymoprh or prodrug thereof lie within the range of from about 0.1 mg to about 2000 mg per day, given as a single once-a-day dose in the morning but preferably as divided doses throughout the day taken with food. More preferably, the daily dose is administered twice daily in equally divided doses. Preferably, a daily dose range should be from about 5 mg to about 500 mg per day, more preferably, between about 10 mg and about 200 mg per day. In managing the patient, the therapy should be initiated at a lower dose, perhaps about 1 mg to about 25 mg, and increased if necessary up to about 200 mg to about 2000 mg per day as either a single dose or divided doses, depending on the patient's global response.

### 4.5.1 ORAL DOSAGE FORMS

Pharmaceutical compositions of the invention that are suitable for oral administration can be presented as discrete dosage forms, such as, but are not limited to, tablets (e.g., chewable tablets), caplets, capsules, and liquids (e.g., flavored syrups). Such dosage forms contain predetermined amounts of active ingredients, and may be prepared by methods of pharmacy well known to those skilled in the art. See generally, Remington's Pharmaceutical Sciences, 18th ed., Mack Publishing, Easton PA (1990).

Typical oral dosage forms of the invention are prepared by combining the active ingredient(s) in an intimate admixture with at least one excipient according to conventional pharmaceutical compounding techniques. Excipients can take a wide variety of forms depending on the form of preparation desired for administration. For example, excipients suitable for use in oral liquid or aerosol dosage forms include, but are not limited to, water, glycols, oils, alcohols, flavoring agents, preservatives, and coloring agents. Examples of excipients suitable for use in solid oral dosage forms (*e*.*g*., powders, tablets, capsules, and caplets) include, but are not limited to, starches, sugars, micro-crystalline cellulose, diluents, granulating agents, lubricants, binders, and disintegrating agents.

In one embodiment, the oral dosage form consists of an effective amount of one or more Indazole Compounds in a capsule or caplet (i.e., without a carrier, diluent or excipient).

Because of their ease of administration, tablets and capsules represent the most advantageous oral dosage unit forms, in which case solid excipients are employed. If desired, tablets can be coated by standard aqueous or nonaqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary.

For example, a tablet can be prepared by compression or molding. Compressed tablets can be prepared by compressing in a suitable machine the active ingredients in a free-flowing form such as powder or granules, optionally mixed with an excipient. Molded tablets can be made by molding in a suitable machine a mixture of the powdered compound moistened with an inert liquid diluent.

Examples of excipients that can be used in oral dosage forms of the invention include, but are not limited to, binders, fillers, disintegrants, and lubricants. Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (*e*.*g*., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, (e.g., Nos. 2208, 2906, 2910), microcrystalline cellulose, and mixtures thereof.

Examples of fillers suitable for use in the pharmaceutical compositions and dosage forms disclosed herein include, but are not limited to, talc, calcium carbonate (e.g., granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions of the invention is typically present in from about 50 to about 99 weight percent of the pharmaceutical composition or dosage form.

Suitable forms of microcrystalline cellulose include, but are not limited to, the materials sold as AVICEL-PH-101, AVICEL-PH-103 AVICEL RC-581, AVICEL-PH-105 (available from FMC Corporation, American Viscose Division, Avicel Sales, Marcus Hook, PA), and mixtures thereof. An specific binder is a mixture of microcrystalline cellulose and sodium carboxymethyl cellulose sold as AVICEL RC-581. Suitable anhydrous or low moisture excipients or additives include AVICEL-PH-103™ and Starch 1500 LM.

Disintegrants are used in the compositions of the invention to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms of the invention. The amount of disintegrant used varies based upon the type of formulation, and is readily discernible to those of ordinary skill in the art. Typical pharmaceutical compositions comprise from about 0.5 to about 15 weight percent of disintegrant, specifically from about 1 to about 5 weight percent of disintegrant.

Disintegrants that can be used in pharmaceutical compositions and dosage forms of the invention include, but are not limited to, agar-agar, alginic acid, calcium carbonate, microcrystalline cellulose, croscarmellose sodium, crospovidone, polacrilin potassium, sodium starch glycolate, potato or tapioca starch, pre-gelatinized starch, other starches, clays, other algins, other celluloses, gums, and mixtures thereof.

Lubricants that can be used in pharmaceutical compositions and dosage forms of the invention include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL 200, manufactured by W.R. Grace Co. of Baltimore, MD), a coagulated aerosol of synthetic silica (marketed by Degussa Co. of Plano, TX), CAB-O-SIL (a pyrogenic silicon dioxide product sold by Cabot Co. of Boston, MA), and mixtures thereof. If used at all, lubricants are typically used in an amount of less than about 1 weight percent of the pharmaceutical compositions or dosage forms into which they are incorporated.

### 4.5.2 DELAYED RELEASE DOSAGE FORMS

Active ingredients of the invention can be administered by controlled release means or by delivery devices that are well known to those of ordinary skill in the art. Examples include, but are not limited to, those described in U.S. Patent Nos.: 3,845,770; 3,916,899; 3,536,809; 3,598,123; and 4,008,719, 5,674,533, 5,059,595, 5,591,767, 5,120,548, 5,073,543, 5,639,476, 5,354,556, and 5,733,566, each of which is incorporated herein by reference. Such dosage forms can be used to provide slow or controlled-release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled-release formulations known to those of ordinary skill in the art, including those described herein, can be readily selected for use with the active ingredients of the invention. The invention thus encompasses single unit dosage forms suitable for oral administration such as, but not limited to, tablets, capsules, gelcaps, and caplets that are adapted for controlled-release.

Controlled-release pharmaceutical products can improve drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency, and increased patient compliance. In addition, controlled-release formulations can be used to affect the time of onset of action or other characteristics, such as blood levels of the drug, and can thus affect the occurrence of side (e.g., adverse) effects.

Most controlled-release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, temperature, enzymes, water, or other physiological conditions or compounds.

### 4.5.3 PARENTERAL DOSAGE FORMS

Parenteral dosage forms can be administered to patients by various routes including, but not limited to, subcutaneous, intravenous (including bolus injection), intramuscular, and intra-arterial. Because their administration typically bypasses patients' natural defenses against contaminants, parenteral dosage forms are preferably sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, and emulsions. For example, lyophilized sterile compositions suitable for reconstitution into particulate-free dosage forms suitable for administration to humans.

Suitable vehicles that can be used to provide parenteral dosage forms of the invention are well known to those skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

Compounds that increase the solubility of one or more of the active ingredients disclosed herein can also be incorporated into the parenteral dosage forms of the invention.

Parenteral dosage forms are preferred for the methods of preventing, treating or managing disease in a cancer patient.

### 4.5.4 TRANSDERMAL AND TOPICAL DOSAGE FORMS

Transdermal and topical dosage forms of the invention include, but are not limited to, creams, lotions, ointments, gels, solutions, emulsions, suspensions, or other forms known to one of skill in the art. See, *e*.*g*., Remington's Pharmaceutical Sciences, 18th eds., Mack Publishing, Easton PA (1990); and Introduction to Pharmaceutical Dosage Forms, 4th ed., Lea & Febiger, Philadelphia (1985). Transdermal dosage forms include "reservoir type" or "matrix type" patches, which can be applied to the skin and worn for a specific period of time to permit the penetration of a desired amount of active ingredients.

Suitable excipients (e.g., carriers and diluents) and other materials that can be used to provide transdermal and topical dosage forms encompassed by this invention are well known to those skilled in the pharmaceutical arts, and depend on the particular tissue to which a given pharmaceutical composition or dosage form will be applied. With that fact in mind, typical excipients include, but are not limited to, water, acetone, ethanol, ethylene glycol, propylene glycol, butane-1,3-diol, isopropyl myristate, isopropyl palmitate, mineral oil, and mixtures thereof to form lotions, tinctures, creams, emulsions, gels or ointments, which are non-toxic and pharmaceutically acceptable. Moisturizers or humectants can also be added to pharmaceutical compositions and dosage forms if desired. Examples of such additional ingredients are well known in the art. See, *e*.*g*., Remington's Pharmaceutical Sciences, 18th eds., Mack Publishing, Easton PA (1990).

Depending on the specific tissue to be treated, additional components may be used prior to, in conjunction with, or subsequent to treatment with active ingredients of the invention. For example, penetration enhancers can be used to assist in delivering the active ingredients to the tissue. Suitable penetration enhancers include, but are not limited to: acetone; various alcohols such as ethanol, oleyl, and tetrahydrofuryl; alkyl sulfoxides such as dimethyl sulfoxide; dimethyl acetamide; dimethyl formamide; polyethylene glycol; pyrrolidones such as polyvinylpyrrolidone; Kollidon grades (Povidone, Polyvidone); urea; and various water-soluble or insoluble sugar esters such as Tween 80 (polysorbate 80) and Span 60 (sorbitan monostearate).

The pH of a pharmaceutical composition or dosage form, or of the tissue to which the pharmaceutical composition or dosage form is applied, may also be adjusted to improve delivery of one or more active ingredients. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery-enhancing or penetration-enhancing agent. Different salts, hydrates or solvates of the active ingredients can be used to further adjust the properties of the resulting composition.

### 4.5.5 MUCOSAL DOSAGE FORMS AND LUNG DELIVERY

Mucosal dosage forms of the invention include, but are not limited to, ophthalmic solutions, sprays and aerosols, or other forms known to one of skill in the art. See, e.g., Remington's Pharmaceutical Sciences, 18th eds., Mack Publishing, Easton PA (1990); and Introduction to Pharmaceutical Dosage Forms, 4th ed., Lea & Febiger, Philadelphia (1985). Dosage forms suitable for treating mucosal tissues within the oral cavity can be formulated as mouthwashes or as oral gels. In one embodiment, the aerosol comprises a carrier. In another embodiment, the aerosol is carrier free.

An Indazole Compound can also be administered directly to the lung by inhalation (see e.g., Tong et al., PCT Application, WO 97/39745; Clark et al, PCT Application, WO 99/47196, which are herein incorporated by reference). For administration by inhalation, an Indazole Compound can be conveniently delivered to the lung by a number of different devices. For example, a Metered Dose Inhaler ("MDI") which utilizes canisters that contain a suitable low boiling propellant, e.g., dichlorodifluoromethane, trichlorofluoromethane, dichlorotetrafluoroethane, carbon dioxide or other suitable gas can be used to deliver an Indazole Compound directly to the lung. MDI devices are available from a number of suppliers such as 3M Corporation, Aventis, Boehringer Ingleheim, Forest Laboratories, Glaxo-Wellcome, Schering Plough and Vectura.

Alternatively, a Dry Powder Inhaler (DPI) device can be used to administer an Indazole Compound to the lung (See, e.g., Raleigh et al., Proc. Amer. Assoc. Cancer Research Annual Meeting, 1999, 40, 397, which is herein incorporated by reference). DPI devices typically use a mechanism such as a burst of gas to create a cloud of dry powder inside a container, which can then be inhaled by the patient. DPI devices are also well known in the art and can be purchased from a number of vendors which include, for example, Fisons, Glaxo-Wellcome, Inhale Therapeutic Systems, ML Laboratories, Qdose and Vectura. A popular variation is the multiple dose DPI ("MDDPI") system, which allows for the delivery of more than one therapeutic dose. MDDPI devices are available from companies such as AstraZeneca, Glaxo Wellcome, IVAX, Schering Plough, SkyePharma and Vectura. For example, capsules and cartridges of gelatin for use in an inhaler or insufflator can be formulated containing a powder mix of the compound and a suitable powder base such as lactose or starch for these systems.

Another type of device that can be used to deliver an Indazole Compound to the lung is a liquid spray device supplied, for example, by Aradigm Corporation. Liquid spray systems use extremely small nozzle holes to aerosolize liquid drug formulations that can then be directly inhaled into the lung.

In a preferred embodiment, a nebulizer device is used to deliver an Indazole Compound to the lung. Nebulizers create aerosols from liquid drug formulations by using, for example, ultrasonic energy to form fine particles that can be readily inhaled (See e.g., Verschoyle et al., British J Cancer, 1999, 80, Suppl 2, 96, which is herein incorporated by reference). Examples of nebulizers include devices supplied by Sheffield/Systemic Pulmonary Delivery Ltd. (See, Armer et al., U.S. Pat. No. 5,954,047; van der Linden et al., U.S. Pat. No. 5,950,619; van der Linden et al., U.S. Pat. No. 5,970,974, which are herein incorporated by reference), Aventis and Batelle Pulmonary Therapeutics. Inhaled compound of the invention, delivered by nebulizer devices, is currently under investigation as a treatment for aerodigestive cancer (Engelke et al., Poster 342 at American Association of Cancer Research, San Francisco, Calif., Apr. 1-5, 2000) and lung cancer (Dahl et al., Poster 524 at American Association of Cancer Research, San Francisco, Calif., April 1-5, 2000).

In a particularly preferred embodiment, an electrohydrodynamic ("EHD") aerosol device is used to deliver an Indazole Compound to the lung. EHD aerosol devices use electrical energy to aerosolize liquid drug solutions or suspensions (see e.g., Noakes et al., U.S. Pat. No. 4,765,539; Coffee, U.S. Pat. No., 4,962,885; Coffee, PCT Application, WO 94/12285; Coffee, PCT Application, WO 94/14543; Coffee, PCT Application, WO 95/26234, Coffee, PCT Application, WO 95/26235, Coffee, PCT Application, WO 95/32807, which are herein incorporated by reference). The electrochemical properties of the compound of the invention formulation may be important parameters to optimize when delivering this drug to the lung with an EHD aerosol device and such optimization is routinely performed by one of skill in the art. EHD aerosol devices may more efficiently delivery drugs to the lung than existing pulmonary delivery technologies. Other methods of intra-pulmonary delivery of an Indazole Compound will be known to the skilled artisan and are within the scope of the invention.

Liquid drug formulations suitable for use with nebulizers and liquid spray devices and EHD aerosol devices will typically include an Indazole Compound with a pharmaceutically acceptable carrier. Preferably, the pharmaceutically acceptable carrier is a liquid such as alcohol, water, polyethylene glycol or a perfluorocarbon. Optionally, another material may be added to alter the aerosol properties of the solution or suspension of an Indazole Compound. Preferably, this material is liquid such as an alcohol, glycol, polyglycol or a fatty acid. Other methods of formulating liquid drug solutions or suspension suitable for use in aerosol devices are known to those of skill in the art (See, e.g., Biesalski, U.S. Pat. Nos. 5,112,598; Biesalski, 5,556,611, which are herein incorporated by reference). An Indazole Compound can also be formulated in rectal or vaginal compositions such as suppositories or retention enemas, e.g., containing conventional suppository bases such as cocoa butter or other glycerides.

In addition to the formulations described previously, an Indazole Compound can also be formulated as a depot preparation. Such long acting formulations can be administered by implantation (for example subcutaneously or intramuscularly) or by intramuscular injection. Thus, for example, the compounds can be formulated with suitable polymeric or hydrophobic materials (for example, as an emulsion in an acceptable oil) or ion exchange resins, or as sparingly soluble derivatives, for example, as a sparingly soluble salt.

Alternatively, other pharmaceutical delivery systems can be employed. Liposomes and emulsions are well known examples of delivery vehicles that can be used to deliver an Indazole Compound. Certain organic solvents such as dimethylsulfoxide can also be employed, although usually at the cost of greater toxicity. An Indazole Compound can also be delivered in a controlled release system. In one embodiment, a pump can be used (Sefton, CRC Crit. RefBiomed Eng., 1987, 14, 201; Buchwald et al., Surgery, 1980, 88, 507; Saudek et al., N. Engl. J Med, 1989, 321, 574). In another embodiment, polymeric materials can be used (see Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Ranger and Peppas, J Macromol. Sci. Rev. Macromol. Chem., 1983, 23, 61; see also Levy et al., Science 1985, 228, 190; During et al., Ann. Neurol., 1989,25,351; Howard et al., 1989, J. Neurosurg. 71, 105). In yet another embodiment, a controlled-release system can be placed in proximity of the target of the compounds of the invention, e.g., the lung, thus requiring only a fraction of the systemic dose (see, e.g., Goodson, in Medical Applications of Controlled Release, supra, vol. 2, pp. 115 (1984)). Other controlled-release system can be used (see *e*.*g*., Langer, Science, 1990, 249, 1527).

Suitable excipients (e.g., carriers and diluents) and other materials that can be used to provide mucosal dosage forms encompassed by this invention are well known to those skilled in the pharmaceutical arts, and depend on the particular site or method which a given pharmaceutical composition or dosage form will be administered. With that fact in mind, typical excipients include, but are not limited to, water, ethanol, ethylene glycol, propylene glycol, butane-1,3-diol, isopropyl myristate, isopropyl palmitate, mineral oil, and mixtures thereof, which are non-toxic and pharmaceutically acceptable. Examples of such additional ingredients are well known in the art. See, e.g., Remington's Pharmaceutical Sciences, 18th eds., Mack Publishing, Easton PA (1990).

The pH of a pharmaceutical composition or dosage form, or of the tissue to which the pharmaceutical composition or dosage form is applied, can also be adjusted to improve delivery of one or more active ingredients. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery-enhancing or penetration-enhancing agent. Different salts, hydrates or solvates of the active ingredients can be used to further adjust the properties of the resulting composition.

The present invention is not to be limited in scope by the specific embodiments disclosed in the examples which are intended as illustrations of a few aspects of the invention and any embodiments that are functionally equivalent are within the scope of this invention. Indeed, various modifications of the invention in addition to those shown and described herein will become apparent to those skilled in the art and are intended to fall within the scope of the appended claims.

It should be noted that one or more hydrogen atoms or methyl groups may be omitted from the drawn structures consistent with accepted shorthand notation of such organic compounds, and that one skilled in the art of organic chemistry would readily appreciate their presence.

### 5. EXAMPLES

### EXAMPLE 1

### SYNTHESIS OF 3-(6-ETHOXY-NAPHTHALEN-2-YL)-5-(1H- [1,2,4] TRIAZOL-3-YL)-1 H-INDAZOLE

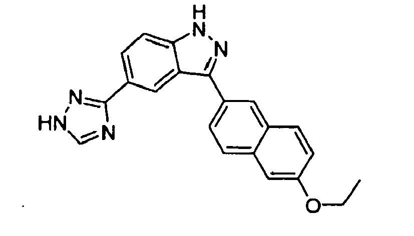

### A. 3-Ethoxynaphthalene-boronic acid

A solution of 2-bromo-6-ethoxynaphthalene (0.492 g, 1.96 mmol) in tetrahydrofuran under a nitrogen atmosphere was prepared. The solution was chilled to - 78° C and n-butyllithium in hexane (1.49 ml, 1.6M) was added dropwise. The reaction was stirred for 1 hour at -78° C then trimethylborate (0.621 g, 5.97 mmol) was added. The reaction was stirred for 1 hour at -78° C then saturated aqueous ammonium chloride (3 ml) was added and the mixture allowed to warm to room temperature. The mixture was diluted with water (50 ml) and extracted with ethyl acetate (3x). The ethyl acetate solution was dried over anhydrous sodium sulfate. The solution was concentrated and solids dried in a vacuum oven to give the title compound (388 mg, 92% yield).

### B. 3-(6-Ethoxy-naphthalen-2-yl)-1H-indazole-5-carbonitrile

A flask was charged with 3-ethoxynaphthalene-boronic acid (382 mg, 1.77 mmol, 1.2 equiv.), 3-bromo-1-perhydro-2H-pyran-2-yl-1H-indazole-5-carbonitrile (0.414 g, 1.35 mmol), [1,1'-bis(diphenylphosphino-ferrocene] complex with dichloromethane (1:1) (0.110 g, 0.135 mmol), powdered potassium phosphate (2.87 g, 13.5 mmol), anhydrous 1,2-dimethoxyethane (30 ml), and water (1ml). The mixture was refluxed for 16 hours. The reaction mixture was diluted with ethyl acetate and filtered through celite. The resulting solution was concentrated and chromatographed on silica gel eluting with ethyl acetate in hexane to give 0.357 g of a yellow solid. Methanol (50 ml), tetrahydrofuran (10 ml), and aqueous hydrochloric acid (50 ml, 6 N) were added to the solid and the mixture was stirred at room temperature for 48 hours. Dioxane (10ml) was added and the mixture heated to 45-60° C for 6 hours. The bulk of organic solvents were removed using a rotary evaporator. Water (50 ml) was added and the solid was filtered and dried in a vacuum oven to give the title compound (0.254 g, 55% yield over two steps): ES-MS (m/z) 314 [M+H]⁺.

### C. 3-(6-Ethoxy-naphthalen-2-yl)-1H-indazole-5-carboxylic acid amide

A mixture of 3-(6-ethoxy-naphthalen-2-yl)-1H-indazole-5-carbonitrile (335 mg, 1.07 mmol), ethanol (10.5 ml), aqueous sodium hydroxide (1.75 ml, 6.0 N), and 30% aqueous hydrogen peroxide (5 ml) was heated to 40° C for 90 minutes. The reaction mixture was diluted with water (100 ml) and acidified with aqueous hydrochloric acid (10 ml, 3N). The solid was filtered and dried in a vacuum oven to give the title compound (332 mg, 94% yield). ES-MS (m/z) 332 [M+H]⁺.

### D. 3-(6-Ethoxy-naphthalen-2-yl)-5-(1H-[1,2,4]triazol-3-yl)-1H-indazole

A mixture of 3-(6-ethoxy-naphthalen-2-yl)-1H-indazole-5-carboxylic acid amide (331 mg, 1.0 mmol) and N, N-dimethylformamide dimethyl acetal (50 ml) was heated to 80° C for 16 hours. The reaction mixture was evaporated and to the concentrate was added glacial acetic acid (30 ml) and anhydrous hydrazine (1.75 ml). The reaction mixture was stirred for at least 30 minutes then diluted with water (150 ml). The solid was filtered and dried in a vacuum oven to give 257 mg. The title compound was isolated after purification by preparative HPLC (168 mg, 47% yield. ¹H NMR (CD₃OD) δ 8.87 (s, 1H), 8.46 (s, 1H), 8.09 (d, 2H), 7.95 (d, 1H), 7.91 (d, 1H), 7.70 (d, 1H), 7.29 (s, 1H), 7.20 (dd, 1H), 4.20 (q, 2H), 1.49 (t, 3H): ES-MS (m/z) 356 [M+H]⁺.

### EXAMPLE 2

### SYNTHESIS OF 3-(6-BUTOXY-NAPHTHALEN-2-YL)-5-(1H-[1,2,4] TRIAZOL-3-YL)-1H-INDAZOLE

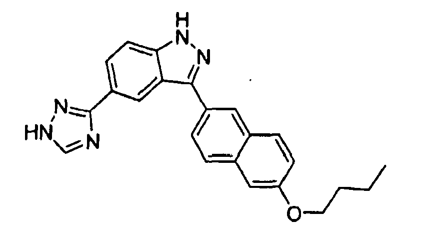

### A. 6-Butoxynaphthalene-2-boronic acid

A solution of 2-bromo-6-butoxynaphthalene (0.492 g, 1.76 mmol) in tetrahydrofuran under a nitrogen atmosphere was prepared. The solution was chilled to - 78° C and n-butyllithium in hexane (1.49 ml, 1.6M) was added dropwise. The reaction was stirred for 1 hour at -78° C then trimethylborate (0.621 g, 5.97 mmol) was added. The reaction was stirred for 1 hour at -78° C then saturated aqueous ammonium chloride (3 ml) was added and the mixture allowed to warm to room temperature. The mixture was diluted with water (50 ml) and extracted with ethyl acetate (3x). The ethyl acetate solution was dried over anhydrous sodium sulfate. The solution was concentrated and solids dried in a vacuum oven to provide the title compound (0.397 g, 92% yield).

### B. 3-(6-Butoxy-naphthalen-2-yl)-1H-indazole-5-carbonitrile

A flask was charged with 6-butoxynaphthalene-2-boronic acid (0.396 g, 1.62 mmol, 1.2 equiv.), 3-bromo-1-perhydro-2H-pyran-2-yl-1H-indazole-5-carbonitrile (0.414 g, 1.35 mmol), [1,1'-bis(diphenylphosphino-ferrocene] complex with dichloromethane (1:1) (0.110 g, 0.135 mmol), powdered potassium phosphate (2.87 g, 13.5 mmol), anhydrous 1,2-dimethoxyethane (30 ml), and water (1ml). The mixture was refluxed for 16 hours. The reaction mixture was diluted with ethyl acetate and filtered through celite. The resulting solution was concentrated and chromatographed on silica gel eluting with ethyl acetate in hexane to provide 0.357 g of a yellow solid. Methanol (50 ml), tetrahydrofuran (10 ml), and aqueous hydrochloric acid (50 ml, 6 N) were added to the solid and the mixture was stirred at room temperature for 48 hours. Dioxane (10ml) was added and the mixture heated to 45-60° C for 6 hours. The bulk of organic solvents were removed using a rotary evaporator. Water (50 ml) was added and the solid was filtered and dried in a vacuum oven to provide the title compound (0.254 g, 55% yield over two steps): ES-MS (m/z) 342 [M+H]⁺.

### C. 3-(6-Butoxy-naphthalen-2-yl)-1H-indazole-5-carboxylic acid amide

A mixture of 3-(6-butoxy-naphthalen-2-yl)-1H-indazole-5-carbonitrile (253 mg, 0.741 mmol), ethanol (10.5 ml), aqueous sodium hydroxide (1.75 ml, 6.0 N), and 30% aqueous hydrogen peroxide (5 ml) was heated to 40° C for 90 minutes. The reaction mixture was diluted with water (100 ml) and acidified with aqueous hydrochloric acid (10 ml, 3N). The solid was filtered and dried in a vacuum oven to provide the title compound (0.250 g, 94% yield): ES-MS (m/z) 360 [M+H]⁺.

### D. 3-(6-Butoxy-naphthalen-2-yl)-5-(1H- [1,2,4] triazol-3-yl)-1H-indazole

A mixture of 3-(6-Butoxy-naphthalen-2-yl)-1H-indazole-5-carboxylic acid amide (250 mg, 0.696 mmol) and N, N-dimethylformamide dimethyl acetal (50 ml) was heated to 80° C for 16 hours. The reaction mixture was evaporated and to the concentrate was added glacial acetic acid (30 ml) and anhydrous hydrazine (1.75 ml). The reaction mixture was stirred for at least 30 minutes then diluted with water (150 ml). The solid was filtered and dried in a vacuum oven to provide 257 mg. of a crude product which was purified using preparative HPLC to provide the title compound (134mg, 50% yield). ¹NMR (DMSO) δ 14.05-14.2 (br, 1H), 13.22 (s, 1H), 8.80 (s, 1H), 8.46 (s, 1H), 8.12 (d, 2H), 8.00 (dd, 2H), 7.72 (d, 1H), 7.40 (s, 1H), (dd, 1H), 4.14 (t, 2H), 1.75-1.85 (m, 2H), 1.45-1.58 (m, 2H), 0.98 (t, 3H): ES-MS (m/z) 384 [M+H]⁺.

### EXAMPLE 3

### SYNTHESIS OF 3-(6-METHOXYNAPHTHALEN-2-YL)-5(5-METHYL-1H-[1,2,4]TRIAZOL-3-YL)-1H-INDAZOLE

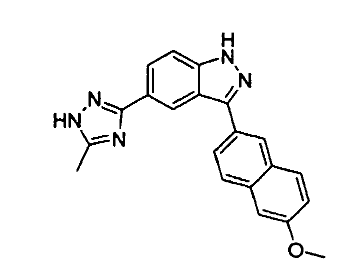

To a flask was charged 3-(6-methoxynaphthalen-2-yl)-1H-indazole-5-carboximidic acid ethyl ester (300 mg, 0.79 mmol), methanol (10 mL), and triethylamine (1.46 mL, 10.5 mmol). After 10 minutes, hydrazide (233 mg, 3.15 mmol, prepared as described in International Publication No. WO 02/10137, Example 422A) was added and the mixture was heated at 90°C for 18 hours. The mixture was concentrated and purified by preparatory HPLC to provide the title compound (81 mg, 29%): ¹H NMR (CD₃O) δ 8.83 (br s, 1H) 8.46 (s, 1H) 8.11 (d, 2H) 7.94 (t, 2H) 7.64 (br d, 1H) 7.31 (s, 1H) 7.20 (d, 1H) 3.95 (s, 3H) 2.44 (br d, 3H); ES-MS (m/z) 356 [M+1]⁺.

### Example 4

### SYNTHESIS OF 5-(5-ISOBUTYL-1H-[1,2,4]TRIAZOL-3-YL)-3-(6-METHOXYNAPTHALEN-2-YL)-1H-INDAZOLE

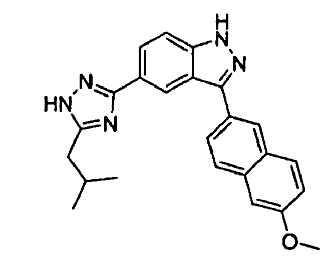

### A. 4-Fluoro-3-[(fluorophenyl)-hydroxymetyl]-benzonitrile

To a flask containing THF (250 mL) was added LDA, which was cooled to - 78°C. A solution of p-fluorobenzonitrile (15.5 g, 128 mmol) in THF (55 mL) was added to the cold mixture dropwise. After 20 minutes, a solution of 6-methoxy-2-naphthaldehyde in THF (100 mL) was added dropwise. After 10 minutes, the reaction mixture was allowed to slowly warm to ambient temperature. Water (20 mL) was added and the THF was evaporated. Ether (200 mL) was added and the layers were partitioned. The ether layer was washed with 5% aqueous HCl (2 x 100 mL), brine (100 mL) and was dried (MgSO₄) and concentrated. The crude product was purified by flash chromatography using a gradient of 5% to 20% ethyl acetate in hexanes to provide pure title compound (11.2 g, 57 %): ES-MS (m/z) 290 [M-17]⁺.

### B. 4-Fluoro-3-(4-fluorobenzoyl)-benzonitrile

To a flask was charged 4-fluoro-3-[(fluorophenyl)-hydroxymethyl]-benzonitrile (1.75 g, 5,71 mmol), dichloromethane (90 mL), and pyridinium chlorochromate (1.29 g, 5.99 mmol). The mixture was heated at 40°C for 3 hours, after which time the solvent was evaporated. The crude product was purified by flash chromatography using dichloromethane as the eluent to provide the title compound (1.59 g, 91 %): ES-MS (m/z) 306 [M+1]⁺.

### C. 3-(6-Methoxynaphthalen-2-yl)-1H-indazole-5-carbonitrile

To a flask was charged 4-fluoro-3-(4-fluorobenzoyl)-benzonitrile (7.00g, 22.9 mmol), THF (250 mL) and hydrazine monohydrate (5.74g, 115 mmol). The mixture was allowed to stir at ambient temperature for 4 hours, after which time water (300 mL) was added and the precipitate was filtered, washed with water and dried to provide the title compound (6.6 g, 96 %): ES-MS (m/z) 300 [M+1]⁺.

### D. 3-(6-Methoxynaphthalen-2-yl)-1H-indazole-5-carboximidic acid ethyl ester

To a flask was charged 3-(6-methoxynaphthalen-2-yl)-1H-indazole-5-carbonitrile (6.4g, 21.4 mmol) and ethanol (650 mL). HCl (g) was bubbled through the stirring solution until saturated. After 18 hours, mixture was concentrated and slurried with diethyl ether. Solid product was filtered and washed with excess diethyl ether. Product was dried to provide the title compound as the HCl salt (6.2g, 76%): ES-MS (m/z) 346 [M+1]⁺.

### E. 5-(5-Isobutyl-1H-[1,2,4]triazol-3-yl)-3-(6-methoxynaphthalen-2-yl)-1H-indazole

To a flask was charged 3-(6-methoxynaphthalen-2-yl)-1H-indazole-5-carboximidic acid ethyl ester (200 mg, 0.52 mmol), methanol (10 mL), and triethylamine (1.46 mL, 10.5 mmol). After 10 minutes, hydrazide (244 mg, 2.10 mmol, prepared as described in International Publication No. WO 02/10137, Example 422A) was added and the mixture was heated at 90°C for 18 hours. The mixture was concentrated and purified by preparatory HPLC to provide the title compound (76 mg, 37 %): ¹H NMR (DMSO) δ 8.74 (d, 1H) 8.46 (d, 1H) 8.20 - 7.96 (m, 4H) 7.69 (dd, 1H) 7.40 (s, 1H) 7.22 (t,- 1H) 3.92 (s, 3H) 2.59 (dd, 2H) 2.18 - 2.02 (m, 1H) 0.94 (d, 6H); ES-MS (m/z) 398 [M+1]⁺.

### Example 5

### SYNTHESIS OF 3-NAPTHALEN-2-YL-5-(5-PYRROLIDIN-1-YLMETHYL-1H-[1,2,4]TRIAZOL-3-YL)-1H-INDAZOLE

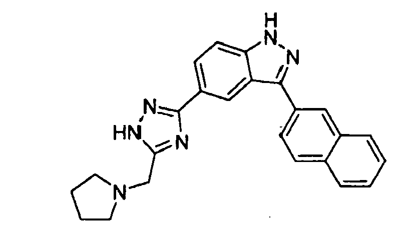

### A. 4-Fluoro-3-(hydroxyl-napthalen-2-yl-methyl)-benzonitrile

Following the procedure of Example 7, step A, using 4-fluorobenzonitrile (3.00g, 24.8mmol), 2-naphthaldehyde (3.44g, 27.3mmol), and 2M lithium diisopropylamide (13.6mL, 27.3mmol) the title compound was prepared (2.18g, 32% yield). ES-MS (m/z) 278 [M+1].

### B. 4-Fluoro-3-(naphthalene-2-carbonyl)-benzonitrile

Following the procedure of Example 6, step D, using 4-fluoro-3-(hydroxyl-napthalen-2-yl-methyl)-benzonitrile (2.18g, 7.8mmol), dimethyl sulfoxide (1.40mL, 19.6mmol), oxalyl chloride (0.85mL, 9.8mmol), and triethylamine (6.20ml, 44.5mmol), the title compound was prepared (0.75g, 35% yield). ES-MS (m/z) 276 [M+1].

### C. 3-Napthalen-2-yl-1H-indazole-5-carbonitrile

Following the procedure of Example 4, step C, using 4-fluoro-3-(naphthalene-2-carbonyl)-benzonitrile (0.75g, 2.7mmol) and hydrazine monohydrate (10mL), the title compound was prepared (0.48g, 65% yield). ES-MS (m/z) 270 [M+1].

### D. 3-Napthalen-2-yl-1H-indazole-5-carboximidic acid ethyl ester

Following the procedure of Example 4, step D, using 3-napthalen-2-yl-1H-indazole-5-carbonitrile (0.48g, 1.8mmol), the title compound was prepared (0.48g, 70% yield). ES-MS (m/z) 316 [M+1].

### E. 3-Napthalen-2-yl-5-(5-pyrrolidin-1-ylmethyl-1H-[1,2,4]triazol-3-yl)-1H-indazole

Following the procedure of Example 4, step E, using 3-napthalen-2-yl-1H-indazole-5-carboximidic acid ethyl ester (0.48g, 1.3mmol), pyrrolidin-1-yl-acetic acid hydrazide (0.21g, 1.5mmol), and 4.63M sodium methoxide (0.53mL, 2.5mmol), the title compound was prepared (0.04g, 8% yield). ¹H NMR (DMSO-d₆) δ 8.83(s, 1H), 8.60(s, 1H), 8.23-8.00 (m, 5H), 7.80-7.60(m, 3H), 3.80(s, 4H), 2.55(m, 4H). ES-MS (m/z) 395 [M+1].

### Example 6

### SYNTHESIS OF 3-(6-ETHOXY-NAPHTHALEN-2-YL)-5-(5-PYRROLIDIN-1-YLMETHYL-1H-[1,2,4]TRIAZOL-3-YL)-1H-INDAZOLE

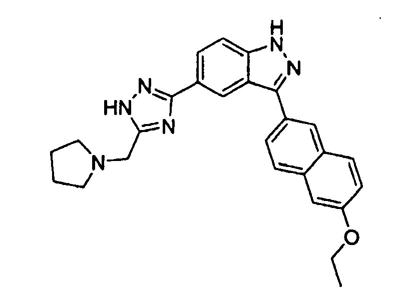

### A. 6-Ethoxy-naphthalene-2-carboxylic acid ethyl ester

To a solution of 6-hydroxy-2-naphtolic acid (5.0 g, 20 mmol) in 50 mL of DMF was added potassium carbonate (0.597 g, 4.32 mmol). After 20 min at room temperature, bromoethane (6.0 mL, 80 mmol) was added. The mixture was heated to 90°C overnight. The solvent was removed under reduced pressure and the crude product was partitioned between ethyl acetate and water. The material was used in the subsequent step without further purification: ¹H NMR (acetone-*d*₆) δ 8.5 (s, 1H), 8.0 (d, 1H), 7.9-7.8 (m, 2H), 7.3 (d, 1H), 7.2 (dd, 1H), 4.3 (q, 2H), 4.1 (q, 2H), 1.42-1.33 (m, 6H).

### B. 6-Ethoxy-naphthalen-2-yl)-methanol

Lithium aluminum hydride (4.55 g, 120 mmol) was added as a solid to anhydrous THF (300 mL) cooled to -78°C. 6-Ethoxy-naphthalene-2-carboxylic acid ethyl ester (20 mmol) was added dropwise to this suspension, as a solution in THF (100 mL). The reaction was slowly warmed to room temperature and was stirred overnight. The reaction was quenched with water and the crude material was extracted with ethyl acetate. The product was used without further purification (3.752 g, 93 % yield): ¹H NMR (DMSO-*d₆*) δ 7.8-7.7 (m, 32H), 7.4 (d, 1H), 7.2 (dd, 1H), 7.1 (dd, 1H), 4.1 (q, 2H), 3.36 (br s), 1.39(t, 3H).

### C. 6-Ethoxy-naphthalene-2-carbaldehyde

In a round-bottom flask containing pyridinium chlorochromate (PCC) and molecular sieves in dichloromethane (100 mL) at 0°C was added a solution of 6-(ethoxy-naphthalen-2-yl)-methanol (4.0 g, 20 mmol). The reaction was stirred at room temperature overnight. The reaction mixture was filtered through celite and the filtrate was concentrated and purified by silica gel column (9:1 hexanes/ethyl acetate) (2.5g, 62% yield): ¹H NMR (DMSO-*d₆*) δ 10.0 (s, 1H), 8.48 (s, 1H), 8.0 (d, 1H), 7.9-7.83 (m, 2H), 7.4 (s, 1H), 7.2 (d, 1H), 4.2 (q, 2H), 1.41 (t, 3H).

### D. (6-Ethoxy-naphthalen-2-yl)-[2-fluoro-5-(5-pyrrolidin-1-ylmethyl-1H [1,2,4]triazol-3-yl)phenyl]-methanone

To 6.25 mL of a commercial solution of LDA (2.0 M in hexanes) was added a solution of 4-fluorobenzonitrile (1.4 g, 11 mmol) in THF (2 mL). The deprotonation reaction was stirred at low temperature for 30 min before 4-fluoro-benzonitrile 6-ethoxynaphthalene-2-carbaldehyde (2.5 g, 12.5 mmol) was added as a solution in THF (1.6 mL). The reaction mixture was stirred at low temperature for 1 h and warmed to room temperature for 2 hours. The reaction was then quenched with ice. The crude product was extracted with ethyl acetate and dried over Na₂SO₄. The material was purified by column chromatography using dichloromethane (3.52 g, 34% yield).

In 2 mL of dichloromethane, was added DMSO (0.58 mL, 8.25 mmol). After cooling to -78°C, oxalyl chloride (0.356 mL, 4 mmol) was added, followed by a suspension of 3-[(6-ethoxy-naphthalen-2-yl)-hydroxy-methyl]-4-fluoro-benzonitrile (1.2 g, 3.75 mmol). After 15 min, triethyl amine was added (2.62 mL, 18.7 mmol). After 30 min at low temperature, the reaction mixture was stirred at room temperature overnight. After work-up, (filtration over Celite and evaporation) the crude product was purified by column chromatography (0.5 g, 42% yield): ES-MS (m/z) 320.

### E. 3-(6-Ethoxy-naphthalen-2-yl)-1H-indazole-5-carbonitrile

To a slurry of 3-(6-ethoxy-naphthalene-2-carbonyl)-4-fluoro-benzonitrile (0.5g, 1.6 mmol) in toluene (6 mL), was added hydrazine monohydrate (0.1 mL, 3.5 mmol). The reaction mixyure was heated to 58°C overnight. Solvents were removed under reduced pressure and the crude material was purified by preparatory HPLC (0.440g, 88% yield): ES-MS (m/z) 314.

F.**3-(6-Ethoxy-naphthalen-2-yl)-1*H*-indazole-5-carboximic acid ethyl** ester dihydrochloride

A solution of 3-(6-ethoxy-naphthalen-2-yl)-1*H*-indazole-5-carbonitrile (0.440 g, 1.4 mmol) in 20 mL of ethanol was prepared and cooled to -78°C before bubbling HCl gas for 15 min. The reaction temperature was then allowed to warm to room temperature and was stirred overnight. The solvent was then removed under reduced pressure and the resulting solid was triturated in diethyl ether before being collected by filtration and dried under vacuum (0.418 g, 69% yield): ES-MS (m/z) 360.

### G. 3-(6-Ethoxy-naphthalen-2-yl)-5-(5-pyrrolidin-1-ylmethylL-1H-[1,2,4]triazol-3-yl)-1H-indazole

To a suspension of 3-(6-ethoxy-naphthalen-2-yl)-1*H*-indazole-5-carboximic acid ethyl ester dihydrochloride (0.200g, 0.56 mmol) in methanol (5 mL) prepared in a sealed tube, was added triethylamine (1.56 mL, 11.2 mmol) followed by pyrrolidin-1-yl-acetic acid hydrazide (0.320g, 2.24 mmol). The reaction mixture was heated to 95°C for 3 hours. The solvent was then removed under reduced pressure and the crude mixture was purified by preparatory HPLC (20-100% acetonitrile-H₂O-0.1% TFA, Rt 9.9 min)to provide the title compound (0.065g, 26% yield). ES-MS (m/z) 439.

### Example 7

### SYNTHESIS OF 6-[5-(5-PYRROLIDIN-1-YLMETHYL-1H-[1,2,4]TRIAZOL-3-YL)-1H-INDAZOL-3-YL]-NAPHTHALENE-2-OL

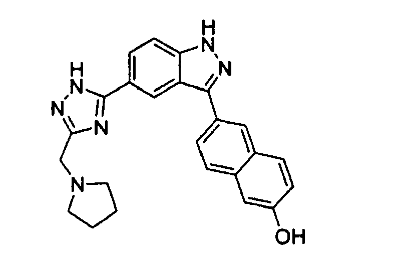

### A. 4-Fluoro-3-(6-hydroxy-naphthalene-2-carbonyl-benzonitrile

A solution of LDA was prepared from di-isopropyl amine (0.83 mL, 5.9 mmol) and *n*-butyl lithium (2.25 mL, 2.4 M) in 2.32 mL of dry THF at low temperature. A solution of 4-fluorobenzonitrile (0.59 g, 4.9 mmol) in THF (2 mL) was slowly added to the solution of LDA generated *in situ.* The deprotonation reaction was stirred at low temperature for 30 min before 6-methoxy-2-naphtaldehyde (1.0 g, 5.4 mmol) was added as a solution in THF (1.6 mL). The reaction mixture was stirred at low temperature for 1 h and warmed to room temperature for 2 hours. The reaction was then quenched with ice. The crude product was extracted with ethyl acetate and dried over Na₂SO₄. The material was purified by column chromatography (SiO₂, 7:3 ethyl acetate/hexanes) (0.440 g, 29% yield).

A slurry of pyridinium chlorochromate (PCC) in dichloromethane was prepared in 60 mL of solvent. Molecular sieves were added. 3-[(6-Methoxy-naphthalen-2-yl)-hydroxy-methyl]-4-fluoro-benzonitrile, (6.08 g, 19.8 mmol) was added to this mixture as a solution in 20 mL of THF. The reaction mixture turned black and was stirred at room temperature for 18 hours. (completion of reaction monitored by LC-MS). The crude mixture was filtered through celite and the material purified by column chromatography (SiO₂, 8:2 hexanes/ethyl acetate) (4.54 g, 75% yield): ES-MS (m/z) 306.

### B. 4-Fluoro-3-(6-hydroxy-naphthalene-2-carbonyl)-benzonitrile

A solution of 3-(6-methoxy-naphthalene-2-carbonyl)-4-fluoro-benzonitrile (3.976 g, 0.013 mmol) in dichloromethane (350 mL) was cooled to 0°C and boron tribromide (12.28 mL, 0.13 mmol) was added slowly. The reaction was stirred at room temperature overnight. The reaction was quenched with ice, neutralized with aqueous NaHCO₃, and extracted with dichloromethane. The crude material was purified by column chromatography (SiO₂, 7:3 hexanes/ethyl acetate) (2.5 g, 66% yield): ES-MS (m/z) 292.

### C. 3-(6-Hydroxy-naphthalen-2-yl)-1H-indazole-5-carbonitrile

To a slurry of 4-fluoro-3-(6-hydroxy-naphthalene-2-carbonyl)-benzonitrile in toluene (2.5 mL), was added hydrazine monohydrate (0.07 mL, 0.69 mmol). The reaction mixyure was heated to 58°C overnight. Solvents were removed under reduced pressure and the crude material was purified by preparatory HPLC (0.130 g, 68% yield): ES-MS (m/z) 286.

### D. 3-(6-Hydroxy-naphthalen-2-yl)-1H-indazole-5-carboximidic acid ethyl ester dihydrochloride

A solution of 3-(6-hydroxy-naphthalen-2-yl)-1H-indazole-5-carbonitrile (0130 g, 0.45 mmol) in ethanol (60 mL) was prepared and cooled to -78°C before bubbling HCl gas for 15 min. The reaction temperature was then allowed to warm to room temperature and was stirred overnight. The solvent was then removed under reduced pressure and the resulting solid was triturated in diethyl ether before being collected by filtration and dried under vacuum (0.200 g, quantitative): ES-MS (m/z) 332.

### E. 6-[5-(5-Pyrrolidin-1-ylmethyl-1H-[1,2,4]triazol-3-yl)-1H-indazol-3-yl]-naphthalene-2-ol

To a suspension of imidate (3-(6-hydroxy-naphthalen-2-yl)-1*H*-indazole-5-carboximidic acid ethyl ester dihydrochloride) in methanol (5 mL) prepared in a sealed tube, was added triethyl amine (1.7 mL, 12 mmol) followed by pyrrolidin-1-yl-acetic acid hydrazide. The reaction mixture was heated to 95°C for 3 hours. The solvent was then removed under reduced pressure and the crude mixture was purified by preparatory HPLC (20-80% acetonitrile- water) to provide the title compound (0.0088g, 4% yield): ¹H NMR (DMSO-*d₆*) δ 13.34 (m, 1H), 9.86 (s, 1H), 8.7 (m, 1H), 8.3 (m, 1H), 8.09 (m, 2H), 7.9 (d, 1H), 7.8 (d, 1H), 7.7 (m, 1H), 7.1 (m, 2H), 3.8 (m, 2H), 2.6 (m, 4H), 1.7 (m, 4H); ES-MS (m/z) 411.

### Example 8

### SYNTHESIS OF 6-[5-(5-PYRROLIDIN-1-YLMETHYL-2H-[1,2,4]TRLAZOL-3-YL)-1H-INDAZOL-3-YL]-NAPHTHALENE-2-CARBOXYLIC ACID ETHYL ESTER

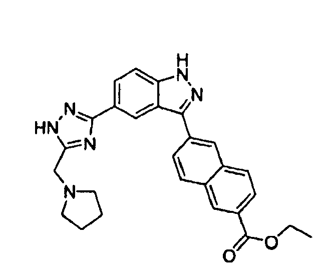

### A. 6-[5-(5-Pyrrolidin-1-ylmethyl-2H-[1,2,4]triazol-3-yl)-1H-indazol-3-yl]-naphthalene-2-carboxylic acid ethyl ester

A solution of 6-(5-ethoxycarbonimidoyl-1*H*-indazol-3-yl)-naphthalene-2-carboxylic acid ethyl ester di-hydrochloric acid salt (500 mg, 1.1 mmol), pyrrolidin-1-yl-acetic acid hydrazide (see WO 02/10137, example 422A) (620 mg, 4.3 mmol), and triethyl amine (2.2 mL, 15.8 mmol) in ethanol (5 mL) was heated in a sealed reaction flask on an 100 °C oil bath overnight. Solvent and excess triethyl amine were then removed under reduced pressure and the crude product purified using reverse-phase preparatory HPLC (20-70% acetonitrile + 0.1% TFA in H₂O + 0.1% TFA, over 30 min). Fractions containing clean product were neutralized with sodium bicarbonate and product extracted using ethyl acetate. The organic fractions were pooled, dried over magnesium sulfate, filtered, and solvent removed under reduced pressure to provide the title compound (131 mg, 26%). ¹H NMR (400 MHz, DMSO-*d₆*) δ 13.60 (bs, 1H), 8.79 (bm, 1H), 8.69 (s, 1H), 8.63 (bm, 1H), 8.28 (overlapping m, 3H), 8.12 (m, 1H), 8.06 (dd, 1H), 7.73 (m, 1H), 4.41 (q, 2H), 3.79 (bs, 2H), 2.58 (bm, 4H), 1.73 (bm, 4H), 1.40 (t, 3H); MS (ESI) *m*/*z* 467.2 [M+1]⁺.

### B. 6-(5-Ethoxycarbonimidoyl-1H-indazol-3-yl)-naphthalene-2-carboxylic acid ethyl ester di-hydrochloric acid salt

A solution of 6-[5-Cyano-1-(tetrahydro-pyran-2-yl)-1*H*-indazol-3-yl]-naphthalene-2-carboxylic acid ethyl ester (500.0 mg, 1.2 mmol) in ethanol (40 mL) was cooled on a dry ice/isopropanol bath and saturated with HCl (g). The resulting solution was allowed to stir, gradually warming to room temperature, overnight. Excess solvent was removed under reduced pressure and the resulting solid was taken up in Et₂O, sonicated 5 min and filtered to provide the title compound as a pale yellow solid (500 mg, 93%). MS (ESI) *m*/*z* 388.1 [free base M+1]⁺.

### C. 6-[5-Cyano-1-(tetrahydro-pyran-2-yl)-1H-indazol-3-yl]-naphthalene-2-carboxylic acid ethyl ester

A solution of 6-bromo-naphthalene-2-carboxylic acid ethyl ester (2 g, 7.2 mmol), bis(pinacolato)diboron (1.8 g, 7.1 mmol), [1,1'-bis(diphenylphosphino)-ferrocene] dichloro palladium complex with dichloromethane (1:1) (0.6 g, 0.73 mmol), and potassium acetate (2.1 g, 21.4 mmol) in DMF (20 mL) was heated in a sealed reaction flask on an 85 °C oil bath 3h. To this reaction mixture was added 3-bromo-1-(tetrahydro-pyran-2-yl)1H-indazole-5-carbonitrile (see WO 02/10137, example 161D) (2.0 g, 6.5 mmol) and potassium phosphate (4.5 g, 21.2 mmol) and the solution was returned to heat overnight. The cooled solution was filtered through Celite and the filter cake washed with EtOAc. The filtrate was washed with water and brine, dried over magnesium sulfate, filtered and solvent removed under reduced pressure to provide crude product. Purification using silica gel flash column chromatography (1:4 EtOAc:hexanes) provided title compound (1.02 g, 73%). MS (ESI) *m*/*z* 426.1 [M+1]⁺

### D. 6-bromo-naphthalene-2-carboxylic acid ethyl ester

A solution of 6-bromo-naphthalene-2-carboxylic acid (5.5 g, 21.9 mmol) and sulfuric acid (1mL, cat.) in ethanol (100 mL) was heated to reflux overnight. The reaction mixture was then cooled and excess ethanol removed under reduced pressure. The resulting solid was taken up in CH₂Cl₂, washed with water, saturated sodium bicarbonate, brine, then dried over magnesium sulfate, filtered and solvent removed under reduced pressure to provide the title compound (5.75 g, 94%). MS (ESI) m/z 279.0 [M+1]⁺, 280.9 [M+1+2]⁺

### Example 9

### SYNTHESIS OF 3-(6-DIFLUOROMETHOXYNAPHTHALEN-2-YL)-5-(5-PYRROLIDIN-1-YLMETHYL-1H-[1,2,4]TRIAZOL-3-YL)-1H-INDAZOLE

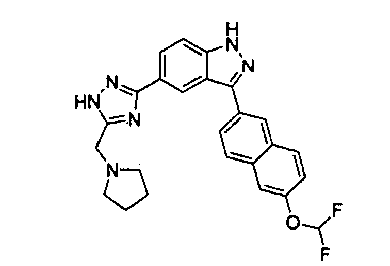

### A. 6-Methoxynaphthalen-2-yl-boronic acid

To a solution of 6-bromo-2-naphthol (10.0 g, 42.9 mmol) in THF (130 mL) at - 78°C was added n-butyllithium (94.4 mmol) and stirred for 45 minutes. Trimethylborate (14.4 mL, 129 mmol) was added and stirred for an additional 45 minutes. Aqueous saturated ammonium chloride (20 mL) was added and the mixture was allowed to warm to ambient temperature. Water (40 mL) was added and the layers were partitioned. The aqueous layer was extracted with ethyl acetate and the combined organic extracts were dried (MgSO₄) and concentrated to provide the title compund (7.55 g, 87 %).

### B. 3-(6-Hydroxynaphthalen-2-yl-1-(tetrahydropyran-2-yl)-1H-indazole-5-carbonitrile

The title compound was prepared as described in Example 149 D in WO 02/10137 from 3-bromo-1-perhydro-2H-pyran-2-yl-1H-indazole-5-carbonitrile (Exmaple 149 C in WO 02/10137, 2.00 g, 6.53 mmol) and 6-methoxynaphthalen-2-yl-boronic acid (1.84 g, 9.80 mmol): ES-MS (m/z) 370 [M+1]⁺.

### C. 3-(6-Difluoromethoxynaphthalen-2-yl)-1-(tetrahydropyran-2-yl)-1H-indazole-5-carbonitrile

To a flask was charged 3-(6-hydroxynaphthalen-2-yl-1-(tetrahydropyran-2-yl)-1H-indazole-5-carbonitrile (0.91 g, 2.46 mmol), 20% aqueous NaOH (10 mL), and 3 mL of THF. The stirred mixture was cooled to 0°C and a cold solution of THF (15 mL) saturated with chlorodifluoromethane was added dropwise. The reaction mixture was allowed to warm to ambient temperature and stirred for 24 hours. Water (25 mL) was added and the THF was removed under reduced pressure. The aqueous layer was extracted with ethyl acetate, dried (MgSO₄) and concentrated to provide the title compound (282 mg, 27 %): ES-MS (m/z) 420 [M+1]⁺.

### D. 3-(6-Difluoromethoxynaphthalen-2-yl)-1H-indazole-5-carboximidic acid ethyl ester

To a flask was charged 3-(6-Difluoromethoxynaphthalen-2-yl)-1-(tetrahydropyran-2-yl)-1H-indazole-5-carbonitrile (6.4g, 21.4 mmol) and ethanol (650 mL). HCl (g) was bubbled through the stirring solution until saturated. After 18 hours, mixture was concentrated and slurried with diethyl ether. Solid product was filtered and washed with excess diethyl ether. Product was dried to provide the title compound as the HCl salt (245 mg, 96 %): ES-MS (m/z) 382 [M+1]⁺.

### E. 3-(6-Difluoromethoxynaphthalen-2-yl)-5-(5-pyrrolidin-1-ylmethyl-1H-[1,2,4]triazol-3-yl)-1H-indazole

To a flask was charged 3-(6-difluoromethoxynaphthalen-2-yl)-1H-indazole-5-carboximidic acid ethyl ester (200 mg, 479 mmol), methanol (10 mL), and triethylamine (1.46 mL, 10.5 mmol). After 10 minutes, hydrazide (prepared according to Example 422 A of WO 02/10137, 274 mg, 1.91 mmol) was added and the mixture was heated at 90°C for 18 hours. The mixture was concentrated and purified by preparatory HPLC to provide the title compound (86.3 mg, 39 %): ¹H NMR (DMSO) δ 8.79 (br s, 1H) 8.56 (br s, 1H) 8.30 - 8.03 (cm, 4H) 7.89 - 7.63 (cm, 3H), 7.51 - 7.38 (cm, 2H) 7.36 - 7.16 (cm, 1H) 3.79 (br s, 2H) 2.57 (br s, 4H) 1.73 (br s, 4H); ES-MS (m/z) 461 [M+1]⁺.

### Example 10

### SYNTHESIS OF 3-(6-FLUORO-NAPHTHALEN-2-YL)-5-(5-PYRROLIDIN-1-YLMETHYL-2H-[1,2,4]TRIAZOL-3-YL)-1H-INDAZOLE

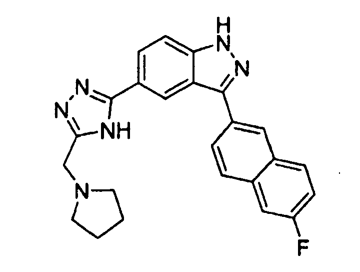

### A. 3-(6-Fluoro-naphthalen-2-yl)-5-(5-pyrrolidin-1-ylmethyl-2H-[1,2,4]triazol-3-yl)-1H-indazole

A solution of 3-(6-fluoro-naphthalen-2-yl)-1*H*-indazole-5-carboximidic acid ethyl ester di-hydrochloric acid salt (460 mg, 1.1 mmol), pyrrolidin-1-yl-acetic acid hydrazide (see WO 02/10137, example 422A) (650 mg, 4.5 mmol), and triethyl amine (3.2 mL, 23.0 mmol) in methanol (6 mL) was heated in a sealed reaction flask on an 100 °C oil bath overnight. Solvent and excess triethyl amine were then removed under reduced pressure and the crude product purified using reverse-phase preparatory HPLC (20-70% acetonitrile +0.1% TFA in H₂O + 0.1% TFA, over 30 min). Fractions containing clean product were neutralized with sodium bicarbonate and product extracted using ethyl acetate. The organic fractions were pooled, dried over magnesium sulfate, filtered, and solvent removed under reduced pressure to provide the title compound (200.2 mg, 43%). ¹H NMR (400 MHz, DMSO-d₆) δ 13.51 (bs, 1H), 8.77 (bm, 1H), 8.59 (bm, 1H), 8.23 (m, 2H), 8.11 (m, 2H), 7.80 (dd, 1H), 7.72 (d, 1H), 7.51 (td, 1H), 3.80 (s, 2H), 2.59 (bm, 4H), 1.73 (quin, 4H); MS (ESI) *m*/*z* 412.8 [M+1]⁺.

### B. 3-(6-Fluoro-naphthalen-2-yl)-1H-indazole-5-carboximidic acid ethyl ester di-hydrochloric acid salt

A solution of 3-[6-fluoro-napthalen-2-yl]-1-(tetrahydro-pyran-2-yl)-1*H*-indazole-5-carbonitrile (500 mg, 1.3 mmol) in ethanol (65 mL) was cooled on a dry ice/isopropanol bath and saturated with HCl (g). The resulting solution was allowed to stir, gradually warming to room temperature, overnight. Excess solvent was removed under reduced pressure and the resulting solid was taken up in Et₂O, sonicated 5 min and filtered to provide the title compound as a pale yellow solid (472.9 mg, 86%). MS (ESI) *m*/*z* 334.2 [free base M+I]⁺.

### C. 3-[6-Fluoro-napthalen-2-yl]-1-(tetrahydro-pyran-2-yl)-1H-indazole-5-carbonitrile

A solution of 2-bromo-6-fluoro-naphthalene (1.2 g, 5.3 mmol), bis(pinacolato)diboron (1.35 g, 5.3 mmol), [1,1'-bis(diphenylphosphino)-ferrocene] dichloro palladium complex with dichloromethane (1:1) (0.43 g, 0.53 mmol), and potassium acetate (1.57 g, 16.0 mmol) in DMF (20 mL) was heated in a sealed reaction flask on an 85 °C oil bath 2h. To this reaction mixture was added 3-bromo-1-(tetrahydro-pyran-2-yl)1H-indazole-5-carbonitrile (see WO 02/10137, example 161D) (1.47 g, 4.8 mmol) and potassium phosphate (3.4 g, 16.0 mmol) and the solution was returned to heat overnight. The cooled solution was filtered through Celite and the filter cake washed with EtOAc. The filtrate was washed with water and brine, dried over magnesium sulfate, filtered and solvent removed under reduced pressure to provide crude product. Purification using silica gel flash column chromatography (1:3 EtOAc:hexanes) provided the title compound (1.1 g, 56%). MS (ESI) *m*/*z* 372.1 [M+1]⁺

### Example 11

### SYNTHESIS OF 6-[5-(5-PYRROLIDIN-1-YLMETHYL-2H-[1,2,4]TRLAZOL-3-YL)-1H-INDAZOL-3-YL]-NAPHTHALENE-2-CARBOXYLIC ACID ETHYL AMIDE

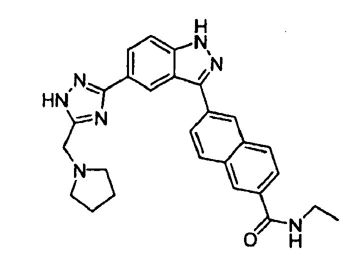

### A. 6-[5-(5-Pyrrolidin-1-ylmethyl-2H-[1,2,4]triazol-3-yl)-1H-indazol-3-yl]-naphthalene-2-carboxylic acid ethyl amide

A solution of 3-(6-ethylcarbamoyl-napthalen-2-yl)-1*H*-indazole-5-carboximidic acid ethyl ester di-hydrochloric acid salt (365 mg, 0.8 mmol), pyrrolidin-1-yl-acetic acid hydrazide (see WO 02/10137, example 422A) (460 mg, 3.2 mmol), and triethyl amine (2.2 mL, 15.8 mmol) in methanol (5 mL) was heated in a sealed reaction flask on an 100 °C oil bath overnight. Solvent and excess triethyl amine were then removed under reduced pressure and the crude product purified using reverse-phase preparatory HPLC (20-60% acetonitrile +0.1% TFA in H₂O + 0.1% TFA, over 30 min). Fractions containing clean product were neutralized with sodium bicarbonate and product extracted using ethyl acetate. The organic fractions were pooled, dried over magnesium sulfate, filtered, and solvent removed under reduced pressure to provide the title compound (65.0 mg, 18%). ¹H NMR (400 MHz, DMSO-d₆) δ 13.55 (bs, 1H), 8.79 (bs, 1H), 8.68 (t, 1H), 8.59 (bs, 1H), 8.51 (s, 1H), 8.24 (bm, 1H), 8.19 (m, 2H), 8.12 (d, 1H), 8.01 (dd, 1H), 7.73 (bm, 1H), 3.79 (bs, 2H), 3.37 (dq, 2H), 2.58 (bs, 4H), 1.73 (bs, 4H), 1.19 (t, 3H); MS (ESI) *m*/*z* 466.3 [M+1]⁺.

### B. 3-(6-Ethylcarbamoyl-oaptbaleo-2-yl)-1H-iodazole-5-carboximidic acid ethyl ester dihydrochloric acid salt

A solution of 6-[5-Cyano-1-(tetrahydro-pyran-2-yl)-1*H*-indazol-3-yl]-naphthalene-2-carboxylic acid ethyl amide (350.0 mg, 0.82 mmol) in ethanol (40 mL) was cooled on a dry ice/isopropanol bath and saturated with HCl (g). The resulting solution was allowed to stir, gradually warming to room temperature, overnight. Excess solvent was removed under reduced pressure and the resulting solid was taken up in Et₂O, sonicated 5 min and filtered to provide the title compound as a pale yellow solid (373.3 mg, 99%). ¹H NMR (400 MHz, DMSO-d₆) δ 9.14 (m, 1H), 8.75 (m, 1H), 8.69 (m, 1H), 8.52 (m, 1H), 8.29 (dd, 1H), 8.21 (m, 2H), 8.02 (m, 2H), 7.86 (d, 1H), 4.67 (q, 2H), 3.36 (m, 2H), 1.56 (t, 3H), 1.19 (t, 3H); MS (ESI) *m*/*z* 387.1 [free base M+1]⁺.

### C. 6-[5-Cyano-1-(tetrabydro-pyrao-2-yl)-1H-iodazol-3-yl]-oaphthalene-2-carboxylic acid ethyl amide

A solution of 6-[5-Cyano-1-(tetrahydro-pyran-2-yl)-1*H*-indazol-3-yl]-naphthalene-2-carboxylic acid (600 mg, 1.5 mmol), HOBT (250 mg, 1.9 mmol), EDCI (350 mg, 1.8 mmol), and ethyl amine (2M in THF, 0.9 mL, 1.8 mmol) in DMF (30 mL) was stirred at room temperature overnight. The solution was then poured into water and filtered to provide a white solid. Purification using silica gel flash column chromatograph (5% methanol in CH₂Cl₂) provided the title compound (387 mg, 60%). ¹H NMR (400 MHz, CDCl₃) δ 8.48 (s, 1H), 8.38 (s, 1H), 8.33 (s, 1H), 8.13 (dd, 1H), 8.03 (m, 2H), 7.90 (dd, 1H), 7.77 (d, 1H), 7.64 (dd, 1H), 6.33 (m, 1H), 5.84 (m, 1H), 4.06 (m, 1H), 3.80 (m, 1H), 3.59 (m, 2H), 2.64 (m, 1H), 2.20 (m, 2H), 1.78 (m, 3H), 1.32 (t, 3H); MS (ESI) *m*/*z* 425.4 [M+1]⁺.

### D. 6-[5-Cyano-1-(tetrahydro-pyran-2-yl)-1H-iodazol-3-yl]-oaphthalene-2-carboxylic acid

A solution of 6-bromo-naphthalene-2-carboxylic acid ethyl ester (2 g, 7.2 mmol), bis(pinacolato)diboron (1.8 g, 7.1 mmol), [1,1'-bis(diphenylphosphino)-ferrocene] dichloro palladium complex with dichloromethane (1:1) (0.6 g, 0.73 mmol), and potassium acetate (2.1 g, 21.4 mmol) in DMF (20 mL) was heated in a sealed reaction flask on an 85 °C oil bath 3h. To this reaction mixture was added 3-bromo-1-(tetrahydro-pyran-2-yl)1H-indazole-5-carbonitrile (2.0 g, 6.5 mmol, prepared as described in International Publication No: WO 02/10137, Example 161D) and potassium phosphate (4.5 g, 21.2 mmol) and the solution was returned to heat overnight. The cooled solution was filtered through Celite and the filter cake washed with EtOAc. The filtrate was washed with water and brine, dried over magnesium sulfate, filtered and solvent removed under reduced pressure to afford crude product. Purification using silica gel flash column chromatography (1:4 EtOAc:hexanes) provided of 6-[5-Cyano-1-(tetrahydro-pyran-2-yl)-1*H-*indazol-3-yl]-naphthalene-2-carboxylic acid ethyl ester (1.02 g, 73%). MS (ESI) *m*/*z* 426.1 [M+1]⁺

A solution of 6-[5-Cyano-1-(tetrahydro-pyran-2-yl)-1*H*-indazol-3-yl]-naphthalene-2-carboxylic acid ethyl ester (1.5 g, 3.5 mmol) and lithium hydroxide monohydrate (0.6 g, 14.3 mmol) in THF (150 mL) and water (20 mL) was stirred at room temperature approximately 96 hours. Excess THF was then removed under reduced pressure and the aqueous solution neutralized with saturated ammonium chloride. The resulting precipitate was recovered by filtration and dried to provide the title compound (1.4 g, 100%). ¹H NMR (400 MHz, DMSO-d₆) δ 9.01 (m, 1H), 8.75 (m, 1H), 8.65 (m, 1H), 8.27 (overlapping m, 3H), 8.06 (m, 2H), 7.88 (dd, 1H), 6.08 (m, 1H), 3.88 (m, 2H), 2.10 (m, 2H), 1.79 (m, 1H), 1.65 (m, 2H); MS (ESI) *mlz* 398.1 [M+1]⁺

### Example 12

### SYNTHESIS OF 3-[6-(3-METHYLBUTOXY)-NAPHTHALEN-2-YL]-5-(5-PYRROLIDIN-1-YLMETHYL-1H-[1,2,4]TRIAZOL-3-YL)-1H-INDAZOLE

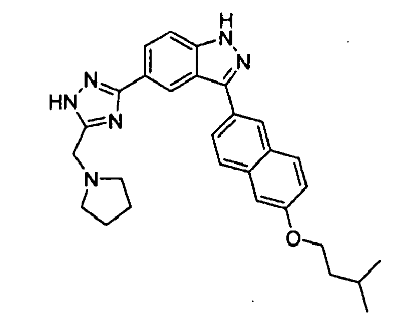

### A. 2-Bromo-6-(3-methylbutoxy)-naphthalene

To a flask was charged 6-bromo-2-naphthol (5.0 g, 21.5 mmol) and DMF (125 mL). NaH (257 mg, 60 % dispersion in mineral oil, 6.44 mmol) was added, followed by a solution of 1-chloro-3-methylbutane (3.1 mL, 25.7 mmol) in DMF (25 mL). The flask contents were heated at 55°C for 24 hours, after which time the mixture was concentrated. Purification was carried out by flash chromatography using 20 % ethyl acetate in hexanes as the eluent to provide the title compound (5.81 g, 92 %): ¹H NMR (DMSO) δ 8.12 (s, 1H) 7.78 (q, 2H) 7.58 (dd, 1H) 7.39 (s, 1H) 7.20 (dd, 1H) 4.09 (t, 2H) 1.81 (m, 1H) 1.68 (m, 2H) 0.98 (d, 6H).

### B. 6-Isobutoxynaphthalen-2-yl-boronic acid

To a solution of 2-bromo-6-(3-methylbutoxy)-naphthalene (4.10g, 14.0 mmol) in THF (130 mL) at -78°C was added n-butyllithium (16.8 mmol) and stirred for 45 minutes. Trimethylborate (4.7 mL, 42.0 mmol) was added and stirred for an additional 45 minutes. Aqueous saturated ammonium chloride (20 mL) was added and the mixture was allowed to warm to ambient temperature. Water (40 mL) was added and the layers were partitioned. The aqueous layer was extracted with ethyl acetate and the combined organic extracts were dried (MgSO₄) and concentrated to provide the title compund (3.54 g, 98 %). ES-MS (m/z) 286 [M+1]⁺.

### C. 3-[6-(3-Methylbutoxy)-naphthalen-2-yl]-1-(tetrahydropyran-2-yl)1H-indazole-5-carbonitrile

The title compound (1.22 g, 50 %) was prepared as described in Example 149 D in WO 02/10137 from 3-bromo-1-perhydro-2H-pyran-2-yl-1H-indazole-5-carbonitrile (Example 149 C in WO 02/10137, 573 mg, 1.87 mmol) and 6-isobutoxynaphthalen-2-yl-boronic acid (725 mg, 2.81 mmol): Rf = 0.22 (9:1 hexanes/ethyl acetate).

### D. 3-[6-(3-Methylbutoxy)-naphthalen-2-yl]-1H-indazole-5-carboximidic acid ethyl ester

To a flask was charged 3-[6-(3-methylbutoxy)-naphthalen-2-yl]-1H-indazole-5-carboximidic acid ethyl ester (3.31 g, 12.5 mmol) and ethanol (650 mL). HCl (g) was bubbled through the stirring solution until saturated. After 18 hours, mixture was concentrated and slurried with diethyl ether. Solid product was filtered and washed with excess diethyl ether. Product was dried to provide the title compound as the HCl salt (5.03 g, 92 %): ES-MS (m/z) 402 [M+1]⁺.

### E. 3-[6-(3-Methylbutoxy)-naphthalen-2-yl]-5-(5-pyrrolidia-1-ylmethyl-1H-[1,2,4]triazol-3-yl)-1H-indazole

To a flask was charged 3-[6-(3-methylbutoxy)-naphthalen-2-yl]-1H-indazole-5-carboximidic acid ethyl ester (800 mg, 1.83 mmol), methanol (10 mL), and triethylamine (1.46 mL, 10.5 mmol). After 10 minutes, the hydrazide prepared as described in Example 422 A of International Publication No. WO 02/10137 (1.05 g, 7.31 mmol):was added and the mixture was heated at 90°C for 18 hours. The mixture was concentrated and purified by preparatory HPLC to provide the title compound (136 mg, 15 %)): ¹H NMR (DMSO) δ 8.75 (br s, 1H) 8.45 (br s, 1H) 8.18 - 8.06 (cm, 2H) 8.05 - 7.93 (cm, 2H) 7.70 (d, 1H) 7.42 (d, 1H) 7.23 (dd, 1H) 4.15 (t, 2H) 3.83 (s, 2H) 2.63 (br s, 4H) 1.95 - 1.74 (m, 1H) 1.80 - 1.63 (cm, 4H) 0.97 (d, 6H); ES-MS (m/z) 481 [M+1]⁺.

### Example 13

### SYNTHESIS OF 3-(6-METHOXYNAPHTHALEN-2-YL)-5(5-PIPERIDIN-1-YLMETHYL-1H-[1,2,4]TRIAZOL-3-YL)-1H-INDAZOLE

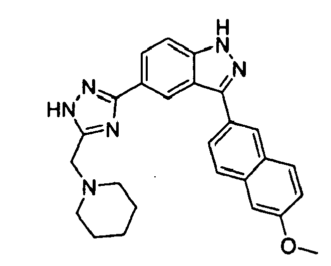

### A. Piperadin-1-yl-acetic acid ethyl ester

The title compound (1.01 g, 32 %) was prepared as described in Example 20, step A, using piperidine (1.72 g, 20.2 mmol) and methyl bromoacetate (2.95 g, 19.3 mmol): R_{f} = 0.6 (ethyl acetate).

### B. Piperidin-1-yl-acetic acid hydrazide

A solution of piperadin-1-yl-acetic acid ethyl ester (1.00 g, 6.36 mmol), ethanol (20 mL) and hydrazine (224 mg, 7.00 mmol) was stirred at 90°C for 18 hours. The mixture was concentrated and dried to provide the title compound (0.94 g, 94 %). R_{f} = 0.8 (ethyl acetate).

### C. 3-(6-Methoxynaphthalen-2-yl)-5(5-piperadin-1-ylmethyl-1H-[1,2,4]triazol-3-yl)-1H-indazole

The title compound (57 mg, 27 %) was prepared as described in Example 12, step E from 3-(6-methoxynaphthalen-2-yl)-1H-indazole-5-carboximidic acid ethyl ester (186 mg, 488 mmol) and piperidin-1-yl-acetic acid hydrazide (307 mg, 1.95 mmol): ¹H NMR (DMSO) δ 8.76 (br d, 1H) 8.46 (br d, 1H), 8.10 (br d, 2H) 8.02 (t, 2H) 7.69 (br s, 1H) 7.41 (s, 1H) 7.24 (br d, 1H) 3.92 (s, 3H) 3.53 (br s, 2H) 2.42 (br s, 4H) 1.53 (br s, 4H) 1.39 (br s, 2H); ES-MS (m/z) 439 [M+1]⁺.

### Example 14

### SYNTHESIS OF 3-(6-METHOXYNAPHTHALEN-2-YL)-5-(5-MORPHOLIN-4-YLMETHYL-1H-[1,2,4]TRIAZOL-3-YL)-1H-INDAZOLE

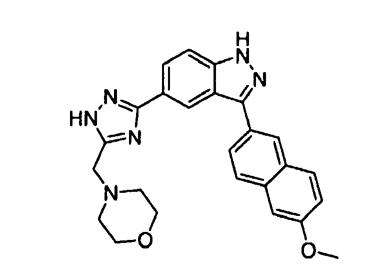

### A. Morpholin-4-yl-acetic acid hydrazide

A solution of methyl morpholinoacetate (2.43g, 15.3 mmol), ethanol (20 mL) and hydrazine (0.53 mL, 16.8 mmol) was stirred at 90°C for 18 hours. The mixture was concentrated and dried to provide the title compound (2.30g, 95%): ES-MS (m/z) 160 [M+1]⁺.

### B. 3-(6-methoxynaphthalen-2-yl)-5-(5-morpholin-4-ylmethyl-1H-[1,2,4]triazol-3-yl)-1H-indazole

The title compound (481mg, 42%) was prepared as described in Example 13, step E from 3-(6-methoxynaphthalen-2-yl)-1H-indazole-5-carboximidic acid ethyl ester (1.0 g, 2.62 mmol) and morpholin-4-yl-acetic acid hydrazide (1.67g, 10.5mmol). ¹H NMR (CD₃OD) δ 8.82 (s, 1H) 8.42 (s, 1H) 8.08 (d, 2H) 7.93 (t, 2H) 7.65 (br s, 1H) 7.30 (s, 1H) 7.21 (d, 1H) 4.15 (s, 3H) 3.72 (m, 6H) 2.59 (br s, 4H); ES-MS (m/z) 441 [M+1]⁺.

### Example 15

### SYNTHESIS OF 3-(6-METHOXYNAPHTHALEN-2-YL)-5-[5-(2-METHYLPYRROLIDIN-1-YLMETHYL)-1H[1,2,4]TRIAZOL-3-YL]-1H-INDAZOLE

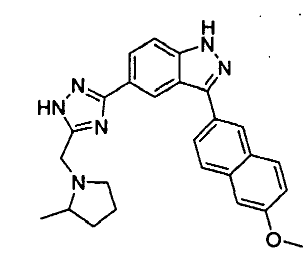

### A. (2-Methylpyrrolidin-1-yl)-acetic acid ethyl ester

To a solution of 2-methylpyrtolidine (racemic, 876 mg, 10.3 mmol) in THF (10 mL) at 0°C was added triethylamine (0.5 mL, 3.59 mmol) and methyl bromoacetate (1.57 g, 10.3 mmol) dropwise. After 18h, the mixture was concentrated and dissolved in dichloromethane. The organic layer was washed with aqueous sodium bicarbonate, dried (MgSO₄ and concentrated to provide crude product. Purification was carried out by flash chromatography, using ethyl acetate as the eluent, to provide the title compound (0.76 g, 47 %): ¹H NMR (DMSO) δ 3.65 (s, 3H) 3.55 (d, 1H) 3.05 (d, 1H) 3.08 - 3.00 (m, 1H) 2.48 (sextet, 1H) 2.28 (q, 1H) 1.93 - 1.80 (cm, 1H) 1.75 - 1.53 (cm, 2H) 1.37 - 1.17 (cm, 1H) 0.98 (d, 3H).

### B. (2-Methylpyrrolodin-1-yl)-acetic acid hydrazide

The title compound (1.49 g, 97 %) was prepared as described in Example 13, step B using (2-methylpyrrolidin-1-yl)-acetic acid ethyl ester (1.54 g, 9.80 mmol).

### C. 3-(6-Methoxynaphthalen-2-yl)-5-[5-(2-methylpyrrolidin-1-ylmethyl)-1H-[1,2,4]triazol-3-yl]-1H-indazole

Compound 22 (56.0 mg, 23 %) was prepared as described in Example 13, step E from 3-(6-methoxynaphthalen-2-yl)-1H-indazole-5-carboximidic acid ethyl ester (216 mg, 0.57 mmol) and (2-methylpyrrilodin-1-yl)-acetic acid hydrazide (356 mg, 2.27 mmol). Product was obtained as a racemic mixture: ¹H NMR (CDCl₃) δ 8.72 (br s, 1H) 8.44 (br s, 1H) 8.09 (d, 2H) 8.01 (t, 2H) 7.70 (br s, 1H) 7.41 (s, 1H) 7.24 (d, 1H) 4.15 - 3.95 (m, 1H) 3.91 (s, 3H) 3.55 (br s, 1H) 2.96 (br s, 1H) 2.30 (br s, 1H) 1.91 (br s, 1H) 1.65 (br s, 2H) 1.36 (br s, 1H) 1.25 - 1.02 (cm, 4H) ; ES-MS (m/z) 439 [M+1]⁺.

### Example 16

### SYNTHESIS OF 5-(5-CYCLOPENTYL-1H-[1,2,4]TRIAZOL-3-YL)-3-(6-METHOXY-NAPHTHALEN-2-YL)-1H-INDAZOLE

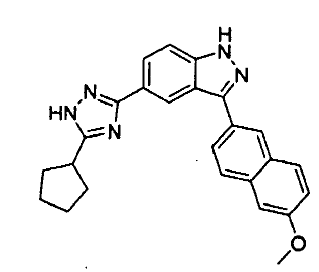

To a solution of 3-(6-methoxy-naphthalen-2yl)-1*H*-indazole-5-carboximidic acid ethyl ester dihydrochloride salt (0.330g, 0.789 mmol, prepared as described in International Publication No. WO 02/10137, Example 423B) in methanol (5ml) was added cyclopentanecarboxylic acid hydrazide (0.404g, 3.16 mmol), then triethylamine (2.2ml, 15.8 mmol). The reaction was stirred at 100°C in a sealed pressure vessel for 2.5h. The mixture was concentrated and purified by HPLC to provide the title compound (195mg, 60%). ¹H NMR (DMSO, *d₆*) δ 8.67 (s, 1H), 8.40 (s, 1H), 8.07-7.98 (m, 4H), 7.66-7.64 (m, 1H), 7.40 (s, 1H), 7.24-7.21 (m, 2H), 3.92 (s, 3H), 2.10-1.59 (m, 9H); ES-MS (m/z) 410 [M+1].

### Example 17

### SYNTHESIS OF 3-(6-METHOXY-NAPHTHALEN-2-YL)-5-[5-(2-PYRROLIDIN-1-YL-ETHYL)-1H-[1,2,4]TRIAZOL-3-YL]-1H-INDAZOLE

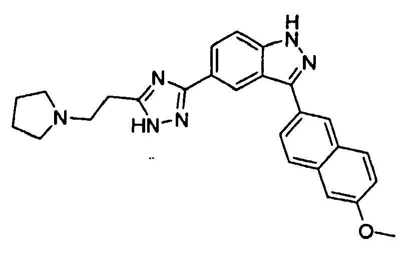

### 3-(6-Methoxy-naphthalen-2-yl)-5-[5-(2-pyrrolidin-1-yl-ethyl)-1H-[1,2,4]triazol-3-yl]-1H-indazole

The title compound (640 mg, 28% yield) was prepared as described in Example 16 using 1-pyrrolidinepropanoic acid hydrazide (1.64 g, 10.4 mmol). ¹H NMR (DMSO-d₆) δ 14.24 (br s, 1H), 13.5 (s, 1H), 8.84 (s, 1H), 8.52 (s, 1H), 8.18-8.04 (m, 3H), 7.98 (d, 1H), 7.73 (d, 1H), 7.41 (s, 1H), 7.24 (dd, 1H), 3.91 (s, 3H), 3.5-3 (m, 6H), 1.9 (br s, 4H), 1.8 (m,2H). ES-MS (m/z) 439 [M+1]⁺.

### Example 18

### SYNTHESIS OF 3-(-6-METHOXY-NAPHTHALEN-2-YL)-5-[5-(1-METHYL-PIPERIDIN-4-YLMETHYL)-1H-[1,2,4]TRIAZOL-3-YL]-1H-INDAZOLE

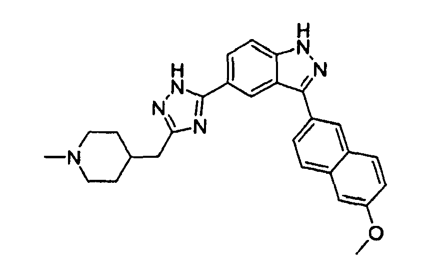

### A. 3-(-6-Methoxy-naphthalen-2-yl)-5-[5-(1-methyl-piperidin4-ylmethyl)-1H-[1,2,4]triazol-3-yl]-1H-indazole

A solution of 3-(6-methoxy-napthalen-2-yl)-1*H*-indazole-5-carboximidic acid ethyl ester di-hydrochloric acid salt (2 g, 4.8 mmol, prepared as described in International Publication No. WO 02/10137, example 423B), (1-methyl-piperidin-4-yl)-acetic acid hydrazide (3.2 g, 18.7 mmol), and triethyl amine (13.4 mL, 96.1 mmol) in methanol (30 mL) was heated in a sealed reaction flask on an 100 °C oil bath overnight. Solvent and excess triethyl amine were then removed under reduced pressure and the crude product purified using reverse-phase preparatory HPLC (20-60% acetonitrile + 0.1 % TFA in H₂O + 0.1 % TFA, over 30 min). Fractions containing clean product were neutralized with sodium bicarbonate and product extracted using ethyl acetate. The organic fractions were pooled, dried over magnesium sulfate, filtered, and solvent removed under reduced pressure to provide the title compound (110 mg, 5%). ¹H NMR (300 MHz, DMSO-d₆) δ 8.73 (s, 1H), 8.45 (s, 1H), 8.06 (m, 4H), 7.69 (d, 1H), 7.41 (d, 1H), 7.24 (dd, 1H), 3.92 (s, 3H), 2.81 (m, 2H), 2.67 (d, 2H), 2.20 (s, 3H), 1.98 (m, 2H), 1.75 (m, 1H), 1.65 (m, 2H), 1.29 (m, 2H); MS (ESI) *m*/*z* 453.2 [M+1]⁺.

### B. (1-Methyl-piperidin-4-yl)-acetic acid hydrazide

A solution of (1-methyl-piperidin-4-yl)-acetic acid ethyl ester (4.5 g, 24.3 mmol) and hydrazine (0.9 mL, 28.7 mmol) in ethanol (20 mL) was heated in a sealed flask on a 100 °C oil bath for 48 hours. The solution was then evaporated to dryness to provide the title compound (4.6 g, 99%). MS (ESI) *m*/*z* 172.1 [M+1]⁺.

### Example 19

### SYNTHESIS OF (5-{3-[3-(3-METHOXY-CYCLOHEXA-2,4-DIENYLIDENE)-1-VINYL-PROPENYL]-1H-INDAZOL-5-YL}-2H-[1,2,4]TRIAZOL-3-YL)-METHANOL

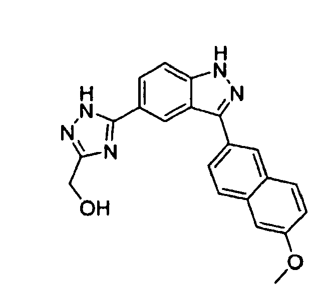

### (5-{3-[3-(3-Methoxy-cyclohexa-2,4-dienylidene)-1-vinyl-propenyl]-1H-indazol-5-yl}-2H-[1,2,4]triazol-3-yl)-methanol

In a sealed tube, 3-[3-(3-methoxy-cyclohexa-2,4-dienylidene)-1-vinyl-propenyl]-1*H*-indazole-5-carboximidic acid ethyl ester dihydrochloride (0.330 mg, 0.8 mmol, prepared as described in International Publication No. WO 02/10137, example 423B) was suspended in methanol (7 mL). Triethyl amine was added (2.23 mL, 16.0 mmol, 20 eq.) followed by 2-hydrazino-prop-2-en-ol (0.288 mg, 3.2 mmol, 4 eq). The reaction mixture was placed under nitrogen and heated to 95°C for 3 hours (reaction was monitored by LC-MS). The crude mixture was purified by preparatory HPLC (20-65% acetonitrile in water) and the title compound was isolated as its free base (0.045 g, 15% yield): ¹H NMR (DMSO-*d₆*, for major tautomer) δ 13.48 (s, 1H), 13.38 (s, 1H), 8.7 (s, 1H), 8.4 (s, 1H), 8.1-7.9 (m, 3H), 7.6 (d, 1H), 7.4 (s, 1H), 7.2 (m, 2H), 5.7 (t, 1H), 4.6 (d, 2H), 3.9 (s, 3H); ES-MS (m/z) 372.

### Example 20

### SYNTHESIS OF 3-(6-METHOXYNAPHTHALEN-2-YL)-5-[5-(1-PYRROLIDIN-1-YL-ETHYL)-1H-[1,2,4]TRIAZOL-3-YL]-1H-INDAZOLE

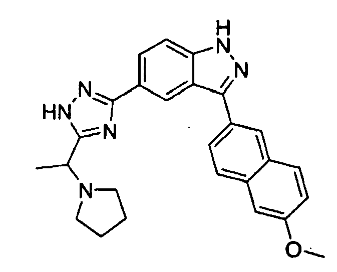

### A. 2-Pyrrolidin-1-yl-propionic acid ethyl ester

To a solution of pyrrolidine (20.0 g, 120 mmol) in THF (10 mL) at 0°C was added triethylamine (0.5 mL, 3.59 mmol) and methyl 2-bromopropionate (8.52 g, 120 nunol) dropwise. After 18h, the mixture was concentrated and dissolved in dichloromethane. The organic layer was washed with aqueous sodium bicarbonate, dried (MgSO₄) and concentrated to provide crude product. Purification was carried out by flash chromatography, using ethyl acetate as the eluent, to provide the title compound (0.99 g, 40 %): ¹H NMR (DMSO) δ 3.62 (s, 3H) 3.23 (q, 1H) 2.63 - 2.46 (m, 4H) 1.75 - 1.60 (m, 4H) 1.22 (d, 3H).

### B. 2-Pyrrolidin-l-yl-propionic acid hydrazide

The title compound (7.52 g, 99 %) was prepared as desccribed in Example 13, step B using 2-pyrrolidin-1-yl-propionic acid ethyl ester (7.0 g, 44.5 mmol) and hydrazine (1.54 mL, 49.0 mmol): ¹H NMR (DMSO) δ 8.90 (s, 1H) 2.84 (q, 1H) 2.58 - 2.32 (cm, 4H) 1.73 - 1.55 (cm, 4H) 1.14 (d, 3H).

### C. 3-(6-methoxynaphthalen-2-yl)-5-[5-(1-pyrrolidin-1-yl-ethyl)-1H-[1,2,4]triazol-3-yl]-1H-indazole

The title compound (24.2 mg, 7 %) was prepared as described in Example 13, step E, from 3-(6-methoxynaphthalen-2-yl)-1H-indazole-5-carboximidic acid ethyl ester (300 mg, 0.79 mmol) and hydrazine (495 mg, 3.14 mmol): ¹H NMR (CD₃OD) δ 8.88 (s, 1H) 8.47 (s, 1H) 8.12 (d, 2H) 7.95 (t, 2H) 7.32 (d, 1H) 7.21 (d, 1H) 3.97 (s, 3H) 3.87 (q, 1H) 2.90 - 2.50 (cm, 4H) 1.95 - 1.70 (cm, 4H) 1.60 (d, 3H); ES-MS (m/z) 439 [M+1]⁺.

### Example 21

### SYNTHESIS OF 3-(6-METHOXY-NAPHTHALEN-2-YL)-5-[5-(2-METHYL-PIPERIDIN-1-YLMETHYL)-1H-[1,2,4] TRIAZOL-3-YL]- 1H-INDAZOLE

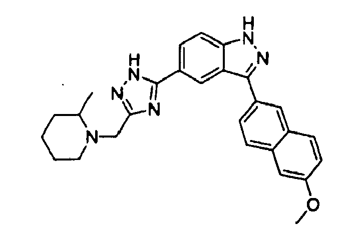

### A. 3-(6-Methoxy-naphthalen-2-yl)-5-[5-(2-methyl-piperidin-1-ylmethyl)-1H-[1,2,4] triazol-3-yl]-1H-indazole

A solution of 3-(6-methoxy-napthalen-2-yl)-1*H*-indazole-5-carboximidic acid ethyl ester di-hydrochloric acid salt (2.0 g, 4.8 mmol, prepared as described in International Publication No. WO 02/10137, Example 423B), (2-methyl-piperdin-1-yl)-acetic acid hydrazide (3.30 g, 19.3 mmol), and triethyl amine (13.3 mL, 95.4 mmol) in methanol (25 mL) was heated in a sealed reaction flask on an 100 °C oil bath for 48 h. Solvent and excess triethyl amine were then removed under reduced pressure. Initial purification using reverse-phase preparatory HPLC (20-80% acetonitrile + 0.1% TFA in H₂O + 0.1% TFA, over 30 min) provided product of 95% purity. Further purification using silica gel flash column chromatography (10% methanol in CH₂Cl₂) provided the title compound, >99 % pure, (125 mg, 6%). ¹H NMR (300 MHz, CD₃OD) δ 8.94 (s, 1H), 8.54 (s, 1H), 8.22 (m, 2H), 7.99 (m, 2H), 7.73 (d, 1H), 7.38 (d, 1H), 7.22 (dd, 1H), 4.04 (m, 1H), 3.96 (s, 3H), 3.79 (m, 1H), 2.90 (m, 1H), 2.39 (m, 2H), 1.61 (m, 4H), 1.32 (m, 2H), 1.20 (d, 3H); MS (ESI) *m*/*z* 453.3 [M+1]⁺.

### B. (2-Methyl-piperidin-yl)-acetic acid hydrazide

A solution of (2-methyl-piperidin-yl)-acetic acid ethyl ester (5.9 g, 31.8 mmol) and hydrazine (1.2 mL, 38.2 mmol) in ethanol (15 mL) was heated in a sealed flask on a 100 °C oil bath for 48 hours. The solution was then evaporated to dryness to provide the title compound (5.3 g, 99%). MS (ESI) *m*/*z* 172.3 [M+1]⁺.

### C. (2-Methyl-piperidin-yl)-acetic acid ethyl ester

To a solution of ethyl-2-bromo acetate (5.6 mL, 50.5 mmol) and triethyl amine (7.0 mL, 50.2 mmol) in THF (150 mL) was added 2-methyl piperidine (5.9 mL, 50.2 mmol). After stirring 48 h, excess THF was removed under reduced pressure and the resulting solid was taken up in EtOAc, washed with water, saturated sodium bicarbonate, brine, then dried over magnesium sulfate, filtered and solvent removed under reduced pressure to provide the title compound (5.6 g, 60%). ¹H NMR (300 MHz, CDCl₃) δ 4.17 (q, 2H), 3.37 (ABq, 2H), 2.86 (m, 1H), 2.52 (m, 2H), 1.63 (overlapping m, 4H), 1.29 (m, 2H), 1.27 (t, 3H), 1.07 (d, 3H).

### Example 22

### SYNTHESIS OF 5-[5-(CIS-2,6-DIMETHYL-PIPERIDIN-1-YLMETHYL)-1H-[1,2,4]TRIAZOL-3-YL]-3-(6-METHOXY-NAPHTHALEN-2-YL)- 1H-INDAZOLE

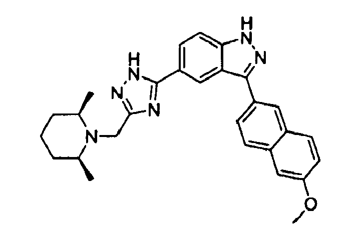

### A. 5-[5-(cis-2,6-Dimethyl-piperidin-1-ylmethyl)-1H-[1,2,4]triazol-3-yl]-3-(6-methoxy-naphthalen-2-yl)-1H-indazole

A solution of 3-(6-methoxy-napthalen-2-yl)-1*H*-indazole-5-carboximidic acid ethyl ester di-hydrochloric acid salt (1.87 g, 4.5 mmol, prepared as described in International Publication No. WO 02/10137, Example 423B), (cis-2,6-dimethyl-piperdin-1-yl)-acetic acid hydrazide (3.30 g, 17.8 mmol), and triethyl amine (12.5 mL, 89.7 mmol) in methanol (25 mL) was heated in a sealed reaction flask on an 100 °C oil bath overnight. Solvent and excess triethyl amine were then removed under reduced pressure. Initial purification using silica gel flash column chromatography (5-15% methanol in CH₂Cl₂) provided crude product. Further purification using reverse-phase preparatory HPLC (20-65% acetonitrile + 0.1% TFA in H₂O + 0.1% TFA, over 30 min). Fractions containing clean product were neutralized with sodium bicarbonate and product extracted using ethyl acetate. The organic fractions were pooled, dried over magnesium sulfate, filtered, and solvent removed under reduced pressure to provide the title compound (243 mg, 12%). ¹H NMR (300 MHz, CD₃OD) δ 8.84 (m, 1H), 8.45 (s, 1H), 8.12 (m, 2H), 7.94 (d, 2H), 7.69 (d, 2H), 7.32 (s, 1H), 7.21 (dd, 1H), 4.19 (s, 2H), 3.96 (s, 3H), 2.78 (bm, 2H), 1.70 (bm, 3H), 1.42 (bm, 3H), 1.30 (d, 6H); MS (ESI) *m*/*z* 467.5 [M+1]⁺.

### B. (cis-2,6-Dimethyl-piperidin-yl)-acetic acid hydrazide

A solution of (*cis*-2,6-Dimethyl-piperidin-yl)-acetic acid ethyl ester (3.6 g, 18.1 mmol) and hydrazine (0.73 mL, 23.3 mmol) in ethanol (15 mL) was heated in a sealed flask on a 100 °C oil bath for 48 hours. The solution was then evaporated to dryness to provide the title compound (3.36 g, 99%). MS (ESI) *m*/*z* 186.3 [M+1]⁺.

### C. (cis-2,6-Dimethyl-piperidin-yl)-acetic acid ethyl ester

To a solution of ethyl-2-bromo acetate (4.9 mL, 44.2 mmol) and triethyl amine (6.2 mL, 44.5 mmol) in THF (100 mL) was added *cis*-2,6-dimethyl piperidine (5.95 mL, 44.2 mmol). After stirring 12 h, excess THF was removed under reduced pressure and the resulting solid was taken up in CH₂Cl₂, washed with water, saturated sodium bicarbonate, brine, then dried over magnesium sulfate, filtered and solvent removed under reduced pressure to provide the title compound (3.72 g, 35%). ¹H NMR (300 MHz, CDCl₃) δ 4.15 (q, 2H), 3.57 (s, 2H), 2.82 (m, 2H), 1.71-1.27 (overlapping m, 6H), 1.27 (t, 3H), 1.11 (d, 6H).

### Example 23

### SYNTHESIS OF 5-(5-AZEPAN-1-YLMETHYL-2H-[1,2,4]TRIAZOL-3-YL-(6-METHOXY-NAPTHALEN-2-YL)-1H-INDAZOLE

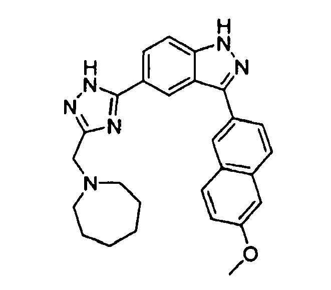

### A. Azepan-1-yl-acetic acid ethyl ester

To a solution of hexamethyleneimine (6.0 g, 60.6 mmol) in THF was added ethyl bromoacetate (10.12 g, 60.6 mmol) and triethylamine (8.4 ml, 60.6 mmol). The solution was stirred at room temperature for 16 hours. The solution was then condensed and extracted with 5% sodium bicarbonate and methylene chloride. The extracts were dried over magnesium sulfate, filtered and concentrated to provide the title compound (10.26 g, 93%). ¹H- NMR (CDCl₃) δ 4.17 (q, 2H), 3.38 (s, 2H), 2.75 (m, 4H), 1.62 (s, 8H), 1.27 (t, 3H).

### B. Azepan-1-yl-acetic acid hydrazide

To a solution of Azepan-1-yl-acetic acid ethyl ester (10.26 g, 55.55 mmol) in ethanol (130 mL) was added hydrazine (2.18 ml, 69.45 mmol). The mixture was allowed to stir at 80°C for 18 hours. An NMR of the solution is obtained to assure product formation. Once confirmed, the reaction mixture is condensed under reduced pressure to provide the title compound (5.4 g, 57%). ¹H- NMR (CDCl₃) δ 8.23 (s, 1H), 3.85 (s, 2H), 3.20 (s, 2H), 2.66 (m, 4H), 1.62 (m, 8H).

### C. 5-(5-Azepan-1-ylmethyl-2H-[1,2,4,]triazol-3-yl)-3-(6-methoxy-naphthalen-2-yl)-1H-indazole

To a mixture containing Azepan-1-yl-acetic acid hydrazide (5.33 g, 31.18 mmol) in methanol (150 mL) was added 5-(5-Azepan-1-ylmethyl-2H-[1,2,4,]triazol-3-yl)-3-(6-methoxy-naphthalen-2-yl)-1H-indazole (6.0 g, 12.47 mmol) and triethylamine (19 mL, 187 mmol). The solution was allowed to stir at 95°C for 18 hours. The mixture was condensed under reduced pressure and extracted with water and methylene chloride. The extracts were dried over magnesium sulfate, filtered and condensed to provide the title compound. ¹H-NMR (CDCl₃) δ 8.87 (s, 1H), 8.43 (s, 1H), 8.21 (dd, 1H), 8.13 (dd, 1H), 7.88 (d, 1H), 7.85 (d, 1H), 7.56 (d, 2H), 7.17 (m, 2H), 3.98 (s, 2H), 3.95 (s, 3 H), 2.80 (t, 4H), 1.67 (m, 8H); ES-MS (m/z) 453 [M+1]⁺.

### Example 24

### SYNTHESIS OF 3-(6-METHOXY-NAPTHALEN-2-YL)-5-PIPERAZIN-Y-YLMETHYL-2H-[1,2,4,TRIAZOL-3-YL)-1H-INDAZOLE

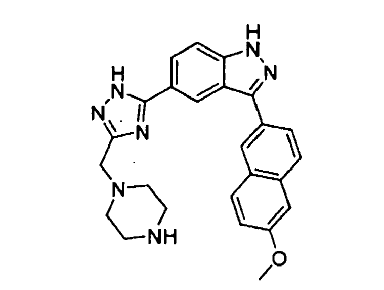

### A. (4-Benzyl-piperazin-1-yl)-acetic acid hydrazide

The title compound was prepared as described in Example 13, step B, using 1-benzyl-4-(ethoxycarbonylmethyl)piperazine (11.79g, 44.93 mmol) to provide the title compound (11.2 g, 100%). ¹H- NMR (CDCl₃) δ 8.15 (s, 1H), 7.3 (m, 5H), 3.85 (s, 2H), 3.5 (s, 2H), 3.1 (s, 2H), 2.45 (m 8H).

### B. 5-[5-(4-Benzyl-piperazin-1-ylmethyl)-2H-[1,2,4,]triazol-3-yl]-3-(6-methoxy-napthalen-2-yl)-1H-indazole

The title compound was prepared as described in Example 13, step C, using (4-Benzyl-piperazin-1-yl)-acetic acid hydrazide (5.19 g, 20.92 mmol), except the crude material was purified via silica gel chromatography (6.8g, 100%). ES-MS (m/z) 528 [M+1]⁺.

### C. 3-(6-Methoxy-naphthalen-2-yl)-5-(5-piperazin-1-ylmethyl-2H-[1,2,4,]triazol-3-yl)-1H-indazole

A suspension of 3-(6-Methoxy-naphthalen-2-yl)-5-(5-piperazin-1-ylmethyl-2H-[1,2,4,] triazol-3-yl)-1H-indazole (5 g, 9.45 mmol) and palladium hydroxide (40% by weight, 2.0g) in ethyl acetate (125 mL), methanol (20 mL) and acetic acid (10 mL) was stirred under hydrogen at room temperature for 18 hours. The solution was filtered through celite and washed with ethyl acetate. The filtration was concentrated and the resulting oil was purified via preparative HPLC (30-80% acetonitrile / water, 60mL/min.) to provide the title compound (1.5 g, 36%). ¹H- NMR (DMSO) δ 8.8 (s, 1H), 8.5 (m, 3H), 8.1 (m, 2H), 8.0 (m, 2H), 7.7 (d, 1H), 7.4 (s, 1H), 7.25 (d, 1H), 3.9 (s, 3H), 3.8 (s, 2H), 3.1 (4H), 2.8 (s, 4 H). ES-MS (m/z) 440 [M+1]⁺.

### Example 25

### SYNTHESIS OF 1-(4-{5-[3-(6-METHOXY-NAPTHALEN-2-YL)-1H-INDAZOL-5-YL-1H-[1,2,4,]TRIAZOL-3-YLMETHYL}-PIPERAZIN-1-YL)-ETHANONE

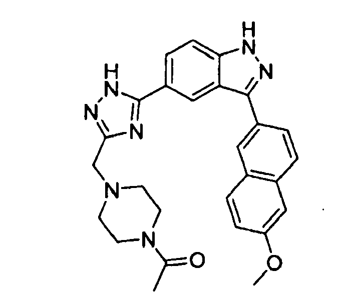

To a solution containing 3-(6-Methoxy-naphthalen-2-yl)-5-(5-piperazin-1-ylmethyl-2H-[1,2,4,]triazol-3-yl)-1H-indazole (1.35 g, 3.07 mmol) in methylene chloride (40 mL) and triethylamine (1.12 mL, 15.35 mmol) was added acetyl chloride (766 uL, 10.74 mmol). The solution stirred for 1 hour until no starting material was present as confirmed by LCMS. The solution was condensed under reduced pressure and the resulting oil subjected to methanol (20 ml) and ammonium hydroxide (1 mL) and allowed to heat at 50°C until only the mono-acetylated product is seen via LCMS. The mixture was condensed under reduced pressure and purified via silica gel to provide the title compound (60 mg, 4.3%). ¹H- NMR (DMSO) δ 8.8 (d, 1H), 8.4 (d, 1H), 8.1 (m, 1H), 8.0 (m, 2H), 7.75 (dd, 1H), 7.4 (s, 1H), 7.2 (t, 1H), 3.9 (s, 2H), 3.75 (s, 1H), 3.6 (s, 1H), 3.45 (m, 4H), 2.4 (m, 2H), 1.95 (s, 3H), 1.2 (s, 3H). ES-MS (m/z) 481 [M+1]⁺.

### Example 26

### SYNTHESIS OF C-{5-[3-(6-METHOXY-NAPTHALEN-2-YL)-1H-[1,2,4,]TRIAZOL-3-YL}-METHYLAMINE

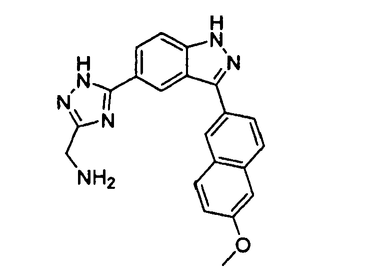

### A. Tert-Butoxycarbonylamino-acetic acid ethyl ester

To a solution containing glycine ethyl ester (5.0 g, 35.82 mmol) in methylene chloride (100 mL) and triethylamine (20 mL, 143.3 mmol) was added di-tert-butyl dicarbonate (19.52 g, 89.55 mmol). The solution was heated at 50°C for four hours. The mixture was then condensed and extracted with sodium bicarbonate (saturated) and ethyl acetate. The organics were combined, dried over magnesium sulfate, filtered and solvent removed under reduced pressure to provide the title compound (5.0 g, 69%). ¹H- NMR (CHCl₃) δ 5.0 (bs, 1H), 4.21 (q, 2H), 3.90 (d, 2H), 1.53 (s, 9H), 1.28 (t, 3H).

### B. Hydrazinocarbonylmethyl-carbamic acid tert-butyl ester

To a mixture containing tert-Butoxycarbonylamino-acetic acid ethyl ester (5.0 g, 26.42 mmol) in ethanol (75 mL) was added hydrazine (2.11 g, 66.0 mmol). The solution was heated to 90°C for 18 hours. Proton NMR was used to determine product formation. The solution was then condensed under reduced pressure to provide the title compound (5.1 g, >100%). ¹H- NMR (CHCl₃) δ 7.25 (bs, 1H), 5.0 (bs, 1H), 3.87 (s, 2H), 3.81 (d, 2H), 1.49 (s, 9H).

### C. {5-[3-(6-Methoxy-naphthalen-2-yl)-1H-indazol-5-yl]-1H-[1,2,4,]triazol-3-ylmethyl}-carbamic acid tert-butyl ester

To a solution containing 3-(6-Methoxy-naphthalen-2-yl)-1H-indazole-5-carboximidic acid ethyl ester (1.0 g, 2.89 mmol) in methanol (30 mL) and triethylamine (6.0 mL, 43.3 mmol) was added Hydrazinocarbonylmethyl- carbamic acid tert-butyl ester (1.64 g, 8.69 mmol). The mixture was allowed to stir at 90°C for 18 hours in a screw-capped reaction vessel. The solution was condensed under reduced pressure and purified via silica gel chromatography (60-90% ethyl acetate/Hexanes) to provide the title compound (0.183 g, 13%). ES-MS (m/z) 471 [M+1]⁺.

### D. C-{5-[3-(6-Methoxy-naphthalen-2-yl)-1H-indazol-5-yl]-1H-[1,2,4,]triazol-3-yl}-methylamine

To an ice cooled mixture containing {5-[3-(6-Methoxy-naphthalen-2-yl)-1H-indazol-5-yl]-1H-[1,2,4,]triazol-3-ylmethyl}-carbamic acid tert-butyl ester (0.793 g, 0.1.68 mmol) in ethanol (100 mL) was added hydrogen chloride gas. The mixture was allowed to stir at room temperature for twenty minutes. Product was monitored by ES-MS. The resulting precipitate was filtered and extracted with minimal water and copious methylene chloride. The organics were combined dried over magnesium sulfate, filtered and solvent removed to provide the title compound (0.100 g, 16%). ¹H- NMR (CHCl₃) δ 8.91 (s, 1H), 8.45 (s, 1H), 8.10 (m, 2H), 7.96 (dd, 2H), 7.81 (d, 1H), 7.32 (d, 1H), 7.21 (dd, 1H), 4.49 (s, 2H), 3.95 (s, 3H). ES-MS (m/z) 371 [M+1]⁺.

### Example 27

### SYNTHESIS OF 3-[6-(2-PYRROLIDIN-1-YL-ETHOXY)-NAPHTHALEN-2-YL]-5-(1H-[1,2,4]TRIAZOL-3-YL)-1H-INDAZOLE

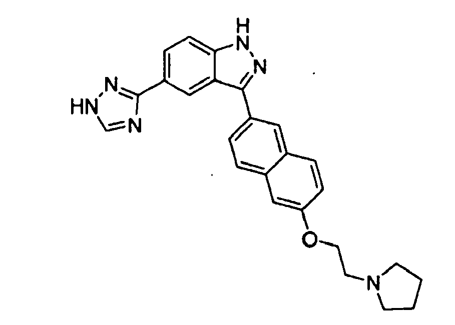

### A. 3-Bromo-1-(tetrahydropyran-2-yl)-1H-indazole-5-carboxylic acid amide

A solution of 3-bromo-1-perhydro-2H-pyran-2-yl-1H-indazole-5-carbonitrile (5.05 g, 16.5 mmol, prepared as described in International Publication No.WO 02/10137, Example 149C) and aqueous sodium hydroxide (6N, 3 mL) in ethanol (15 mL) was heated until all solids dissolved. Hydrogen peroxide (30% aqueous, 5 mL) was added dropwise and stirred for 1 hour at ambient temperature. The mixture was diluted with water and acidified with aqueous HCl, resulting in a white precipitate that was subsequently filtered and washed with excess water. The residue was dried to give the title compound (4.70 g, 88 %): ES-MS (m/z) 324 [M+1]⁺.

### B. 3-Bromo-1-(tetrahydropyran-2-yl)-5-(1H-[1,2,4]triazol-3-yl)-1H-indazole

A solution of 3-bromo-1-(tetrahydropyran-2-yl)-1H-indazole-5-carboxylic acid amide (4.50 g, 13.9 mmol) in dimethylformamide dimethyl acetal (40 mL) was heated to 80°C for two hours. Solvent was removed and the residue was dissolved in glacial acetic acid (40 mL) followed by addition of hydrazine (0.70 mL, 21.8 mmol). The mixture was heated at 110°C for two hours. Water (50 mL) was added and the mixture was allowed to stir at room temperature until a precipitate formed. The precipitate was filtered and dried to give the title compound (3.80 g, 79 %): ES-MS (m/z) 348 [M+1]⁺.

### C. 3-Bromo-1-(tetrahydropyran-2-yl)-5-(1-trityl-1H-[1,2,4]triazol-3-yl)-1H-indazole

To a flask was charged 3-bromo-1-(tetrahydropyran-2-yl)-5-(1H-[1,2,4]triazol-3-yl)-1H-indazole (2.53 g, 7.27 mmol), pyridine (30 mL), triethylamine (1.52 mL, 10.9 mmol) and trityl chloride (3.04 g, 10.9 mmol). After heating for 12 hours at 55°C, methanol (3 mL) was added and the mixture was concentrated. The residue was dissolved in ethyl acetate (30 mL) and washed with aqueous saturated NaHCO₃. The organic layer was dried (MgSO₄) and concentrated to provide the title compound (4.06 g, 95 %): Rf = 0.85 (1:1 ethyl acetate/hexanes).

### D. 3-[6-(2-Pyrrolidin-1-yl-ethoxy)-naphthalen-2-yl]-1-(tetrahydropyran-2-yl)-5-(1-trityl-1H-[1,2,4]triazol-3-yl)-1H-indazole

The title compound (828 mg, 32 %) was prepared as described in International Publication No. WO 02/10137, Example 149 D, using 3-bromo-1-(tetrahydropyran-2-yl)-5-(1-thtyl-1H-[1,2,4]triazol-3-yl)-1H-indazole (2.00 g, 3.39 mmol) and 6-(2-pyrrolidin-1-yl-ethoxy)-naphthalen-2-boronic acid (1.45 g, 5.08 mmol): ES-MS (m/z) 751 [M+1]⁺.

### E. 3-[6-(2-Pyrrolidin-1-yl-ethoxy)-naphthalen-2-yl]-5-(1H-[1,2,4]triazol-3-yl)-1H-indazole

A solution of 3-[6-(2-pyrrolidin-1-yl-ethoxy)-naphthalen-2-yl]-1-(tetrahydropyran-2-yl)-5-(1-trityl-1H-[1,2,4]triazol-3-yl)-1H-indazole (0.83 g, 1.10 mmol) in methanol (50 mL) at 0°C was saturated with HCl (g). After 30 minutes, the mixture was concentrated and purified by preparatory HPLC to provide the title compound (38.0 mg, 8 %): ¹H NMR (DMSO) δ 8.81 (s, 1H) 8.48 (s, 1H) 8.19 - 7.94 (cm, 5H) 7.73 (d, 1H) 7.45 (d, 1H) 7.27 (dd, 1H) 4.32 (t, 2H) 3.13 (br s, 2H) 2.82 (br s, 4H) 1.79 (br s, 4H); ES-MS (m/z) 425 [M+1]⁺.

### Example 28

### SYNTHESIS OF 3-[6-(2-PIPERIDIN-1-YL-ETHOXY)-NAPHTHALEN-2-YL]-5-(1H-[1,2,4]TRIAZOL-3-YL)-1H-TNDAZOLE

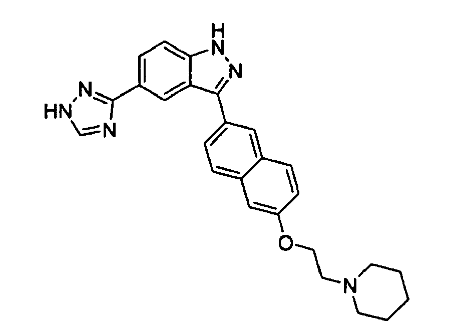

### A. 1-[2-(6-Bromonaphthalen-2-yloxy)-ethyl]-piperidine

A solution of sodium hydroxide (5.74 g, 144 mmol), water (100 mL), THF (100 mL) and 6-bromo-2-naphthol (8.0 g, 35.9 mmol) was stirred for 10 minutes. To this mixture was added 1-(2-chloroethyl)-piperidine-HCl (9.90 g, 53.8 mmol). After heating for 14 hours at 55°C, reaction was removed from heat and the layers were partitioned. The aqueous layer was extracted with ethyl acetate, dried (MgSO₄) and the combined organic layers were concentrated to give crude product. Purification was carried out by flash chromatography using a gradient of 50 % to 100 % ethyl acetate in hexanes as the eluent to give the title compound (4.35 g, 36 %): ES-MS (m/z) 334 [M+1]⁺.

### B. 6-(2-Piperidin-1-yl-ethoxy)-naphthalen-2-boronic acid

The title compound (3.66 g, 97 %) was prepared using the procedure described in Example 12, step B, using 1-[2-(6-bromonaphthalen-2-yloxy)-ethyl]-piperidine (4.20 g, 12.6 mmol).

### C. 3-[6-(2-Piperidin-1-yl-ethoxy)-naphthalen-2-yl]-1-(tetrahydropyran-2-yl)-5-(5-trityl-1H-[1,2,4]triazol-3-yl)-1H-indazole

The title compound (645 mg, 25 %) was prepared as described in International Publication No. WO 02/10137, Example 149C, using 3-Bromo-1-(tetrahydropyran-2-yl)-5-(1-trityl-1H-[1,2,4]triazol-3-yl)-1H-indazole (2.00 g, 3.39 mmol) and 6-(2-piperidin-1-yl-ethoxy)-naphthalen-2-boronic acid (1.52 g, 5.08 mmol): ES-MS (m/z) 765 [M+1]⁺.

### D. 3-[6-(2-Piperidin-1-yl-ethoxy)-naphthalen-2-yl]-5-(1H-[1,2,4]triazol-3-yl)-1H-indazole

A solution of using 3-[6-(2-piperidin-1-yl-ethoxy)-naphthalen-2-yl]-1-(tetrahydropyran-2-yl)-5-(5-trityl-1H-[1,2,4]triazol-3-yl)-1H-indazole (645 mg, 0.84 mmol) in methanol (50 mL) at 0°C was saturated with HCl (g). After 30 minutes, the mixture was concentrated and purified by preparatory HPLC to provide the title compound (52.1 mg, 14 %): ¹H NMR (DMSO) δ 8.81 (s, 1H) 8.47 (s, 1H) 8.23 - 7.91 (cm, 5H) 7.73 (d, 1H) 7.45 (d, 1H) 7.45 (d, 1H) 7.25 (dd, 1H) 4.28 (t, 2H) 2.87 (br s, 2H) 2.60 (br s, 4H) 1.63 - 1.49 (cm, 4H) 1.48 - 1.37 (cm, 2H); ES-MS (m/z) 439 [M+1]⁺.

### Example 29

### SYNETHESIS OF 3-[6-(2-AZEPAN-1-YL-ETHOXY)-NAPHTHALEN-2-YL]-5-(5-METHYL-1H-[1,2,4]TRIAZOL-3-YL)-1H-INDAZOLE

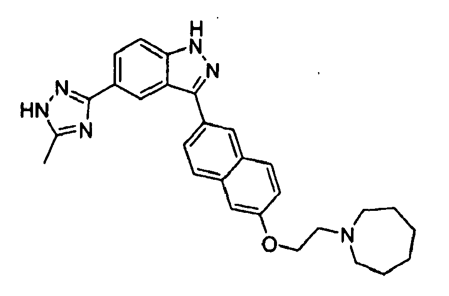

To a solution of 3-[6-(2-azepan-1-yl-ethoxy)-naphthalen-2-yl]-1*H*-indazole-5-carboximidic acid ethyl ester dihydrochloride salt (0.100g, 0.95 mmol) in methanol (5ml) was added acetic acid hydrazide (0.334g, 3.8 mmol), then triethylamine (0.8ml, 5.7 mmol). The reaction was stirred at 100°C in a sealed pressure vessel for 4h. The mixture was concentrated and purified by HPLC to provide the title compound (89mg, 20%). ¹H NMR (DMSO, *d₆*) δ 8.81 (s, 1H), 8.46 (s, 1H), 8.13-7.90 (m, 4H), 7.65-7.62 (d, 1H), 7.36-7.35 (d, 1H), 7.27-7.23 (dd, 1H), 4.43-4.40 (t, 2H), 3.50-3.46 (m, 2H), 3.35-3.28 (m, 4H), 2.48 (s, 3H), 1.87-1.70 (m, 8H); ES-MS (m/z) 467[M+1]⁺.

### Example 30

### 5-(5-METHYL-1H-[1,2,4]TRIAZOL-3-YL)-3-[6-(2-PYRROLIDIN-1-YL-ETHOXY)-NAPTHALEN-2-YL]-1H-INDAZOLE

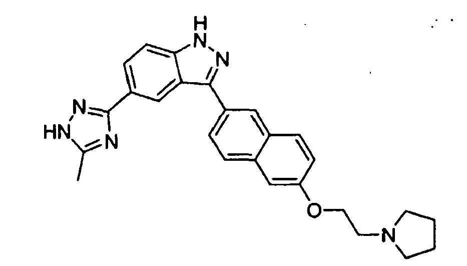

Following the procedure described in Example 30, using 3-[6-(2-pyrrolidin-1-yl-ethoxy)-napthalen-2-yl]-1H-indazole-5-carboximidic acid ethyl ester (0.43g, 1.60mmol), acetic acid hydrazide (0.47g, 6.3mmol), and triethyamine (4.40mL, 31.4mmol) to provide the title compound as a white solid (0.14g, 20% yield). ¹H NMR (DMSO-d₆) δ 8.80(s, 1H), 8.50(s, 1H), 8.30-8.05(m, 5H), 7.75(m, 1H), 7.50(s, 1H), 7.30(d, 2H), 4.30(t, 2H), 2.99(t, 3H), 2.70(m, 4H), 2.60(s, 3H), 1.80(m, 4H). ES-MS (m/z) 439 [M+1].

### Example 31

### SYNTHESIS OF 3-{6-(2,6-DIMETHYL-PIPERIDIN-1-YL)-ETHOXY]NAPHTHALEN-2-YL}-5-(5-METHYL-2H-[1,2,4,]TRIAZOL-3-YL)-1H-INDAZOLE.

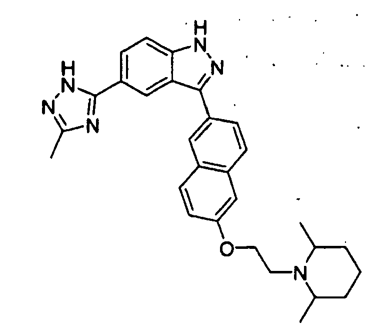

### 3-{6-(2,6-Dimethyl-piperidin-1-yl)-ethoxy]naplhthalen-2-yl}-5-(5-methyl-2H-(1,2,4,)triazol-3-yl)-1H-indazole

The title compound was prepared as described in Example 4.D, using acetic acid hydrazide (0.560 g, 7.57 mmol) and 3-{6-[2-(2,6-Dimethyl-piperidin-l-yl)-ethoxy]-naphthalen-2-yl}-1H-indazole-5-carboximidic acid ethyl ester (0.890 g, 1.89 mmol). The product was isolated by preparative-HPLC (20-50% acetonitrile:water) to provide the title compound as a white solid (94 mg, 10%). ¹H- NMR (CHCl₃) δ 8.82 (s, 1H), 8.39 (s, 1H), 8.17 (d, 1H), 8.10 (d, 1H), 7.84 (t, 2H), 7.58 (d, 1H), 7.24 (d, 2H), 4.17 (bs, 2H), 3.17 (bs, 2H), 2.58 (s, 3H), 1.25 (bm, 14H). ES-MS (m/z) 480 [M+1]⁺.

### Example 32

### SYNTHESIS OF 5-(5-ISOBUTYL-1H-[1,2,4]TRIAZOL-3-YL)-3-[6-(2-PIPERIDIN-1-YL-ETHOXY)-NAPHTHALEN-2-YL]-1H-INDAZOLE

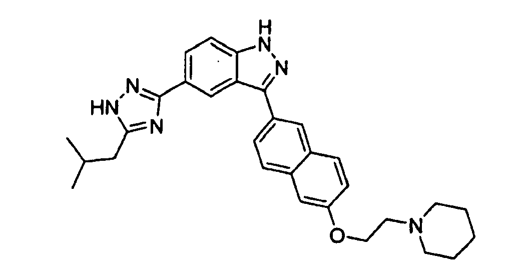

### A. 1-[2-(Bromo-naphthalen-2-yloxy)-ethyl]-piperidine

To a solution of sodium hydroxide (3.6 g, 89.7 mmol) in water (50 mL) was added a solution of 6-bromo-2-naphthol (5 g, 22.4 mmol) in tetrahydrofuran (80 mL). The mixture was stirred at room temperature for ten minutes, after which 1-(2-chloroethyl)piperidine hydrochloride (7 g, 38.0 mmol) was added. The reaction was stirred at 55°C overnight. Tetrahydrofuran was removed *in vacuo* and the aqueous phase was extracted with ethyl acetate. The organic layer was dried over magnesium sulfate, filtered and the solvent was removed *in vacuo.* The crude material was purified by column chromatography (SiO₂, 1:1 hexanes: ethyl acetate) to provide the title compound (7.2 g, 96% yield). ES-MS (m/z) 335 [M+1]⁺.

### B. 6-(2-Piperidin-1-yl-ethoxy)-naphthalene-2-boronic acid

A solution of 1-[2-(bromo-naphthalen-2-yloxy)-ethyl]-piperidine (7.2 g, 21.55 mmol) in tetrahydrofuran (100 mL) was purged with nitrogen and cooled to -78°C. N-Butyllithium (16.2 mL, 25.87 mmol) (1.6 M solution in hexanes) was added over a 10-minute period and the reaction mixture was stirred at -78°C for 1.5 hr. Trimethyl borate (7.24 mL, 64.65 mL) was added, the reaction was further stirred at -78°C for 45 minutes and then quenched by the addition of saturated solution of ammonium chloride. The reaction mixture was allowed to warm to room temperature and tetrahydrofuran was removed *in vacuo.* The aqueous phase was extracted with ethyl acetate. The organic layer was dried over magnesium sulfate, filtered and the solvent was removed *in vacuo.* The crude material was carried over to the next step without further purification (6.5 g, 100% yield). ES-MS (m/z) 300 [M+1]⁺.

### C. 3-[6-(2-Piperidin-1-yl-ethoxy)-naphthalen-2-yl]-1-(tetrahydro-pyran-2-yl)-1H-indazole-5-carbonitrile

To a stirred solution of 3-bromo-1-(tetrahydro-pyran-2-yl)-1H-indazole-5-carbonitrile (4.4 g, 14.37 mmol, prepared as described in International Publication No. WO 02/10137, Example 149C) in dimethoxyethane (250 mL) was added 6-(2-piperidin-1-yl-ethoxy)-naphthalene-2-boronic acid (6.4 g, 21.55 mmol), dichloro[1,1'-bis(diphenylphosphino) ferrocene] palladium (1.17 g, 1.44 mmol) and potassium phosphate (30.5 g, 143.7 mmol) and the mixture was heated at reflux for 24 h. Dimethoxyethane was removed in vacuo, the residue was dissolved in ethyl acetate/water (1:1, 200 mL) and filtered through Celite. The two layers were separated and the aqueous layer was extracted thoroughly with ethyl acetate. The combined organic layers were dried over magnesium sulfate, filtered and the solvent was removed *in vacuo.* The crude material was purified by column chromatography (SiO₂, 1% triethylamine/ ethyl acetate) to provide the title compound (6 g, 87% yield). ES-MS (m/z) 481 [M+1]⁺.

### D. 3-[6-(2-Piperidin-1-yl-ethoxy)-naphthalen-2-yl]-1H-indazole-5-carboximidic acid ethyl ester, hydrochloride

A solution of 3-[6-(2-piperidin-1-yl-ethoxy)-naphthalen-2-yl]-1-(tetrahydro-pyran-2-yl)-1H-indazole-5-carbonitrile (2 g, 5.23 mmol) in 400 mL ethanol was cooled to 0 °C. HCl gas was bubbled through the reaction mixture for 30 minutes. The reaction vessel was sealed and the mixture stirred at room temperature for 20 h. The reaction mixture was diluted with diethyl ether and the precipitate was filtered and washed with diethyl ether. The white solid was dried in a 40 °C vacuum overnight to provide the title compound as a yellow solid (1.8 g, 82% yield). ES-MS (m/z) 443 [M+1]⁺.

### E. 5-(5-isobutyl-1H-[1,2,4]triazol-3-yl)-3-[6-(2-pyrrolidin-1-yl-ethoxy)-naphthalen-2-yl]-1H-indazole

The title compound was prepared according to the procedure described in Example 30, using 3-[6-(2-piperidin-1-yl-ethoxy)-naphthalen-2-yl]-1H-indazole-5-carboximidic acid ethyl ester hydrochloride (1.8 g, 4 mmol), 3-methyl-butyric acid hydrazide (1.39 g, 12 mmol), triethylamine (11.2 mL, 80 mmol) in methanol (20 mL) to yield 550 mg (28% yield) of the title compound after purification by HPLC (20-65% water/ acetonitrile). ¹H NMR (methanol-d₄, 300 MHz) δ 8.85 (s, 1H), 8.5 (s, 1H), 8.13 (ddd, 2H), 8.00 (dd, 2H), 7.71 (dd, 1H), 7.42 (d, 1H), 7.31 (dd, 1H), 4.53 (t, 2H), 3.67 (m, 4H), 3.12 (m, 2H), 2.77 (d, 2H), 2.20 (m, 1H), 1.92 (m, 5H), 1.59 (m, 1H), 1.03 (d, 6H). ES-MS (m/z) 495 [M+1]⁺.

### Example 33

### SYNTHESIS OF 5-(5-ISOBUTYL-2H-[1,2,4,]TRL4ZOL-3-YLO)-3-[6-(3-PIPERIDIN-1-YL-PROPOXY)-NAPHTHALEN-2-YL]-1H-INDAZOLE

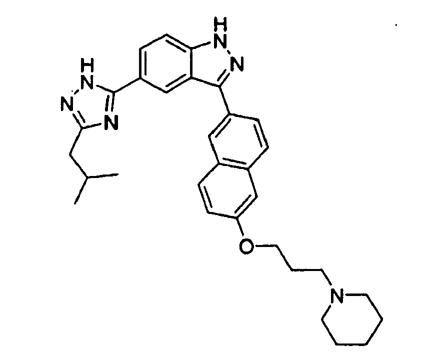

### A. 1-[3-(6-Bromo-napthalen-2-yloxy)-propyl]-piperidine

To a solution of 6-bromo-2-naphthol (5.0 g, 44.6 mmol) in tetrahydrofuran (120 mL) was added triphenylphosphine (23 g, 89 mmol) and 1-piperidinepropanol (12.7 g, 44.6 mmol). Diisopropyl Azodicarboxylate (17.5 mL, 89 mmol) was then added drop wise. The solution was allowed to stir for one half hour. The reaction mixture was condensed under reduced pressure and ether: hexanes (1:1) added to precipitate triphenylphosphine. The precipitate was filtered and the filtrate condensed. The resulting crude material was purified by preparative normal phase chromatography to provide the title compound (11.3 g, >100%). ES-MS (m/z) 348/350 [M+1]⁺.

### B. 3-[6-(3-Piperidin-1-yl-propoxy)-napthalen-2-yl]-1-(tetrahydro-pyran-2-yl)-1H-indole-5-carbonitrile

To a mixture of 1-[3-(6-Bromo-napthalen-2-yloxy)-propyl]-piperidine (3.02 g, 8.69 mmol) in dimethylformamide (80 mL) was added bis (pinacolato) diboron (2.20 g, 8.69 mmol), potassium acetate (2.55 g, 26.07 mmol) and [1,1'-bis(diphenylphosphino-ferrocene] complex with dichloromethane (1:1) (0.709 g, 0.869 mmol). The solution was heated to 90°C for three hours. Reaction was monitored by TLC and ES-MS to assure boronate ester formation. 3-Bromo-1-(tetrahydro-pyran-2-yl)-1H-indazole-5-carbonitrile (2.39 g, 7.82 mmol) and potassium phosphate (5.52 g, 26.07 mmol) added and heating continued for 18 hours. The solution was filtered through celite and washed with ethyl acetate. The filtrate was condensed the resulting oil purified via silica gel chromatography (5% Methanol/Ethyl Acetate) to provide the title compound (1.50 g, 39%). ES-MS (m/z) 495 [M+1]⁺.

### C. 3-[6-(3-Piperidin-1-yl-propoxy-naphthalen-2-yl]-1H-indazole-5-carboximidic acid ethyl ester

To an ice bath cooled solution of 3-[6-(3-Piperidin-1-yl-propoxy)-napthalen-2-yl]-1-(tetrahydro-pyran-2-yl)-1H-indole-5-carbonitrile (1.45 g, 2.93 mmol) in ethanol (75 mL) was added hydrogen chloride gas until the mixture was saturated. The solution was allowed to stir at room temperature until product formed via ES-MS confirmation. The solution was then condensed under reduced pressure to provide a dihydrochloride salt of the title compound (0.850 g, 55%). ES-MS (m/z) 456 [M+1]⁺.

### D. 5-(5-Isobutyl-2H-[1,2,4,triazol-3-yl)-3[6-(3-piperidin-1-yl-propoxy)-naphthalen-2-[yl]-1H-indazole

To a solution of 3-[6-(3-Piperidin-1-yl-propoxy-naphthalen-2-yl]-1H-indazole-5-carboximidic acid ethyl ester (0.650 g, 1.190 mmol) in methanol (20 mL) was added N-amino-3-methylbutanamide (0.552 g, 4.76 mmol) and triethylamine (2.4 g, 23.8 mmol). The mixture was allowed to stir at 95°C for 18 hours. The solution was condensed under reduced pressure and the resulting oil quenched with sodium bicarbonate (saturated). The aqueous layer was then extracted with ethyl acetate and the subsequent organic layer dried over sodium sulfate and concentrated to an oil. The product was isolated by preparative-HPLC (15-80% acetonitrile:water) to provide the title compound as a white solid (170 mg, 28%). ¹H- NMR (CHCl₃) δ 8.76 (s, 1H), 8.29 (s, 1H), 8.15 (d, 1H), 8.0 (d, 1H), 7.29 (t, 2H), 7.52 (d, 1H), 7.09 (d, 2H), 4.14 (t, 2H), 2.74 (d, 2H), 2.60 (bm, 5H), 2.21 (m, 1H), 2.09 (m, 2H), 1.65 (bm, 8H), 1.03 (d, 6H). ES-MS (m/z) 509 [M+1]⁺

### Example 34

### SYNTHESIS OF 5-(5-ISOBUTYL-1H-[1,2,4]TRIAZOL-3-YL)-3-[6-(2-PYRROLIDIN-1-YL-ETHOXY)-NAPHTHALEN-2-YL]-1H-INDAZOLE

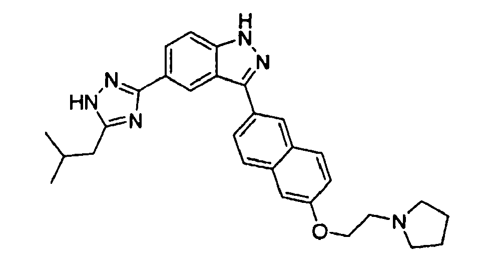

### A. 4-Fluoro-3-[hydroxy-(6-methoxy-naphthalen-2-yl)-methyl]-benzonitrile

To a cooled solution (-78°C) of LDA (24.5 mL, 49 mmol) in THF (40 mL) was added 4-fluorobenzonitrile (5.45 g, 45 mmol) in THF (30 mL) and 6-methoxy-2-naphthaldehyde (9.13g, 49 mmol) in THF (40 mL). The reaction was stirred at -78°C for 1 hour and then allowed to warm to room temperature over a period of 2 hours. The reaction was quenched with ice and THF was removed *in vacuo.* The aqueous solution was then extracted with ethyl acetate. The organic phase was dried over magnesium sulfate, filtered and the solvent was removed *in vacuo.* The crude material was purified by column chromatography. (SiO₂, 4:1 hexanes: ethyl acetate) to provide the title compound (5.5 g, 40% yield). ES-MS (m/z) 308 [M+1]⁺.

### B. 4-Fluoro-3-(6-methoxy-naphthalene-2-carbonyl)-benzonitrile

In a round bottom flask, pyridinium chlorochromate (6.4 g, 29.7 mmol) was taken up in a slurry of dichloromethane (40 mL). 4-Fluoro-3-[hydroxy-(6-methoxy-naphthalen-2-yl)-methyl]-benzonitrile (6.08 g, 19.8 mmol) in dichloromethane (40 mL) was added. The reaction turned black. The mixture was stirred at room temperature for 5 hours, after which it was filtered through a pad of Celite and the solvent removed *in vacuo.* The crude material was purified by column chromatography (SiO₂, 4:1 hexanes: ethyl acetate - 1:1 hexanes: ethyl acetate) to provide the title compound (4.54 g, 75% yield). ES-MS (m/z) 306 [M+1]⁺.

### C. 4-Fluoro-3-(6-hydroxy-naphthalene-2-carbonyl)-benzonitrile

4-Fluoro-3-(6-methoxy-naphthalene-2-carbonyl)-benzonitrile (3.976 g, 13 mmol) was dissolved in dichloromethane (350 mL) and cooled under nitrogen in an ice bath. Boron tribromide (12.28 mL, 130 mmol) was slowly added and the reaction was allowed to stir overnight at room temperature. The reaction was quenched with ice, neutralized with sodium bicarbonate and extracted with dichloromethane. The organic layer was dried over magnesium sulfate, filtered and the solvent was removed *in vacuo.* The crude material was purified by column chromatography (SiO₂, 7:3 hexanes: ethyl acetate) to provide the title compound (2.5 g, 66% yield). ES-MS (m/z) 292 [M+1]⁺.

### D. 4-Fluoro-3-[6-(2-pyrrolidin-1-yl-ethoxy)-naphthalene-2-carbonyl]-benzonitrile

4-Fluoro-3-(6-hydroxy-naphthalene-2-carbonyl)-benzonitrile (2.7 g, 9.3 mmol) was dissolved in dioxane (22 mL). Tetraethylammonium bromide (195 mg, 0.93 mmol) was added, followed by sodium hydroxide (1.12 g in 1.6 mL of water). 1-(2-chloroethyl) pyrrolidine hydrochloride (1.74 g, 10.23 mmol) was added and the reaction mixture was stirred at 55°C for 4 hrs. Solvent was removed *in vacuo* and water was added. Reaction mixture was neutralized to pH=7 and thoroughly extracted with ethyl acetate. The organic layer was dried over magnesium sulfate, filtered and solvent removed *in vacuo* to provide the title compound (3.16 g, 87% yield). ES-MS (m/z) 389 [M+1]⁺.

### E. 3-[6-(2-pyrrolidin-1-yl-ethoxy)-naphthalen-2-yl]-1H-indazole-5-carbonitrile

To a solution of 4-fluoro-3-[6-(2-pyrrolidin-1-yl-ethoxy)-naphthalene-2-carbonyl]-benzonitrile (3.16 g, 8.14 mmol) in toluene (40 mL) hydrazine monohydrate (0.87 mL, 17.9 mmol) was added and the reaction mixture was heated at 65°C overnight. The solvent was removed *in vacuo* and the crude material was purified by column chromatography (SiO₂, 1% triethylamine/ ethyl acetate) to provide the title compound (2.0 g, 65% yield). ES-MS (m/z) 383 [M+1]⁺.

### F. 3-[6-(2-Pyrrolidin-1-yl-ethoxy)-naphthalen-2-yl]-1H-indazole-5-carboximidic acid ethyl ester, hydrochloride

The title compound was prepared according to the procedure described in Example 32, step D using 3-[6-(2-pyrrolidin-1-yl-ethoxy)-naphthalen-2-yl]-1H-indazole-5-carbonitrile (2 g, 5.23 mmol). 2 g of a yellow solid were obtained (89% yield). ES-MS (m/z) 429 [M+1]⁺.

### G. 5-(5-isobutyl-1H-[1,2,4]triazol-3-yl)-3-[6-(2-pyrrolidin-1-yl-ethoxy)-naphthalen-2-yl]-1H-indazole

The title compound was prepared according to the procedure described in Example 30, using 3-[6-(2-pyrrolidin-1-yl-ethoxy)-naphthalen-2-yl]-1H-indazole-5-carboximidic acid ethyl ester (2 g, 4.67 mmol), 3-methyl-butyric acid hydrazide (1.63 g, 14.04 mmol), triethylamine (13.04 mL, 93.44 mmol) in methanol (20 mL) to yield 378.5 mg (17% yield) of the title compound after purification by HPLC (20-65% water/acetonitrile). ¹H NMR (methanol-d₄, 300 MHz) δ 8.82 (s, 1H), 8.47 (s, 1H), 8.10 (dd, 2H), 7.97 (dd, 2H), 7.69 (d, 1H), 7.39 (s, 1H), 7.29 (d, 1H), 4.47 (t, 2H), 3.72 (m, 4H), 3.28 (m, 2H), 2.75 (d, 2H), 2.07 (m, 5H), 1.01 (d, 6H). ES-MS (m/z) 481 [M+1]⁺.

### Example 35

### SYNTHESIS OF 3-[6-(2-AZEPAN-1-YL-ETHOXY)-NAPHTHALEN-2-YL]-5-(5-ISOBUTYL-1H-[1,2,4]TRIAZOL-3-YL)-1H-INDAZOLE

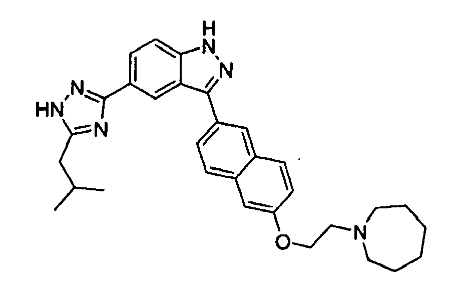

### A. 1-[2-(6-Bromo-naphthalen-2-yloxy)-ethyl]-azepane

To a solution of 6-bromo-2-naphthol (8.0g, 35.9 mmol) and 2-(hexamethyleneimino)ethyl chloride hydrochloride (7.53g, 46.6mmol) in 220 ml of THF was added 24ml of 6N NaOH (143.6mmo1) and stirred for 18h at 50°C. The reaction mixture was extracted into ethyl acetate, washed with water then dried over MgSO₄. Purification was done by column chromatography (hexanes/EtOAc, 1:1) to yield the pale yellow oil (9.34g, 75%); ES-MS (m/z) 348, 350 [M, M+2]⁺.

### B. 3-[6-(2-Azepan-1yl-ethoxy)-naphthalen-2yl]-1H-indazole-5-carbonitrile

To a solution of 1-[2-(6-bromo-naphthalen-2-yloxy)-ethyl]-azepane (11.28g, 32.4mmol) in THF (190ml) at -78°C, was added n-butyllithium (24.3ml, 38.9mmol) then stirred for 1.5h. Trimethyl borate (10.9ml, 97.2mmol) was then added and after 45 min was quenched with ammonium chloride. After warming to room temperature, the reaction mixture was extracted into ethyl acetate, dried over MgSO₄ and concentrated to provide 8.6g of the boronic acid.

The above boronic acid was stirred with 3-bromo-1-(tetrahydropyran-2-yl)-1*H-*indazole-5-carbonitrile (8.6g, 27.5mmol, prepared as described in International Publication No. WO 02/10137, Example 161D), potassium phosphate (19.4g, 91.5mmol) and [1,1'-bis(diphenylphosphino)-ferrocene] dichloro palladium complex with dichloromethane (1:1) in 200ml ofDME at reflux for 12h. The reaction mixture was then filtered through a pad of celite, washing with ethyl acetate. The filtrate was then concentrated and purified by column chromatography (hexanes/EtOAc, 4:1) to provide 6.0g (48%) of the title compound; ES-MS (m/z) 343 [M+1]⁺.

### C. 3-[6-(2-Azepan-1-yl-ethoxy)-naphthalen-2-yl]-1H-indazole-5-carboximidic acid ethyl ester dihydrochloride salt

To a solution of 3-[6-(2-azepan-1yl-ethoxy)-naphthalen-2yl]-1*H*-indazole-5-carbonitrile (6.0g, 12.1mmol) in ethanol (300ml) was bubbled HClg for 10min, then stirred for 12h. The yellow solid was then filtered, washed with ether and dried *in vacuo* to provide the title compound (5.9g, 92%); ES-MS (m/z) 457 [M+1]⁺.

### D. 3-[6-(2-Azepan-1-yl-ethoxy)-naphthalen-2-yl]-5-(5-isobutyl-1H-[1,2,4]triazol-3-yl)-1H-indazole

To a solution of 3-[6-(2-azepan-1-yl-ethoxy)-naphthalen-2-yl)-1*H*-indazole-5 carboximidic acid ethyl ester dihydrochloride salt (0.100g, 0.95 mmol) in methanol (5ml) was added 3-methyl-butyric acid hydrazide (0.441 g, 3.8 mmol), then triethylamine (0.8ml, 5.7 mmol). The reaction was stirred at 100°C in a sealed pressure vessel for 4h. The mixture was concentrated and purified by HPLC to provide the title compound (78mg, 16%). ¹H NMR (DMSO, *d₆)* δ 8.82 (s, 1H), 8.49 (s, 1H), 8.15-7.92 (m, 4H), 7.67-7.65 (d, 1H), 7.41-7.40 (d, 1H), 7.30-7.26 (dd, 1H), 4.52-4.48 (t, 2H), 3.71-3.55 (m, 4H), 2.72-2.69 (d, 2H), 2.20-1.75 (m, 9H), 1.35-1.25 (m, 2H), 1.00-0.98 (d, 6h); ES-MS (m/z) 509 [M+1]⁺.

### Example 36

### SYNTHESIS OF 3-{6-[2-(2,6-DIMETHYL-PIPERIDIN-1-YL)-ETHOXY]-NAPTHALEN-2-YL}-5-(5-ISOBUTYL-2H-[1,2,4,]TRIAZOL-3-YL)-1H-INDAZOLE

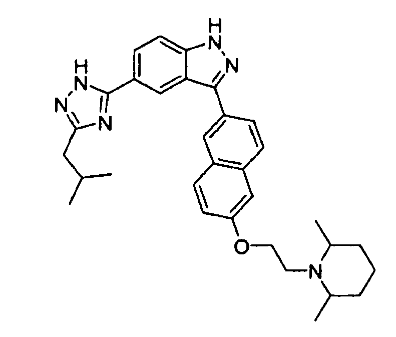

### A. 6-Dimethyl-piperidin-1-yl)-acetic acid ethyl ester

To a solution containing 2,6-dimethylpiperidine (6.0 g, 53.00 mmol) was added tetrahydrofuran (150 mL), triethylamine (5.35 g, 53.00 mmol) and bromoethylacetate (8.85 g, 53.00 mmol). The mixture was allowed to stir at ambient temperature for 18 hours. The resulting thick solution was condensed and extracted with water and methylene chloride. The organics were combined, dried over magnesium sulfate, filtered and solvent condensed under reduced pressure to provide the title compound (4.3 g, 41%). ¹H- NMR (CHCl₃) δ 4.15 (q, 2H), 3.57 (s, 2H), 2.82 (m, 2H), 1.65 (m, 2H), 1.57 (bd, 2H), 1.27 (t, 4H), 1.11 (d, 6H).

### B. (2,6-Dimethyl-piperidin-1-yl)-ethanol

To an ice cooled solution containing 6-Dimethyl-piperidin-1-yl)-acetic acid ethyl ester (4.30 g, 21.6 mmol) in tetrahydrofuran (50 mL) was added lithium aluminum hydride (615 mg, 16.20 mmol). The solution was allowed to stir for one hour until reaction is done. The mixture was then quenched drop wise with water until a white precipitate formed. Solution was then condensed and extracted with ethyl acetate. The organics were dried over magnesium sulfate, filtered and solvent removed under reduced pressure to provide the title compound (2.66 g, 78%). ¹H- NMR (CHCl₃) δ 3.54 (t, 2H), 2.72 (t, 2H), 2.5 (m, 2H), 1.65 (m, 1H), 1.53 (m, 2H), 1.27 (bm, 3H), 1.10 (d, 6H).

### C. 1-[2-(6-Bromo-naphthalen-2-yloxy)-ethyl]-2,6-dimethyl-piperidine

The title compound was prepared as described in Example 34, step A, using (2,6-Dimethyl-piperidin-1-yl)-ethanol (4.53 g, 28.85 mmol) and 6-bromo-2-naphthol (2.57 g, 11.54 mmol) to provide the title compound (3.23 g, 77%). ¹H- NMR (CHCl₃) δ 7.90 (d, 1H), 7.63 (d, 1H), 7.58 (d, 1H), 7.49 (dd, 1H), 7.14 (dd, 1H), 7.09 (d, 1H), 4.98 (m, 3H), 4.10 (t, 2H), 3.53 (bs, 2H), 3.12 (t, 2H), 2.87 (bs, 2H), 2.58 (m, 2H), 2.43 (m, 2H), 1.56 (t, 4H), 1.1 (d, 6H)

### D. 3-{6-[2,6-Dimethyl-piperidin-1-yl)-ethoxy]napthalen-2-yl}-1-(tetrahydro-pyran-2-yl)-1H-indazole-5-carbonitrile

The title compound was prepared as described in Example 34, step B, using 1-[2-(6-Bromo-naphthalen-2-yloxy)-ethyl]-2,6-dimethyl-piperidine (3.23 g, 8.93 mmol) to provide the title compound (2.18 g, 41%). ES-MS (m/z) 509 [M+1]⁺.

### E. 3-{6-[2-(2,6-Dimethyl-piperidin-1-yl)-ethoxy]-napthalen-2-yl}-1H-indazole-5-carboximidic acid ethyl ester

The title compound was prepared as described in Example 34, step C, using 3-{6-[2,6-Dimethyl-piperidin-1-yl)-ethoxy]napthalen-2-yl}-1-(tetrahydro-pyran-2-yl)-1H-indazole-5-carbonitrile (2.18 g, 72%).

### F. 3-{6-[2,6-Dimethyl-piperidin-1-yl)-ethoxy]-naphthalen-2-yl}-5-(5-isobutyl-2H-[1,2,4]triazol-3-yl)-1H-indazole

The title compound was prepared as described in Example 34, step E, using 3-{6-[2-(2,6-Dimethyl-piperidin-1-yl)-ethoxy]-napthalen-2-yl}-1H-indazole-5-carboximidic acid ethyl ester (0.600 g, 1.27 mmol) to provide the title compound (0.090 g, 15%). ¹H-NMR (CHCl₃) δ 8.80 (s, 1H), 8.36 (s, 1H), 8.15 (d, 1H), 8.06 (d, 1H), 7.80 (t, 2H), 7.54 (d, 1H), 7.15 (d, 2H), 4.18 (t, 2H), 3.2 (bs, 2H), 2.73 (d, 2H), 2.1 (m, 1H), 1.26 (bm, 8H), 1.02 (d, 6H). ES-MS (m/z) 523 [M+1]⁺.

### Example 37

### SYNTHESIS OF (2-{6-[5-(5-ISOBUTYL-1H-[1,2,4]TRIAZOL-3-YL)-1H-INDAZOL-3-YL]-NAPHTHALEN-2-YLOXY}-ETHYL-DIMETHYL-AMINE

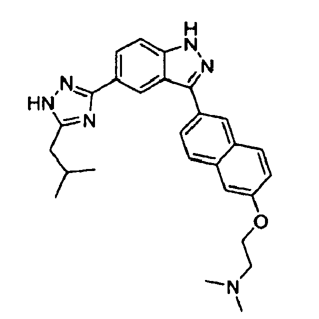

### A. 3-[6-(2-Dimethylamino-ethoxy)-naphthalene-2-carbonyl]-4-fluorobenzonitrile

To a solution of 4-fluoro-3-(6-hydroxy-naphthalene-2-carbonyl)-benzonitrile (0.630 g, 2.16 mmol) in 8 mL of acetone was added potassium carbonate (0.597 g, 4.32 mmol). After stirring the mixture at room temperature for 20 min, 2-dimethylamino ethyl chloride dihydrochloride. The mixture was heated to reflux temperature of the solvent for 4 h. The solvent was removed under reduced pressure and the crude product partitioned between ethyl acetate and water. Separation of the product from side products was attempted by column chromatography and failed so the product was used in the subsequent step without further purification attempts (0.440 g, 56% yield): ES-MS (m/z) 363.

### B. 3-[6-(2-Dimethylamino-ethoxy)-naphthaten-2-yl]-1H-indazole-5-carbonitrile

To a slurry of 3-[6-(2-dimethylamino-ethoxy)-naphthalene-2-carbonyl]-4-fluorobenzonitrile (0.440 g, 1.21 mmol) in toluene (5 mL) was added hydrazine monohydrate (0.14 mL, 2.66 mmol). The reaction mixture was heated to 65°C for 5 hours. The solvent was removed under reduced pressure and the crude material was purified by preparatory HPLC (0.150 g, 35% yield): ES-MS (m/z) 357.

### C. 3-[6-(2-Dimethylamino-ethoxy)-naphthalen-2-yl]-1H-indazole-5-carboximidic acid ethyl ester

A solution of 3-[6-(2-dimethylamino-ethoxy)-naphthalen-2-yl]-1*H*-indazole-5-carbonitrile (0.150 g, 0.42 mmol) in ethanol (150 mL) was prepared and cooled to-78°C. HCl gas was bubbled through the solution for 15 min. The solution was stirred overnight and the temperature was raised to room temperature. The solvent was then removed under reduced pressure and the solid was maintained under vacuum (0.236g, quantitative yield): ES-MS (m/z) 403.

### D. (2-{6-[5-(5-Isobutyl-1H-[1,2,4]triazolL-3-yl)-1H-indazol-3-yl]-naphthalen-2-yloxy}-ethyl-dimethyl-amine

To a solution of 3-[6-(2-dimethylamino-ethoxy)-naphthalen-2-yl]-1*H*-indazole-5-carboximidic acid ethyl ester (0.236 g, 0.5 mmol) in methanol (4 mL) was added triethyl amine (1.4 mL, 10 mmol), followed by 3-methyl butyric acid (0.232 g, 2.0 mmol). The reaction mixture was heated to 95°C for 3 h. The solvent was removed under reduced pressure and the crude mixture was purified by preparatory HPLC to provide the title compound (0.022 g, 10% yield): ¹H NMR (CD₃OD) δ 8.8 (s, 1H), 8.5 (s, 1H), 8.1 (m, 2H), 8.0 (dd, 2H), 7.6 (d, 1H), 7.4 (s, 1H), 7.28 (d, 1H), 4.47 (m, 2H), 3.5 (m, 2H), 2.9 (s, 6H), 2.7 (d, 2H), 2.2 (m, 1H), 1.0 (d, 6H); ES-MS (m/z) 455.

### Example 38

### SYNTHESIS OF 5-(5-TERT-BUTYL-1H-[1,2,4]TRIAZOL-3-YL)-3-[6-(2-PYRROLIDIN-1-YL-ETHOXY)-NAPHTHALEN-2-YL]-1H-INDAZOLE

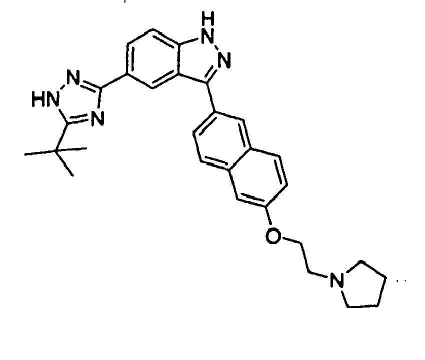

### A. 1-[2-(6-Bromonaphthalen-2-yloxy)-ethyl]-pyrrolidine

A solution of sodium hydroxide (5.74 g, 144 mmol), water (100 mL), THF (100 mL) and 6-bromo-2-naphthol (8.0 g, 35.9 mmol) was stirred for 10 minutes. To this mixture was added 1-(2-chloroethyl)-pyrrolidine·HCl (9.15 g, 53.8 mmol). After heating for 14 hours at 55°C, reaction was removed from heat and the layers were partitioned. The aqueous layer was extracted with ethyl acetate, dried (MgSO₄) and the combined organic layers were concentrated to provide crude product. Purification was carried out by flash chromatography using a gradient of 50 % to 100 % ethyl acetate in hexanes as the eluent to provide the title compound (10.6 g, 92 %): ES-MS (m/z) 321 [M+1]⁺.

### B. 6-(2-Pyrrolidin-1-yl-ethoxy)-naphthalen-2-boronic acid

To a solution of 1-[2-(6-bromonaphthalen-2-yloxy)-ethyl]-pyrrolidine (10.5 g, 32.8 mmol) in THF (130 mL) at -78°C was added n-butyllithium (39.4 mmol) and stirred for 45 minutes. Trimethylborate (11.0 mL, 98.5 mmol) was added and stirred for an additional 45 minutes. Aqueous saturated ammonium chloride (20 mL) was added and the mixture was allowed to warm to ambient temperature. Water (40 mL) was added and the layers were partitioned. The aqueous layer was extracted with ethyl acetate and the combined organic extracts were dried (MgSO₄) and concentrated to provide the title compund (8.12 g, 87 %). ES-MS (m/z) 286 [M+1]⁺.

### C. 3-[6-(2-Pyrrolidin-1-yl-ethoxy)-naphthalen-2-yl]-1-(tetrahydropyran-2-yl)-1H-indazole-5-carbonitrile

The title compound (1.05 g, 12 %) was prepared as described in International Publication No. WO 02/10137, Example 149D using 3-bromo-1-perhydro-2H-pyran-2-yl-1H-indazole-5-carbonitrile (5.82 g, 19.0 mmol, prepared as described in International Publication No. WO 02/10137, Example 149C) and 6-(2-pyrrolidin-1-yl-ethoxy)-naphthalen-2-boronic acid (8.13 g, 28.5 mmol): ES-MS (m/z) 467 [M+1]⁺.

### D. 3-[6-(2-Pyrrolidin-1-yl-ethoxy)-naphthalen-2-yl]-1H-indazole-5-carboximidic acid ethyl ester

The title compound (1.01 g, 99 %) was prepared as described in Example 9, step D, using 3-[6-(2-pyrrolidin-1-yl)-ethoxy)-naphthalen-2-yl]-1-(tetrahydropyran-2-yl)-1H-indazole-5-carbonitrile (1.01 g, 2.17 mmol): ES-MS (m/z) 429 [M+1]⁺.

### E. 5-(5-tert-Butyl-1H-[1,2,4]triazol-3-yl)-3-[6-(2-pyrrolidin-1-yl-ethoxy)-naphthalen-2-yl]-1H-indazole

The title compound (129 mg, 42 %) was prepared as described in Example 13, step E, using 3-[6-(2-pyrrolidin-1-yl-ethoxy)-naphthalen-2-yl]-1H-indazole-5-carboximidic acid ethyl ester (300 mg, 0.65 mmol) and pivalic acid hydrazide (150 mg, 1.29 mmol): ¹H NMR (DMSO) δ 8.71 (s, 1H) 8.44 (s, 1H) 8.17 - 7.93 (cm, 4H) 7.68 (d, 1H) 7.24 (dd, 1H) 4.25 (t, 2H) 2.92 (br s, 2H) 2.61 (br s, 4H) 1.73 (br s, 4H) 1.39 (s, 9H); ES-MS (m/z) 481 [M+1]⁺.

### Example 39

### SYNTHESIS OF 3-[6-(2-AZEPAN-1-YL-ETHOXY)-NAPHTHALEN-2-YL]-5-(5-TERT-BUTYL-2H-[1,2,4]TRIAZOL-3-YL)-1H-INDAZOLE

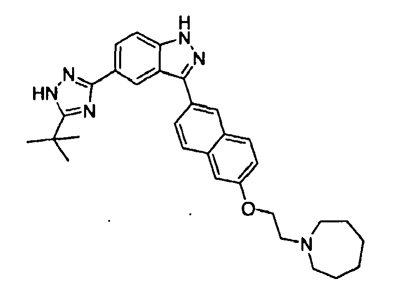

### 3-[6-(2-Azepan-1-yl-ethoxy)-naphthalen-2-yl]-5-(5-tert-butyl-2H-[1,2,4]triazol-3-yl)-1H-indazole

A solution of 3-[6-(2-Azepan-1-yl-ethoxy)-napthalen-2-yl]-1*H*-indazole-5 carboximidic acid ethyl ester di-hydrochloric acid salt (1 g, 1.8 mmol), 2,2-dimethyl-propionic acid hydrazide (0.88 g, 7.6 mmol), and triethyl amine (5.3 mL, 38 mmol) in methanol (15 mL) was heated in a sealed reaction flask on an 100°C oil bath for three hours. Solvent and excess triethyl amine were then removed under reduced pressure and the crude product purified using reverse-phase preparatory HPLC (20-60% acetonitrile + 0.1 % TFA in H₂O + 0.1% TFA, over 30 min). Fractions containing clean product were neutralized with sodium bicarbonate and product extracted using ethyl acetate. The organic fractions were pooled, dried over magnesium sulfate, filtered, and solvent removed under reduced pressure to provide the title compound (266 mg, 28%). ¹H NMR (300 MHz, Acetone-d*₆*) δ 8.92 (s, 1H), 8.52 (d, 1H), 8.21 (m, 2H), 7.97 (dd, 2H), 7.73 (d, 1H), 7.40 (d, 1H), 7.23 (dd, 1H), 4.24 (t, 2H), 3.01 (t, 2H), 2.80 (m, 4H), 1.63 (m, 8H), 1.46 (s, 9H); MS (ESI) *m*/z 509.3 [M+1]⁺.

### Example 40

### SYNTHESIS OF 5-(5-TERT-BUTYL-1H-[1,2,4]TRIAZOL-3-YL)-3-[6-(2-PIPERIDIN-1-YL-ETHOXY)-NAPHTHALEN-2-YL]-1H-INDAZOLE

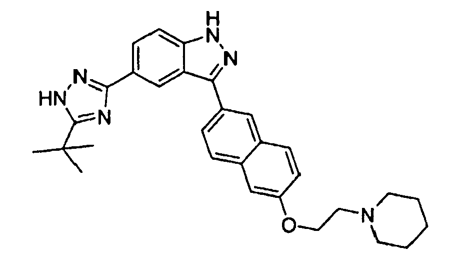

### 5-(5-tert-Butyl-1H-[1,2,4]triazol-3-yl)-3-[6-(2-piperidin-1-yl-ethoxy)-naphthalen-2-yl]-1H-indazole

The title compound was prepared according to the procedure described in Example 30, using 3-[6-(2-piperidin-1-yl-ethoxy)-naphthalen-2-yl]-1H-indazole-5-carboximidic acid ethyl ester hydrochloride (0.45 g, 1.02 mmol), 2,2-dimethyl-propionic acid hydrazide (0.35 g, 4.2 mmol), triethylamine (2.84 mL, 20.36 mmol) in methanol (15 mL) to yield 180 mg (36% yield) of pure material after purification by HPLC (20-70% water/ acetonitrile). ¹H NMR (methanol-d₄, 300 MHz) δ 8.83 (s, 1H), 8.46 (s, 1H), 8.11 (bd, 2H), 7.93 (dd, 2H), 7.66 (d, 1H), 7.31 (bs, 1H), 7.21 (bd, 1H), 4.29 (bs, 2H), 2.90 (bs, 2H), 2.64 (bs, 4H), 1.66 (bs, 4H), 1.46 (bs, 11H). ES-MS (m/z) 495 [M+1]⁺.

### Example 41

### SYNTHESIS OF 3-[6-(2-PIPERIDIN-1-YL-ETHOXY)-NAPHTHALEN-2-YL]-5-(5-PYRROLIDIN-1-YLMETHYL-1H-[1,2,4]TRIAZOL-3-YL)-1H-INDAZOLE

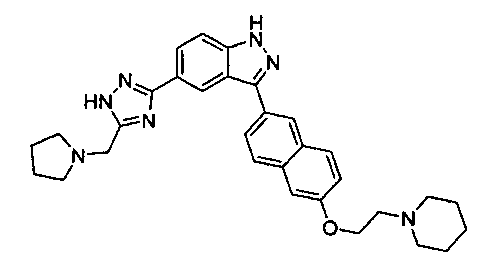

The title compound was prepared according to the procedure described in Example 30, using 3-[6-(2-piperidin-1-yl-ethoxy)-naphthalen-2-yl]-1H-indazole-5-carboximidic acid ethyl ester hydrochloride (1.35 g, 3.05 mmol), pyrrolidin-1-yl-acetic acid hydrazide (1.31 g, 9.15 mmol, prepared as described in International Publication No. WO 02/10137, Example 422A), triethylamine (8.5 mL, 61 mmol) in methanol (20 mL) to yield 550 mg (35% yield) of pure material after purification by HPLC (20-65% water/ acetonitrile). ¹H NMR (methanol-d₄, 300 MHz) δ 8.82 (s, 1H), 8.42 (s, 1H), 8.08 (d, 2H), 7.89 (dd, 2H), 7.65 (d, 1H), 7.27 (d, 1H), 7.18 (dd, 1H), 4.25 (t, 2H), 3.85 (s, 2H), 2.86 (t, 2H), 2.65 (bd, 8H), 1.82 (bs, 4H), 1.64 (m, 4H), 1.49 (m, 2H). ES-MS (m/z) 522 [M+1]⁺.

### Example 42

### SYNTHESIS OF 3-[6-(2-PYRROLIDIN-1-YL-ETHOXY)-NAPTHALEN-2-YL]-5-(5-PYRROLIDIN-1-YLMETRYL-1H-[1,2,4]TRIAZOL-3-YL)-1H-INDAZOLE

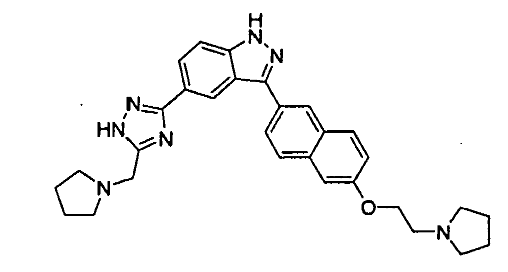

Following the procedure described in Example 30, using 3-[6-(2-pyrrolidin-1-yl-ethoxy)-napthalen-2-yl]-1H-indazole-5-carboximidic acid ethyl ester (1.41g, 3.3mmol), pyrrolidin-1-yl-acetic acid hyrazide (1.88g, 13.2mmol), and triethylamine (9.15mL, 65.8mmol) to provide C the title compound as a white solid (0.25g, 15% yield). ¹H NMR (DMSO-d₆) δ 8.80(s, 1H), 8.55(s, 1H), 8.25-8.0(m, 5H), 7.80(d, 1H), 7.52(s, 1H), 7.35(d, 1H), 4.35(t, 2H), 3.35(s, 2H), 3.00(t, 2H), 2.65(m, 8H), 1.80(m, 8H). ES-MS (m/z) 508 [M+1].

### Example 43

### SYNTHESIS OF 3-[6-(2-AZEPAN-1-YL-ETHOXY)-NAPHTHALEN-2-YL]-5-(5-PYRROLEDIN-1-YLMETHYL-1H-[1,2,4]TRIAZOL-3-YL)-1H-INDAZOLE

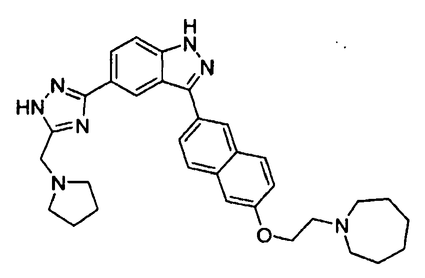

### 3-[6-(2-Azepan-1-yl-ethoxy)-naphthalen-2-yl]-5-(5-pyrrolidin-1-ylmethyl-1H-[1,2,4]triazol-3-yl)-1H-indazole

To a solution of 3-[6-(2-azepan-1-yl-ethoxy)-naphthalen-2-yl]-1*H*-indazole-5-carboximidic acid ethyl ester dihydrochloride salt (0.100g, 0.95 mmol) in methanol (5ml) was added pyrrolidin-1-yl-acetic acid hydrazide (0.542g, 3.8 mmol, prepared as described in International Publication No. WO 02/10137, Example 321B), then triethylamine (0.8ml, 5.7 mmol). The reaction was stirred at 100°C in a sealed pressure vessel for 4h. The mixture was concentrated and purified by HPLC to provide the title compound (64mg, 13%). ¹H NMR (DMSO, *d₆)* δ 8.82 (s, 1H), 8.43 (s, 1H), 8.10-7.88 (m, 3H), 7.67-7.63 (d, 1H), 7.32-7.31 (d, 1H), 7.22-7.19 (dd, 2H), 4.32-4.28 (t, 2H), 3.86 (s, 2H), 3.27-3.16 (m, 2H), 3.01-2.98 (m, 4H), 2.83-2.65 (m, 4H), 1.86-1.64 (m, 12H); ES-MS (m/z) 536 [M+1].

### Example 44

### SYNTHESIS OF 3-[6-(2-CYCLOPENTYL-ETHOXY)-NAPHTHALEN-2-YL]-5-(5-PYRROLIDIN-1-YLMETHYL-1H-[1,2,4]TRIAZOL-3-YL)-1H-INDAZOLE

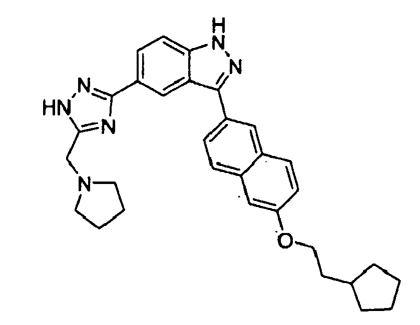

### A. Cyclopentyl ethanol

To a flask was charged cyclopentyl acetic acid (2.60 g, 20.0 mmol) and THF (100 mL). The flask contents were cooled on ice and LiAlH (0.57 g, 15.0 mmol) was added. After 1.0 h, water (10 mL) was added and the entire mixture was filtered over Celite. The filtrate was concentrated and the aqueous layer was extracted with ethyl acetate, dried (MgSO₄) and concentrated to provide the title compound (1.06 g, 46 %): R_{f}= 0.68 (30 % ethyl acetate in hexanes).

### B. 2-Bromo-6-(2-cyclopentyl-ethoxy)-naphthalene

A mixture of 6-bromo-2-naphthol (2.86 g, 12.3 mmol), cyclopentyl ethanol (2.80 g, 24.5 mmol), triphenylphosphine (3 mmol P / gram polymer supported, 8.20 g, 24.5 mmol) and diisopropyl azodicarboxylate (4.96 g, 24.5 mmol) in THF (75 mL) was stirred at ambient temperature for 1 hour. The entire mixture was filtered over Celite and concentrated to provide the crude product. Purification was carried out by flash chromatography, using hexanes as the eluent, to provide the title compound (3.59 g, 91 %): R_{f} = 0.35 (hexanes).

### C. 6-(2-Cyclopentyl-ethoxy)-naphthalen-2-yl-boronic acid

The title compound (2.92 g, 93 %) was prepared as described in Example 12, step B, using 2-bromo-6-(2-cyclopentyl-ethoxy)-naphthalene (3.54 g, 11.1 mmol): ES-MS (m/z) = 285 [M+1]⁺.

### D. 3-[6-(2-Cyclopentyl-ethoxy)-naphthalen-2-yl]-1-(tetrahydro-pyran-2-yl)-1H-indazole-5-carbonitrile

The title compound (3.17 g, 71 %) was prepared as described in International Publication No. WO 02/10137, Example 149C using 3-bromo-1-perhydro-2H-pyran-2-yl-1H-indazole-5-carbonitrile (2.10 g, 6.80 mmol, prepared as described in International Publication No. WO 02/10137, Example 149C) and 6-(2-cyclopentyl-ethoxy)-naphthalen-2-yl-boronic acid (2.90 g, 10.2 mmol): R_{f} = 0.56 (1:4 ethyl acteate/hexanes).

### E. 3-[6-(2-Cyclopentyl-ethoxy)-naphthalen-2-yl]-1H-indazole-5-carboximidic acid ethyl ester

The title compound (0.47 g, 85 %) was prepared as described in Example 9, step D using 3-[6-(2-cyclopentyl-ethoxy)-naphthalen-2-yl]-1-(tetrahydro-pyran-2-yl)-1H-indazole-5-carbonitrile (0.56 g, 1.20 mmol): ES-MS (m/z) 428 [M+1]⁺.

### F. 3-[6-(2-Cyclopentyl-ethoxy)-naphthalen-2-yl]-5-(S-pyrrolidin-1-ylmethyl-1H-[1,2,4]triazol-3-yl)-1H-indazole

The title compound (139 mg, 31 %) was prepared as desribed in Example 13, step E using 3-[6-(2-cyclopentyl-ethoxy)-naphthalen-2-yl]-1H-indazole-5-carboximidic acid ethyl ester (415 mg, 0.89 mmol) and the hydrazide (512 mg, 3.58 mmol, prepared as described in International Publication No. WO 02/10137, Example 422A): ¹H NMR (DMSO) δ 8.74 (br s, 1H) 8.44 (br s, 1H) 8.18 - 7.94 (cm, 4H) 7.70 (br d, 1H) 7.41 (d, 1H) 7.22 (dd, 1H) 4.15 (t, 2H) 3.79 (s, 2H) 2.57 (br s, 2H) 1.90 - 1.75 (cm, 4H) 1.73 (br s, 4H) 1.68 - 1.42 (cm, 4H) 1.30 - 1.10 (cm, 4H); ES-MS (m/z) 507 [M+1]⁺.

### Example 45

### SYNTHESIS OF PYRROLIDIN-1-YL-{6-[5-(5-PYRROLIDIN-1-YLMETHYL-2H-[1,2,4]TRIAZOL-3-YL)-1H-INDAZOL-3-YL]-NAPHTHALEN-2-YL}-METHANONE

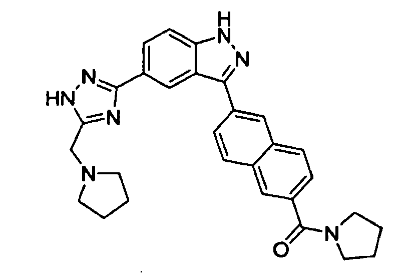

### A. Pyrrolidin-1-yl-{6-[5-(5-pyrrolidin-1-ylmethyl-2H-[1,2,4]triazol-3-yl)-1H-indazol-3-yl]-naphthalen-2-yl}-methanone

A solution of 3-[6-(pyrrolidine-1-carbonyl)-napthalen-2-yl]-1*H*-indazole-5-carboximidic acid ethyl ester di-hydrochloric acid salt (420 mg, 0.87 mmol), pyrrolidin-1-yl-acetic acid hydrazide (500 mg, 3.5 mmol, prepared as described in International Publication No. WO 02/10137, Example 422A), and triethyl amine (2.4 mL, 17.2 mmol) in methanol (5 mL) was heated in a sealed reaction flask on an 100 °C oil bath overnight. Solvent and excess triethyl amine were then removed under reduced pressure and the crude product purified using reverse-phase preparatory HPLC (20-60% acetonitrile + 0.1% TFA in H₂O + 0.1% TFA, over 30 min). Fractions containing clean product were neutralized with sodium bicarbonate and product extracted using ethyl acetate. The organic fractions were pooled, dried over magnesium sulfate, filtered, and solvent removed under reduced pressure to provide the title compound (160 mg, 38%). ¹H NMR (400 MHz, DMSO-d*₆*) δ 13.56 (bs, 1H), 8.80 (bm, 1H), 8.58 (bm, 1H), 8.24 (m, 1H), 8.18 (overlapping m, 3H), 8.12 (m, 1H), 7.74 (m, 1H), 7.70 (dd, 1H), 3.85 (bs, 2H), 3.53 (m, 4H), 2.63 (bm, 4H), 1.89 (m, 4H), 1.75 (m, 4H); MS (ESI) *m*/*z* 492.3 [M+1]⁺.

### B. 3-[6-(Pyrrolidine-1-carbonyl)-napthalen-2-yl]-1H-indazole-5-carboximidic acid ethyl ester di-hydrochloric acid salt

A solution of 3-[6-(pyrrolidine-1-carbonyl)-napthalen-2-yl]-1-(tetrahydro-pyran-2-yl)-1*H*-indazole-5-carbonitrile (420.0 mg, 0.93 mmol) in ethanol (40 mL) was cooled on a dry ice/isopropanol bath and saturated with HCl (g). The resulting solution was allowed to stir, gradually warming to room temperature, overnight. Excess solvent was removed under reduced pressure and the resulting solid was taken up in Et₂O, sonicated 5 min and filtered to provide the title compound as a pale yellow solid (431.6 mg, 95%). MS (ESI) *m*/*z* 413.2 [free base M+1]⁺.

### C. 3-[6-(pyrrolidine-1-carbonyl)-napthalen-2-yl]-1-(tetrahydro-pyran-2-yl)-1H-indazole-5-carbonitrile

A solution of 6-[5-Cyano-1-(tetrahydro-pyran-2-yl)-1*H*-indazol-3-yl]-naphthalene-2-carboxylic acid (600 mg, 1.5 mmol), HOBT (250 mg, 1.9 mmol), EDCI (350 mg, 1.8 mmol), and pyrrolidine (0.15 mL, 1.8 mmol) in DMF (30 mL) was stirred at room temperature overnight. The solution was then poured into water to provide a white precipitate, which was filtered and dried to provide the title compound (540 mg, 79%). MS (ESI) *m*/*z* 451.5 [M+1]⁺.

### Example 46

### SYNTHESIS 5-(5-MORPHOLIN-4-YLMETHYL-1H-[1,2,4\TRIAZOL-3-YL)-3-[6-(2-PYRROLIDIN-1-YL-ETHOXY)-NAPHTHALEN-2-YL]-1H-INDAZOLE

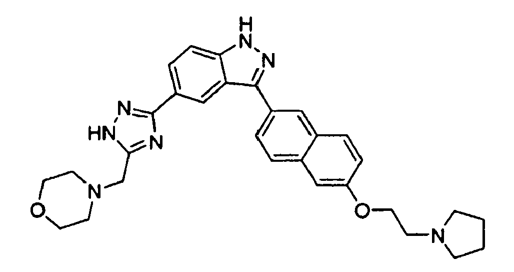

Following the procedure described in Example 30, using 3-[6-(2-pyrrolidin-1-yl-ethoxy)-napthalen-2-yl]-1H-indazole-5-carboximidic acid ethyl ester (1.70g, 3.9mmol), morpholin-4-yl-acetic acid hydrazide (2.52g, 15.8mmol), and triethylamine (8.40mL, 79.4mmol) to provide the title compound as a white solid (0.30g, 15% yield). ¹H NMR (DMSO-d₆) δ 8.40(s, 1H), 8.45(s, 1H), 8.25(d, 1H), 8.15(d, 1H), 7.85(d, 2H), 7.62(d, 1H), 7.23(m, 3H), 4:40(t, 2H), 3.85(m, 8H), 3.20(t, 2H), 2.85(m, 4H), 2.00(m, 4H). ES-MS (m/z) 524 [M+1].

### Example 47

### SYNTHESIS OF 5-(5-MORPHOLIN-4-YLMETHYL-1H-[1,2,4]TRIAZOL-3-YL)-3-[6-(2-PIPERIDIN-1-YL-ETHOXY)-NAPHTHALEN-2-YL]-1H-INDAZOLE

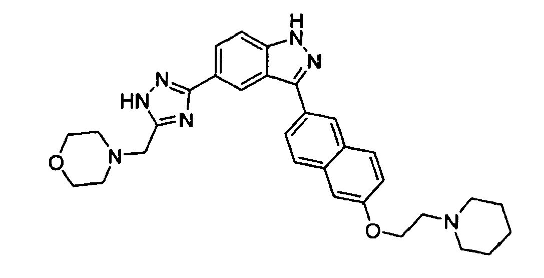

### A. Morpholin-4-yl-acetic acid hydrazide

To a solution of morpholine (52.4 mL, 0.601 mol) in tetrahydrofuran (250 mL) at 0°C was added ethyl bromoacetate (80 mL, 0.722 mol) followed by triethylamine (167.5 mL, 1.202 mol). The reaction was allowed to reach room temperature and stirred for 48 h. Tetrahydrofuran was removed *in vacuo* and the aqueous phase was extracted with ethyl acetate. The organic layer was dried over magnesium sulfate, filtered and the solvent was removed *in vacuo.* The crude material (98 g) was dissolved in ethanol (150mL) and anhydrous hydrazine (19.5 mL, 0.623 mmol) was added and the reaction was refluxed for 48 h. Solvent was removed in vacuo to yield morpholin-4-yl-acetic acid hydrazide (90 g, 94% yield). ¹H NMR (methanol-d₄, 300 MHz) δ 3.72 (m, 4H), 3.05 (s, 2H), 2.52 (m, 4H).

### B. 5-(5-Morpholio-4-ylmethyl-1H-[1,2,4]triazol-3-yl)-3-[6-(2-piperidin-1-yl-ethoxy)-naphthalen-2-yl]-1H-indazole

The title compound was prepared according to the procedure described in Example 30, using 3-[6-(2-piperidin-1-yl-ethoxy)-naphthalen-2-yl]-1H-indazole-5-carboximidic acid ethyl ester hydrochloride (0.615 g, 1.4 mmol), morpholin-4-yl-acetic acid hydrazide (0.67 g, 4.2 mmol), triethylamine (3.9 mL, 27.9 mmol) in methanol (15 mL) to yield 128 mg (17% yield) of the title compound after purification by HPLC (20-70% water/ acetonitrile). ¹H NMR (methanol-d₄, 300 MHz) δ 8.85 (s, 1H), 8.46 (s, 1H), 8.10 (ddd, 2H), 7.95 (dd, 2H), 7.68 (d, 1H), 7.33 (d, 1H), 7.23 (dd, 1H), 4.31 (t, 2H), 3.73 (m, 6H), 2.94 (t, 2H), 2.63 (bd, 8H), 1.68 (m, 4H), 1.51 (m, 2H). ES-MS (m/z) 538 [M+1]⁺.

### Example 48

### SYNTHESIS OF 5-(5-CYCLOPROPYLMETHYL-1H-[1,2,4]TRIAZOL-3-YL)-3-[6-(2-PIPERIDIN-1-YL-ETHOXY)-NAPHTHALEN-2-YL]-1H-INDAZOLE

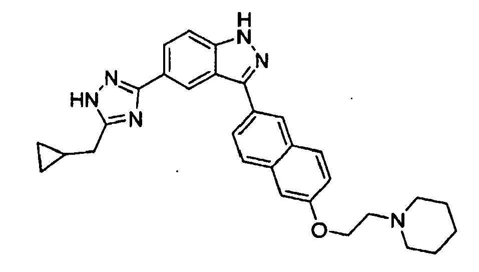

The title compound was prepared according to the procedure described in Example 30, using 3-[6-(2-piperidin-1-yl-ethoxy)-naphthalen-2-yl]-1H-indazole-5-carboximidic acid ethyl ester hydrochloride (0.61 g, 1.39 mmol), cyclopropyl-acetic acid hydrazide (0.48 g, 4.18 mmol), triethylamine (3.9 mL, 27.8 mmol) in methanol (15 mL) to yield 247.5 mg (36% yield) of pure material after purification by HPLC (20-60% water/ acetonitrile). ¹H NMR (methanol-d₄, 300 MHz) δ 8.72 (s, 1H), 8.34 (s, 1H), 7.98 (d, 2H), 7.81 (dd, 2H), 7.54 (d, 1H), 7.18 (d, 1H), 7.09 (dd, 1H), 4.16 (t, 2H), 2.80 (t, 2H), 2.64 (d, 2H), 2.54 (bs, 4H), 1.55 (m, 4H), 1.39 (m, 2H), 1.11 (m, 1H), 0.48 (m, 2H), 0.21 (m, 2H). ES-MS (m/z) 493 [M+1]⁺.

### Example 49

### SYNTHESIS OF 5-(5-CYCLOPROPYLMETHYL-1H-[1,2,4]TRIAZOL-3-YL)-3-[6-(2-PYRROLIDIN-1-YL-ETHOXY)-NAPHTHALEN-2-YL]-1H-INDAZOLE

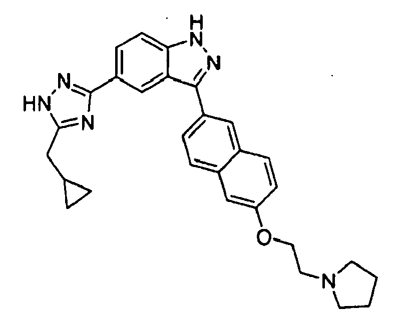

### 5-(5-Cyclopropylmethyl-1H-[1,2,4]triazol-3-yl)-3-[6-(2-pyrrolidin-1-yl-ethoxy)-naphthalen-2-yl]-1H-indazole

The title compound (79.5 mg, 26 %) was prepared as described in Example 13, step E, using 3-[6-(2-pyrrolidin-1-yl-ethoxy)-naphthalen-2-yl]-1H-indazole-5-carboximidic acid ethyl ester (300 mg, 0.65 mmol, prepared as described in Example 37, step D) and hydrazide (147 mg, 1.29 mmol, prepared as described in International Publication No. WO 02/10137, Example 422A). ¹H NMR (DMSO) 8.71 (s, 1H) 8.46 (s, 1H) 8.21 - 7.93 (cm, 4H) 7.68 (d, 1H) 7.43 (s, 1H) 7.25 (d, 1H) 4.28 (s, 2H) 2.98 (br s, 2H) 2.67 (br s, 4H) 1.75 (br s, 4H) 1.16 (cm, 2H) 0.80 (br s, 1H) 0.50 (d, 2H) 0.25 (d, 2H);ES-MS (m/z) 479 [M+1]+.

### Example 50

### SYNTHESIS OF 3-[6-(2-AZEPAN-1-YL-ETHOXY]-NAPHTHALEN-2-YL]-5-(5-CYCLOPROPYMETHYLL-1H-[1,2,4]TRIAZOL-3-YL)-1H-INDAZOLE

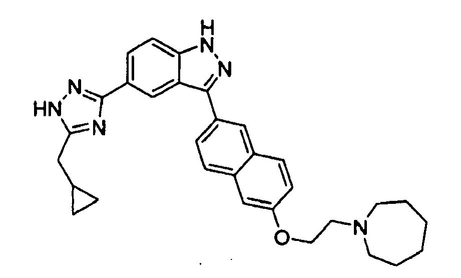

To a solution of 3-[6-(2-azepan-1-yl-ethoxy)-naphthalen-2-yl]-1H-indazole-5-carboximidic acid ethyl ester (0.600g, 1.21 mmol) in methanol (7ml) was added cyclopropyl-acetic acid hydrazide (0.553g, 4.85 mmol), then triethylamine (3.37ml, 24.2 mmol). The reaction was stirred at 100°C in a sealed pressure vessel for 4h. The mixture was concentrated and purified by HPLC to provide the title compound (159mg, 26%). 1H NMR (CD₃OD) δ 8.96 (s, 1H), 8.59 (s, 1H), 8.24-8.20 (m, 2H), 8.20-8.05 (q, 2H), 7.80-7.77 (d, 1H), 7.45-7.44 (d, 1H), 7.36-7.32 (dd, 1H), 4.42-4.39 (t, 2H), 3.24-3.20 (t, 2H), 3.06-3.02 (m, 4H), 2.89-2.86 (d, 2H), 1.87-1.79 (m, 8H), 1.39-1.31 (m, 3H), 0.73-0.42 (m, 2H); ES-MS (m/z) 507[M+1]⁺.

### Example 51

### PREPARATION OF 3-[6-(2-PYRROLIDIN-1-YL-ETHOXY)-NAPTHALEN-2-YL]-1H-INDAZOLE-5-CARBONITRILE

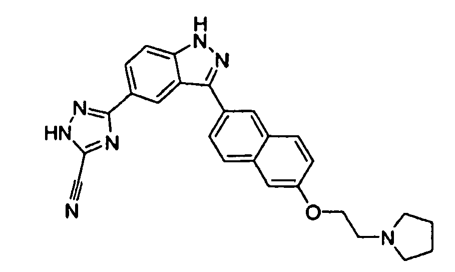

3-[6-(2-pyrrolidin-1-yl-ethoxy)-napthalen-2-yl]-1-(tetrahydro-pyran-2-yl)-1H-indazole-5-carbonitrile (0.10g, 1.8mmol) was heated in a sealed tube in 1N HCl overnight. Crude material subjected to prep HPLC, extracted with ethyl acetate to provide the title compound as a white solid (0.005g, 0.72% yield). ¹H NMR (DMSO-d₆) δ 9.00(s, 1H), 8.65(s, 1H), 8.30-8.15(m 2H), 8.00(d, 1H), 7.85(m, 2H), 7.35(dd, 1H), 4.30(t, 2H), 2.99(t, 2H), 2.65(m, 4H), 1.80(m, 4H). ES-MS (m/z) 383 [M+1].

### EXAMPLE 52

### SYNTHESIS OF 2-[2-(CIS-2, 6-DIMETHYLPIPERIDYL)ETHOXY]-6-{5-[3-(CYCLOPENTYLMETHYL)(1H-1,2,4-TRIAZOL-5-YL)](1H-INDAZOL-3-YL)}NAPTHALENE

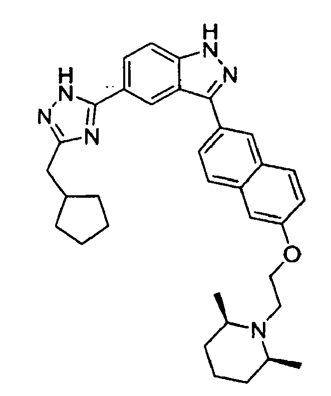

### A. 2-[2-(cis-2, 6-dimethylpiperidyl)ethoxy]-6-bromonapthalene

To a solution of 6-bromo-2-napthol (6.95 g., 31.2 mmol), 2-(cis-2,6-dimethylpiperidyl)ethan-1-ol (7.35 g., 46.8 mmol), and triphenylphosphine (12.26 g., 46.8 mmol) in THF was added diisobutylazodicarboxylate (9.23 mL, 46.8 mmol). The solution stirred for twenty minutes at ambient temperature and monitored via TLC until completion of the reaction. The solvent was evaporated under reduced pressure to give an oil. A mixture of diethyl ether: hexanes (1:1) was added to the oil and sonicated for 5 minutes to precipitate out triphenylphosphine oxide. The white solid was filtered through celite and the resultant filtrate condensed under reduced pressure to an oil. The oil was purified via silica gel chromatography (30-70% ethyl acetate/hexanes) to afford the title compound in excess yield (22 g. >100% yield). ES-MS (m/z) 361 [M+1]⁺, 361 [M+2]⁺.

### B. 3-{6-[2-(cis-2,6-dimethylpiperidyl)ethoxy](2-naphthyl)-1-perhydro-2H-pyran-2-yl-1H-indazole-5-carbonitrile

A mixture of 2-[2-cis-2,6-dimethylpiperidyl)ethoxy)-6-bromonaphthalene (5.0 g., 13.85 mmol), [1,1'-bis(diphenylphosphinoferrocene) complex with dichloromethane (1:1) (1.13 g., 1.385 mmol), potassium acetate (4.07 g., 41.55 mmol), and bis(pinnacolato)-diboron (3.51 g., 13.85 g) in dimethylformamide (70 mL) was heated to 95°C for three hours. The reaction was monitored by TLC and ES-MS to assure boronate ester formation. 3-Bromo-1-(tetrahydro-pyran-2-yl)-1H-indazole-5-carbonitrile (4.23 g., 13.85 mmol) and potassium carbonate (10.85 g., 41.55 mmol) were then added and heated for an additional 16 hours at 95°C. The resultant mixture was then condensed under reduced pressure to afford a black oil. The oil was then diluted with ethyl acetate and filtered through celite and solvent removed under reduced pressure. The resultant oil was purified via silica gel chromatography (10% methanol/methylene chloride) to afford the title compound (4.7 g., 64% yield). ES-MS (m/z) 509 [M+1]⁺.

### C. (3-{6-[2-(cis-2,6-dimethylpiperidyl)ethoxy](2-naphthyl)}(1H-indazol-5-yl))ethoxymethanimine

3- {6-[2-(cis-2,6-dimethylpiperidyl)ethoxy](2-naphthyl}-1-perhydro-2H-pyran-2-yl-1H-indazole-5-carbonitrile (4.6 g., 8.85 mmol) was dissolved in ethanol (800 mL) and cooled to 0°C. Hydrogen chloride gas was then bubbled into solution for twenty minutes. The acidified mixture was then stirred at room temperature for 16 hours. The resultant solution was condensed under reduced pressure to afford a solid. The solid was washed with diethyl ether and filtered through a buchner funnel and dried under vacuum to afford the title compound (4.8 g., 99% yield). ES-MS (m/z) 472 [M+1]⁺.

### D. 2-[2-(cis-2,6-dimethylpiperidyl)ethoxy]-6-{5-[3-(cyclopentylmethyl)(1H-1,2, 4-triazol-5-yl)](1H-indazol-3-yl)}naphthalene

To a solution of (3-{6-[2-(cis-2,6-dimethylpiperidyl)ethoxy](2-naphthyl)}(1H-indazol-5-yl))ethoxymethanimine (2.68 g., 4.94 mmol) in methanol (45 mL) was added cyclopentyl methyl hydrazide (1.4 g., 9.89 mmol) and triethylamine (4.98 g., 49.4 mmol). The mixture was heated to 95°C in a sealed tube for 16 hours. The solvent was then removed under reduced pressure and the oil purified via preparative HPLC (20-70% acetonitrile/water, 60mL/min.) to afford the title compound in 99% purity by analytical HPLC (0.375 g., 12% yield). ¹H- NMR (CD₃OD) δ 8.83 (qs, 1H), 8.48 (s, 1H), 8.12 (T, 2h), 7.96 (dd, 2H), 7.68 (d, 1H), 7.34 (s, 1H), 7.21 (dd, 1H), (4.25 (t, 2H), 3.3 (t, 3H), 2.85 (d, 2H), 2.75 (bs, 2H), 2.40 (m, 1H), 1.85 (m, 2H), 1.60 (m, 6H), 1.30 (m, 4H), 1.29 (d, 6H). ES-MS (m/z) 549 [M+1]⁺.

### EXAMPLE 53

### SYNTHESIS OF 2-[2-(CIS-2,6-DIMETHYLPIPERIDYL)ETHOXY]-6-{5-[3-(TERT-BUTYL)(1H-1,2,4-TRIAZOL-5-YL)](1H-INDAZOL-3-YL)}NAPHTHALENE

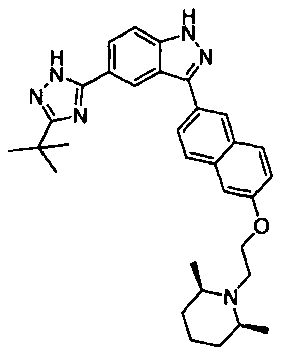

### A. 2-[2-(cis-2,6-dimethylpiperidyl)ethoxy]-6-{5-[3-(cyclopentylmethyl)(1H-1,2,4-triazol-5-yl)](1H-indazol-3-yl)}naphthalene

The title compound was prepared as described in Example 52, step D using N-amino-2,2-dimethylpropanamide (0.856 g., 3.69 mmol) to provide the title compound in 98% purity by analytical HPLC(0.395 g., 18% yield). ¹H- NMR (DMSO) δ 8.75 (s, 1H), 8.43 (s, 1H), 8.10 (d, 4H), 8.0 (t, 4H), 7.68 (d, 1H), 7.40 (s, 1H), 7.20 (d, 1H), 4.10 (t, 2H), 3.15 (d, 1H), 3.05 (t, 2H), 2.50 (m, 3H), 1.40 (s, 9H), 1.15 (d, 6H). ES-MS (m/z) 523[M+1]⁺.

### Example 54

### SYNTHESIS OF 2-[((2R)-1-METHYLPYRROLIDIN-2-YL)METHOXY]-6-{5-[3-(2,2-DIMETHYLPROPYL)(1H-1,2,4-TRIAZOL-5-YL)](1H-INDAZOL-3-YL)}NAPHTHALENE

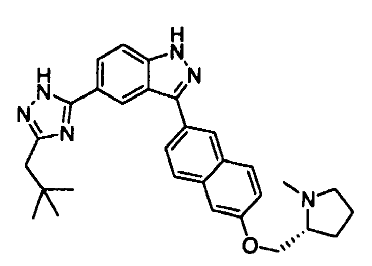

### A. 3-{6-[((2R)-1-methylpyrrolidin-2-yl)methoxy](2-naphthyl))-1-perhydro-2H-pyran-2-yl-1H_indazole-5-carbonitrile

To a solution of 3-(6-hydroxy(2-naphthyl)-1-perhydro-2H-pyran-2-yl-1H-indazole-5-carbonitrile (2.9 g., 7.86 mmol), triphenylphosphine (3.08 g., 11.78 mmol) and ((2R)-1-methypyrrolidin-2-yl)methan-1-ol (1.35 g., 11.78 mmol) in THF was added diisopropylazodicarboxylate (2.368 g., 11.78 mmol). The mixture was allowed to stir at ambient temperature for 30 minutes. The reaction was monitored via TLC and ES-MS. The mixture was condensed under reduced pressure and the resultant oil was sonicated with a mixture of diethyl ether/hexanes (1:1) until a white precipitate was present. The precipitate was filtered through celite and the filtrate condensed under reduced pressure. The resultant oil was purified via silica gel chromatography (3-10% methanol/dichloromethane) to afford the title compound (1.90 g., 52% yield). ES-MS (m/z) 467[M+1]⁺.

### B. (3-{6-[((2R)-1-methylpyrrolidin-2-yl)methoxy](2-naphthyl)}(1H-indazol-5- yl))ethoxymethanimine

The title compound was prepared as described in Example 52, step C using 3-{6-[((2R)-1-methylpyrrolidin-2-yl)methoxy](2-naphthyl)}-1-perhydro-2H-pyran-2-yl-1H-indazole-5-carbonitrile (1.90 g., 92% yield). ES-MS (m/z) 429[M+1]⁺.

### C. 2-[((2R)-1-methylpyrrolidin-2-yl)-methoxy]-6-{5-[3-(2,2dimethylpropyl)(1H-1,2,4-triazol-5-yl](1H_indazol-3-yl)}naphthalene

The title compound was prepared as described in Example 52, step D using (3-{6-[((2R)-1-methylpyrrolidin-2-yl)methoxy](2-naphthyl)}(1H-indazol-5-yl))ethoxymethanimine (0.900 g., 2.10 mmol) and N-amino-3,3-dimethylbutanamide (0.546 g., 4.20 mmol) to afford the title compound in 100% purity by analytical HPLC (0.031 g., 3% yield). ¹H- NMR (CHCl₃) δ 8.84 (s, 1H), 8.41 (s, 1H), 8.20 (d, 1H), 8.10 (d, 1H), 7.85 (t, 2H), 7.59 (d, 1H), 7.21 (m, 2H), 4.10 (d, 2H), 2.76 (s, 2H), 2.57 (s, 2H), 2.05 (m, 8H), 1.05 (s, 9H). ES-MS (m/z) 496[M+1]⁺.

### EXAMPLE 55

### SYNTHESIS OF 2-[((2R)-1-METHYLPYRROLIDIN-2-YL)METHOXY]-6-{5-[3-(2-METHYLPROPYL)(1H-1,2,4-TRIAZOL-5-YL)](1H-INDAZOL-3-YL)}NAPHTHALENE

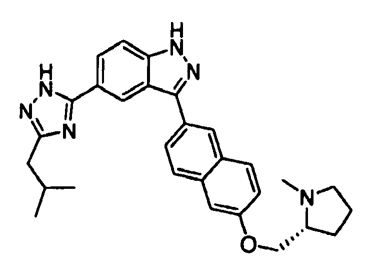

### A. 2-[((2R)-1-methylpyrrolidin-2-yl)-methoxy]-6-{5-[3-(2-methylpropyl)(1H-1,2,4-triazol-5-yl](1H_indazol-3-yl)}naphthalene

The title compound was prepared as described in Example 52, step D using (3-{6-[((2R)-1-methylpyrrolidin-2-yl)methoxy](2-naphthyl)}(1H-indazol-5-yl))ethoxymethanimine (1.0 g., 2.37 mmol) and N-amino-3-methylbutanamide (0.540 g., 4.67 mmol) to afford the title compound in 98% purity by analytical HPLC (0.084 g., 7.5% yield). ¹H- NMR (CHCl₃) δ 8.79 (s, 1H). 8.35 (s, 1H), 8.13 (d, 1H), 8.05 (d, 1H), 7.80 (dd, 2H), 7.53 (d, 1H), 7.18 (m, 2H), 4.11 (m, 2H), 3.18 (t, 1H), 2.74 (m, 3H), 2.56 (s, 3H), 2.36 (m, 1H), 2.21 (m, 1H), 2.11 (m, 1H), 1.83 (m, 4H). ES-MS (m/z) 481[M+1]⁺.

### EXAMPLE 56

### SYNTHESIS OF 2-[((2S)-1-ETHYLPYRROLIDIN-2-YL)METHOXY]-6-{5-[3-(2,2-DI METHYLPROPYL)(1H-1,2,4,-TRIAZOL-5-YL)](1H-INDAZOL-3-YL)}NAPHTHALENE

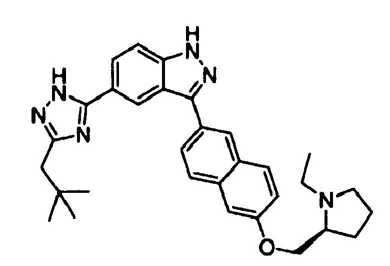

### A. 2-[((2S)-1-ethylpyrrolidin-2-yl)methoxy]-6-bromonapthalene

The title compound was prepared as described in Example 52, step A using ((2S)-1-ethylpyrrolidin-2-yl)methan-1-ol (6.20 g., 48.13 mmol) and purified via silica gel chromatography (3-10% methanol/dichloromethane) to afford the title compound (11.0 g., >100% yield). ES-MS (m/z) 334[M+1]⁺, 336 [M+2]+.

### B. 3-[6-[((2S)-1-ethylpyrrolidin-2-yl)methoxy](2-naphthyl)]-1-perhydro-2H-pyran-2-yl-1H-indazole-5-carbonitrile

The title compound was prepared as described in Example 52, step B using 2-[((2S)-1-ethylpyrrolidin-2-yl)methoxy]-6-bromonaphthalene (4.46 g, 13.39 mmol) and purified via silica gel chromatography (10-15% methanol/dichloromethane) to afford the title compound (1.05 g., 16% yield). ES-MS (m/z) 481[M+1]+,

### C. (3-{6-[((2S)-1-ethylpyrrolidin-2-yl)methoxy](2-naphthyl}(1H-indazol-5-yl))ethoxymethanimine

The title compound was prepared as described in Example 52, step C using 3-{6-[((2S)-1-ethylpyrrolidin-2-yl)methoxy](2-naphthyl)}-1-perhydro-2H-pyran-2-yl-1H-indazole-5-carbonitrile (1.10 g., 98% yield). ES-MS (m/z) 443[M+1]+.

### D. 2-[((2S)-1-ethylpyrrolidin-2-yl)methoxy]-6-{5-[3-(2,2-dimethylpropyl)(1H-1,2,4,-triazol-5-yl)](1H-indazol-3-yl)}naphthalene

The title compound was prepared as described in Example 52, step D using (3-{6-[((2S)-1-ethylpyrrolidin-2-y1)methoxy](2-naphthyl}(1H-indazol-5-yl))ethoxymethanimine (0.550 g., 1.24 mmol) and N-amino-3,3-dimethylbutanamide (0.323 g., 2.488 mmol) and purified via preparative HPLC (20-80% acetonitrile /water, 60 mL/min.) to provide the title compound in 100% purity by analytical HPLC (0.061 g., 9.6% yield). 1H- NMR (CHCl3) δ 8.83 (s, 1H), 8.39 (s, 1H), 8.20 (d, 1H), 8.09 (d, 1H), 7.83 (m, 2H), 7.58 (d, 1H), 7.19 (m, 2H), 4.16 (m, 1H), 4.0 (m, 1H), 3.26 (t, 1H), 3.05 (m, 1H), 2.97 (m, 1H), 2.78 (s, 2H), 2.50 (m, 1H), 2.33 (m, 1H), 2.21 (m, 1H), 2.05 (m, 1H), 1.85 (m, 4H), 1.10 (t, 3H), 1.04 (q, 2H). ES-MS (m/z) 509[M+1]⁺.

### EXAMPLE 57

### SYNTHESIS OF 2-[((2S)-1-ETHYLPYRROLIDIN-2-YL)METHOXY]-6-{5-[3-(2-METHYLPROPYL)(1H-1,2,4,-TRIAZOL-5-YL)](1H-INDAZOL-3-YL)}NAPHTHALENE

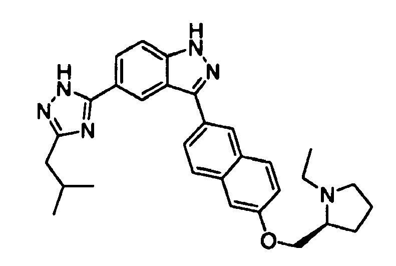

### A. 2-{((2S)-1-ethylpyrrolidin-2-yl)methoxy]-6-{5-[3-(2-methylpropyl)(1H-1,2,4-triazol-5-yl)](1H-indazol-3-yl)}naphthalene

The title compound was prepared as described in Example 52, step D using (3-{6-[((2S)-1-ethylpyrrolidin-2-yl)methoxy](2-naphthyl}(1H-indazol-5-yl))ethoxymethanimine (0.550 g., 1.244 mmol), N-amino-e-methylbutanamide (0.288 g., 2.48 mmol) and purified via preparative HPLC (20-80% acetonitrile/water, 60mL/min.) to afford the title compound in 98% purity by analytical HPLC (0.110 g., 18% yield). ¹H- NMR (CHCl₃) δ 8.80 (s, 1H), 8.36 (s, 1H), 8.15 (d, 1H), 8.05 (d, 1H), 7.8 (dd, 2H), 7.54 (d, 1H), 7.17 (m, 2H), 4.16 (m, 1H), 4.0 (t, 2H), 3.26 (t, 1H), 3.06 (m, 1H), 2.97 (m, 1H), 2.74 (d, 2H), 2.50 (m, 1H), 2.33 (m, 1H), 2.21 (m, 1H), 2.05 (m, 1H), 1.85 (m, 4H), 1.19 (t, 3H), 1.02 (d, 6H). ES-MS (m/z) 495[M+1]⁺.

### EXAMPLE 58

### SYNTHESIS OF 1-((6S,2R)-2,6-DIMETHYPIPERIDYL)-2-(6-[5-[3-(2-METHYLPROPYL)(1H-1,2,4-TRIAZOL-5-YL)](1H-INDAZOL-3-YL)}(2-NAPHTHYLOXY))ETHAN-1-ONE

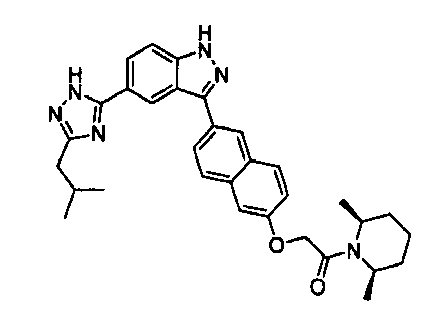

### A. 1-((6S,2R)-2,6-dimethypiperidyl)-2-chloroethan-1-one

To a solution of cis-2,6-dimethylpiperidine (10.0 g., 88.33 mmol) in benzene (500 mL) was added triethylamine (8.92 g., 88.33 mmol) and chloroacetyl chloride (9.97 g., 88.33 mmol). The solution was stirred at ambient temperature for 16 hours. The resultant precipitate was filtered through a fritted glass funnel and the filtrate condensed under reduced pressure to afford a brown solid (12.83 g., 76% yield). Characterization via ¹H-NMR was inconclusive. The material was taken on to the next step.

### B. 1-((6S,2R)-2,6-dimethylpiperidyl)-2-(6-bromo(2-naphthyloxy))ethan-1-one

1-((65,2R)-2,6-dimethypiperidyl)-2-chloroethan-1-one (12.83 g., 67.88 mmol), 6-bromo-2-naphthol (14.38 g., 64.48 mmol) and potassium carbonate (18.76 g., 135.76 mmol) were stirred in dimethylformamide (300 mL) at 85°C for 16 hours. The solution was condensed under reduced pressure and partitioned between water and ethyl acetate (3X). The organics were combined, dried over magnesium sulfate, magnesium sulfate filtered and solvent removed under reduced pressure. The resultant oil was purified via silica gel chromatography (20-40% ethyl acetate/hexanes) to afford the title compound (11.65 g., 46% yield). ES-MS (m/z) 376[M+1]⁺, 378 [M+2]⁺.

### C. 3-{6-[2-((6S,2R)-2,6-dimethylpiperidyl)-2-oxoethyl](2-naphthyl)}-1-perhydro-2H-pyran-2-yl-1H-indazole-5-carbonitrile

The title compound was prepared as described in example 52, step B using 1-((6S,2R)-2,6-dimethylpiperidyl)-2-(6-bromo(2-naphthyloxy))ethan-1-one (6.0 g., 15.95 mmol), 3-Bromo-1-(tetrahydro-pyran-2-yl)-1H-indazole-5-carbonitrile (4.88 g., 15.94 mmol) and purified via silica gel chromatography (30-50% ethyl acetate/hexanes) to afford the title compound (2.0 g., 24% yield). ES-MS (m/z) 523[M+1]⁺.

### D. 1-((6S,2R)-2,6-dimethylpiperidyl)-2-{6-[5-(ethoxyiminomethyl)(1H-indazol-3- yl)](2-naphthyloxy)}ethan-1-one

3-{6-[2-((6S,2R)-2,6-dimethylpiperidyl)-2-oxoethyl](2-naphthyl)}-1-perhydro-2H-pyran-2-yl-1H-indazole-5-carbonitrile (2.0 g., 3.83 mmol) was dissolved in ethanol and cooled to 0°C using an ice bath. Hydrogen chloride gas was bubbled into the solution for 15 minutes and then allowed to stir at ambient temperature for 16 hours. The solution was condensed under reduced pressure to afford the title compound (1.79 g., 84% yield). ES-MS (m/z) 485 [M+1]⁺.

### E. 1-((6S, 2R)-2,6-dimethylpiperidyl)-2-(6-{5-[3-(2-methylpropyl)(1H_1,2,4,-triazol-5-yl)](1H-indazol-3-yl)}(2-naphthyloxy))ethan-1-one

The title compound was prepared as described in Example 52, step D using 1-((6S, 2R)-2,6-dimethylpiperidyl)-2-{6-[5-(ethoxyiminomethyl)(1H-indazol-3-yl)](2-naphthyloxy)}ethan-1-one (0.895 g., 1.85 mmol), N-amino-3-methylbutanamide (0.644 g., 5.55 mmol) and purified via preparative HPLC (30-100% acetonitrile/water) to afford the title compound in 99% purity via HPLC (0.44 g., 44% yield). ¹H- NMR (CD₃OD) δ 8.81 (s, 1H), 8.46 (s, 1H), 8.09 (m, 2H), 7.97 (d, 1H), 7.89 (d, 1H), 7.64 (d, 1H), 7.28 (m, 2H). 2.70 (d, 2H), 2.15 (m, 1H), 1.91 (bs, 1H), 1.67 (bs, 3H), 1.40 (bm, 6H), 0.98 (d, 6H). ES-MS (m/z) 537[M+1]⁺.

### EXAMPLE 59

### SYNTHESIS OF 1-((6S,2R)-2,6-DIMETHYPIPERIDYL)-2-(6-[5-[3-(2,2-DIMETHYLPROPYL)(1H-1,2,4,-TRIAZOL-5-YL)](1H-INDAZOL-3-YL)}(2-NAPHTHYLOXY))ETHAN-1-ONE

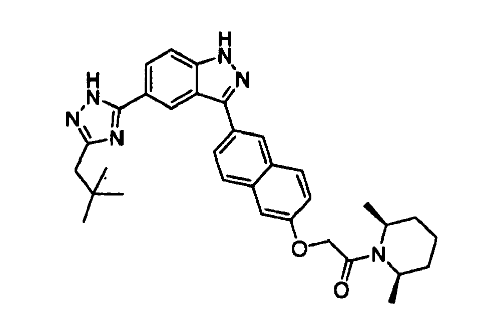

The title compound was prepared as described in Example 52, step D using 1-((6S, 2R)-2,6-dimethylpiperidyl)-2-{6-[5-(ethoxyiminomethyl)(1H-indazol-3-yl)](2-naphthyloxy)}ethan-1-one (0.895 g., 1.85 mmol), N-amino-3,3-dimethylbutanamide (0.721 g., 5.55 mmol) and purified via preparative HPLC (30-100% acetonitrile/water) to afford the title compound in 99% purity by analytical HPLC(0.140 g., 14% yield). ¹H-NMR (DMSO) δ 8.73 (s, 1H), 8.44 (s, 1H), 8.09 (m, 2H), 8.02 (d, 2H), 7.94 (d, 2H), 7.68 (d, 1H), 7.34 (d, 1H), 7.27 (dd, 1H), 4.02 (s, 1H), 2.64 (s, 2H), 1.6 (bm, 3H), 1.3 (bm, 6H), 1.00 (s, 9H). ES-MS (m/z) 551[M+1]⁺.

### EXAMPLE 60

### SYNTHESIS OF 2-[((2R)-1-ETHYLPYRROLIDIN-2-YL)METHOXY]-6-{5-[3-(2-METHYLPROPYL)(1H-1,2,4-TRIAZOL-5-YL)](1H-INDAZOL-3-YL)}NAPHTHALENE

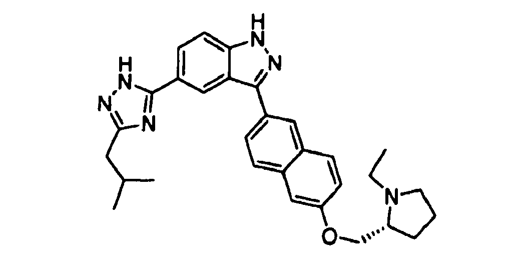

### A. 2-[((2R)-1-ethylpyrrolidin-2-yl)methoxy]-6-bromonsphthalene

The title compound was prepared as described in Example 52, step A using ((2R)-1-ethylpyrrolidin-2-yl)methan-1-ol (6.55 g., 50.77 mmol), 6-bromo-2-naphthol (7.54 g., 33.84 mmol) and the resultant oil purified via silica gel chromatography (3-10% methanol/dichloromethane) to afford the title compound (6.0 g., 56% yield). ES-MS (m/z) 334[M+1]⁺, 335 [M+2]⁺.

### B. 3-{6-[((2R)-1-ethylpyrrolidin-2-yl)methoxy](2-naphthyl)}-1-perhydro-2H-pyran-2-yl-1H-indazole-5-carbonitrile

The title compound was prepared as described in Example 52, step B using 2-[((2R)-1-ethylpyrrolidin-2-yl)methoxy]-6-bromonaphthalene (5.95 g, 17.86 mmol) and purified via silica gel chromatography (10-15% methanol/dichloromethane) to afford the title compound (3.4 g., 16% yield). ES-MS (m/z) 481[M+1]⁺.

### C. (3-{6-[((2R)-1-ethylpyrrolidin-2-yl)methoxy](2-naphthyl}(1H-indazol-5-yl))ethoxymethanimine

The title compound was prepared as described in Example 52, step C using 3-{6-[((2R)-1-ethylpyrrolidin-2-yl)methoxy](2-naphthyl)}-1-perhydro-2H-pyran-2-yl-1H-indazole-5-carbonitrile (3.4 g., 0.833 mmol) to afford the title compound (3.75 g., >100% yield). ES-ES-MS (m/z) 443[M+1]⁺.

### D. 2-{((2R)-1-ethylpyrrolidin-2-yl)methoxy]-6-{5-[3-(2-methylpropyl)(1H-1,2,4-triazol-5-yl)](1H-indazol-3-yl)}naphthalene

The title compound was prepared as described in Example 52, step D using (3-{6-[((2R)-1-ethylpyrrolidin-2-yl)methoxy](2-naphthyl}(1H-indazol-5-yl))ethoxymethanimine (1.8 g., 4.23 mmol), N-amino-3-methylbutanamide (0.981 g., 8.46 mmol) and purified via preparative HPLC (20-80% acetonitrile/water, 60mL/min.) to afford the title compound in 99% purity by analytical HPLC(0.196 g., 9.3% yield). ¹H- NMR (CHCl₃) δ 8.80 (s, 1H), 8.36 (s, 1H), 8.15 (d, 1H), 8.04 (d, 1H), 7.80 (m, 2H), 7.56 (d, 1H), 7.17 (bs, 2H), 4.05 (m, 1H), 3.02 (bm, 2H), 2.73 (d, 2H), 2.49 (m, 1H), 2.19 (bm, 1H), 1.84 (m, 3H), 1.16 (m, 3H), 1.02 (d, 3H). ES-MS (m/z) 495[M+1]⁺.

### EXAMPLE 61

### SYNTHESIS OF 2-(2-(2-PYRIDYL)ETHOXY)-6-{5-[3-(PYRROLIDINYLMETHYL)(1H,1,2,4-TRIAZOL-5-YL)(1H-INDAZOL-3-YL)}NAPHTHALENE

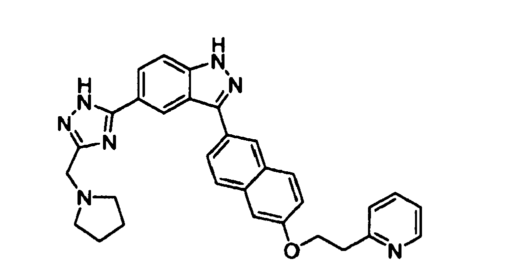

### A. 1-perhydro-2H-pyran-2-yl-3-(6-(2-(2-pyridyl)ethoxy)(2-naphthyl)]-1H-indazole-5-Carbonitrile

To a solution of 3-(6-hydroxy(2-naphthyl))-1-perhydro-2H-pyran-2-yl-1H-indazole-5-carbonitrile (2.3 g., 6.23 mmol), triphenyl phosphine (2.44 g., 9.34 mmol) and 2-(2-hydroxyethylpyridine) (1.15 g., 9.34 mmol) in tetrahydrofuran was added diethylazodicarboxylate (1.88 g., 9.34 mmol). The solution stirred at ambient and followed by TLC and ES-MS to assure consumption of starting material. The solution was condensed under reduced pressure and the resultant oil partitioned between water and ethyl acetate (3X). The organics were dried over magnesium sulfate, magnesium sulfate filtered, and solvent removed. The oil was then purified via silica gel chromatography (30-60% ethyl acetate/hexanes) to afford the title compound (1.6 g., 54% yield). ES-MS (m/z) 475 [M+1]⁺.

### B. ethoxy{3-[6-(2-(2-pyridyl)ethoxy)(2-naphthyl)](1H-indazol-5-yl)}methanimine

The title compound was prepared as described in Example 52, step C using 1-perhydro-2H-pyran-2-yl-3-(6-(2-(2-pyridyl)ethoxy)(2-naphthyl)]-1H-indazole-5-carbonitrile (1.60 g., 3.37 mmol) to afford the title compound (1.83 g., >100% yield). ES-MS (m/z) 437 [M+1]⁺.

### C. 2-(2-(2-pyridyl)ethoxy)-6-{5-[3-(pyrrolidinylmethyl)(1H-1,2,4-triazol-5-yl)](1H-indazol-3-yl)}naphthalene

The title compound was prepared as described in Example 52, step D using ethoxy{3-[6-(2-(2-pyridyl)ethoxy)(2-naphthyl)](1H-indazol-5-yl)}methanimine (0.915 g., 1.790 mmol), N-amino-2-pyrrolidinylacetamide (0.60 g., 3.59 mmol) and purified via preparative HPLC (20-80% acetonitrile/water). The resultant solid was further purified via silica gel chromatography (3-12% methanol/methylene chloride) to afford the title compound in 99% purity by analytical HPLC (0.178 g., 19% yield). ¹H- NMR (CDCl₃) δ 8.82 (s, 1H), 8.62 (d, 1H), 8.31 (s, 1H), 8.16 (d, 1H), 8.03 (d, 1H), 7.69 (bm, 3H), 7.50 (d, 1H), 7.33 (d, 1H), d7.20 (t, 1H), 7.09 (bm, 2H), 4.49 (t, 2H), 3.98 (s, 2H), 3.64 (t, 2H), 2.73 (bs, 4H), 1.86 (bs, 4H). ES-MS (m/z) 516 [M+1]⁺.

### EXAMPLE 62

### SYNTHESIS OF 6-{5-[3-(MORPHOLIN-4-YLMETHYL)(1H-1,2,4-TRIAZOL-5-YL)](1H-INDAZOL-3-YL)}-2-(2-(2-PYRIDYL)ETHOXY)NAPHTHALENE

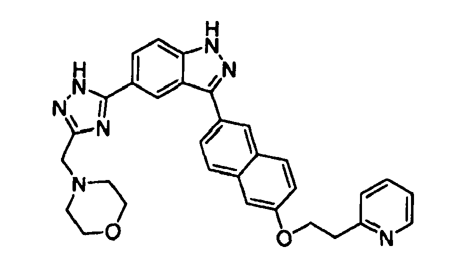

### A. 1-perhydro-2H-pyran-2-yl-3-(6-(2-(2-pyridyl)ethoxy)(2-naphthyl)]-1H-indazole-5-Carbonitrile

To a solution of 3-(6-hydroxy(2-naphthyl))-1-perhydro-2H-pyran-2-yl-1H-indazole-5-carbonitrile (2.3 g., 6.23 mmol), triphenyl phosphine (2.44 g., 9.34 mmol) and 2-(2-hydroxyethylpyridine) (1.15 g., 9.34 mmol) in tetrahydrofuran was added diethylazodicarboxylate (1.88 g., 9.34 mmol). The solution stirred at ambient and followed by TLC and ES-MS to assure consumption of starting material. The solution was condensed under reduced pressure and the resultant oil partitioned between water and ethyl acetate (3X). The organics were dried over magnesium sulfate, magnesium sulfate filtered, and solvent removed. The oil was then purified via silica gel chromatography (30-60% ethyl acetate/hexanes) to afford the title compound (1.6 g., 54% yield). ES-MS (m/z) 475 [M+1]⁺.

### B. Ethoxy{3-[6-(2-(2-pyridyl)ethoxy)(2-naphthyl)](1H-indazol-5-yl)}methanimine

The title compound was prepared as described in Example 1, step C using 1-perhydro-2H-pyran-2-yl-3-(6-(2-(2-pyridyl)ethoxy)(2-naphthyl)]-1H-indazole-5-carbonitrile (1.60 g., 3.37 mmol) to afford the title compound (1.83 g., >100% yield). ES-MS (m/z) 437 [M+1]⁺.

### C. 6-{5-[3-(morpholin4-ylmethyl)(1H-1,2,4,-triazol-5-yl)](1H-indazol-3-yl)}-2-(2-(2-pyridyl)ethoxy)naphthalene

To a solution of ethoxy{3-[6-(2-(2-pyridyl)ethoxy)(2-naphthyl)](1H-indazol-5-yl)}methanimine (0.915 g., 1.79 mmol) and triethylamine (0.903 g., 8.95 mmol) in tetrahydrofuran (20 mL) was added N-amino-2-morpholin-4-ylacetamide (0.571 g., 3.59 mmol). The solution was stirred at 95°C for 16 hours. The solution was partitioned between water and ethyl acetate (3X). The organics were dried over magnesium sulfate, magnesium sulfate was filtered and solvent removed under reduced pressure. The resulting solid was purified via preparative HPLC (20-80% acetonitrile/water) to afford the title compound in 100% purity by analytical HPLC (0.077 g., 8% yield). ¹H- NMR (CDCl₃) δ 8.81 (s, 1H), 8.61 (d, 1H), 8.36 (s, 1H), 8.16 (d, 1H), 8.06 (d, 1H), 7.80 (dd, 2H), 7.67 (m, 1H), 7.57 (d, 1H), 7.34 (d, 1H), 7.16 (m, 3H), 4.53 (t, 2H), 3.80 (s, 2H), 3.76 (t, 4H), 3.38 (t, 2H), 2.61 (t, 4H). ES-MS (m/z) 532 [M+1]⁺.

### EXAMPLE 63

### SYNTHESIS OF 2-[((2R)-1-ETHYLPYRROLIDIN-2-YL)METHOXY]-6-{5-[3-(2,2-DIMETHYLPROPYL)(1 H-1,2,4-TRIAZOL-5-YL)](1H-INDAZOL-3-YL)}NAPHTHALENE

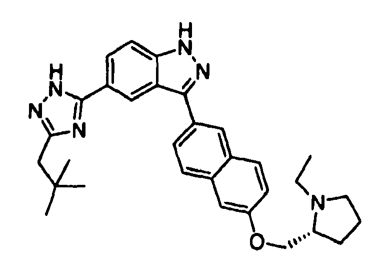

The title compound was prepared as described in Example 60, step D using (3-{6-[((2R)-1-ethylpyrrolidin-2-yl)methoxy](2-naphthyl}(1H-indazol-5-yl))ethoxymethanimine (1.8 g., 4.23 mmol). N-amino-3,3-dimethylbutanamide (1.05 g., 8.12 mmol). The resultant sold was purified via preparative HPLC (20-60% acetonitrile/water, 60mL/min.) to afford the title compound in 98% purity by analytical HPLC (0.013 g., 0.6% yield). ¹H- NMR (CD₃OD) δ 8.95 (s, 1H), 8.58 (s, 1H), 8.22 (dd, 2H), 8.06 (dd, 2H), 7.79 (d, 1H), 7.44 (s, 1H), 7.34 (dd, 1H), 4.3 (m, 2H), 2.92 (s, 2H), 2.64 (bm, 3H), 2.0 (bm, 4H), 1.35 (bs, 5H), 1.05 (s, 9H). ES-MS (m/z) 509[M+1]⁺.

### EXAMPLE 64

### SYNTHESIS OF 6-{5-[3-(2,2-DIMETHYLPROPYL)(1H-1,2,4-TRIAZOL-5-YL)](1H-INDAZOL-3-YL)}-2-(2-(2-PYRIDYL)ETHOXY)NAPHTHALENE

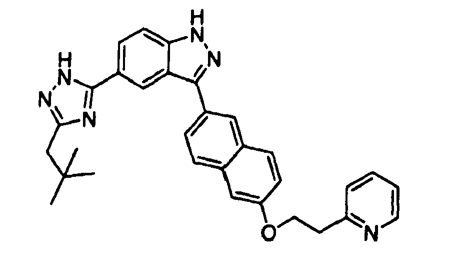

### A. 6-{5-[3-(2,2-dimethylpropyl)(1H-1,2,4,-triazol-3-yl)}-2-(2-(2-pyridyl)ethoxy)naphthalene

The title compound was prepared as described in Example 60, step C using ethoxy{ 3-[6-(2-(2-pyridyl)ethoxy)(2-naphthyl)](1H-indazol-5-yl) }methanimine (0.790 g., 1.55 mmol), N-amino-3,3-dimethylbutanamide (0.403 g., 2.76 mmol) and purified via preparative HPLC (10-50% acetonitrile/water) to afford the title compound in 100% yield by analytical HPLC (0.07 g., 9% yield). ¹H- NMR (CD₃OD) δ 8.88 (s, 1H), 8.54 (d, 1H), 8.49 (s, 1H), 8.14 (dd, 1H), 7.96 (m, 2H), 7.85 (td, 1H), 7.71 (bs, 1H), 7.53 (d, 1H), 7.37 (m, 2H), 7.19 (dd, 1H), 4.54 (t, 2H), 3.40 (t, 2H), 2.79 (s, 2H), 1.09 (s, 9H). ES-MS (m/z) 502 [M+1]⁺.

### EXAMPLE 65

### SYNTHESIS OF 4-{[5-(3-(6-QUINOLYL)-1H-INDAZOL-5-YL)-1H,1,2,4-TRIAZOL-3-YL]METHYL}MORPHOLINE

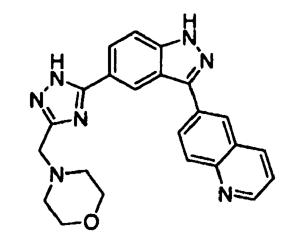

### A. 6-quinolyl(trifluoromethyl)sulfonate

To a solution of 6-hydroxyquinoline (2.0 g., 13.77 mmol) in methanol (50 mL) was added N-phenyltrifluoromethanesulfonate (5.4 g., 15.15 mmol) and diisopropylethylamine (3.55 g., 27.5 mmol). The mixture stirred at ambient temperature for one hour until starting material was consumed by TLC. The solution was condensed under reduced pressure and partitioned between water and ethyl acetate (3X) to afford an oil. The oil was purified via silica gel chromatography (30-40% ethyl acetate/hexanes) to afford the title compound (2.75 g., 72% yield). ES-MS (m/z) 278 [M+1]⁺.

### B. 1-perhydro-2H-pyran-2-yl-(6-quinolyl)-1H-indazol-5-carbonitrile

The title compound was prepared as described in Example 5, step **B** using 6-quinolyl(trifluoromethyl)sulfonate (2.0 g., 7.22 mmol) and purified via silica gel chromatography (40-50% ethyl acetate/hexanes) to afford the title compound (0.546 g., 21% yield). ES-MS (m/z) 354 [M+1]⁺.

### C. Ethoxy(1-perhydro-2H-pyran-2-yl-3-(6-quinolyl)(1H-indazol-5-yl))methanimine

The title compound was prepared as described in Example 52, step C using 1-perhydro-2H-pyran-2-yl-(6-quinolyl)-1H-indazol-5-carbonitrile (0.545 g., 1.2 mmol) to afford the title compound (0.288 g., 77% yield). ES-MS (m/z) 317 [M+1]⁺.

### D. 4-{[5-(3-(6-quinolyl)-1H-indazol-5-yl)-1H-1,2,4-triazol-3-yl]methyl)morpholine

The title compound was prepared as described in Example 52, step D using ethoxy(1-perhydro-2H-pyran-2-yl-3-(6-quinolyl)(1H-indazol-5-yl))methanimine (0.288 g., 0.911 mmol) and N-amino-2-morpholin-4-ylacetamide (0.217 g., 1.37 mmol) to afford the title compound in 100% purity by analytical HPLC (0.071 g., 23% yield).). ¹H- NMR (CD₃OD) δ 8.86 (s, 1H), 8.84 (dd, 1H), 8.63 (d, 1H), 8.54 (d, 1H), 8.48 (dd, 1H). 8.15 (d, 1H), 8.5 (bs, 1H), 7.68 (bs, 1H), 7.58 (dd, 1H), 3.75 (bs, 2H), 3.70 (t, 4H), 2.56 (bs, 4H). ES-MS (m/z) 412 [M+1]⁺.

### EXAMPLE 66

### SYNTHESIS OF 3-[6-(1,4-DIMETHYL-PIPERAZIN-2-YLMETHOXY)-NAPHTHALEN-2-YL]-5-(5-ISOBUTYL-1H-[1,2,4]TRIAZOL-3-YL)1H-INDAZOLE

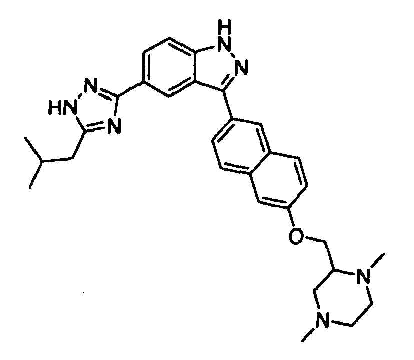

### A. 3-[6-(1,4-Dimethyl-piperazin-2-ylmethoxy)-naphthalen-2-yl]-1-(tetrahydro-pyran-2-yl)1H-indazole-5-carbonitrile

To an ice bath cooled solution of 3-(6-Hydroxy-naphthalen-2-yl)-1-(tetrahydro-pyran-2-yl)-1H-indazole-5-carbonitrile (1.20g, 3.25mmol), (1,4-Dimethyl-piperain-2-yl)-methanol (0.67g, 4.75mmol, 1.5eq.), and triphenylphosphine (1.24g, 4.75mmol, 1.5eq.) in THF (20mL) was added diisopropyl azodicarboxylate (0.95g, 4.75mmo1, 1.5eq.). Reaction was monitored by TLC (10%Methanol/Dichloromethane) and was complete after four hours. Solvent was removed in *vacuo* and subjected to Biotage column chromatography to afford 1.00g (63% yield) of the title compound as yellow solids. ES-MS (m/z) 495 [M+1].

### B. 3-[6-(1,4-Dimethyl-piperazin-2-ylmethoxy)-naphthalen-2-yl]-1H-indazole-5-carboximidic acid ethyl ester

To a dry ice/acetone bath cooled solution of 3-[6-(1,4-Dimethyl-piperazin-2-ylmethoxy)-naphthalen-2-yl]-1-(tetrahydro-pyran-2-yl)-1H-indazole-5-carbonitrile (1.00g, 2.00mmol) in ethanol (100mL) was bubbled through HCI(g) for twenty minutes. Reaction was monitored by LCMS and was complete after 48 hours. Solvent was removed *in vacuo* and was triturated with diethyl ether. The solids were filtered to afford 0.66g (91% yield) of the title compound as yellow solids. ES-MS (m/z) 457 [M+1].

### C. 3-[6-(1,4-Dimethyl-piperazin-2-ylmethoxy)-naphthalen-2-yl]-5-(5-isobutyl-1H-[1,2,4]triazol-3-yl)1H-indazole

To a solution of 3-[6-(1,4-Dimethyl-piperazin-2-ylmethoxy)-naphthalen-2-yl]-1H-indazole-5-carboximidic acid ethyl ester (0.30g, .065mmol) in methanol (6ml) was added 3-Methyl-butyric acid hydrazide (0.30g, 2.62mmol, 4.0eq.) and triethylamine (1.82mL, 13.0mmol, 20 eq.). The reaction was heated to 90 degrees in a sealed tube overnight. The reaction was monitored by LCMS and was complete after 24 hours. The solvent was removed *in vacuo* and was subjected to Prep HPLC (20-80% acetonitrile/water +0.1 % TFA) to afford 76mg (23% yield) of the title compound as white solids. ¹H NMR (CD3OD) δ 8.81(s, 1H), 8.42(s, 1H), 8.10(dd, 2H), 7.90-7.99(q, 2H), 7.62(dd, 1H), 7.20-7.30(dd, 2H), 4.20(m, 1H), 3.45(s, 2H), 3.30(s, 2H), 3.1-2.5(mm, 6H), 2.45(s, 3H), 2.40(s, 2H), 1.0(ds, 6H). ES-MS(m/z) 509 [M+1].

### EXAMPLE 67

### 3-[6-(1,4-DIMETHYL-PIPERAZIN-2-YLMETHOXY)-NAPHTHALEN-2:YL]-5-[5-(2,2-DIMETHYL-PROPYL)-1H-[1,2,4]TRIAZOL-3-YL]1H-INDAZOLE

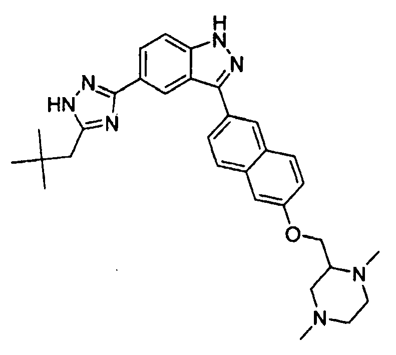

To a solution of 3-[6-(1,4-Dimethyl-piperazin-2-ylmethoxy)-naphthalen-2-yl]-1H-indazole-5-carboximidic acid ethyl ester (0.30g, .065mmol) in methanol (6ml) was added 3,3-Dimethyl-butyric acid hydrazide (0.34g, 2.62mmol, 4.0eq.) and triethylamine (1.82mL, 13.0mmol, 20eq.). The reaction was heated to 90 degrees in a sealed tube overnight. The reaction was monitored by LCMS and was complete after 24 hours. The solvent was removed *in vacuo* and was subjected to Prep HPLC (20-80% acetonitrile/water +0.1% TFA) to afford 76mg (23% yield) of the title compound as white solids. ¹H NMR (CD30D) δ 8.90(s, 1H), 8.60(s, 1H), 8.25(dd, 2H), 8.15(q, 2H), 7.80(dd, 1H), 7.45(d, 1H), 7.35(dd, 1H), 4.35(m, 1H), 3.45(s, 2H), 3.30-2.60(mm, 6H), 2.55(s, 3H), 2.50(s, 1H), 1.25(s, 9H). ES-MS (m/z) 523 [M+1].

### EXAMPLE 68

### 5-[5-(2,2-DIMETHYL-PROPYL)-1H-[1,2,4]TRIAZOL-3-YL]-3-[6-(2-IMIDAZOL-1-YL-ETHOXY)-NAPHTHALEN-2-YL]-1H-INDAZOLE

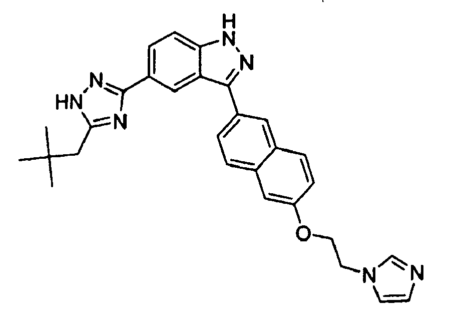

### A. 3-[6-(2-Imidazol-1-yl-ethoxyrnaphthalen-2-yl]-1-(tetrahydro-pyran-2-yl)-1H-indazole-5-carbonitrile

To an ice bath cooled solution of 3-(6-Hydroxy-naphthalen-2-yl)-1-(tetrahydro-pyran-2-yl)-1H-indazole-5-carbonitrile (1.50g 4.00mmol), 2-Imidazol-1-yl-ethanol (0.67g, 6.00mmol, 1.5eq.), and triphenylphosphine (1.57g, 6.00mmol, 1.5eq.) in THF (100mL) was added diisopropyl azodicarboxylate (1.18mL, 6.0mmol, 1.5eq.). Reaction was monitored by TLC (10%Methanol/Dichloromethane) and was complete after four hours. Solvent was removed *in vacuo* and subjected to Biotage column chromatography to afford 0.60g (38% yield) of the title compound as yellow solids. ES-MS (m/z) 463 [M+1].

### B. 3-[6-(2-Imidazol-1-yl-ethoxy)-naphthalen-2-yl]-1H-indazole-5-carboximidic acid ethyl ester

To a dry ice/acetone bath cooled solution of 3-[6-(2-Imidazol-1-yl-ethoxy)-naphthalen-2-yl]-1-(tetrahydro-pyran-2-yl)-1H-indazole-5-carbonitrile (0.60g, 13.Ommol) in ethanol (100mL) was bubbled through HCl(g) for twenty minutes. Reaction was monitored by LCMS and was complete after 48 hours. Solvent was removed *in vacuo* and was triturated with diethyl ether. The solids were filtered to afford 0.34g (62% yield) of the title compound as yellow solids. ES-MS (m/z) 425 [M+1].

### C. 5-[5-(2,2-Dimethyl-propyl)-1H-[1,2,4]triazol-3-yl]-3-[6-(2-imidazol-1-yl-ethoxy)-naphthalen-2-yl]-1H-indazole

To a solution of 3-[6-(2-Imidazol-1-yl-ethoxy)-naphthalen-2-yl]-1H-indazole-5-carboximidic acid ethyl ester (0.34g, 0.80mmol) in methanol (6mL) was added 3,3-Dimethyl-butyric acid hydrazide (0.42g, 3.2mmol, 4.0eq.) and triethylamine (1.11mL, 8.00mmol, 10.0eq.). The reaction was heated to 90 degrees in a sealed tube overnight. The reaction was monitored by LCMS and was complete after 24 hours. The solvent was removed *in vacuo* and was subjected to Prep HPLC (20-80% acetonitrile/water +0.1% TFA) to afford 25.8mg (7% yield) of the title compound as white solids. ¹H NMR (CD3OD) δ 8.78(s, 1H), 8.37(s, 1H), 8.00(t, 2H), 7.80(dd, 2H), 7.65(s, 1H), 7.55(d, 1H), 7.15(dd, 2H), 7.05(dd, 1H), 6.90(s, 1H), 4.40-4.30(m, 4H), 2.60(s, 2H), 0.95(s, 9H). ES-MS (m/z) 492 [M+1].

### EXAMPLE 69

### 3-{6-[2-(2,6-DIMETHYL-PIPERIDIN-1-YL)-ETHOXY]-NAPHTHALEN-2-YL}-5-[5-(2,2,-DIMETHYL-PROPYL)-1H-[1,2,4]TRIAZOL-3-YL]-1H-INDAZOLE

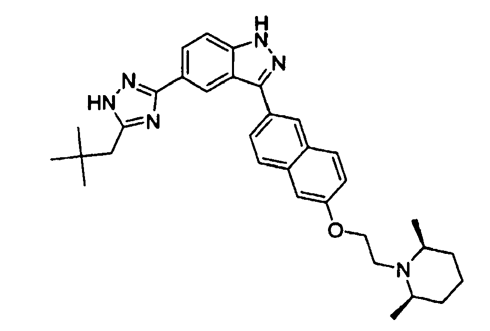

To a solution of 3-{6-[2-(2,6-Dimethyl-piperidin-1-yl)-ethoxy]-naphthalen-2-yl}-1H-indazole-5-carboximidic acid ethyl ester (0.50g, 1.06mmol) was added Dimethyl-butyric acid hydrazide (0.55g, 4.25mmol, 4.0eq.) and triethylamine (1.47mL, 10.60mmol, 10.0eq.). The reaction was heated to 90 degrees in a sealed tube overnight. The reaction was monitored by LCMS and was complete after 24 hours. The solvent was removed *in vacuo* and was subjected to Prep HPLC (20-80% acetonitrile/water +0.1% TFA) to afford 26mg (5% yield) of the title compound as white solids. ¹H NMR (CD3OD)δ 8.75(s, 1H), 8.35(s, 1H), 8.00(d, 2H), 7.82(q, 2H), 7.55(d, 1H), 7.20(s, 1H), 7.05(dd, 1H), 4.10(m, 2H), 3.20(m, 2H), 3.10(m, 2H), 2.60(m, 4H), 2.05(s, 2H), 1.15(ds, 6H), 0.95(s, 9H). ES-MS (m/z) 536 [M+1].

### EXAMPLE 70

### 5-[5-(2,2-DIMETHYL-PROPYL)-1H-[1,2,4]TRIAZOL-3-YL]-3-[6-(PYRIDINE-2-YLMETHOXY)-NAPHTHALEN-2-YL]-1H-INDAZOLE

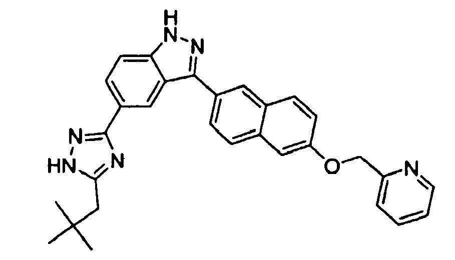

### A. 2-(6-Bromo-naphthalen-2-yloxymethyl)-pyridine

To an ice bath cooled solution of 6-bromonaphthol (6.13g, 27.5mmol), pyridine-2-yl-methanol (2.64mL, 27.5mmol, 1.0eq.), and triphenylphosphine (10.8g, 41.30mmol, 1.5eq.) in THF was added diisopropyl azodicarboxylate (8.12mL, 41.3mmol, 1.5eq.). The reaction was monitored by TLC (30% ethyl acetate/hexanes) and was complete after 24 hours. Solvent was removed *in vacuo* and subjected to Biotage column chromatography to afford 9.00g (100% yield) of the title compound as tan solids. ES-MS (m/z) 313 [M+1].

### B. 3-[6-(Pyridin-2-ylmethoxy)-naphthalen-2-yl]-1-(tetrahydro-pyran-2-yl)-1H-indazole-5-carbonitrile

To a solution of 2-(6-Bromo-naphthalen-2-yloxymethyl)-pyridine (4.97g, 15.8mmol) in DMF (50mL) was added bis(pinnacalato)diboron (4.02g, 15.8mmol, 1.0eq.) and potassium acetate (4.66g, 47.6mmol, 3.0eq.) and palladium II chloride (bis-diphenyl phosphino ferrocene) dichloromethane (1.29g, 10%mmol). Reaction was heated at 80°C overnight and LCMS confirmed formation of boronate ester complex. To the reaction was added 3-bromo-1-perhydro-2H-pyran-2-yl-1H-indazote-5-carbonitrile (4.85g, 15.8mmol, 1.0eq.) and potassium phosphate (10.09g, 47.6mmol, 3.0eq.) and was stirred at 80°C overnight. Reaction was monitored by LCMS and was complete after 24 hours. The solvent was removed *in vacuo* and the residue was washed with water, extracted with ethyl acetate, and subjected to Biotage column chromatography (60% ethyl acetate/hexanes) to afford 2.10g (30% yield) of the title compound as tan solids. ES-MS (m/z) 460 [M+1].

### C. 3-[6-(Pyridin-2-ylmethoxy)-naphthalen-2-yl]-1H-indazole-5-carboximidic acid ethyl ester

To a dry ice/acetone bath cooled solution of 3-[6-(Pyridin-2-ylmethoxy)-naphthalen-2-yl]-1-(tetrahydro-pyran-2-yl)-1H-indazole-5-carbonitrile (2.00g, 4.30mmol) in ethanol (500mL) was bubbled through HCI(g) for twenty minutes. Reaction was monitored by LCMS and was complete after 72 hours. Solvent was removed *in vacuo* and was triturated with diethyl ether. The solids were filtered to afford 1.05g (58% yield) of the title compound as off white solids. ES-MS (m/z) 422 [M+1].

### D. 5-[5-(2,2-Dimethyl-propyl)-1H-[1,2,4]triazol-3-yl]-3-[6-(pyridine-2-ylmethoxy)-naphthalen-2-yl]-1H-indazole

To a solution of 3-[6-(Pyridin-2-ylmethoxy)-naphthalen-2-yl]-1H-indazole-5-carboximidic acid ethyl ester (0.50g, 1.18mmol) was added Dimethyl-butyric acid hydrazide (0.62g, 4.72mmol, 4.0eq.) and triethylamine (3.28mL, 23.6mmol, 20.0eq.). The reaction was heated to 90 degrees in a sealed tube overnight. The reaction was monitored by LCMS and was complete after 24 hours. The solvent was removed *in vacuo* and was subjected to Prep HPLC (20-80% acetonitrile/water +0.1 % TFA) to afford 60mgs (9% yield) of the title compound as white solids. ¹H NMR (CD30D) δ 8.98(s, 1H), 8.70(d, 1H), 8.60(s, 1H), 8.35(dd, 2H), 8.10(d, 1H), 8.00(dd, 2H), 7.80(m, 2H), 7.55-7.40(m, 3H), 5.45(s, 2H), 2.95(s, 2H), 1.05(s, 9H). ES-MS (m/z) 566 [M+1].

### EXAMPLE 71

### 5-(5-ISOBUTYL-1H-[1,2,4]TRIAZOLE-3-YL)-3-[6-(PYRIDINE-2YLMETHOXY)-NAPHTHALEN-2-YL]-1H-INDAZOLE

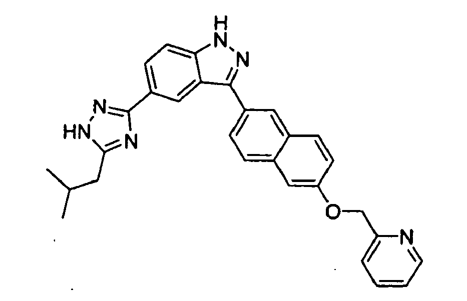

To a solution of 3-[6-(Pyridin-2-ylmethoxy)-naphthalen-2-yl]-1H-indazole-5-carboximidic acid ethyl ester (0.50g, 1.18mmol) was added 3-Methyl-butyric acid hydrazide (0.55g, 4.72mmol, 4.0eq.) and triethylamine (3.28mL, 23.6mmol, 20.0eq.). The reaction was heated to 90 degrees in a sealed tube overnight. The reaction was monitored by LCMS and was complete after 24 hours. The solvent was removed in vacuo and was subjected to Prep HPLC (20-80% acetonitrile/water +0.1% TFA) to afford 171mgs (26% yield) of the title compound as white solids. 1H NMR (CD3OD) δ 8.90(s, 1H), 8.70(dd, 1H), 8.60(s, 1H), 8.25(dd, 2H), 8.15(d, 1H), 8.00(dd, 2H), 7.80(m, 2H), 7.55-7.40(m, 3H), 5.50(s, 2H), 2.95(m, 2H), 2.30(m, 1H), 1.15(ds, 6H). ES-MS (m/z) 552 [M+1].

### EXAMPLE 72

### 5-(5-ISOBUTYL-1H-[1,2,4]TRIAZOL-3-YL)-3-[6-(3-METHYL-3H-IMIDAZOL-4-YLMETHOXY)-NAPHTHALEN-2-YL]-1H-INDAZOLE

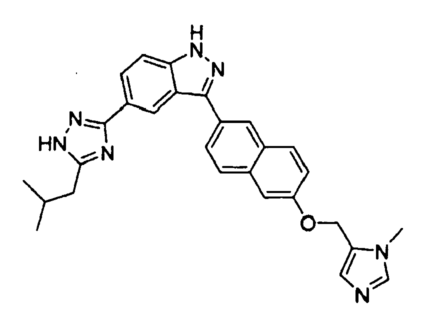

### A. 3-[6-(3-Methyl-3H-imidazol-4-ylmethoxy)-naphthaten-2-yl]-1-(tetrahydro-pyran-2-yl)-1 H-indazole-5-carbonitrile

To a solution of 3-bromo-1-perhydro-2H-pyran-2-yl-1H-indazole-5-carbonitrile (1.27g, 3.43mmol), 3-(Methyl-3H-imidazol-4-yl)-methanol (0.65g, 5.15mmol, 1.5eq.), and triphenylphosphine (2.69g, 10.29mmol, 3.0eq.) cooled to 0 degrees in THF was added diisopropyl azodicarboxylate (2.07g, 10.29mmol, 3.0eq.). Reaction was warmed to room temperature and stirred over the weekend. The solvent was removed *in vacuo* and the oil was subjected to Biotage chromatography (2-5% methanol/dichloromethane) to get 600mg (48% yield) of the title compound as yellow solids. ES-MS (m/z) 381 [M+1].

### B. 3-[6-(3-Methyl-3H-imidazol-4-ylmethoxy)-naphthalen-2-yl]-1H-indazole-5-carboximidic acid ethyl ester

To a solution of 3-[6-(3-Methyl-3H-imidazol-4-ylmethoxy)-naphthalen-2-yl]-1-(tetrahydro-pyran-2-yl)-1H-indazole-5-carbonitrile (0.60g, 1.6mmol) in ethanol cooled to -78 degrees was bubbled through HCI (g) for 30 minutes. Reaction was warmed to room temperature overnight. Solvent was removed and solids were washed with diethyl ether and filtered to get 600mg (100% yield) of the title compound as yellow solids. ES-MS (m/z) 425 [M+1].

### C. 5-(5-Isobutyl-1H-[1,2,4]triazol-3-yl)-3-[6-(3-methyl-3H-imidazol-4-ylmethoxy)-naphthalen-2-yl]-1H-indazole

To a solution of 3-[6-(3-Methyl-3H-imidazol-4-ylmethoxy)-naphthalen-2-yl]-1H-indazole-5-carboximidic acid ethyl ester (600mg, 1.4mmol) was added 3-Methyl-butyric acid hydrazide (0.63g, 5.2mmol, 4.0eq.) and triethylamine (3.64 mL, 26mmol, 20.0eq.). The reaction was heated to 90 degrees in a sealed tube overnight. The reaction was monitored by LCMS and was complete after 24 hours. The solvent was removed in *vacuo* and was subjected to Prep HPLC (20-80% acetonitrile/water +0.1 % TFA) to afford 40mgs (5% yield) of the title compound as white solids. ¹H NMR (CD3OD) δ 8.95(s, 1H), 8.52(s, 1H), 8.15(dd, 2H), 8.0(d, 1H), 7.9(d, 1H), 7.63(s, 1H), 7.55(d, 1H), 7.42(d, 1H), 7.20(dd, 1H), 7.10(d, 1H), 3.75(s, 1H), 3.60(m, 4H), 3.35(m, 1H), 2.00(s, 3H), 1.98(s, 3H), 1.96(s, 6H). ES-MS (m/z) 477 [M+1].

### EXAMPLE 73

### 5-[5-(2,2-DIMETHYL-PROPYL)-1H-[1,2,4]TRIAZOL-3-YL]-3-[6-(2-PYRIDIN-3-YL-ETHOXY)-NAPHTHALEN-2-YL]-1H-INDAZOLE

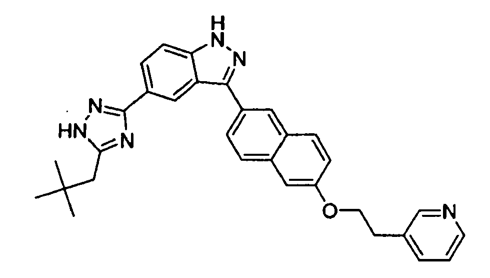

### A. 3-[2-(6-Bromo-naphthalen-2-yloxy)-ethyl]-pyridine

To a solution of 6-Bromo-naphthol (604mg, 2.70mmol), 3-Pyrdin-ethanol (1.00g, 8.10mmol, 3.0eq.), and triphenylphosphine (2.12g, 8.10mmol, 3.0eq.) in THF cooled to 0 degrees was added diisopropyl azodicarboxylate (1.63g, 8.10mmol, 3.0eq.). Reaction was warmed to room temperature and stirred over the weekend. The solvent was removed *in vacuo* and the oil was subjected to Biotage chromatography (30-60% ethyl acetate/hexanes) to afford 800mg (90% yield) of the title compound as yellow solids. ES-MS (m/z) 328 [M+1].

### B. 3-[6-(2-Pyridin-3-yl-ethoxy)-naphthalen-2-yl]-1-(tetrahydro-pyran-2-yl)-1H-indazole-5-carbonitrile

A solution of 3-[2-(6-Bromo-naphthalen-2-yloxy)-ethyl]-pyridine (800mg, 2.43mmol) in DMF was subjected to standard one-pot Suzuki coupling conditions using bis(pinnacalato)diboron (615mg, 2.43mmol, 1.0et.), potassium acetate (714mg, 7.24mmol, 3.0eq.), palladium II chloride (bis-diphenyl phosphino ferrocene) dichloromethane (816mg, 10%mmol), 3-bromo-1-perhydro-2H-pyran-2-yl-1H-indazole-5-carbonitrile (743mg, 2.43mmol, 1.0eq.), and potassium phosphate (1.54g, 7.29mmol, 3.0eq.) to afford 800mg (71% yield) of the title compound as yellow solids. ES-MS (m/z) 474 [M+1].

### C. 3-[6-(2-Pyridin-3-yl-ethoxy)-naphthalen-2-yl]-1H-indazole-5-carboxomidic acid ethyl ester

To a solution of 3-[6-(2-Pyridin-3-yl-ethoxy)-naphthalen-2-yl]-1-(tetrahydro pyran-2-yl)-1H-indazole-5-carbonitrile (0.80g, 1.7mmol) in ethanol cooled to-78 degrees was bubbled through HCI (g) for 30 minutes. Reaction was warmed to room temperature overnight. Solvent was removed and solids were washed with diethyl ether and filtered to get 800mg (100% yield) of the title compound as yellow solids. ES-MS (m/z) 436 [M+1].

### D. 5-[5-(2,2-Dimethyl-propyl)-1H-[1,2,4]triazol-3-yl]-3-[6-(2-pyridin-3-yl-ethoxy)-naphthalen-2-yl]-1H-indazole

To a solution of 3-[6-(2-Pyridin-3-yl-ethoxy)-naphthalen-2-yl]-1H-indazole-5-carboxomidic acid ethyl ester (0.40g, 0.91mmol) was added Dimethyl-butyric acid hydrazide (0.48g, 3.70mmol, 4.0eq.) and triethylamine (2.50mL, 18.0mmol, 20.0eq.). The reaction was heated to 90 degrees in a sealed tube overnight. The reaction was monitored by LCMS and was complete after 24 hours. The solvent was removed in *vacuo* and was subjected to Prep HPLC (20-80% acetonitrile/water +0.1 % TFA) to afford 40mg (2% yield) of the title compound as white solids. ¹H NMR (CD30D) δ 8.60(dd, 2H), 8.42(dd, 1H), 8.38(s, 1H), 8.18(dd, 1H), 8.0-7.7(m, 3H), 7.85(dd, 1H), 7.40(m, 2H), 7.20(dd, 1H), 4.40(t, 2H0, 3.15(t, 2H), 1.50(s, 1H), 1.00(s, 9H). ES-MS (m/z) 502 [M+1].

### EXAMPLE 74

### 3-[6-(2-PYRIDIN-3-YL-ETHOXY)-NAPHTHALEN-2-YL]-5-(5-PYRROLIDIN-1-YLMETHYL-1H-[1,2,4]TRIAZOL-3-YL)-1H-INDAZOLE

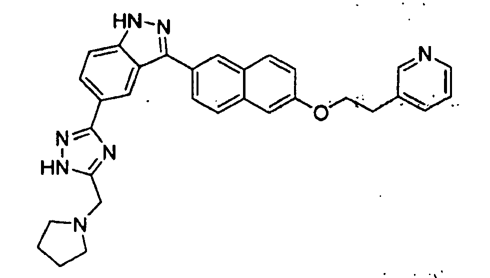

To a solution of 3-[6-(2-Pyridin-3-yl-ethoxy)-naphthalen-2-yl]-1H-indazole-5-carboxomidic acid ethyl ester (0.40g, 0.91mmol) was added pyrrolidin-1-yl-acetic acid hydrazide (529mg, 3.70mmol, 4.0eq.) and triethylamine (2.50mL, 18.0mmol, 20.0eq.). The reaction was heated to 90 degrees in a sealed tube overnight. The reaction was monitored by LCMS and was complete after 24 hours. The solvent was removed in *vacuo* and was subjected to Prep HPLC (20-80% acetonitrile/water +0.1% TFA) to afford 40mg (2% yield) of the title compound as white solids. ¹H NMR (CD30D) δ 8.95(d, 1H), 8.65(d, 1H), 8.50(m, 2H), 8.20(dd, 2H), 8.00(m, 3H), 7.80(dd, 1H), 7.55-7.50(m, 1H), 7.45(d, 1h), 7.30(dd, 1H), 4.50(t, 2H), 4.0(s, 2H), 3.30(t, 2H), 2.80(bm, 4H), 1.95(bm, 4H). ES-MS (m/z) 515 [M+1].

### EXAMPLE 75

### 5-{6-[5-(5-ISOBUTYL-1H-[1,2,4]TRIAZOL-3-YL]-1H-NAPHTHALEN-2-YLOXYMETHYL}-1-METHYL-PYRROLIDIN-2-ONE

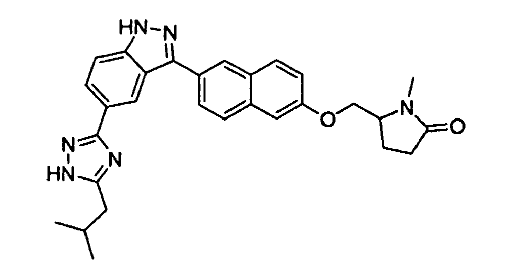

### A. 5-(6-Bromo-naphthalen-2-yloxymethyl-1-methyl-pyrrolidin-2-one

To a solution of 6-Bromo-naphthol (324mg, 1.45mmol) 5-Hyroxymethyl-1-methyl-pyrrolidin-2-one (750mg, 5.81mmol, 4.0eq.), and triphenylphosphine (1.52g, 5.81mmol, 3.0eq.) in THF cooled to 0 degrees was added diisopropyl azodicarboxylate (1.17g, 5.81mmol, 3.0eq.). Reaction was warmed to room temperature and stirred over the weekend. The solvent was removed *in vacuo* and the oil was subjected to Biotage chromatography (0-5% methanol/dichloromethane) to afford 484mg (50% yield) of the title compound as yellow solids. ES-MS (m/z) 334 [M+1].

### B. 3-[6-(1-Methyl-5-oxo-pyrrolidin-2-ylmetboxy)-naphthalen-2yl]-1-(tetrabydro-pyran-2-yl)-1 H-indazole-5-carbonitrile

A solution of 5-(6-Bromo-naphthalen-2-yloxymethyl-1-methyl-pyrrolidin-2one (484mg, 1.45mmol) in DMF was subjected to standard one-pot Suzuki coupling conditions using bis(pinnacalato)diboron (366mg, 1.45mmol, 1.0eq.), potassium acetate (426mg, 4.35mmol, 3.0eq.), palladium II chloride (bis-diphenyl phosphino ferrocene) dichloromethane (118mg, 10%mmol), 3-bromo-1-perhydro-2H-pyran-2-yl-1H-indazole-5-carbonitrile (443mg, 1.45mmol, 1.0eq.), and potassium phosphate (922mg, 4.35mmol, 3.0eq.) to afford 600mg (86% yield) of the title compound as yellow solids. ES-MS (m/z) 480 [M+1].

### C. 3-[6-(1-Methyl-5-oxo-pyrrolidin-2-ylmethoxy)-naphthalen-2-yl]-1H-indazole-5-carboxomidic acid ethyl ester

To a solution of 3-[6-(1-Methyl-5-oxo-pyrrolidin-2-ylmethoxy)-naphthalen-2yl]-1-(tetrahydro-pyran-2-yl)-1H-indazole-5-carbonitrile (0.40g, 1.7mmol) in ethanol cooled to -78 degrees was bubbled through HCI (g) for 30 minutes. Reaction was warmed to room temperature overnight. Solvent was removed and solids were washed with diethyl ether and filtered to get 400mg (100% yield) of the title compound as yellow solids. ES-MS (m/z) 442 [M+1].

### D. 5-{6-[5-(5-Isobutyl-1H-[1,2,4]triazol-3-yl]-1H-naphthalen-2-yloxymethyl}-1-methyl-pyrrolidin-2-one

To a solution of 3-[6-(1-Methyl-5-oxo-pyrrolidin-2-ylmethoxy)-naphthalen-2-yl]-1H-indazole-5-carboxomidic acid ethyl ester (400mg, 0.9mmol) was added 3-Methyl-butyric acid hydrazide (0.63g, 3.6mmol, 4.0eq.) and triethylamine (3.64 mL, 26mmol, 20.0eq.). The reaction was heated to 90 degrees in a sealed tube overnight. The reaction was monitored by LCMS and was complete after 24 hours. The solvent was removed in *vacuo* and was subjected to Prep HPLC (20-80% acetonitrile/water +0.1 % TFA) to afford 40mgs (5% yield) of the title compound as white solids. ¹H NMR (CD30D) δ 8.85(s, 1H), 8.45(s, 1H), 8.10(m, 2H), 8.00-7.90(dd, 2H), 7.60(d, 1H), 7.35(d, 1H), 7.20(dd, 1H), 4.35(dd, 1H), 4.20(dd, 1H), 4.10(m, 1H), 2.90(s, 3H), 2.70(d, 2H), 2.60-2.0(m, 4H), 0.99(ds, 6H). ES-MS (m/z) 494 [M+1].

### EXAMPLE 76

### SYNTHESIS OF 1-METHYL-5-{6-[5-(5-PYRROLIDIN-1-YLMETHYL-1H-[1,2,4]TRIAZOL-3-YL)-1H-INDAZOL-3-YL]-NAPHTHALEN-2-YLOXYMETHYL}-PYRROLIDIN-2-ONE

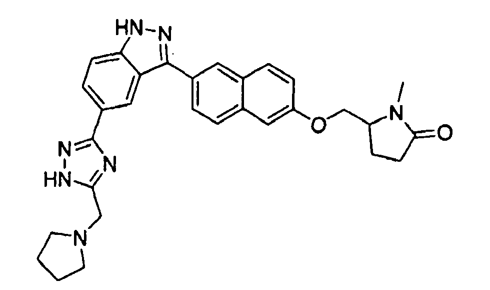

To a solution of 3-[6-(1-Methyl-5-oxo-pyrrolidin-2-ylmethoxy)-naphthalen-2-yl]-1H-indazole-5-carboxomidic acid ethyl ester (0.40g, 0.91mmol) was added pyrrolidin-1-yl-acetic acid hydrazide (529mg, 3.70mmol, 4.0eq.) and triethylamine (2.50mL, 18.0mmol, 20.0eq.). The reaction was heated to 90 degrees in a sealed tube overnight. The reaction was monitored by LCMS and was complete after 24 hours. The solvent was removed *in vacuo* and was subjected to Prep HPLC (20-80% acetonitrile/water +0.1% TFA) to afford 60mg (5% yield) of the title compound as white solids. ¹H NMR (CD30D) δ 8.81(d, 1H), 8.45(s, 1H), 8.15(dd, 2H), 8.0-7.8(dd, 2H), 7.60(d, 1H), 7.35(d, 1H), 7.20(dd, 1H), 4.45(dd, 1H), 4.20(dd, 1H), 4.05(m, 6H), 3.85(s, 2H), 3.55(dd, 2H), 2.90(s, 3H), 2.70(m, 4H), 1.80(m, 4H) ES-MS (m/z) 521 [M+1].

### EXAMPLE 77

### SYNTHESIS OF 2-METHOXY-6-[5-PYRROLIDIN-1-YLMETHYL-1H-[1,2,4]TRIAZOL-3-YL)-1H-INDAZOL-3-YL]QUINOLINE

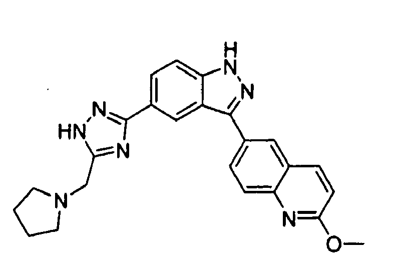

### A. 3-Ethoxy-acryloyl chloride

To an ice bath cooled solution of oxalyl chloride (137mL, 1.58mol) was added ethyl vinyl ether (151.3mL, 1.58mol). The solution was allowed to warm to room temperature and was stirred overnight. Oxalyl chloride (10mL) was distilled from the reaction and then it was heated to 120 degrees C for 30 minutes. Reaction was distilled under vacuum (90-120 degrees C) to afford 67.4g (32% yield) of the title compound as a yellow oil. ES-MS (m/z) 134 [M+1].

### B. N-(4-Bromo-phenyl)-3-ethoxy-acrylamide

To an ice bath cooled solution of 4-Bromoaniline (86.5g, 502mmol) in dichloromethane (610mL) and pyridine (85mL) was added 3-Ethoxy-acryloyl chloride (67.4g, 502mmol, 1.0eq.). The reaction was warmed to room temperature and stirred for two hours. The solids were filtered and washed with water to afford 79.0g (58% yield) of the title compound as a yellow solid. ES-MS (m/z) 269 [M+1].

### C. 6-Bromo-1H-quinolin-2-one

To an ice bath cooled solution of sulfuric acid (480mL) was slowly added N-(4-Bromo-phenyl)-3-ethoxy-acrylamide (72.6g, 269mmol). The reaction was warmed to room temperature, stirred for one hour, and quenched in 4L water and ice. The solids were filtered and dried in dessicator overnight to afford 59.9g (100% yield) of title compound as yellow solids. ES-MS (m/z) 223 [M+1].

### D. 6-Bromo-2-chloro-quinoline

6-Bromo-1H-quinolin-2-one (46.9g, 210mmol) was refluxed in phosphorus oxychloride (50mL, 537mmol, 2.0eq.) with Dimethylformamide (2mL) overnight. Reaction was quenched into 4L water and ice and the solids were filtered and dried in a dessicator to afford 46.6g (90% yield) of the title compound as tan solids. ES-MS (m/z) 243 [M+1].

### E. 6-Bromo-2-methoxy-quinoline

A solution of 6-Bromo-2-chloro-quinoline (37.9g, 155mol) and sodium methoxide (50mL, 232mmol, 1.5eq.) in methanol (150mL) was heated in a sealed flask to 90 degrees C for two hours. Reaction was cooled to room temperature and the solids were filtered to afford 36.9g (100% yield) of the title compound as off white solids. ES-MS (m/z) 238 [M+1].

### F. 3-(2-Methoxy-quinotin-6-yl)-1-(tetrahydro-pyran-2-yl)-1H-indazole-5-carbonitrile

To a solution of 6-Bromo-2-methoxy-quinoline (2.50g, 10.46mmol) in THF (100mL) cooled to -78 degrees C was slowly added 1.7M t-butyl lithium (12.2.mL, 20.9mmol, 2.0eq.). After 10 minutes a cooled solution of zinc chloride (1.42g, 10.46mmol, 1.0eq.) in THF (30mL) was added and the reaction was slowly warmed to room temperature. 3-bromo-1-perhydro-2H-pyran-2-yl-1H-indazole-5-carbonitrile (3.20g, 10.46mmol, 1.0eq.) and tetrakis(triphenylphosphine) palladium (150mg) was added to the reaction and it was refluxed for 45 minutes. Solvent was removed, Biotage chromatography (10% ethyl acetate/hexanes) to afford 800mg of a 1:1 mixture of regioisomers that was carried onto next step. ES-MS (m/z) 384 [M+1].

### G. 3-(2-Metboxy-quinolin-6-yl)-1H-indazole-5-carbonitrile

A solution of the regioisomers of 3-(2-Methoxy-quinolin-6-yl)-1-(tetrahydro-pyran-2-yl)-1H-indazole-5-carbonitrile (800mg, 2.0mmol)in 6N hydrochloric acid and methanol was heated to 40 degrees C for two hours. The solids were filtered to afford 150mg (25% yield) of desired regioisomer that was carried into the next step. ES-MS (m/z) 300 [M+1].

### F. 3-(2-Metboxy-quinolin-6-yl)-1H-indazole-5-carboximidic acid ethyl ester

To an ice bath cooled solution of 3-(2-Methoxy-quinolin-6-yl)-1H-indazole-5-carbonitrile (150mg, 0.50mmol) was bubbled hydrochloric gas for 30 minutes. Reaction was allowed to warm to room temperature and stirred overnight. Solvent was removed and solids were washed with diethyl ether to afford 126mg (73% yield) of the title compound as yellow solids. ES-MS (m/z) 346 [M+1].

### G. 2-Methoxy-6-[5-pyrrolidin-1-ylmethyl-1H-[1,2,4]triazol-3-yl)-1H-indazol-3-yl]quinoline

To a solution of 3-(2-Methoxy-quinolin-6-yl)-1H-indazole-5-carboximidic acid ethyl ester (126mg. 0.36mmol) was added pyrrolidin-1-yl-acetic acid hydrazide (207mg, 1.45mmol, 4.0eq.) and triethylamine (1.0mL, 7.2mmol, 20.0eq.). The reaction was heated to 90 degrees in a sealed tube overnight. The reaction was monitored by LCMS and was complete after 24 hours. The solvent was removed *in vacuo* and was subjected to Prep HPLC (20-80% acetonitrile/water +0.1% TFA) to afford 26mg (17% yield) of the title compound as white solids. ¹H NMR (CD30D) 8.70(s, 1H), 8.30(s, 1H), 8.20-8.10(dd, 2H), 7.98(dd, 1H), 7.55(dd, 1H), 6.88(dd, 1H), 4.00(s, 3H), 3.82(s, 2H), 2.70(m, 4H), 1.75(m, 4H). ES-MS (m/z) 425 [M+1].

### EXAMPLE 78

### SYNTHESIS OF 3-(6-METHOXY-NAPHTHALEN-2-YL)-1H-INDAZOLE-5-CARBOXYLIC ACID ETHYLAMIDE

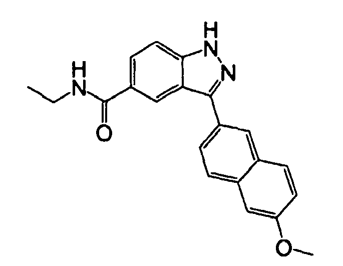

### A. 3-(6-Methoxy-naphthaten-2-yl)-1-(tetrahydro-pyran-2-yl)-1H-indazole-5-carboxylic acid

Potassium hydroxide (8.55 g, 152 mmol) was dissolved in a mixture of ethanol (200 mL) and water (100 mL). 3-(6-methoxy-naphthalen-2-yl)-1-(tetrahydropyran-2-yl)-1*H*-indazole-5-carbonitrile (6.0 g, 15 mmol) was then added to this solution and the temperature was brought to 110 °C with stirring. After 24 h, the resulting white solid was filtered, washed with saturated ammonium chloride (300 mL), and suspended in diethyl ether (200 mL). The precipitate was filtered and dried under reduced pressure to give the title compound as a fine white powder (5.6 g, 14 mmol, 91 %): LC-MS (m/z) 403 [M+1]⁺.

### B. 3-(6-Methoxy-naphthalen-2-yl)-1-(tetrahydro-pyran-2-yl)-1H-indazole-5-carboxylic acid ethylamide

Triethylamine (100 mg, 1.0 mmol) was added dropwise to a solution of 3-(6-methoxy-naphthalen-2-yl)-1-(tetrahydro-pyran-2-yl)-1*H*-indazole-5-carboxylic acid (200 mg, 0.50 mmol), HOBt (200 mg, 1.5 mmol), and EDACHCl (290 mg, 1.5 mmol) in methylene chloride (2.0 mL). Ethyl amine (1.0 mL, 2.0 mmol, 2.0 M in THF) was then added and the reaction was allowed to stir for 12 h. The resulting white precipitate was dissolved by adding 0.1 N NaOH (40 mL) and extracted with ethyl acetate (4 x 100 mL). The organic layers were combined, dried (MgSO₄) and concentrated to give the title compound as a white solid (50 mg, 0.12mmol, 23%): LC-MS (m/z) 430 [M+1]⁺.

### C. 3-(6-Methoxy-naphthalen-2-yl)-1H-indazole-5-carboxylic acid ethylamide

3-(6-Methoxy-naphthalen-2-yl)-1-(tetrahydro-pyran-2-yl)-1*H*-indazole-5-carboxylic acid ethylamide (50 mg, 0.12 mmol) was dissolved in ethanol (50 mL) at 0 °C. Hydrogen chloride gas was then bubbled through the reaction mixture until excess was indicated. The reaction mixture was allowed to warm to room temperature and stirred over 12 h. The resulting yellow solid was filtered and washed with diethyl ether (30 mL). The impure residue was then recrystallized from ethyl acetate and hexanes to provide the title compound as a pale yellow solid with 97% purity by analytical HPLC (30 mg, 0.09 mmol, 90%): ¹H NMR (CD₃OD) 8.62 (s, 1H) 8.39 (s, 1H) 8.03 (dd, 1H) 7.92-7.88 (m, 3H) 7.60 (d, 1H) 7.28 (d, 1H) 7.16 (dd, 1H) 3.92 (s, 3H) 3.43 (quartet, 2H) 1.23 (t, 3H); LC-MS (*m*/*z*) 346 [M+1]⁺.

### EXAMPLE 79

### SYNTHESIS OF 3-(6-METHOXY-NAPHTRALEN-2-YL)-1H-INDAZOLE-5-CARBOXYLIC ACID (2-MORPHOLIN-4-YL-ETHYL)-AMIDE

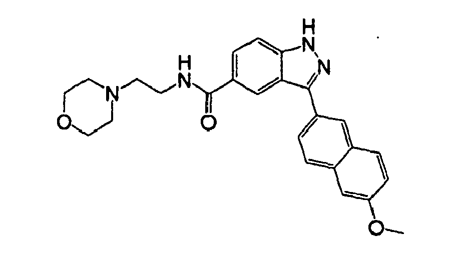

### A. 3-(6-Methoxy-naphthalen-2-yl)-1-(tetrahydro-pyran-2-yl)-1H-indazole-5-carboxylic acid (2-morpbolin-4-yl-ethyl)-amide

3-(6-Methoxy-naphthalen-2-yl)-1-(tetrahydro-pyran-2-yl)-1*H-*indazole-5-carboxylic acid (Example 76, step A, 0.79 g, 2.0 mmol) was dissolved at room temperature in a solution of methylene chloride (4 mL) and THF (2 mL). HOBt (0.79 g, 5.9 mmol) and EDACHCl (1.1 g, 5.7 mmol) were then added to the solution in one portion. After stirring for 5 min, 4-(2-aminoethyl)morpholine (1.0 g, 8 mmol) was added dropwise via syringe. The reaction was stirred for 12 h at room temperature. The excess solvent was filtered from the resulting precipitate. After washing with hexanes (50 mL), the precipitate was concentrated under reduced pressure to give the title compound as a white solid (0.50 g, 0.97 mmol, 50%): LC-MS (m/z) 515 [M+1]⁺.

### B. 3-(6-Methoxy-naphthalen-2-yl)-1H-indazole-5-carboxylic acid (2-morpholin-4-yl-ethyl)-amide

3-(6-Methoxy-napbthalen-2-yl)-1-(tetrahydro-pyran-2-yl)-1*H*-indazole-5-carboxylic acid (2-morpholin-4-yl-ethyl)-amide (0.50 mg, 0.97 mmol) was dissolved in methanol (60 mL) at 0 °C. Hydrogen chloride gas was then bubbled through the reaction mixture until excess was indicated. The reaction mixture was allowed to warm to room temperature and stirred over 12 h. Excess HCI(g) was removed by first filtering the residue and then rinsing it with diethyl ether (100 mL). The resulting residue was then dried under reduced pressure at 40 °C to provide the title compound as a pale yellow solid with 98% purity by analytical HPLC (0.34 g, 0.79 mmol, 81%): ¹H NMR (CD₃OD) 8.74 (s, 1H) 8.41 (s, 1H) 8.04 (dd, 1H) 7.99 (dd, 1H) 7.93 (m, 2H) 7.64 (dd, 1H) 7.29 (d, 1H) 7.17 (dd, 1H) 4.06 (dd, 2H) 3.92 (s, 3H) 3.83-3.75 (m, 4H) 3.68 (d, 2H) 3.42 (t, 2H) 3.22 (dt, 2H); LC-MS (m/z) 431 [M+1]⁺.

### EXAMPLE 80

### SYNTHESIS OF 3-(6-METHOXY-NAPHTHALEN-2-YL)-1H-INDAZOLE-5-CARBOXYLIC ACID (2-PYRROLIDIN-1-YL-ETHYL)-AMIDE

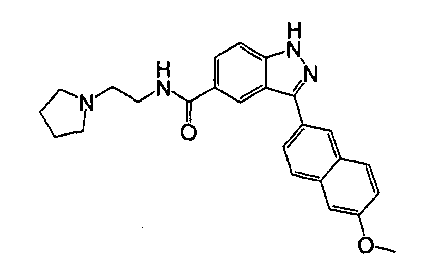

### A. 3-(6-Methoxy-naphthalen-2-yl)-1-(tetrahydro-pyran-2-yl)-1H-indazole-5-carboxylic acid (2-pyrrolidin-1-yl-ethyl)-amide

3-(6-Methoxy-naphthalen-2-yl)-1-(tetrahydro-pyran-2-yl)-1*H*-indazole-5-carboxylic acid (Example 76, step A, 0.75 g, 1.9 mmol) was dissolved at room temperature in DMF (4 mL). HOBt (0.76 g, 5.6 mmol) and EDACHCI (1.1 g, 5.7 mmol) were then added to the solution in one portion. After stirring for 10 min, 1-(2-aminoethyl)pyrrolidine (0.97 g, 8.5 mmol) was added dropwise via syringe. The solution was stirred for 18 h at room temperature. The mixture was concentrated and purified by preparatory HPLC to give the title compound as a yellow oil (0.62 g, 1.2 mmol, 67%): LC-MS (m/z) 499 [M+1]⁺.

### B. 3-(6-Methoxy-naphthalen-2-yl)-1H-indazole-5-carboxylic acid (2-pyrrolidin-1-yl-ethyl)-amide

3-(6-Methoxy-naphthalen-2-yl)-1-(tetrahydro-pyran-2-yl)-1*H*-indazole-5-carboxylic acid (2-pyrrolidin-1-yl-ethyl)-amide (0.62 g, 1.2 mmol) was dissolved in methanol (60 mL) at 0 °C. Hydrogen chloride gas was then bubbled through the reaction mixture until excess was indicated. The reaction mixture was allowed to warm to room temperature and stirred over 18 h. Excess HCl(g) was removed by first filtering the residue and then rinsing it with diethyl ether (100 mL). The resulting residue was purified by preparatory HPLC to provide the title compound with 99% purity by analytical HPLC (0.14 g, 0.34 mmol, 27%): ¹HMNR (CD₃OD) 8.72 (s, 1H) 8.38 (s, 1H) 8.04 (dd, 1H) 7.95 (dd, 1H) 7.90 (m, 2H) 7.63 (d, 1H) 7.28 (d, 1H) 7.17 (dd, 1H) 3.92 (s, 3H) 3.77 (t, 4H) 3.44 (t, 2H) 3.14 (m, 2H) 2.15 (m, 2H) 2.00 (m, 2H); LC-MS (*m*/*z*) 414 [M+1]⁺.

### EXAMPLE 81

### SYNTHESIS OF 3-(6-METHOXY-NAPHTHALEN-2-YL)-1H-INDAZOLE-5-CARBOXYLIC ACID (2-METHOXY-ETHYL)-AMIDE

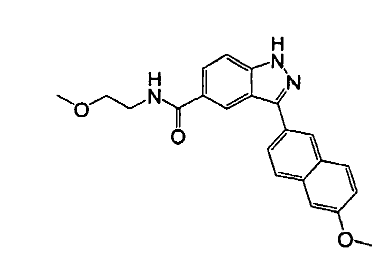

### A. 3-(6-Methoxy-naphthalen-2-yl)-1-(tetrahydro-pyran-2-yl)-1H-indazole-5-carboxylic acid (2-methoxy-ethyl)-amide

The title compound (100 mg, 2.18 mmol, 9%) was prepared as described in Example 76, step A from 3-(6-methoxy-naphthalen-2-yl)-1-(tetrahydro-pyran-2-yl)-1*H-*indazole-5-carboxylic acid (Example 76, step A, 1.0 g, 2.5 mmol) and 2-methoxyethylamine (0.75 g, 9.9 mmol): %): LC-MS (*m*/*z*) 460 [M+1]⁺.

### B. 3-(6-Methoxy-naphthalen-2-yl)-1H-indazole-5-carboxylic acid (2-methoxy-ethyl)-amide

The title compound (51 mg, 0.14 mmol, 62%) was prepared with 98% purity by analytical HPLC, as described in Example 77, step B from 3-(6-Methoxy-naphthalen-2-yl)-1-(tetrahydro-pyran-2-yl)-1H-indazole-5-carboxylic acid (2-methoxy-ethyl)-amide (100 mg, 2.18 mmol): 1HNMR (DMSO) 8.70 (s, 1H) 8.51 (s, 1H) 8.15 (dd, 1H) 7.96 (ddd, 3H) 7.64 (d, 1H) 7.41 (d, 1H) 7.24 (dd, 1H) 3.92 (s, 3H) 3.50 (m, 4H) 3.29 (s, 3H); LC-MS (m/z) 376 [M+1]⁺.

### EXAMPLE 82

### SYNTHESIS OF 3-{6-[3-CIS-(2,6-DIMETHYL-PIPERIDIN-1-YL)-PROPOXY]-NAPHTHALEN-2-YL}-5-(5-ISOBUTYL-4H-[1,2,4]TRIAZOL-3-YL)-1H-INDAZOLE

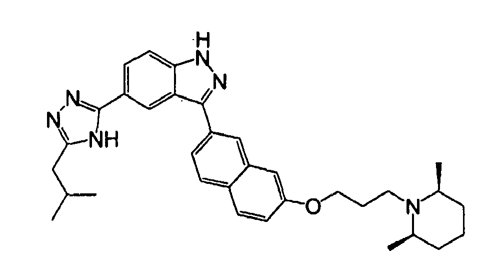

### A. 3-(cis-2, 6-Dimethyl-piperidin-1-ylrpropan-1-ol

Potassium iodide (1.0 g, 6.0 mmol) was added to a solution of 3-chloro-1-propanol (5.0 g, 53 mmol) in xylenes (10 mL). After stirring for 10 min, cis-2, 6-dimethylpiperidine (12.0 g, 106 mmol) was added dropwise via syringe. The mixture was refluxed at 150 °C in a sealed glass tube for 2.5 h. After cooling to room temperature, solid impurities were removed by filtering the reaction mixture through a bed of celite (50 g) with diethyl ether (200 mL). The filtrate was concentrated under reduced pressure. The resulting yellow oil was purified by vacuum distillation to provide the title compound as a colorless oil (4.2 g, 25 mmol, 47%): LC-MS (m/z) 172 [M+1]+.

### B. 7-Bromo-naphthalen-2-ol

Bromine (32.7 g, 205 mmol) was slowly added to a solution of triphenyl phosphine (53.9 g, 205 mmol) in acetonitrile (250 mL) at 0 °C. After stirring for 30 min, a solution of 2,7-dihydroxynaphthalene (30.0 g, 187 mmol) in acetonitrile (125 mL) was added dropwise via addition funnel. The addition funnel was replaced with a cold-water condenser and the reaction mixture was refluxed at 85 °C under an inert atmosphere. After 3 h, the mixture was concentrated under reduced pressure to yield a pale grey solid, which was subsequently heated at 350 °C in a sand bath until fuming was no longer observed. The resulting black residue was purified by flash chromatography using a gradient of 10% to 50% ethyl acetate in hexanes to provide the title compound (7.2 g, 32 mmol, 17%): LC-MS (*m*/*z*) 224 [M+1]⁺.

### C. 1-[3-(7-Bromo-naphthalen-2-yloxy)-propyl]-cis-2,6-dimethyl-piperidine

To a solution of 7-bromo-2-naphthol (3.5 g, 16 mmol) in THF (63 mL) at room temperature was added triphenyl phosphine (6.2 g, 24 mmol) and 3-(cis-2,6-dimethyl-piperidin-1-yl)-propan-1-ol (3.5 g, 21 mmol). Diisopropyl azodicarboxylate (4.8 g, 24 mmol) was then added and the reaction mixture was monitored via thin layer chromatography and LC-MS. After 1 h, the reaction mixture was concentrated and the excess triphenyl phophine was precipitated out using a 1:1 mixture of diethyl ether and hexanes. Purification was carried out by flash chromatography using a gradient of 1% to 5% methanol in methylene chloride to provide the title compound as an orange oil (4.1 g, 11 mmol, 69%): LC-MS (*m*/*z*) 378 [M+1].

### D. 3-{7-[3-cis-(2,6-Dimethyl-piperidin-1-yl)-propoxy]-naphtbaten-2-yl}-1-(tetrahydro-pyran-2-yl)-1H-indazole-5-carbonitrile

A mixture of 1-[3-(7-bromo-naphthalen-2-yloxy)-propyl]-cis-2,6-dimethylpiperidine (4.1 g, 11 mmol), bis(pinacolato)diboron (2.8 g, 11 mmol), PdCl₂(dppf)CH₂Cl₂ (0.89 g, 1.1 mmol), and potassium acetate (3.2 g, 33 mmol) was dissolved in DMF (80 mL). The reaction mixture was refluxed at 85 °C in a sealed glass tube. After 3.5 h, the presence of *cis*-2,6-dimethyl-1-{3-[7-(4,4,5,5-tetramethyl-[1,3,2]dioxaborolan-2-yl)-naphthalen-2-yloxy]-propyl-piperidine was confirmed via LC-MS (m/z) 424 [M+1]⁺. 3-Bromo-1-(tetrahydro-pyran-2-yl)-1*H*-indazole-5-carbonitrile (3.7 g, 12 mmol) and potassium phosphate (7.0 g, 33 mmol) were then added to the reaction mixture in one portion and refluxed at 85 °C over 12 h. The crude residue was filtered through a bed of celite (150 g) and further purified by flash chromatography using a gradient of 1% to 10% methanol in methylene chloride. The title compound was recovered as a pale yellow oil (3.3 g, 6.3 mmol, 57%): LC-MS (*m*/*z*) 524 [M+1]⁺.

### E. 3-{7-[3-cis-(2,6-Dimethyl-piperidin-1-yl)-propoxy]-naphthalen-2-yl}-1H-indazole-5-carboximidic acid ethyl ester

3-{7-[3-*cis*-(2,6-Dimethyl-piperidin-1-yl)-propoxy]-naphthalen-2-yl}-1-(tetrahydro-pyran-2-yl)-1*H*-indazole-5-carbonitrile (3.3 g, 6.3 mmol) was dissolved in a mixture of methylene chloride (20 mL) and ethanol (60 mL) at 0 °C. Hydrogen chloride gas was then bubbled through the reaction mixture until excess was indicated. The reaction mixture was allowed to warm to room temperature and stirred over 24 h. Excess HCI(g) was removed by first filtering the residue and then rinsing it with diethyl ether (100 mL). The resulting residue was dried under reduced pressure to give the title compound as a white solid (2.0 g, 4.1 mmol, 66%): LC-MS (*m*/*z*) 485 [M+1]⁺.

### F. 3-{7-[3-cis-(2,6-Dimethyl-piperidin-1-yl)-propoxy]-naphthalen-2-yl}-5-(5-isobutyl-4H-[1,2,4]triazol-3-yl)-1H-indazole

To a solution of 3-{7-[3-*cis*-(2,6-Dimethyl-piperidin-1-yl)-propoxy]-naphthalen-2-yl}-1*H*-indazole-5-carboximidic acid ethyl ester (1.8 g, 3.6 mmol) in methanol (24 mL) was added 3-methyl-butyric acid hydrazide (1.7 g, 15 mmol). Triethylamine (7.3 g, 72 mmol) was then added to the mixture under an inert atmosphere. After stirring for 30 min, the reaction mixture was refluxed at 85 °C over 24 h. The resulting crude residue was concentrated under reduced pressure and purified by flash chromatography using a gradient of 5% to 10% methanol in methylene chloride. Further purification via preparatory HPLC provided the title compound as a pale yellow solid with 97% purity by analytical HPLC (5 mg, 0.009 mmol, 0.25%): ¹HNMR (CD₃OD) 8.77 (s, 1H) 8.37 (s, 1H) 7.96 (dd, 1H) 7.85 (d, 1H) 7.75 (d, 1H) 7.59 (d, 1H) 7.38 (d, 1H) 7.10 (dd, 1H) 4.21 (t, 2H) 3.48 (m, 2H) 3.32 (m, 2H) 2.64 (d, 2H) 2.17 (m, 2H) 2.10 (sp, 1H) 1.92 (m, 2H) 1.74 (m, 1H) 1.55 (m, 4H) 1.37 (d, 6H) 0.92 (d, 6H); LC-MS (*m*/*z*) 537.6 [M+1]⁺.

### EXAMPLE 83

### SYNTHESIS OF 3-{6-[3-CIS-(2,6-DIMETHYL-PIPERIDIN-1-YL)-PROPOXY]-NAPHTHALEN-2-YL}-5-(5-ISOBUTYL-4H-[1,2,4]TRIAZOL-3-YL)-1H-INDAZOLE

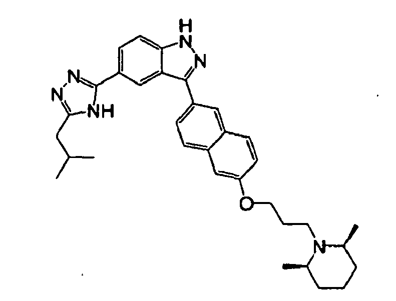

### A. 1-[3-(6-Bromo-naphthalen-2-yloxy)-propyl]-cis-2,6-dimethyl-piperidine

To a solution of 6-bromo-2-naphthol (3.8 g, 17 mmol) in THF (68 mL) at room temperature was added triphenyl phosphine (67 g, 26 mmol). Diisopropyl azodicarboxylate (5.2 g, 26 mmol) was added and the reaction mixture was monitored via thin layer chromatography and LC-MS. After 12 h, the reaction mixture was concentrated and the excess triphenyl phosphine was precipitated out using a 1:1 mixture of diethyl ether and hexanes. Purification was carried out by flash chromatography using a gradient of 1% to 5% methanol in methylene chloride to provide the title compound as an orange oil (3.7 g, 9.4 mmol, 55%): LC-MS (*m*/*z*) 378 [M+1]⁺.

### B. 3-{6-[3-cis-(2,6-Dimethyl-piperidin-1-yl)-propoxy]-naphthalen-2-yl}-1-(tetrahydro-pyran-2-yl)-1H-indazole-5-carbonitrile

A mixture of 1-[3-(6-bromo-naphthalen-2-yloxy)-propyl]-*cis*-2,6-dimethylpiperidine (3.6 g, 9.4 mmol), bis(pinacolato)diboron (2.4 g, 9.4 mmol), PdCl₂(dppf)CH₂Cl₂ (0.77 g, 0.94 mmol), and potassium acetate (2.8 g, 28 mmol) was dissolved in DMF (80 mL). The reaction mixture was refluxed at 85 °C in a sealed glass tube. After 2.5 h, the presence of *cis*-2,6-dimethyl-1-{3-[6-(4,4,5,5-tetramethyl-[1,3,2]dioxaborolan-2-yl)-naphthalen-2-yloxy]-propyl-piperidine was confirmed via LC-MS (*m*/*z*) 424 [M+1]⁺. 3-Bromo-1-(tetrahydro-pyran-2-yl)-1*H-*indazole-5-carbonitrile (3.2 g, 10 mmol) and potassium phosphate (6.0 g, 28 mmol) were then added to the reaction mixture in one portion and refluxed at 85 °C over 12 h. The crude residue was purified by flash chromatography using a gradient of 1% to 10% methanol in methylene chloride. The title compound was recovered as a yellow oil (1.6 g, 3.1 mmol, 33%): LC-MS *(m*/*z)* 524 [M+1]⁺.

### C. 3-{6-[3-cis-(2,6-Dimethyl-piperidin-1-yl)-propoxy]-naphthalen-2-yl}-1H-indazole-5-carboximidic acid ethyl ester

The title compound (1.4 g, 2.9 mmol, 94%) was prepared as described in Example 80, step E from 3-{6-[3-*cis*-(2,6-dimethyl-piperidin-1-yl)-propoxy]-naphthalen-2-yl}-1-(tetrahydro-pyran-2-yl)-1*H-*indazole-5-carbonitrile (1.6 g, 3.1 mmol): LC-MS (*m*/*z*) 485.4 [M+1]⁺.

### D. 3-{6-[3-cis-(2,6-Dimethyl-piperidia-1-yl)-propoxy]-naphthalen-2-yl}-5-(5-isobutyl-4H-[1,2,4]triazol-3-yl)-1H-indazole

To a solution of 3-{6-[3-*cis*-(2,6-dimethyl-piperidin-1-yl)-propoxy]-naphthalen-2-yl}-1*H*-indazole-5-carboximidic acid ethyl ester (1.7 g, 3.5 mmol) in methanol (23 mL) was added 3-methyl-butyric acid hydrazide (1.6 g, 14 mmol). Triethylamine (6.9 g, 68 mmol) was then added to the mixture under an inert atmosphere. The reaction mixture was refluxed at 85°C over 16 h. The resulting crude residue was concentrated under reduced pressure and purified by flash chromatography using 10% methanol in methylene chloride as an eluent. Further purification via preparatory HPLC provided the title compound as a pale yellow solid with 99% purity by analytical HPLC (0.10 g, 0.19 mmol, 6%): ¹HNMR (CD₃OD) 8.89 (s, 1H) 8.4 (s, 1H) 8.09 (dt, 2H), 7.90 (d, 1H) 7.82 (d, 1H) 7.49 (d, 1H) 7.23 (dd, 1H) 7.15 (dd, 1H) 4.18 (t, 2H) 3.35 (m, 4H) 2.61 (d, 2H) 2.10 (m, 3H) 1.75 (m, 3H) 1.45 (m, 3H) 1.31 (d, 6H) 0.95 (d, 6H); LC-MS (m/z) 537.6 [M+1]⁺.

### EXAMPLE 84

### SYNTHESIS OF 3-{7-[3-CIS-(2,6-DIMETHYL-PIPERIDIN-1-YL)-PROPOXY]-NAPHTHALEN-2-YL}-5-[5-(2,2-DIMETHYL-PROPYL)-4H-[1,2,4]TRIAZOL-3-YL]-1H-INDAZOLE

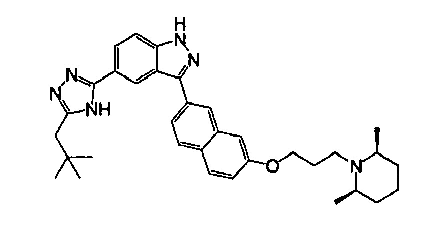

### A. 3-{7-[3-cis-(2,6-Dimetbyl-piperidin-1-yl)-propoxy]-naphthalen-2-yl}-5-[5-(2,2-(dimethyl-propyl)-4H-[1,2,4]triazol-3-yl]-1H-indazole

To a solution of 3-{7-[3-*cis*-(2,6-dimethyl-piperidin-1-yl)-propoxy]-naphthalen-2-yl)-1*H*-indazole-5-carboximidic acid ethyl ester (Example 80, step E, 0.95 g, 2.0 mmol) in methanol (13 mL) was added 3,3-dimethyl-butyric acid hydrazide (1.0 g, 7.9 mmol). Triethylamine (4.0 g, 39 mmol) was then added to the mixture under an inert atmosphere. After stirring for 20 min, the reaction mixture was refluxed at 85°C over 24 h. The resulting crude residue was concentrated under vacuum and purified by preparatory HPLC provided the title compound as a pale yellow solid with 97% purity by analytical HPLC (23 mg, 0.04 mmol, 2%): ¹HNMR (CD₃OD) 8.79 (s, 1H) 8.37 (s, 1H) 8.03 (dd, 1H) 7.92 (dd, 1H) 7.83 (d, 1H) 7.73 (d, 1H) 7.59 (d, 1H) 7.3 (d, 1H) 7.07 (dd, 1H) 4.10 (t, 2H) 3.08 (m, 4H) 1.96 (m, 2H) 1.61 (m, 4H) 1.39-1.18 (m, 4H) 1.15 (d, 6H) 0.97 (s, 9H); LC-MS (m/z) 551 [M+1]⁺.

### EXAMPLE 85

### SYNTHESIS OF 3-{7-[3-CIS-(2,6-DIMETHYL-PIPERIDIN-1-YL)-ETHOXY]-NAPHTHALEN-2-YL}-5-(5-ISOBUTYL-4H-[1,2,4]TRIAZOL-3-YL)-1H-INDAZOLE

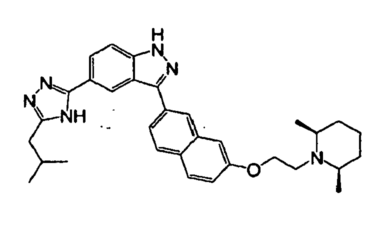

### A. 2-(cis-2,6-Dimethyl-piperidin-1-yl)-ethanol

Triethylamine (9.8 g, 97 mmol) in THF (20 mL) was added to a solution of *cis-2,* 6-dimethylpiperidine (10.0 g, 88 mmol) in THF (80 mL) at room temperature under an inert atmosphere. After stirring for 45 min, ethyl bromoacetate (15 g, 90 mmol) in THF (25 mL) was added dropwise at 0 °C over 20 min. The reaction mixture was slowly warmed to room temperature and stirred an additional 16 h. The resulting orange solution was diluted with methylene chloride (150 mL) and several washes of the organic phase were performed with H₂O (3 x 150 mL). The combined organic phases were dried (MgSO₄) and concentrated under vacuum. The crude residue was once again dissolved in THF (40 mL) and LiAlH₄ (5.5 g, 0.14 mol) was added at 0 °C. The reaction mixture was stirred an additional 8 h at 0 °C before sodium sulfate decahydrate in celite (1:1,20 g) was added to quench the excess LiAlH₄. The mixture was then filtered and rinsed with ethyl acetate (200 mL). The ethereal layers were concentrated and the crude product was purified by flash chromatography using 20% ethyl acetate in hexanes as the eluent to provide the title compound as a colorless oil (5.5 g, 0.16 mol, 40%): LC-MS (*m*/*z*) 158 [M+1]⁺.

### B. 1-(2-(7-Bromo-naphthalen-2-yloxy)-ethyl]-cis-2,6-dimethyl-piperidine

To a solution of 7-bromo-2-naphthol (5.0 g, 22 mmol) in THF (80 mL) at room temperature was added triphenyl phosphine (8.8 g, 34 mmol) and 2-(cis-2, 6-dimethyl-piperidin-1-yl)-ethanol (4.6 g, 29 mmol). Diisopropyl azodicarboxylate (6.8 g, 34 mmol) was then added and the reaction mixture was monitored via thin layer chromatography and LC-MS. After 1 h, the reaction mixture was concentrated and the excess triphenyl phosphine was precipitated out using a 1:1 mixture of diethyl ether and hexanes. Purification was carried out by flash chromatography using a gradient of 1% to 2% , methanol in methylene chloride to provide the title compound as an orange oil (6.1 g, 17 mmol, 75%): LC-MS (*mlz*) 362 [M+1]⁺.

### C. 3-{7-[2-cis-(2,6-Dimethyl-piperidin-1-yl)-ethoxy]-naphthalen-2-yl}-1-(tetrahydro-pyran-2-yl)-1H-indazole-5-carbonitrile

A mixture of 1-[2-(7-bromo-naphthalen-2-yloxy)-ethyl]-cis-2, 6-dimethylpiperidine (1.0 g, 2.8 mmol), bis(pinacolato)diboron (0.70 g, 2.8 mmol), PdCl₂(dppf)CH₂Cl₂ (0.23 g, 0.28 mmol), and potassium acetate (0.82 g, 8.3 mmol) were dissolved in DMF (20 mL). The reaction mixture was refluxed at 85°C in a sealed glass tube. After 3 h, the presence of *cis*-2,6-dimethyl-1-{2-[7-(4,4,5,5-tetramethyl-[1,3,2] dioxaborolan-2-yl)-naphthalen-2-yloxy]-ethyl}-piperidine was confirmed via LC-MS (*m*/*z*) 410.6 [M+1]⁺. 3-Bromo-1- (tetrahydro-pyran-2-yl)-1*H*-indazole-5-carbonitrile (0.93 g, 3.1 mmol) and potassium phosphate (1.8 g, 8.3 mmol) were then added to the reaction mixture in one portion and refluxed at 85°C over 12 h. The crude residue was purified by flash chromatography using a gradient of 1% to 5% methanol in methylene chloride. The title compound was recovered as a yellow oil (0.78 g, 1.5 mmol, 54%): LC-MS (*m*/*z*) 509.4 [M+1]⁺.

### D. 3-{7-[2-cis-(2,6-Dimethyl-piperidin-1-yl)-ethoxy]-naphthalen-2-yl}-1H-indazole-5-carboximidic acid ethyl ester

3-{7-[2-*cis*-(2,6-Dimethyl-piperidin-1-yl)-ethoxy]-naphthalen-2-yl}-1-(tetrahydro-pyran-2-yl)-1*H*-indazole-5-carbonitrile (0.78 g, 1.5 mmol) was dissolved in ethanol (50 mL) at 0 °C. Hydrogen chloride gas was then bubbled through the reaction mixture until excess was indicated. The reaction mixture was allowed to warm to room temperature and stirred over 24 h. Excess HCI(g) was removed by first filtering the residue and then rinsing it with diethyl ether (100 mL). The resulting residue was dried under reduced pressure to give the title compound as a white solid (0.52 g, 1.1 mmol, 73%): LC-MS (*m*/*z*) 472 [M+1]⁺.

### E. 3-{7-12-cis-(2,6-Dimethyl-piperidin-1-yl)-etboxy]-naphthalen-2-yl}-5-(5-isobutyl-4H-[1,2,4]triazol-3-yl)-1H-indazole

To a solution of 3-{7-[2-*cis*-(2,6-dimethyl-piperidin-1-yl)-ethoxy]-naphthalen-2-yl}-1*H*-indazole-5-carboximidic acid ethyl ester (0.52 g, 1.1 mmol) in methanol (7 mL) was added 3-methyl-butyric acid hydrazide (0.51 g, 4.4 mmol). Triethylamine (2.3 g, 22 mmol) was then added to the mixture under an inert atmosphere. After stirring for 30 min, the reaction mixture was refluxed at 85°C over 24 h. The resulting crude residue was concentrated under vacuum and purified by flash chromatography using 10% methanol in methylene chloride as an eluent. Further purification via preparatory HPLC provided the title compound as a pale yellow solid with 98% purity by analytical HPLC (38 mg, 0.07 mmol, 7%): ¹HNTMR (CD₃OD) 8.81 (s, 1H) 8.41 (s, 1H) 8.1 (d, 1H) 7.98 (dd, 1H) 7.90 (d, 2H) 7.79 (d, 2H) 7.65 (d, 1H) 7.41 (dd, 1H) 7.16 (dd, 1H) 4.29 (m, 2H) 3.37 (m, 2H) 2.68 (d, 2H) 2.15 (m, 1H) 1.64 (m, 3H) 1.40 (m, 3H) 1.29 (d, 6H) 0.99 (d, 6H); LC-MS (*m*/*z*) 523.6 [M+1]⁺.

### EXAMPLE 86

### SYNTHESIS OF 5-[5-(2,2-DIMETHYL-PROPYL)-4H-[1,2,4] TRIAZOL-3-YL]-3-[7-(2-PYRROLIDIN-1-YL-ETHOXY)-NAPHTHALEN-2-YL]-1H-NDAZOLE

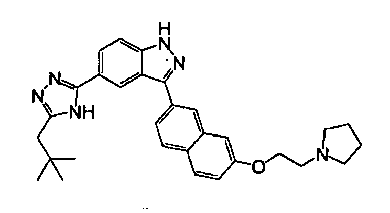

### A. 1-[2-(7-Bromo-naphthalen-2-yloxy)-ethyl]-pyrrolidine

7-Bromo-naphthalen-2-ol (5.0 g, 22 mmol) was added to a suspension of sodium hydride (6.0 g, 90 mmol, 60% in mineral oil) in THF (120 mL) at room temperature under an inert atmosphere. After stirring for 30 minutes, 1-(2-chloroethyl) pyrrolidine hydrochloride (6.5 g, 38 mmol) was added. The reaction mixture was refluxed at 85 °C over 14 h and then allowed to cool to room temperature. Excess base was quenched by first pouring the reaction mixture over ice and then adding 1 N HCI (100 mL). The aqueous layer was extracted with methylene chloride (3 x 250 mL) and the combined organic layers were dried (MgSO₄) and concentrated under reduced pressure. The crude residue was purified by flash chromatography using a gradient of 1% to 8% methanol in methylene chloride. The title compound was isolated as pale yellow oil (6.3 g, 20 mmol, 69%): LC-MS (m/z) 322 [M+1]⁺.

### B. 3-[7-(2-Pyrrolidin-1-yl-etboxy)-naphthalen-2-yl]-1-(tetrabydro-pyran-2-yl)-1H-indazote-5-carbonitrile

A mixture of 1-[2-(7-bromo-naphthalen-2-yloxy)-ethyl]-pyrrolidine (2.0 g, 6.3 mmol), bis(pinacolato)diboron (1.6 g, 6.3 mmol), PdCl₂(dppf)CH₂Cl₂ (0.50 g, 0.63 mmol), and potassium acetate (1.8 g, 19 mmol) were dissolved in DMF (40 mL). The reaction mixture was refluxed at 85°C in a sealed glass tube. After 3 h, the presence of 1-{2-[7-(4,4,5,5-tetramethyl- [1,3,2] dioxaborolan-2-yl)-naphthalen-2-yloxy]-ethyl}-pyrrolidine was confirmed via LC-MS *(mlz)* 368.4 [M+1]⁺. 3-Bromo-1-(tetrahydro-pyran-2-yl)-1*H*-indazole-5-carbonitrile (2.1 g, 6.9 mmol) and potassium phosphate (4.0 g, 19 mmol) were then added to the reaction mixture in one portion and refluxed at 85°C over 12 h. The crude residue was purified by flash chromatography using a gradient of 1% to 5% methanol in methylene chloride as an eluent. The title compound was recovered as an oil (2.4 g, 5.2 mmol, 83%): LC-MS *(mlz)* 467.4 [M+1]⁺.

### C. 3-[7-(2-Pyrrolidin-1-yl-ethoxy)-naphthalen-2-yl]-1H-indazole-5-carboximidic acid ethyl ester

The title compound (1.8 g, 2.9 mmol, 81%) was prepared as described in Example 83, step D from 3-[7-(2-pyrrolidin-1-yl-ethoxy)-naphthalen-2-yl]-1-(tetrahydro-pyran-2-yl)-1H-indazole-5-carbonitrile (2.4 g, 5.2 mmol): LC-MS (m/z) 429 [M+1]⁺.

### D. 5-[5-(2,2-Dimethyl-propyl)-4H-[1,2,4] triazol-3-yl]-3-[7-(2-pyrrolidin-1-yl-ethoxy)-naphthalen-2-yl]-1H-indazole

To a solution of 3-[7-(2-pyrrolidin-1-yl-ethoxy)-naphthalen-2-yl]-1H-indazole-5-carboximidic acid ethyl ester (1.8 g, 4.2 mmol) in methanol (27 mL) was added 3,3-dimethyl-butyric acid hydrazide (2.1 g, 16 mmol). Triethylamine (8.1 g, 80 mmol) was then added to the mixture under an inert atmosphere. After stirring for 30 min, the reaction mixture was refluxed at 85 °C over 24 h. The resulting crude residue was concentrated under reduced pressure and purified by flash chromatography using 10% methanol in methylene chloride as an eluent. Further purification was achieved via preparatory HPLC to provide the title compound as a white solid with 98% purity by analytical HPLC (69 mg, 0.14 mmol, 3%): ¹HMR (CD₃OD) 8.81 (s, 1H) 8.40 (s, 1H) 8.09 (d, 1H) 7.95 (d, 1H) 7.84 (d, 1H) 7.75 (d, 1H) 7.61 (d, 1H) 7.39 (dd, 1H) 7.15 (dd, 1H) 4.22 (t, 2H) 3.27 (m, 2H) 2.98 (t, 2H) 2.64 (m, 4H) 1.78 (m, 4H) 1.10 (s, 9H); LC-MS (m/z) 495.5 [M+1]⁺.

### EXAMPLE 87

### SYNTHESIS OF 5-[5-(2,2-DINMTHYL-PROPYL)-4H-[1,2,4] TRIAZOL-3-YL]-3-[6-(1-METHYL-PIPERIDIN-2-YLMETHOXY)-NAPHTHALEN-2-YL]-1H-INDAZOLE

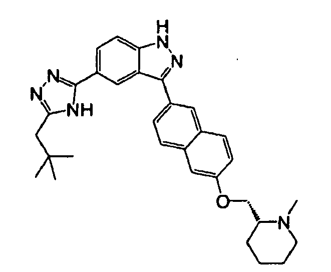

### A. (1-Methyl-piperidin-2-y]-methanol

To a solution of piperidin-2-yl-methanol (1.1 g, 2.5 mmol) in formaldehyde (5 mL, 37 wt. % solution in water) was added formic acid (2.6 g, 57 mmol) at 0 °C under an inert atmosphere. The reaction mixture was refluxed at 100 °C for 24 h. After cooling to room temperature the mixture was concentrated under reduced pressure and then diluted in THF (20 mL). Lithium aluminum hydride (1.5 g, 40 mmol, 1.0 M in THF) was added dropwise to this solution at 0 °C. After slowly warming to room temperature over 1 h, the excess LiAlH₄ was quenched by stirring the reaction mixture with sodium sulfate decahydrate in celite (1:1, 40 g). The mixture was then filtered and rinsed with ethyl acetate (200 mL). The ethereal layers were concentrated to provide the title compound as a yellow oil (1.1 g, 8.4 mol, 60%): LC-MS (m/z) 130 [M+1]⁺.

### B. 3-[6-(1-Methyl-piperidin-2-ylmethoxy)-naphthalen-2-yl]-1-(tetrahydro-pyran-2-yl)-1H-iadazole-5-carbonitrile

To a solution of (1-methyl-piperidin-2-yl)-methanol (0.9 g, 7 mmol) in THF (22 mL) at room temperature was added 3-(6-hydroxy-naphthalen-2-yl)-1-(tetrahydro-pyran-2-yl)-1*H*-indazole-5-carbonitrile (2.6 g, 7.0 mmol). Diisopropyl azodicarboxylate (6.8 g, 34 mmol) and triphenyl phosphine (2.1 g, 8.1 mmol) were then added and the reaction mixture was stirred over 24 h. The impure residue was concentrated and purification was carried out by flash chromatography using a gradient of 2% to 5% methanol in methylene chloride to provide the title compound as a yellow oil (1.5 g, 3.1 mmol, 44%): LC-MS (m/z) 481.4 [M+1]⁺.

### C. 3-16-(Methyl-piperidin-2-ylmethoxy)-naphthalen-2-yl]-1H-indazole-5-carboximidic acid ethyl ester

The title compound (1.1 g, 2.5 mmol, 81%) was prepared as described in Example 83, step D from 3-[6-(1-Methyl-piperidin-2-ylmethoxy)-naphthalen-2-yl]-1-(tetrahydro-pyran-2-yl)-1*H*-indazole-5-carbonitrile (1.5 g, 3.1 mmol): LC-MS (m/z) 443.8 [M+1]⁺.

### D. 5-[5-(2,2-Dimethyl-propyl)-4H-[1,2,4] triazol-3-yl]-3-[6-(1-methylpiperidin-2-ylmethoxy)-naphthalen-2-yl]-1H-indazole

To a solution of 3-[6-(methyl-piperidin-2-ylmethoxy)-naphthalen-2-yl]-1*H-*indazole-5-carboximidic acid ethyl ester (1.1 g, 2.5 mmol) in methanol (17 mL) was added 3,3-dimethyl-butyric acid hydrazide (1.3 g, 10 mmol). Triethylamine (5.1 g, 51 mmol) was then added to the mixture under an inert atmosphere. After stirring for 30 min, the reaction mixture was refluxed at 85°C over 16 h. The resulting crude residue was concentrated under vacuum and purified by flash chromatography using a gradient of 1% to 10% methanol in methylene chloride. Further purification was achieved via preparatory HPLC to provide the title compound as a white solid with 99% purity by analytical HPLC (26 mg, 0.05 mmol, 2%): ¹HNMR (CD₃OD) 8.76 (s, 1H) 8.39 (s, 1H) 8.02 (ddd, 2H) 7.87 (d, 1H) 7.81 (d, 1H) 7.57 (d, 1H) 7.21 (d, 1H) 7.10 (dd, 1H) 2.92 (dd, 1H) 2.77 (dd, 1H) 2.64 (s, 3H) 2.62-2.45 (m, 2H) 2.13 (m, 2H) 1.89-1.81 (m, 2H) 1.79 (s, 2H) 1.76-1.67 (m, 3H) 0.97 (s, 9H); LC-MS (m/z) 510 [M+1]⁺.

### EXAMPLE 88

### SYNTHESIS OF 6-{5-[5-(2,2-DIMETHYL-PROPYL)-2h-[1.2.4)TRLIAZOL-3-YL]-1h-INDAZOL-3-YL}-NAPHTHALENE-2-CARBOXYLIC ACID (2-PYRROLIDIN-1-YL-ETHYL)-AMIDE

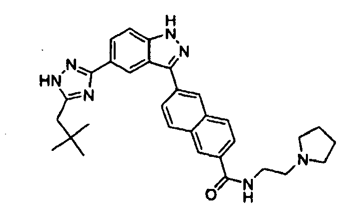

### A. 6-{5-15-(2,2-Dimethyl-propyl)-2H-[1.2.4]triazol-3-yl]-1H-indazol-3-yl}-naphthalene-2-carboxylic acid (2-pyrrolidin-1-yl-ethyl)-amide

A solution of 3-(6-(2-pyrrolidin-1-yl-ethylcarbarnoyl)-napthalen-2-yl)-1*H-*indazole-5-carboximidic acid ethyl ester di-hydrochloric acid salt (240 mg, 0.45 mmol), 3,3-dimethyl-butyric acid hydrazide (230 mg, 1.8 mmol), and triethyl amine (1.25 mL, 9.0 mmol) in methanol (5 mL) was heated in a sealed reaction flask on an 100 °C oil bath overnight. Solvent and excess triethyl amine were then removed under reduced pressure and the crude product was purified using reverse-phase preparatory HPLC (20-60% acetonitrile +0.1% TFA in H₂O + 0.1 % TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound, 99.25% pure by analytical HPLC (62.0 mg, 26% yield). ¹H NMR (400 MHz, DMSO-d*₆*) δ 13.56 (bs, 1H), 8.76 (bm, 2H), 8.58 (bs, 1H), 8.52 (s, 1H), 8.26-8.17 (overlapping m, 3H), 8.23 (m, 1H), 8.01 (m, 1H), 7.72 (m, 1H), 3.50 (m, 2H), 3.33 (s, 2H), 2.76 (bm, 2H), 2.65 (bm, 4H), 1.74 (bm, 4H), 1.00 (s, 9H); MS (ESI) *m*/*z* 522.4 [M+1]⁺.

### B. 3-(6-(2-pyrrolidin-1-yl-ethytcarbamoyl)-napthaten-2-yl)-1H-indazote-5-carboximidic acid ethyl ester di-hydrochloric acid salt

A solution of 6-[5-Cyano-1-(tetrahydro-pyran-2-yl)-1*H*-indazol-3-yl]-naphthalene-2-carboxylic acid (2-pyrrolidin-1-yl-ethyl) amide (300.0 mg, 0.61 mmol) in ethanol (100 mL) was cooled on a dry ice/isopropanol bath and saturated with HCl (g). The resulting solution was allowed to stir, gradually warming to room temperature, overnight. Excess solvent was removed under reduced pressure and.the resulting solid was taken up in Et₂O, sonicated 5 min and filtered to provide the title compound as a pale yellow solid (248.0 mg, 77%). MS (ESI) *m*/*z* 456.3 [free base M+1]⁺.

### C. 6-[5-Cyano-1-(tetrahydro-pyran-2-yl)-1H-indazol-3-yl]-naphthalene-2-carboxylic acid (2-pyrrolidin-1-yl-ethyl) amide

A solution of 6-[5-Cyano-1-(tetrahydro-pyran-2-yl)-1*H*-indazol-3-yl]-naphthalene-2-carboxylic acid (260 mg, 0.65 mmol), HOBT (100 mg, 0.74 mmol), EDCI (150 mg, 0.78 mmol), and 1-(2-aminoethyl)-pyrrolidine (0.1 mL, 0.78 mmol) in DMF (30 mL) was stirred at room temperature overnight. The solution was then poured into water and filtered to give the title compound (310 mg, 96%). MS (ESI) *m*/*z* 494.5 [M+1]⁺.

### EXAMPLE 89

### SYNTHESIS OF 6-[5-(PYRROLIDIN-1-YLMEETHYL-2H-[1.2.4]TRIAZOL-3-YL)-IH-INDAZOL-3-YL]-NAPHTHALENE-2-CARBOXYLIC ACID AMIDE

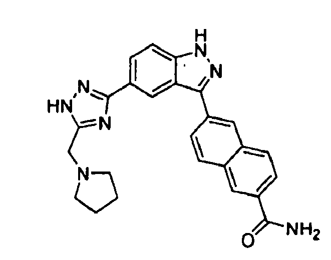

### A. 6-15-(Pyrrolidin-1-ylmethyl-2H-[1.2.4]triazol-3-yl)-1H-indazol-3-yl]-naphthelene-2-carboxylic acid amide

A solution of 3-(6-carbamoyl)-napthalen-2-yl)-1*H*-indazole-5-carboximidic acid ethyl ester di-hydrochloric acid salt (210 mg, 0.49 mmol), pyrrolidin-1-yl-acetic acid hydrazide (see WO 02/10137, example 422A) (280 mg, 2.0 mmol), and triethyl amine (1.4 mL, 10.0 mmol) in methanol (5 mL) was heated in a sealed reaction flask on an 100 °C oil bath overnight. Solvent and excess triethyl amine were then removed under reduced pressure and the crude product was purified using reverse-phase preparatory HPLC (20-80% acetonitrile +0.1% TFA in H₂O +0.1% TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound, 99.25% pure by analytical HPLC (69.0 mg, 32% yield). ¹H NMR (300 MHz, DMSO-d*₆*) δ 8.77 (bm, 1H), 8.55 (bs, 1H), 8.24-8.16 (overlapping m, 3H), 8.11 (m, 1H), 8.03 (m, 1H), 7.74 (bm, 1H), 7.52 (bs, 1H), 3.82 (s, 2H), 2.56 (bm, 4H), 1.73 (bm, 4H); MS (ESI) *mlz* 438.3 [M+1]⁺.

### B. 3-(6-Carbamoyl)napthalen-2-yl)-1H-indazole-5-carboximidic acid ethyl ester di-hydrochloric acid salt

A solution of 6-[5-Cyano-1-(tetrahydro-pyran-2-yl)-1*H*-indazol-3-yl]-naphthalene-2-carboxylic acid amide (200.0 mg, 0.5 mmol) in ethanol (60 mL) was cooled on a dry ice/isopropanol bath and saturated with HCl (g). The resulting solution was allowed to stir, gradually warming to room temperature, overnight. The reaction mixture was recharged daily with HCl (g) until reaction reached completion, 7 days total. Excess solvent was removed under reduced pressure and the resulting solid was taken up in Et₂O, sonicated 5 min and filtered to provide the title compound as a pale yellow solid (215.0 mg, 99%). MS (ESI) *mlz* 359.1 [free base M+1]⁺.

### C. 6-[5-Cyano-1-(tetrahydro-pyran-2-yl)-1H-indazol-3-yl]-naphthalene-2-carboxylic acid amide

A solution of 6-[5-Cyano-l-(tetrahydro-pyran-2-yl)-1*H*-indazol-3-yl]-naphthalene-2-carboxylic acid (350 mg, 0.88 mmol), HOBT (238 mg, 1.8 mmol), EDCI (210 mg, 1.3 mmol), ammonium chloride (95 mg, 1.8 mmol), and *N*-ethyl-morpholine (0.23 mL, 1.8 mmol) in DMF (30 mL) was stirred at room temperature overnight. The solution was then poured into water and filtered. The resulting solid was taken up in EtOAc, dried over magnesium sulfate, filtered and solvent removed under reduced pressure to give the title compound (215 mg, 62%). MS (ESI) *mlz* 397.1 [M+1]⁺.

### EXAMPLE 90

### SYNTHESIS OF 5-[5-(2,2-DIMETHYL-PROPYL)-2H-[1,2,4]TRIAZOL-3-YL]-3-(6-FLUORO-NAPHTHALEN-2-YL)-1H-INDAZOLE

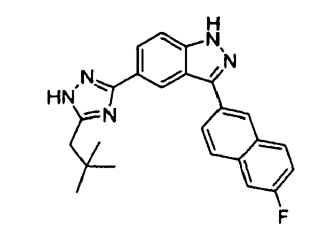

A solution of 3-(6-fluoro-naphthalen-2-yl)-1*H*-indazole-5-carboximidic acid ethyl ester di-hydrochloric acid salt (250 mg, 0.62 mmol), 3,3-dimethyl-butyric acid hydrazide (320 mg, 2.5 mmol), and triethyl amine (1.7 mL, 12.2 mmol) in methanol (6 mL) was heated in a sealed reaction flask on an 100 °C oil bath overnight. Solvent and excess triethyl amine were then removed under reduced pressure and the crude product was purified using reverse-phase preparatory HPLC (20-70% acetonitrile +0.1% TFA in H₂O +0.1% TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound, 99.7% pure by analytical HPLC (115 mg, 47%). ¹H NMR (300 MHz, DMSO-d*₆*) δ 8.76 (m, 1H), 8.60 (m, 1H), 8.24-8.08 (overlapping m, 4H), 7.80 (m, 1 H), 7.68 (m, 1H), 7.51 (m, 1H), 2.66 (s, 2H), 1.00 (s, 9H); MS (ESI) *m*/*z* 400.3 [M+1]⁺.

### EXAMPLE 91

### SYNTHESIS OF 3-(6-CHLORO-NAPHTHALEN-2-YL)-5-(5-PYRROLIDIN-1-YLMETHYL-2H-[1,2,4]TRLAZOL-3-YL)-1H-INDAZOLE

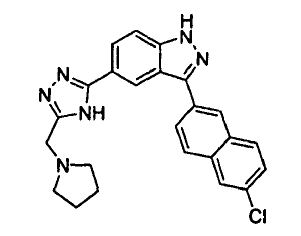

### A. 3-(6-Cbloro-naphtbalen-2-yl)-5-(5-pyrrolidin-1-ylmethyl-2H-[1,2,4]triazol-3-yl)-1H-indazole

A solution of 3-(6-chloro-naphthalen-2-yl)-1*H*-indazole-5-carboximidic acid ethyl ester di-hydrochloric acid salt (200 mg, 0.47 mmol), pyrrolidin-1-yl-acetic acid hydrazide (see WO 02/10137, example 422A) (270 mg, 1.9 mmol), and triethyl amine (1.3 mL, 9.3 mmol) in methanol (5 mL) was heated in a sealed reaction flask on an 100 °C oil bath overnight. Solvent and excess triethyl amine were then removed under reduced pressure and the crude product was purified using reverse-phase preparatory HPLC (20-80% acetonitrile + 0.1% TFA in H₂O +0.1% TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound (38.0 mg, 19%). ¹H NMR (300 MHz, acetone-d*₆*) δ 8.95 (s, 1H), 8.60 (s, 1H); 8.34 (dd, 1H), 8.24 (dd, 1H), 8.16 (d, 1H), 8.08 (d, 1H), 8.05 (d, 1H), 7.76 (d, 1H), 7.57 (dd, 1H), 3.97 (s, 2H), 2.76 (bm, 4H), 1.82 (quin, 4H); MS (ESI) *mlz* 429.1 [M+1]⁺.

### B. 3-(6-Chloro-naphthalen-2-yl)-1H-indazole-5-carboximidic acid ethyl ester di-bydrochloric acid salt

A solution of 3-[6-chloro-napthalen-2-yl]-1-(tetrahydro-pyran-2-yl)-1*H*-indazole-5-carbonitrile (310 mg, 0.8 mmol) in ethanol (100 mL) was cooled on a dry ice/isopropanol bath and saturated with HCl (g). The resulting solution was allowed to stir, gradually warming to room temperature, overnight. Excess solvent was removed under reduced pressure and the resulting solid was taken up in Et₂O, sonicated 5 min and filtered to provide the title compound as a pale yellow solid (205.6 mg, 61%). MS (ESI) *mlz* 350.1 [free base M+1]⁺.

### C. 3-[6-Chloro-napthalen-2-yl]-1-(tetrahydro-pyran-2-yl)-1H-indazole-5-carbonitrile

A solution of 2-chloro-6-bromo-naphthalene (0.6 g, 2.5 mmol), bis(pinacolato)diboron (0.63 g, 2.5 mmol), [1,1'-bis(diphenylphosphino)-ferrocene] dichloro palladium complex with dichloromethane (1:1) (0.2 g, 0.25 mmol), and potassium acetate (0.73 g, 7.4 mmol) in DMF (60 mL) was heated in a sealed reaction flask on an 80°C oil bath 2h. To this reaction mixture was added 3-bromo-1-(tetrahydro-pyran-2-yl)1H-indazole-5-carbonitrile (see WO 02/10137, example 161D) (0.68 g, 2.2 mmol) and potassium phosphate (1.6 g, 7.5 mmol) and the solution was returned to heat overnight. The cooled solution was filtered through Celite and the filter cake washed with EtOAc. The filtrate was washed with water and brine, dried over magnesium sulfate, filtered and solvent removed under reduced pressure to afford crude product. Purification using silica gel flash column chromatography (1:4 EtOAc:hexanes) and a second column (CH₂Cl₂ provided title compound (0.32 g, 37%). MS (ESI) *mlz* 388.1 [M]⁺.

### EXAMPLE 92

### SYNTHESIS OF BIS-(2-METHOXY-ETHYL)-{5-[3-(6-METHOXY-NAPHTHALEN-2-YL)-1H-INDAZOL-5-YL]-1H-[1.2.4]TRLAZOL-3-YLMETHYL}-AMINE

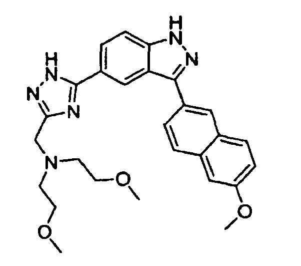

A solution of 3-(6-methoxy-napthalen-2-yl)-1*H*-indazole-5-carboximidic acid ethyl ester di-hydrochloric acid salt (see WO 02/10137, example 423B) (1.5 g, 3.6 mmol), [bis-(2-methoxy-ethyl)-amino]-acetic acid hydrazide (3.0 g, 14.6 mmol), and triethyl amine (10.0 mL, 71.7 mmol) in methanol (20 mL) was heated in a sealed reaction flask on an 100 °C oil bath overnight. Solvent and excess triethyl amine were then removed under reduced pressure. Initial purification using reverse-phase preparatory HPLC (20-70% acetonitrile + 0.1 % TFA in H₂O + 0.1 % TFA, over 30 min) provided crude product. Further purification using reverse-phase preparatory HPLC (20-60% acetonitrile +0.1% TFA in H₂O +0.1% TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound (103 mg, 6%). ¹H NMR (400 MHz, acetone-d*₆*) δ 12.65 (bs, 1H), 8.90 (s, 1H), 8.49 (s, 1H), 8.18 (m, 2H), 7.94 (m, 2H), 7.68 (m, 1H), 7.33 (s, 1H), 7.18 (m, 1H), 3.99 (s, 2H), 3.92 (s, 3H), 3.50 (bm, 4H), 3.28 (s, 6H), 2.83 (bm, 4H); MS (ESI) *m*/*z* 487.4 [M+1]⁺.

### EXAMPLE 93

### SYNTHESIS OF (S)-5-(5-ISOBUTYL-2H-[1,2,4]TRLkZOL-3-YL)-3-[6-(1-METHYLPYRROLIDIN-2-YLMETHOXY)-NAPHTHALEN-2-YL]-1H-INDAZOLE

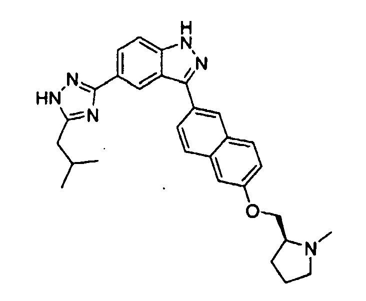

### A. (S)-5-(5-Isobutyl-2H-[1,2,4]triazot-3-yt)-3-[6-(1-metbyt-pyrrotidin-2-ylmethoxy)-naphthalen-2-yl)-1H-indazole

A solution of *(S)*-3-[6-(1-methyl-pyrrolidin-2-ylmethoxy)-napthalen-2-yl]-1*H-*indazole-5-carboximidic acid ethyl ester di-hydrochloric acid salt (0.5 g, 1.0 mmol), 3-methyl-butyric acid hydrazide (0.46 g, 4.0 mmol), and triethyl amine (2.8 mL, 20 mmol) in methanol (10 mL) was heated in a sealed reaction flask on an 100 °C oil bath for three hours. Solvent and excess triethyl amine were then removed under reduced pressure and the crude product was purified using reverse-phase preparatory HPLC (20-60% acetonitrile +0.1% TFA in H₂O +0.1% TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound, 100% pure by analytical HPLC (170 mg, 36%). ¹H NMR (400 MHz, DMSO-d*₆*) δ 13.40 (bs, 1H), 8.73 (s, 1H), 8.43 (s, 1H), 8.10 (m, 2H), 8.00 (m, 2H), 7.68 (d, 1H), 7.44 (m, 1H), 7.24 (dd, 1H), 4.08 (m, 2H), 3.02 (m, 1H), 2.61 (d, 2H), 2.45 (s, 3H), 2.23 (m, 1H), 2.14-1.99 (overlapping m, 3H), 1.78-1.63 (overlapping m, 3H), 0.95 (d, 6H); MS (ESI) *m*/*z* 481.4 [M+1]⁺.

### B. (S)-3-[6-(1-Methyl-pyrrolidin-2-ytmethoxy)-napthaten-2-yl]-1H-indazole-5-carboximidic acid ethyl ester di-hydrochloric acid salt

A solution of *(S)*-3-[6-(1-methyl-pyrrolidin-2-ylmethoxy)-napthalen-2-yl]-1-(tetrahydro-pyran-2-yl)-1*H*-indazole-5-carbonitrile (1.7 g, 3.6 mmol) in ethanol (75 mL) was cooled on a dry ice/isopropanol bath and saturated with HCl (g). The resulting solution was allowed to stir, gradually warming to room temperature, overnight. The reaction mixture was recharged daily with HCl (g) until reaction reached completion, 3 days total. Excess solvent was removed under reduced pressure and the resulting solid was taken up in Et₂O, sonicated 5 min and filtered to provide the title compound as a pale yellow solid (1.47 g, 80%). MS (ESI) *mlz* 429.3 [free base M+1]⁺.

### C. (S)-3-[6-(1-methyt-pyrroldin-2-ytmethoxy)-naptha!en-2-yl]-1-(tetrahydro-pyran-2-yl)-1H-indazole-5-carbonitrile

A solution of 3-(6-hydroxy-napthalen-2-yl)-1-(tetrahydro-pyran-2-yl)-1*H-*indazole-5-carbonitrile (2.0 g, 5.4 mmol) and triphenyl-phosphine (2.8 g, 10.7 mmol) in THF (100 mL) was treated with *(S)*-(1-methyl-pyrrolidin-2-yl)-methanol (2.5 g, 21.7 mmol) and DIAD (2.1 mL, 10.7 mmol). The resulting solution was allowed to stir at room temperature 1.5 h. Excess THF was removed under reduced pressure and the resulting solid was taken up in EtOAc, washed with water, brine, then dried over magnesium sulfate, filtered and solvent removed under reduced pressure to provide crude material. Purification using silica gel flash column chromatograph (10% methanol in CH₂Cl₂) provided the title compound (2.13 g, 84%). MS (ESI) *mlz* 467.4 [M+1]⁺.

### D. 3-(6-Hydroxy-napthalen-2-yl)-1-(tetrahydro-pyran-2-yl)-1H-indazole-5 carbonitrile

A solution of 2-bromo-6-hydroxy-naphthalene (5.0 g, 22.4 mmol), bis(pinacolato)diboron (5.7 g, 22.4 mmol), [1,1'-bis(diphenylphosphino)-ferrocene] dichloro palladium complex with dichloromethane (1:1) (1.8 g, 2.20 mmol), and potassium acetate (6.6 g, 67.2 mmol) in DMF (200 mL) was heated in a sealed reaction flask on an 85°C oil bath 2.5 h. To this reaction mixture was added 3-bromo-1-(tetrahydro-pyran-2-yl)1H-indazole-5-carbonitrile (see WO 02/10137, example 161D) (6.86 g, 22.4 mmol) and potassium phosphate (14.3 g, 67.5 mmol) and the solution was returned to heat overnight. Excess DMF was removed under reduced pressure and the resulting residue taken up in EtOAc and water, filtered through Celite and the filter cake washed with EtOAc. The filtrate was washed with water and brine, dried over magnesium sulfate, filtered and solvent removed under reduced pressure to afford crude product. Purification using silica gel flash column chromatography (1:2 EtOAc:hexanes) provided title compound (4.45 g, 54%). MS (ESI) *mlz* 370.1 [M]⁺.

### EXAMPLE 94

### SYNTHESIS OF (S)-5-[5-(2,2-DEMETHYL-PYROPYL)-2H-[1,2,4]TRIAZOL-3-YL]-3-[6-( 1-METHYL-PYRROLIDIN-2-YLMETHOX)-NAPHTHALEN-2-YL)-1H-INDAZOLE

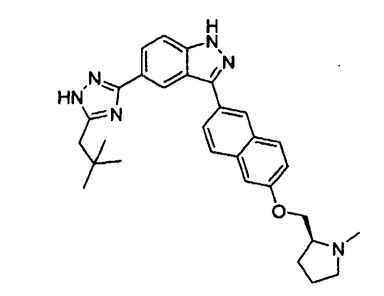

A solution of *(S)*-3-[6-(1-methyl-pyrrolidin-2-ylmethoxy)-napthalen-2-yl]-1*H-*indazole-5-carboximidic acid ethyl ester di-hydrochloric acid salt (see Example 93, step B) (0.5 g, 1.0 mmol), 3,3-dimethyl-butyric acid hydrazide (0.52 g, 4.0 mmol), and triethyl amine (2.8 mL, 20 mmol) in methanol (5 mL) was heated in a sealed reaction flask on an 100 °C oil bath for three hours. Solvent and excess triethyl amine were then removed under reduced pressure and the crude product was purified using reverse-phase preparatory HPLC (20-60% acetonitrile +0.1% TFA in H₂O +0.1% TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound, 100% pure by analytical HPLC (173.2 mg, 35%). ¹H NMR (300 MHz, DMSO-d*₆*) δ 8.72 (s, 1H), 8.42 (s, 1H), 8.09 (m, 2H), 7.99 (m, 2H), 7.68 (d, 1H), 7.45 (m, 1H), 7.24 (dd, 1H), 4.09 (m, 2H), 3.02 (m, 1H), 2.64 (s, 2H), 2.45 (s, 3H), 2.23 (m, 2H), 2.05 (m, 2H), 1.72 (m, 2H), 1.00 (s, 9H); MS (ESI) *m*/*z* 495.4 [M+1]⁺.

### EXAMPLE 95

### SYNTHESIS OF (S)-5-(5-TERT-BUTYL-2H-[1,2,4]TRIAZOL-3-YL)-3-[6-(1-METHYL-PYRROLIDIN-2-YLMETHOXY)-NAPHTHALEN-2-YL]-1H-INDAZOLE

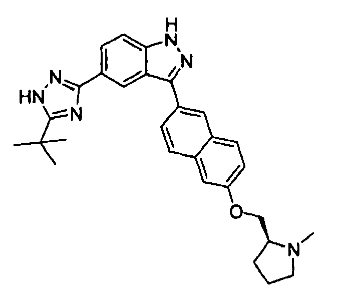

A solution of *(S)*-3-[6-(1-methyl-pyrrolidin-2-ylmethoxy)-napthalen-2-yl]-1*H-*indazole-5-carboximidic acid ethyl ester di-hydrochloric acid salt (see Example 93, step B) (0.43 g, 0.8 mmol), pivalic acid hydrazide (0.39 g, 3.4 mmol), and triethyl amine (2.4 mL, 17.2 mmol) in methanol (5 mL) was heated in a sealed reaction flask on an 100 °C oil bath for three hours. Solvent and excess triethyl amine were then removed under reduced pressure and the crude product was purified using reverse-phase preparatory HPLC (20-60% acetonitrile +0.1% TFA in H₂O +0.1% TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound, 100% pure by analytical HPLC (96.3 mg, 23%). ¹H NMR (400 MHz, DMSO-d*₆*) δ 8.69 (s, 1H), 8.38 (s, 1H), 8.06 (m, 2H), 7.97 (m, 2H), 7.63 (d, 1H), 7.38 (m, 1H), 7.19 (dd, 1H), 4.14 (m, 2H), 3.02 (m, 1H), 2.68 (m, 2H), 2.45 (s, 3H), 2.26 (m, 1H), 2.05 (m, 1H), 1.73 (m, 2H), 1.42 (s, 9H); MS (ESI) *m*/*z* 481.4 [M+1]⁺.

### EXAMPLE 96

### SYNTHESIS OF (S)-3-[6-(1-MITHYL-PYRROLDIN-2-YLMETHOXY)-NAPHTHALEN-2-YL]-5-(5-PYRROLIDIN-1-YLMETHYL-2H-[1,2,4]TRIAZOL-3-YL)-1H-INDAZOLE

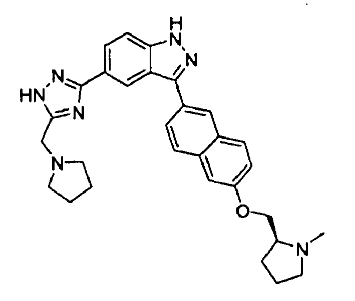

A solution of *(S)*-3-[6-(1-methyl-pyrrolidin-2-ylmethoxy)-napthalen-2-yl]-1*H-*indazole-5-carboximidic acid ethyl ester di-hydrochloric acid salt (see Example 93, step B) (0.5 g, 1.0 mmol), pyrrolidin-1-yl-acetic acid hydrazide (see WO 02/10137, example 422A) (0.57 g, 4.0 mmol), and triethyl amine (2.8 mL, 20.0 mmol) in methanol (6 mL) was heated in a sealed reaction flask on an 100 °C oil bath for three hours. Solvent and excess triethyl amine were then removed under reduced pressure and the crude product was purified using reverse-phase preparatory HPLC (20-60% acetonitrile +0.1% TFA in H₂O + 0.1% TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound, 100% pure by analytical HPLC (103 mg, 20%). ¹H NMR (400 MHz, CD₃OD) δ 8.87 (s, 1H), 8.48 (s, 1H), 8.12 (m, 2H), 7.98 (d, 1H), 7.94 (d, 1H), 7.70 (d, 1H), 7.34 (m, 1H), 7.25 (m, 1H), 4.18 (d, 2H), 3.89 (s, 2H), 3.14 (m, 1H), 2.85 (m, 2H), 2.72 (m, 4H), 2.41 (m, 1H), 2.17 (m, 1H), 1.86 (overlapping m, 6H); MS (ESI) *m*/*z* 481.4 [M+1]⁺.

### EXAMPLE 97

### SYNTHESIS OF 3-(6-HYDROXY-NAPTHALEN-2-YL)-1H INDAZOLE-5-CARBONITRILE

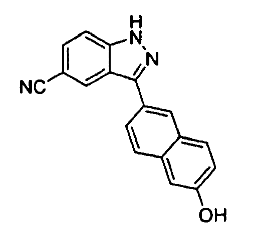

A solution of 3-(6-hydroxy-napthalen-2-yl)-1-(tetrahydro-pyran-2-yl)-1*H-*indazole-5-carbonitrile (see Example 93, step B) (0.25 g, 0.46 mmol) in ethanol (100 mL) was cooled on a dry ice/isopropanol bath and treated briefly with HCl (g) and allowed to stir 20 min. Excess solvent was then removed under reduced pressure and the crude product was purified using reverse-phase preparatory HPLC (30-100% acetonitrile + 0.1 % TFA in H₂O +0.1% TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound, 99.4% pure by analytical HPLC (185 mg, 96%). ¹H NMR (400 MHz, DMSO-d*₆*) δ 13.72 (s, 1H), 9.85 (s, 1H), 8.89 (s, 1H), 8.52 (s, 1H), 8.08 (dd, 1H), 8.02 (d, 1H), 7.81 (d, 1H), 7.76 (m, 2H), 7.17 (m, 1H), 7.14 (m, 1H); MS (ESI) *m*/*z* 286.2 [M+1]⁺.

### EXAMPLE 98

### SYNTHESIS OF [2-(6-{5-[5-(2,2-DIMETHYL-PYROPYL)-2H-[1,2,4]TRIAZOL-3-YL]-1H-INDAZOL-3-YL}-NAPHTHALEN-2-YLOXY)-ETHYL]-ETHYL-ISOPROPYL-AMINE

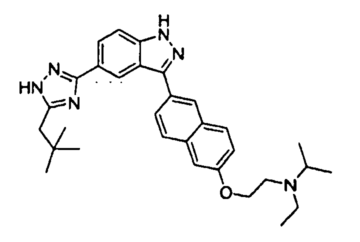

### A. [2-(6-{5-[5-(2,2-Dimethyl-pyropyl)-2H-[1,2,4]triazol-3-yl]-1H-indazol-3-yl}-naphthalen-2-yloxy)-ethyl]-ethyl-isopropyl-amine

A solution of 3-{6-[2-(ethyl-isopyropy-arnino)-ethoxy]-napthalen-2-yl)-1*H-*indazole-5-carboximidic acid ethyl ester di-hydrochloric acid salt (0.4 g, 0.8 mmol), 3,3-dimethyl-butyric acid hydrazide (0.4 g, 3.1 mmol), and triethyl amine (2.2 mL, 15.8 mmol) in methanol (5 mL) was heated in a sealed reaction flask on an 100 °C oil bath for three hours. Solvent and excess triethyl amine were then removed under reduced pressure and the crude product was purified using reverse-phase preparatory HPLC (20-60% acetonitrile + 0.1% TFA in H₂O +0.1% TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound, 100% pure by analytical HPLC (173.2 mg, 35%). ¹H NMR (300 MHz, CD₃OD) δ 8.78 (s, 1H), 8.39 (s, 1H), 8.05 (m, 2H), 7.87 (d, 1H), 7.83 (d, 1H), 7.56 (d, 1H), 7.20 (d, 1H), 7.12 (dd, 1H), 4.11 (t, 2H), 3.10 (sep, 1H), 2.91 (t, 2H), 2.68 (q, 2H), 2.67 (s, 2H), 1.08 (t, 3H), 1.05 (d, 6H), 0.99 (s, 9H); MS (ESI) m/z 511.3 [M+1]⁺.

### B. 3-{6-[2-(Ethyl-isopyropy-amino)-ethoxy]-napthalen-2-yl)-1H-indazole-5-carboximidic acid ethyl ester di-hydrochloric acid salt

A solution of 3-{6-[2-(ethyl-isopyropy-amino)-ethoxy]-napthalen-2-yl}-1-(tetrahydro-pyran-2-yl)-1*H*-indazole-5-carbonitrile (0.45 g, 0.9 mmol) in ethanol (100 mL) was cooled on a dry ice/isopropanol bath and saturated with HCl (g). The resulting solution was allowed to stir, gradually warming to room temperature, overnight. Excess solvent was removed under reduced pressure and the resulting solid was taken up in Et₂O, sonicated 5 min and filtered to provide the title compound as a pale yellow solid (0.4 g, 83%). MS (ESI) m/z 445.4 [free base M+1]⁺.

### C. 3-{6-[2-(Ethyl-isopyropy-amino)-ethoxy]-napthalen-2-yl)-1-(tetrahydro-pyran-2-yl)-1H-indazole-5-carbonitrile

A solution of 3-(6-hydroxy-napthalen-2-yl)-1-(tetrahydro-pyran-2-yl)-1H-indazole-5-carbonitrile (see Example 93, step D) (0.7 g, 1.9 mmol) and triphenylphosphine (1.6 g, 6.1 mmol) in THF (30 mL) was treated with 2-(ethyl-isopropylamino)-ethanol (0.5 g, 3.8 mmol) and DIAD (1.2 mL, 6.1 mmol). The resulting solution was allowed to stir at room temperature 1.5 h. Excess THF was removed under reduced pressure and the resulting solid was taken up in EtOAc, washed with water, brine, then dried over magnesium sulfate, filtered and solvent removed under reduced pressure to provide crude material. Purification using silica gel flash column chromatograph (10% methanol in CH₂Cl₂) provided the title compound (0.47 g, 52%). MS (ESI) *m*/*z* 483.0 [M+1]⁺.

### EXAMPLE 99

### SYNTHESIS 3-{6-[2-(2,6-CIS-DIMETHYL-PIPERIDI-1-YL)-ETHOXY]-NAPHTHALEN-2-YL}-5-[5-(3-METHYL-BUTYL)-2H-[1,2,4]TRIAZOL-3-YL]-1H-INDAZOLE

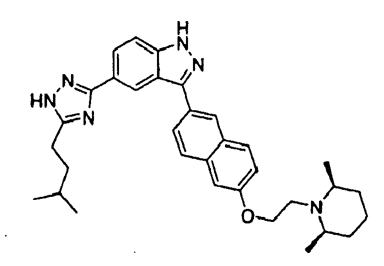

A solution of 3- {6-[2-(2,6-*cis*-Dimethyl-piperidi-1-yl)-ethoxy]-naphthalen-2-yl}-1*H-*indazole-5-carboximidic acid ethyl ester di-hydrochloric acid salt (0.37 g, 0.7 mmol), iso-valeric acid hydrazide (0.35 g, 2.7 mmol), and triethyl amine (1.9 mL, 13.6 mmol) in methanol (4 mL) was heated in a sealed reaction flask on an 100 °C oil bath for three hours. Solvent and excess triethyl amine were then removed under reduced pressure and the crude product was purified using reverse-phase preparatory HPLC (20-60% acetonitrile +0.1% TFA in H₂O +0.1% TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound. Further purification using silica gel flash column chromatograph (10% methanol in CH₂Cl₂) provided the title compound, 99% pure by analytical HPLC (43.0 mg, 12%). ¹H NMR (400 MHz, CD₃OD) δ 8.85 (s, 1H), 8.48 (s, 1H), 8.13 (m, 2H), 7.99 (d, 1H), 7.94 (d, 1H), 7.68 (d, 1H), 7.34 (d, 1H), 7.22 (dd, 1H), 4.28 (t, 2H), 3.33 (m, 2H), 2.87 (t, 2H), 2.84 (br m, 2H), 1.68 (br m, 6H), 1.42 (overlapping m, 3H), 1.29 (d, 6H), 1.00 (d, 6H); MS (ESI) *m*/*z* 537.4 [M+1]⁺.

### EXAMPLE 100

### SYNTHESIS OF (2-{6-[5-(5-ISOBUTYL-2H-[1,2,4]TRIAZOL-3-YL)-1H-INDAZOL-3-YL]-NAPHTHALEN-2-YLOXY}-ETHYL)-DIISOPROPYL-AMINE

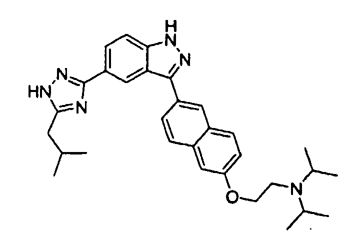

### A. (2-{6-[5-(5-Isobutyl-2H-[1,2,4]triazol-3-yl)-1H-indazol-3-yl]-naphthalen-2-yioxy}-ethyl)-diisopropyl-amine

A solution of 3-[6-(2-diisopyropyamino-ethoxy)-napthalen-2-yl]-1*H*-indazole-5-carboximidic acid ethyl ester di-hydrochloric acid salt (0.4 g, 0.75 mmol), 3-methyl-butyric acid hydrazide (0.35 g, 3.0 mmol), and triethyl amine (2.1 mL, 15.1 mmol) in methanol (4.5 mL) was heated in a sealed reaction flask on an 100°C oil bath overnight. Solvent and excess triethyl amine were then removed under reduced pressure and the crude product was purified using reverse-phase preparatory HPLC (20-60% acetonitrile +0.1% TFA in H₂O +0.1% TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound, 100% pure by analytical HPLC (96.0 mg, 25%). ¹H NMR (400 MHz, CD₃OD) δ 8.84 (s, 1H), 8.46 (s, 1H), 8.11 (m, 2H), 7.96 (d, 1H), 7.91 (d, 1H), 7.67 (m, 1H), 7.28 (m, 1H), 7.16 (dd, 1H), 4.10 (t, 2H), 3.17 (sep, 2H), 2.96 (t, 2H), 2.72 (d, 2H), 2.19 (sep, 1H), 1.12 (d, 12H), 1.02 (d, 6H); MS (ESI) *m*/*z* 512.1 [M+1]⁺.

### B. 3-[6-(2-Diisopyropyamino-ethoxy)-napthalen-2-yl]-1H-indazole-5-carboximidic acid ethyl ester di-hydrochloric acid salt

A solution of 3-[6-(2-diisopyropyamino-ethoxy)-napthalen-2-yl]-1-(tetrahydro-pyran-2-yl)-1*H*-indazole-5-carbonitrile (2.68 g, 5.4 mmol) in ethanol (350 mL) was cooled on a dry ice/isopropanol bath and saturated with HCl (g). The resulting solution was allowed to stir, gradually warming to room temperature, overnight. Excess solvent was removed under reduced pressure and the resulting solid was taken up in Et₂O, sonicated 5 min and filtered to provide the title compound as a pale yellow solid (2.01 g, 71%). MS (ESI) *m*/*z* 459.9 [free base M+1]⁺.

### C. 3-[6-(2-Diisopyropyamino-ethoxy)-napthalen-2-yl]-1-(tetrahydro-pyran-2-yl)-1H-indazole-5-carbonitrile

A solution of 3-(6-hydroxy-napthalen-2-yl)-1-(tetrahydro-pyran-2-yl)-1*H* indazole-5-carbonitrile (see Example 93, step D) (2.0 g, 5.4 mmol) and triphenylphosphine (4.3 g, 16.4 mmol) in THF (100 mL) was treated with 2-(diisopropylamino)-ethanol (2.9 mL, 15.8 mmol) and DIAD (3.2 mL, 16.3 mmol). The resulting solution was allowed to stir at room temperature overnight. Excess THF was removed under reduced pressure and the resulting solid was taken up in EtOAc, washed with water, brine, then dried over magnesium sulfate, filtered and solvent removed under reduced pressure to provide crude material. Purification using silica gel flash column chromatograph (10% methanol in CH₂Cl₂) provided the title compound, >80% clean (3.5 g). MS (ESI) *m*/*z* 498.1 [M+1]⁺.

### EXAMPLE 101

### SYNTHESIS DIISOPROPYL-[2-(6-{5-[5-(3-METHYL-BUTYL)-2H-[1,2,4]TRIAZOL-3-YL]-1H-INDAZOL-3-YL}-NAPHTHALEN-2-YLOXY)-ETHYL]-AMINE

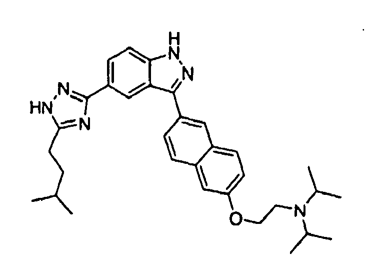

A solution of 3-[6-(2-diisopyropyamino-ethoxy)-napthalen-2-yl]-1*H*-indazole-5-carboximidic acid ethyl ester di-hydrochloric acid salt (see Example 100, step B) (0.4 g, 0.75 mmol), iso-valeric acid hydrazide (0.39 g, 3.0 mmol), and triethyl amine (2.1 mL, 15.1 mmol) in methanol (5.0 mL) was heated in a sealed reaction flask on an 100 °C oil bath overnight. Solvent and excess triethyl amine were then removed under reduced pressure and the crude product was purified using reverse-phase preparatory HPLC (20-60% acetonitrile +0.1% TFA in H₂O +0.1% TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound, 100% pure by analytical HPLC (52.0 mg, 13%). ¹H NMR (300 MHz, CD₃OD) δ 8.83 (s, 1H), 8.46 (s, 1H), 8.10 (m, 2H), 7.96 (d, 1H), 7.91 (d, 1H), 7.67 (dd, 1H), 7.28 (d, 1H), 7.20 (dd, 1H), 4.13 (t, 2H), 3.20 (sep, 2H), 3.02 (t, 2H), 2.86 (m, 2H), 1.70 (m, 2H), 1.64 (m, 1H), 1.15 (d, 12H), 0.99 (d, 6H); MS (ESI) *m*/*z* 524.9 [M+1]⁺.

### EXAMPLE 102

### SYNTHESIS DIISOPROPYL-(2-{6-[5-(5-PYRROLIDIN-1-YLMETHYL-2H-[1,2,4]TRIAZOL-3-YL)-1H-INDAZOL-3-YL]-NAPHTHALEN-2-YLOXY}-ETHYL)-AMINE

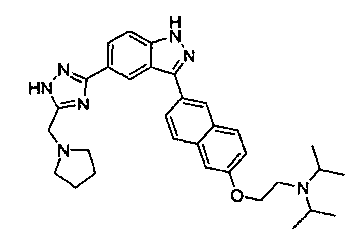

A solution of 3-[6-(2-diisopyropyamino-ethoxy)-napthalen-2-yl]-1*H*-indazole-5-carboximidic acid ethyl ester di-hydrochloric acid salt (see Example 100, step B) (0.4 g, 0.75 mmol), pyrrolidin-1-yl-acetic acid hydrazide (0.43 g, 3.0 mmol), and triethyl amine (2.1 mL, 15.1 mmol) in methanol (4.5 mL) was heated in a sealed reaction flask on an 100°C oil bath overnight. Solvent and excess triethyl amine were then removed under reduced pressure and the crude product was purified using reverse-phase preparatory HPLC (20-60% acetonitrile +0.1% TFA in H₂O +0.1% TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound, 99.53% pure by analytical HPLC (105.0 mg, 27%). ¹H NMR (400 MHz, CD₃OD) δ 8.86 (m, 1H), 8.47 (s, 1H), 8.11 (m, 2H), 7.97 (d, 1H), 7.93 (d, 1H), 7.69 (dd, 1H), 7.31 (d, 1H), 7.22 (dd, 1H), 4.14 (t, 2H), 3.89 (s, 2H), 3.21 (sep, 2H), 3.03 (t, 2H), 2.72 (m, 4H), 1.85 (m, 4H), 1.16 (d, 12H); MS (ESI) *m*/*z* 512.3 [M+1]^{+]}.

### EXAMPLE 103

### SYNTHESIS [2-(6-{5-[5-(2,2-DIMETHYL-PROPYL)-2H-[1,2,4]TRIAZOL-3-YL]-1H-INDAZOL-3-YL}-NAPHTHALEN-2-YLOXY)-ETHYL]-DIISOPROPYL-AMINE

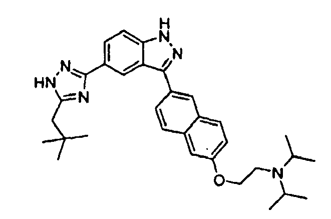

A solution of 3-[6-(2-diisopyropyamino-ethoxy)-napthalen-2-yl]-1*H*-indazole-5-carboximidic acid ethyl ester di-hydrochloric acid salt (see Example 100, step B) (0.4 g, 0.75 mmol), 3,3-dimethyl-butyric acid hydrazide (0.39 g, 3.0 mmol), and triethyl amine (2.1 mL, 15.1 mmol) in methanol (5.0 mL) was heated in a sealed reaction flask on an 100°C oil bath overnight. Solvent and excess triethyl amine were then removed under reduced pressure and the crude product was purified using reverse-phase preparatory HPLC (20-60% acetonitrile + 0.1% TFA in H₂O + 0.1% TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound, 99.8% pure by analytical HPLC (120.0 mg, 30%). ¹H NMR (300 MHz, CD₃OD) δ 8.84 (s, 1H), 8.46 (s, 1H), 8.11 (m, 2H), 7.95 (d, 1H), 7.91 (d, 1H), 7.67 (d, 1H), 7.29 (d, 1H), 7.17 (dd, 1H), 4.11 (t, 2H), 3.17 (sep, 2H), 2.98 (t, 2H), 2.74 (s, 2H), 1.13 (d, 12H), 1.05 (s, 9H); MS (ESI) *m*/*z* 525.8 [M+1]⁺.

### EXAMPLE 104

### SYNTHESIS 3-{6-[2-(2,6-CIS-DEWETHYL-PIPERIDI-1-YL)-ETHOXY]-NAPHTHALEN-2-YL}-5-(5-ISOBUTYL)-2H 1,2,4]TRIAZOL-3-YL]-1-METHYL-1H-INDAZOLE

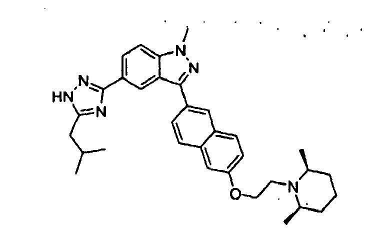

### A. 3-{6-[2-(2,6-cis-Dimethyl-piperidi-1-yl)-ethoxy]-naphthalen-2-yl)-5-(5-isobutyl)-2H-[1,2,4]triazol-3-yl]-1-methyl-1H-indazole

A solution of 3-{6-[2-(2,6-*cis*-Dimethyl-piperidi-1-yl)-ethoxy]-naphthalen-2-yl}-1-methyl-1*H*-indazole-5-carboximidic acid ethyl ester di-hydrochloric acid salt (0.34 g, 0.65 mmol), 3-methyl-butyric acid hydrazide (0.30 g, 2.5 mmol), and triethyl amine (1.8 mL, 12.9 mmol) in methanol (3.5 mL) was heated in a sealed reaction flask on an 100 °C oil bath for three hours. Solvent and excess triethyl amine were then removed under reduced pressure and the crude product was purified using reverse-phase preparatory HPLC (20-80% acetonitrile + 0.1 % TFA in H₂O +0.1% TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound, 99% pure by analytical HPLC (30.0 mg, 8.6%). ¹H NMR (400 MHz, CD₃OD) δ 8.84 (s, 1H), 8.47 (s, 1H), 8.14 (m, 2H), 7.98 (d, 1H), 7.93 (d, 1H), 7.70 (d, 1H), 7.33 (m, 1H), 7.21 (m, 1H), 4.25 (t, 2H), 4.18 (s, 3H), 3.26 (t, 2H), 2.74 (overlapping m, 4H), 2.20 (sep, 1H), 1.68 (overlapping m, 3H), 1.39 (overlapping m, 3H), 1.27 (d, 6H), 1.03 (d, 6H); MS (ESI) *m*/*z* 538.2 [M+1]⁺.

### B. 3-{6-[2-(2,6-cis-Dimethyt-piperidi-1-yl)-ethoxy]-naphthaten-2-yl}-1-methyl-1H-indazole-5-carboximidic acid ethyl ester di-hydrochloric acid salt

A solution of 3-{6-[2-(2,6-*cis*-dimethyl-piperidi-1-yl)-ethoxy]-naphthalen-2-yl}-1-methyl-1*H*-indazole-5-carbonitrile (0.32 g, 0.7 mmol) in ethanol (50 mL) was cooled on a dry ice/isopropanol bath and saturated with HCl (g). The resulting solution was allowed to stir, gradually warming to room temperature, overnight. Excess solvent was removed under reduced pressure and the resulting solid was taken up in Et₂O, sonicated 5 min and solvent removed under reduced pressure to provide the title compound as a yellow residue (0.34 g, 89%). MS (ESI) *m*/*z* 486.0 [free base M+1]⁺.

### C. 3-{6-[2-(2,6-cis-Dimethyl-piperidi-1-yl)-ethoxy]-naphthalen-2-yl}-1-methyl-1H-indazole-5-carbonitrile

A solution of 2-bromo-6-[2-(2,6-*cis*-dimethyl-piperidi-1-yl)-ethoxy]-naphthalene (0.5 g, 1.4 mmol), bis(pinacolato)diboron (0.35 g, 1.4 mmol), [1,1'-bis(diphenylphosphino)-ferrocene] dichloro palladium complex with dichloromethane (1:1) (0.2 g, 0.25 mmol), and potassium acetate (0.11 g, 0.13 mmol) in DMF (75 mL) was heated on an 85°C oil bath 2h. To this reaction mixture was added 3-bromo-1-methyl-1*H*-indazole-5-carbonitrile (0.33 g, 1.4 mmol) and potassium phosphate (0.88 g, 4.2 mmol) and the solution was returned to heat overnight. The cooled solution was filtered through Celite and the filter cake washed with EtOAc. The filtrate was washed with water and brine, dried over magnesium sulfate, filtered and solvent removed under reduced pressure to afford crude product. Purification using silica gel flash column chromatography (1:4 EtOAc:hexanes) and a second column (CH₂Cl₂) provided title compound (0.32 g, 53%). MS (ESI) m/z 439.8[M]⁺

### D. 3-Bromo-1-methyl-1H-indazole-5-carboaitrile

3-Bromo-1H-indazole-5-carbonitrile (5.0 g., 22.62 mmol), potassium carbonate (3.21 g., 45.24 mol) and methyl iodide (3.21 g., 22.62 mmol)were added to dimethylformamide (100 mL). The solution was stirred at 80°C for two hours or until TLC shows consumption of starting materials. The solution was condensed under reduced pressure to give a solid. The solid was partitioned between water and ethyl acetate (3X). The organics were dried over magnesium sulfate, magnesium sulfate was filtered and solvent removed under reduced pressure. The resultant solid was recrystallized in 100% ethyl acetate to afford the title compound (1.25 g., 23% yield). 8.802 (t, 1H), 7.63 (dd, 1H), 7.47 (dd, 1H), 4.10 (s, 3H). ES-MS (m/z) 236[M+1]+, 238 [M+2]+.

### E. 3-Bromo-1H-indazole-5-carbonitrile

To a solution containing 3-Bromo-1-(tetrahydro-pyran-2-yl)-1H-indazole-5-carbonitrile (7.0 g., 22.87 mmol) in ethanol was added hydrogen chloride gas for five minutes. The mixture was monitored by TLC until starting material was fully deprotected. The solution was condensed under reduced pressure and partitioned between water and ethyl acetate (3X). The organics were dried over magnesium sulfate, magnesium sulfate was filtered and solvent removed under reduced pressure to afford the title compound (7.3 g., >100% yield). ES-MS (m/z) 221[M+1]⁺, 222 [M+2]⁺.

### EXAMPLE 105

### SYNTHESIS 5-[5-(2,2-DIN4ETHYL-PROPYL)-2H-[1,2,4]TRIAZOL-3-YL]-3-{6-[2-(2,2.6,6-TETRAMETHYL-PIPERIDI-1-YL)-ETHOXY]-NAPHTHALEN-2-YL} -1H-INDAZOLE

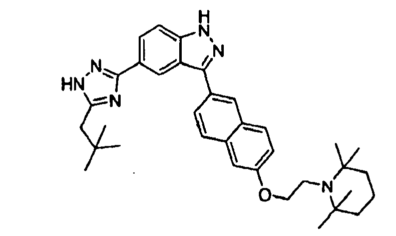

### A. 5-[5-(2,2-Dimethyl-propyl)-2H-[1,2,4]triazol-3-yl]-3-{6-[2-(2,2,6,6-tetramethyl-piperidi-1-yl)-ethoxy]-oaphtbaleo-2-yl}-1 H-indazole

A solution of 3-{6-[2-(2,2,6,6-tetramethyl-piperidi-1-yl)-ethoxy]-naphthalen-2-yl}-1*H*-indazole-5-carboximidic acid ethyl ester di-hydrochloric acid salt (0.5 g, 0.9 mmol), 3,3-dimethyl-butyric acid hydrazide (0.46 g, 3.5 mmol), and triethyl amine (2.4 mL, 17.2 mmol) in methanol (5 mL) was heated in a sealed reaction flask on an 100 °C oil bath for three hours. Solvent and excess triethyl amine were then removed under reduced pressure and the crude product was purified using reverse-phase preparatory HPLC (20-60% acetonitrile +0.1% TFA in H₂O +0.1% TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound, 98.5% pure by analytical HPLC (9.0 mg, 2%). ¹H NMR (400 MHz, CD₃OD) δ 8.85 (s, 1H), 8.47 (s, 1H), 8.12 (m, 2H), 7.97 (d, 1H), 7.93 (d, 1H), 7.68 (m, 1H), 7.29 (m, 1H), 7.21 (dd, 1H), 4.07 (t, 2H), 3.03 (bm, 2H), 2.75 (s, 2H), 1.63 (bm, 2H), 1.51 (bm, 4H), 1.17 (s, 12H), 1.06 (s, 9H); MS (ESI) *m*/*z* 566.3 [M+1]⁺.

### B. 3-{6-[2-(2,2,6,6.tetramethyt-piperidi-l-yl)-ethoxy]-naphthaten-2-yl}-1H-indazole-5-carboximidic acid ethyl ester di-hydrochlotic acid salt

A solution of 1-(tetrahydro-pyran-2-yl)-3-{6-[2-(2,2,6,6-tetramethyl-piperidi-1-yl)-ethoxy]-naphthalen-2-yl)-1*H*-indazole-5-carbonitrile (1.6 g, 3.0 mmol) in ethanol (300 mL) was cooled on a dry ice/isopropanol bath and saturated with HCl (g). The resulting solution was allowed to stir, gradually warming to room temperature, overnight. The reaction mixture was recharged daily with HCl (g) until reaction reached completion, 11 days total. Excess solvent was removed under reduced pressure and the resulting solid was taken up in Et₂O, sonicated 5 min and filtered to provide the title compound as a pale yellow solid (1.36 g, 80%). MS (ESI) *m*/*z* 499.7 [free base M+1]⁺.

### C. 1-(Tetrahydro-pyran-2-yl)-3-{6-[2-(2,2,6,6-tetrametbyl-piperidi-1-yl)-ethoxy]-naphthalen-2-yl}-1H-indazole-5-carbonitrile

A solution of 3-(6-hydroxy-napthalen-2-yl)-1-(tetrahydro-pyran-2-yl)-1*H-*indazole-5-carbonitrile (see Example 93, step D) (1.5 g, 4.1 mmol) and triphenylphosphine (1.6 g, 6.1 mmol) in THF (80 mL) was treated with 2-(2,2,6,6-tetramethylpiperidi-1-yl)-ethanol (1.5 g, 8.1 mmol) and DIAD (1.2 mL, 6.1 mmol). The resulting solution was allowed to stir at room temperature 1 h. Excess THF was removed under reduced pressure and the resulting solid was taken up in EtOAc, washed with water, brine, then dried over magnesium sulfate, filtered and solvent removed under reduced pressure to provide crude material. Purification using silica gel flash column chromatograph (5% methanol in CH₂Cl₂) provided the title compound (1.66 g, 90% yield). MS (ESI) *m*/*z* 537.5[M+1]⁺.

### EXAMPLE 106

### SYNTHESIS 5-(5-ISOBUTYL-2H-[1,2,4]TRIAZOL-3-YL)-3-{6-[2-(2,2,6,6-TETRAMETHYL-PIPERIDI-1- YL)-ETHOXY]-NAPHTHALEN-2-YL} -1H-INDAZOLE

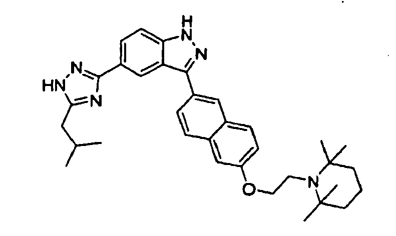

A solution of 3-{6-[2-(2,2,6,6-tetramethyl-piperidi-1-yl)-ethoxy)-naphthalen-2-yl}-1*H*-indazole-5-carboximidic acid ethyl ester di-hydrochloric acid salt (0.5 g, 0.9 mmol), 3-methyl-butyric acid hydrazide (0.41 g, 3.5 mmol), and triethyl amine (2.4 mL, 17.2 mmol) in methanol (5 mL) was heated in a sealed reaction flask on an 100°C oil bath for three hours. Solvent and excess triethyl amine were then removed under reduced pressure and the crude product was purified using reverse-phase preparatory HPLC (20-60% acetonitrile + 0.1 % TFA in H₂O + 0.1 % TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound, 99.3% pure by analytical HPLC (40 mg, 8.3%). ¹H NMR (400 MHz, CD₃OD) δ 8.85 (s, 1H), 8.47 (s, 1H), 8.11 (m, 2H), 7.97 (d, 1H), 7.91 (d, 1H), 7.68 (m, 1H), 7.27 (m, 1H), 7.20 (dd, 1H), 4.06 (t, 2H), 3.02 (t, 2H), 2.73 (d, 2H), 2.19 (sep, 1H), 1.63 (bm, 2H), 1.51 (bm, 4H), 1.15 (s, 12H), 1.02 (d, 6H); MS (ESI) *m*/*z* 551.6 [M+1]⁺.

### EXAMPLE 107

### SYNTHESIS 5-[5-(2,2-DIMETHYL-PROPYL)-2H-[1,2,4]TRIAZOL-3-YL]-3-{6-[2-(2,5-CIS-DIMETHYL-PYRROLIDIN-1-YL)-ETHOXY]-NAPHTHALEN-2-YL)-1H-INDAZOLE

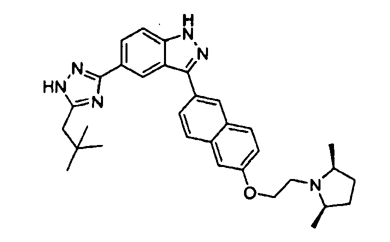

### A. 5-[5-(2,2-Dimetbyl-propyl)-2H-[1,2,4]triazol-3-yl]-3-{6-[2-(2,5-cis-Dimethyl-pyrrolidin-1-yl)-ethoxy]-naphthalen-2-yl}-1H-indazole

A solution of 3-(6-[2-(2,5-dimethyl-pyrrolidin-1-yl)-ethoxy]-naphthalen-2-yl}-1*H*-indazole-5-carboximidic acid ethyl ester di-hydrochloric acid salt (0.1 g, 0.2 mmol), 3,3-dimethyl-butyric acid hydrazide (0.1 g, 0.8 mmol), and triethyl amine (0.5 mL, 3.6 mmol) in methanol (2 mL) was heated in a sealed reaction flask on an 100°C oil bath overnight. Solvent and excess triethyl amine were then removed under reduced pressure and the crude product was purified using reverse-phase preparatory HPLC (20-60% acetonitrile +0.1% TFA in H₂O +0.1% TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound, 99.9% pure by analytical HPLC (15.0 mg, 15%). ¹H NMR (300 MHz, CD₃OD) δ 8.84 (s, 1H), 8.47 (s, 1H), 8.12 (dd, 2H), 7.96 (d, 1H), 7.92 (d, 1H), 7.67 (dd, 1H), 7.31 (d, 1H), 7.20 (dd, 1H), 4.26 (t, 2H), 3.16 (t, 2H), 2.89 (m, 2H), 2.74 (s, 2H), 1.94 (m, 2H), 1.44 (m, 2H), 1.23 (d, 6H), 1.05 (s, 9H); MS (ESI) m/z 524.2 [M+1]⁺.

### B. 3-{6-[2-(2,5-dimethyl-pyrrolidin-1-yl)-ethoxy]-naphthalen-2-yl}-1H-indazole-5-carboximidic acid ethyl ester di-hydrochloric acid salt

A solution of 3-{6-(2-(2,5-dimethyl-pyrrolidin-1-yl)-ethoxy]-naphthalen-2-yl}-1-(tetrahydro-pyran-2-yl)-1H*-*indazole-5-carbonitrile (0.39 g, 0.8 mmol) in ethanol (50 mL) was cooled on a dry ice/isopropanol bath and saturated with HCl (g). The resulting solution was allowed to stir, gradually warming to room temperature, overnight. Excess solvent was removed under reduced pressure to provide the title compound as yellow/brown residue (0.41 g, 99%). MS (ESI) *m*/*z* 457.9 [free base M+1)⁺.

### C. 3-{6-[2-(2,5-dimethyl-pyrrolidin-1-yl)-ethoxy]-naphtbalen-2-yl}-1-(tetrahydro-pyran-2-yl)1H-indazole-5-carbonitrile

A solution of 3-(6-hydroxy-napthalen-2-yl)-1-(tetrahydro-pyran-2-yl)-1*H-*indazole-5-carbonitrile (see Example 93, step D) (1.0 g, 2.7 mmol) and triphenyl-phosphine (2.8 g, 10.7 mmol) in THF (50 mL) was treated with 2-(2,5-cis-dimethyl-pyrrolidin-1-yl)-ethanol (1.0 g, 7.0 mmol) and DIAD (2.1 mL, 10.7 mmol). The resulting solution was allowed to stir at room temperature overnight. Excess THF was removed under reduced pressure and the resulting solid was taken up in EtOAc, washed with water, brine, then dried over magnesium sulfate, filtered and solvent removed under reduced pressure to provide crude material. Purification using silica gel flash column chromatograph (5% methanol in CH₂Cl₂) provided the title compound (0.39 g, 29% yield). MS (ESI) *m*/*z* 495.4 [M+1]⁺.

### EXAMPLE 108

### SYNTHESIS 3-{6-[2-(2,5-DIMETHYL-PYRROLIDlN-1-YL)-ETHOXY]-NAPHTHALEN-2-YL}-5-(5-ISOBUTYL-2H-[1,2,4]TRIAZOL-3-YL)-1H-INDAZOLE

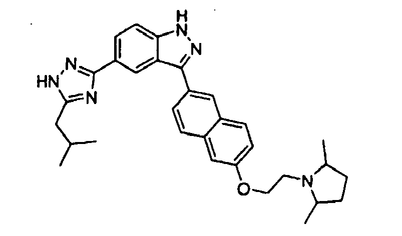

A solution of 3-(6-[2-(2,5-dimethyl-pyrrolidin-1-yl)-ethoxy]-naphthalen-2-yl)-1*H*-indazole-5-carboximidic acid ethyl ester di-hydrochloric acid salt (0.41 g, 0.8 mmol), 3-methyl-butyric acid hydrazide (0.36 g, 3.1 mmol), and triethyl amine (2.2 mL, 15.8 mmol) in methanol (5 mL) was heated in a sealed reaction flask on an 100°C oil bath for three hours. Solvent and excess triethyl amine were then removed under reduced pressure and the crude product was purified using reverse-phase preparatory HPLC (20-60% acetonitrile + 0.1% TFA in H₂O +0.1% TFA, over 30 min). Fractions containing clean product were neutralized with potassium carbonate and product extracted using ethyl acetate. The organic fractions were pooled, dried over magnesium sulfate, filtered, and solvent removed under reduced pressure to afford the title compound. Further purification using silica gel flash column chromatograph (10% methanol in CH₂Cl₂) provided the title compound, 99.5% pure by analytical HPLC (16.8 mg, 4.3%). (∼3:1 mixture of cis:trans diastereomers) ¹H NMR (400 MHz, CD₃OD) (major isomer, cis) δ 8.854 (s, 1H), 8.47 (s, 1H), 8.11 (m, 2H), 7.97 (d, 1H), 7.92 (d, 1H), 7.67 (d, 1H), 7.30 (m, 1H), 7.20 (dd, 1H), 4.25 (t, 2H), 3.14 (t, 2H), 2.88 (m, 2H), 2.72 (d, 2H), 2.18 (sep, 1H), 1.93 (m, 2H), 1.43 (m, 2H), 1.23 (d, 6H), 1.02 (d, 6H); MS (ESI) m/z 510.1 [M+1]⁺.

### EXAMPLE 109

### SYNTHESIS OF 3-(7-METHOXY-NAPHTHALEN-2-YL)-5-(5-PIPERIDIN-1-YLMETHYL-1H-[11,2,4]TRIAZOL-3-YL)-1H-INDAZOLE

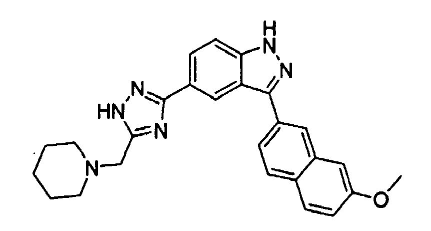

### A. Piperidin-1-yl-acetic acid hydrazide

To a solution of piperidine (9.9 mL, 0.100 mol) in tetrahydrofuran (100 mL) at 0°C was added ethyl bromoacetate (13.3 mL, 0.120 mol) followed by triethylamine (27.8 mL, 0.200 mol). The reaction was allowed to reach room temperature and stirred for 48 h. Tetrahydrofuran was removed *in vacuo* and the aqueous phase was extracted with ethyl acetate. The organic layer was dried over magnesium sulfate, filtered and the solvent was removed *in vacuo.* The crude material (20.4 g) was dissolved in ethanol (60 mL) and anhydrous hydrazine (4 mL, 0.131 mmol) was added and the reaction was refluxed for 48 h. Solvent was removed in vacuo to yield the title compound (16.14 g) in 86% yield. ¹H NMR ](methanol-d₄, 300 MHz) δ 4.98 (s, 2H), 3.09 (s, 2H), 2.55 (m, 4H), 1.71 (m, 4H), 1.57 (m, 2H).

### B. 7-Bromo-naphthalen-2-ol

To a suspension of triphenylphosphine (30 g, 114 mmol) in acetonitrile (120 mL) was added bromine (5.9 mL, 114 mmol) dropwise, followed by a solution of 2,7-dihydroxynaphthalene (16.7 g, 104 mmol) in 20 mL of acetonitrile. The reaction mixture was refluxed for 3 hrs, cooled down to room temperature and acetonitrile was removed in vacuo. The residue was then heated at 180°C in an oil bath until it became a grey solid that was then heated in a sand bath at 350°C for 1h. After cooling down the reaction, the black thick oil formed was taken up in methylene chloride and loaded onto a chromatography column (SiO2, 15% ethyl acetate/hexanes) to yield 16.7 g (64% yield). ES-MS (m/z) 223 [M+1]⁺.

### C. 2-bromo-7-methoacy -naphthalene

In a round bottom flask, 7-bromo-naphthalen-2-ol (9.4 g, 42.15 mmol) was dissolved in 80 mL ofDMF. Potassium carbonate (8.7 g, 63.2 mmol) and iodomethane (3.94 mL, 63.2 mmol) were added and the reaction was stirred at room temperature for 2 h. The reaction mixture was then filtered and solvent was removed *in vacuo.* Residue was dissolved in ethyl acetate (150 mL) and washed with water (3 x 50 mL). The organic layer was then dried over magnesium sulfate, filtered and the solvent was removed in *vacuo* to give the title compound (10 g, 100% yield). ES-MS (m/z) 238 [M+1]⁺.

### D. 7-metboxy-napbthalene-2-boronic acid

A solution of 2-bromo-7-methoxy-naphthalene (10 g, 42.2 mmol) in tetrahydrofuran (200 mL) was purged with nitrogen and cooled to -78°C. N-Butyllithium (31.7 mL, 50.6 mmol) (1.6 M solution in hexanes) was added over a 10-minute period and the reaction mixture was stirred at -78°C for 1.5 hr. Trimethyl borate (14.2 mL, 126.6 mL) was added, the reaction was further stirred at -78°C for 45 minutes and then quenched by the addition of saturated solution of ammonium chloride. The reaction mixture was allowed to warm to room temperature and tetrahydrofuran was removed in *vacuo.* The aqueous phase was extracted with ethyl acetate. The organic layer was dried over magnesium sulfate, filtered and the solvent was removed *in vacuo.* The crude material was carried over to the next step without further purification (6.5 g, 74% yield). ES-MS (m/z) 203 [M+1]⁺.

### E. 3-(7-methoxy-naphthalen-2-yl)-1-(tetrahydro-pyran-2-yl)-1H-indazote-5-carbonitrile

To a stirred solution of 3-bromo-1-(tetrahydro-pyran-2-yl)-1H-indazole-5-carbonitrile (see WO 02/10137, example 149C) (5 g, 16.33 mmol) in dimethoxyethane (400 mL) was added 7-methoxy-naphthalene-2-boronic acid (6.5 g, 32.17 mmol), dichloro[1,1'-bis(diphenylphosphino) ferrocene] palladium (1.74 g, 2.1 mmol) and potassium phosphate (44.5 g, 211.9 mmol) and the mixture was heated at reflux for 2 h. Reaction mixture was filtered through Celite and the precipitate was washed with ethyl acetate. The solvent was removed *in vacuo,* the residue was purified by column chromatography (SiO₂, 4:1 n-hexanes/ ethyl acetate) to give the title compound (4.37 g, 70% yield). ES-MS (m/z) 384 [M+1]⁺.

### F. 3-(7-metboxy-naphthalen-2-yl]-1H-indazole-5-carboximidic acid ethyl ester, hydrochloride

The title compound was prepared according to the procedure described in Example 12, step D using 3-(7-methoxy-naphthalen-2-yl)-1-(tetrahydro-pyran-2-yl)-1H-indazole-5-carbonitrile (3 g, 7.83 mmol). 3 g of a yellow solid were obtained (100% yield). ES-MS (m/z) 346 [M+1]⁺.

### G. 3-(7-metboxy-naphthalen-2-yl)-5-(5-piperidin-1-ylmethyl-1H-[1,2,4]triazol-3-yl]-1H-indazole

The title compound was prepared according to the procedure described in Example 12, step E using 3-(7-methoxy-naphthalen-2-yl]-1H-indazole-5-carboximidic acid ethyl ester, hydrochloride (568 mg, 1.49 mmol), piperidin-1-yl-acetic acid hydrazide (702 mg, 4.47 mmol), triethylamine (4.2 mL, 29.8 mmol) in ethanol (10 mL) to yield 28.5 mg (4% yield, 98.1% purity) of the title compound after purification by column chromatography (SiO₂, 5% MeOH/EtOAc + 1% triethylamine) and precipitation from ethyl acetate. ¹H NMR (methanol-d₄, 300 MHz) δ 8.82 (s, 1H), 8.40 (s, 1H), 8.07 (dd, 1 H), 7.95 (dd, 1H), 7.88 (d, 1H), 7.77 (d, 1H), 7.66 (d, 1H), 7.39 (d, 1H), 7.13 (dd, 1H), 3.93 (s, 3H), 3.81 (s, 2H), 2.66 (br s, 4H), 1.65 (m, 4H), 1.47 (m, 2H). ES-MS (m/z) 439 [M+1]⁺.

### EXAMPLE 110

### SYNTHESIS OF 3-(7-METHOXY-NAPHTHALEN-2-YL)-5-(5-MORPHOLIN-4-YLMETHYL-1H-[1,2,4]TRIAZOL-3-YL)-1H-INDAZOLE

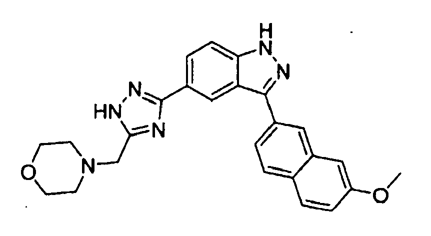

### A. 3-(7-methoxy-naphthalen-2-yl)-5-(5-morpholin-4-ylmethyl-1H-[1,2,4]triazol-3-yl]-1H-indazole

The title compound was prepared according to the procedure described in example Example 12, step D using 3-(7-methoxy-naphthalen-2-yl]-1H-indazole-5-carboximidic acid ethyl ester, hydrochloride (368 mg, 0.96 mmol), morpholin-4-yl-acetic acid hydrazide (460 mg, 2.90 mmol), triethylamine (2.7 mL, 19.29 mmol) in ethanol (7 mL) to yield 67.6 mg (16% yield, 99.5% purity) of the title compound after purification by column chromatography (SiO₂, 5% MeOH/EtOAc + 1% triethylamine) and precipitation from ethyl acetate. ¹H NMR (methanol-d₄, 400 MHz) δ 8.84 (s, 1H), 8.43 (s, 1H), 8.08 (br s, 1H), 7.97 (dd, 1H), 7.90 (d, 1H), 7.78 (d, 1H), 7.67 (br s, 1H), 7.42 (d, 1H), 7.14 (dd, 1H), 3.94 (s, 3H), 3.71 (m, 6H), 2.56 (m, 4H). ES-MS (m/z) 441 [M+1]⁺.

### EXAMPLE 111

### SYNTHESIS OF 3-(7-METHOXY-NAPHTHALEN-2-YL)-5-(5-PYRROLIDIN-1-YLMETHYL-1H-[1,2,4]TRIAZOL-3-YL)-1H-INDAZOLE

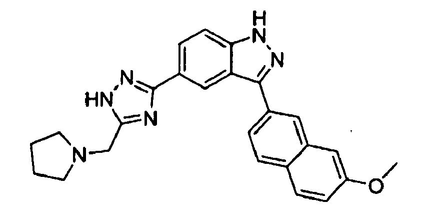

The title compound was prepared according to the procedure described in example Example 12 using 3-(7-methoxy-naphthalen-2-yl]-1H-indazole-5-carboximidic acid ethyl ester, hydrochloride (685 mg, 1.79 mmol), pyrrolidin-1-yl-acetic acid hydrazide (770 mg, 5.38 mmol), triethylamine (5.0 mL, 35.9 mmol) in ethanol (20 mL) to yield 334.9 mg (44% yield, 99.0% purity) of the title compound after purification by column chromatography (SiO₂ 10% MeOH/EtOAc + 1% triethylamine) and precipitation from ethyl acetate. ¹H NMR (methanol-d₄, 400 MHz) δ 8.80 (s, 1H), 8.38 (s, 1H), 8.05 (dd, 1H), 7.93 (dd, 1H), 7.86 (d, 1H), 7.75 (d, 1H), 7.64 (d, 1H), 7.37 (d, 1H), 7.11 (dd, 1H), 3.91 (s, 5H), 2.75 (m, 4H), 1.82 (m, 4H). ES-MS (m/z) 425 [M+1]⁺.

### EXAMPLE 112

### SYNTHESIS OF (S) 5-[5-(2,2-DIMETHYL-PROPYL)-1H-[1,2,4]TRIAZOL-3-YL)-3-[6-(3-METHYL-2-PYRROLIDIN-1-YL-BUTOXY)-NAPHTHALEN-2-YL]-1H-INDAZOLE

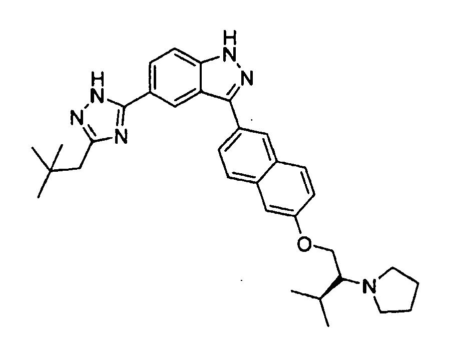

### A. (S) [1-(6-Bromo-naphthalen-2-yloxymethyl)-2-methyl-propyll-carbamic acid tert-butyl ester

To a solution of 6-bromo-2-naphthol (4.57 g, 20.5 mmol) in THF (90 mL) were added sequentially N-(tert-butoxycarbonyl)-L-valinol (5 g, 24.6 mmol), triphenylphosphine (5.37 g, 20.5 mmol) and diisopropyl azodicarboxylate (4.04 mL, 20.5 mmol). The reaction mixture was stirred at room temperature for 18 h, after which the solvent was removed *in vacuo* and the crude material was purified by column chromatography (SiO₂, 5% ethyl acetate/ n-hexanes → 10% ethyl acetate/ n-hexanes) to yield 1.97 g of the title compound (24% yield). ES-MS (m/z) 408 [M+1]⁺.

### B. (S) 1-[1-(6-Bromo-naphthalen-2-yloxymethyl)-2-methyl-propyl]-pyrrolidine

To a stirred solution of (2S) [1-(6-bromo-naphthalen-2-yloxymethyl)-2-methylpropyl]-carbamic acid tert-butyl ester (1.5 g, 3.7 mmol) in methylene chloride (50 mL), trifluoroacetic acid was added (5 mL). After stirring at room temperature for 30 minutes, solvent was removed *in vacuo* and the compound dried under high vacuum. This crude material was taken up in toluene and molecular sieves were added (0.5 g), followed by 1,4-dibromobutane (0.49 mL, 4.07 mmol) and sodium bicarbonate (0.93 g, 11.1 mmol). The reaction mixture was heated in a sealed tube at 120°C for 72 h, cooled down and di-tert-butyl dicarbonate (403.8 mg, 1.85 mmol) were added. The reaction was stirred at room temperature until LCMS analysis showed that all remaining starting material had been converted to N-boc protected amine. Solvent was removed in vacuo and crude was purified by HPLC (20 → 80% acetonitrile/ water) to yield 545 mg of the title compound (41% yield). ES-MS (m/z) 363 [M+1]⁺.

### C. (S) 3-[6-(3-Methyl-2-pyrrolidin-1-yl-butoxy)-naphthalen-2-yl)-1-(tetrahydro-pyran-2-yl)-1H-indazole-5-carbonitrile

To a stirred solution of (2S) 1-[1-(6-bromo-naphthalen-2-yloxymethyl)-2-methylpropyl]-pyrrolidine (345 mg, 0.95 mmol) in DMF (6 mL) were added sequentially bis(pinacolato) diboron (242 mg, 0.95 mmol), potassium acetate (280.6 mg, 2.86 mmol) and [1,1'-bis(diphenylphosphino)ferrocene]dichloropalladium (II), complex with dichloromethane (1:1) catalyst (77.8 mg, 0.095 mmol). Reaction mixture was heated using microwave irradiation at 100°C for 20 minutes. LCMS showed formation of the corresponding boronate ester. 3-Bromo-1-(tetrahydro-pyran-2-yl)-1H-indazole-5-carbonitrile (see WO 02/10137, example 149C) (262.4 mg, 0.858 mmol) and potassium phosphate (600 mg, 2.859 mmol) were added and the reaction mixture was heated again at 100°C using microwave irradiation for 20 minutes. Reaction mixture was filtered through Celite and solvent was removed *in vacuo.* Crude was purified by semiprep HPLC (20 → 80% acetonitrile/ water) to yield 49 mgs of the title compound (11% yield). ES-MS (m/z) 509 [M+1]⁺.

### D. (S) 3-(6-(3-Methyl-2-pyrrolidin-1-butoxy)-naphthalen-2-yl]-1H-indazole-5-carboximidic acid ethyl ester, hydrochloride

The title compound was prepared according to the procedure described in Example 12, step D using (S) 3-[6-(3-methyl-2-pyrrolidin-1-yl-butoxy)-naphthalen-2-yl]-1-(tetrahydro-pyran-2-yl)-1H-indazole-5-carbonitrile (42 mg, 0.083 mmol). 42 mg of a yellow solid were obtained (100% yield). ES-MS (m/z) 471 [M+1]⁺.

### E. (S)-5-[5-(2,2-Dimethyl-propyl)-1H-[1,2,4)triazol-3-yl]-3-[6-(3-methyl-2-pyrrolidin-1-yl-butoxy)-naphthalen-2-yl]-1H-indazole

The title compound was prepared according to the procedure described in example Example 12, step E using (S) 3-[6-(3-methyl-2-pyrrolidin-1-butoxy)-naphthalen-2-yl]-1H-indazole-5-carboximidic acid ethyl ester, hydrochloride (42 mg, 0.083 mmol), 3,3-dimethyl-butyric acid hydrazide (32 mg, 0.247 mmol), triethylamine (0.228 mL, 1.64 mmol) in ethanol (3 mL) and heating for 48 h, to yield 12.9 mg (29% yield, 96.8% purity) of the title compound after purification by HPLC (20 → 80% acetonitrile/ water). ¹H NMR (CDCl₃, 400 MHz) δ 8.71 (s, 1H), 8.26 (s, 1H), 8.02 (d, 1H), 7.97 (d, 1H), 7.67 (t; 2H), 7.36 (d, 1H), 7.08 (d, 2H), 4.20 (d, 2H), 2.79 (br s, 4H), 2.72 (s, 2H), 2.59 (m, 1H), 2.17 (m, 1H), 1.79 (br s, 4H), 1.05 (m, 15 H). ES-MS (m/z) 537 [M+1]⁺.

### EXAMPLE 113

### SYNTHESIS OF (S) 3-[6-(1-METHYL-PYRROLIDIN-2-YLMETHOXY)-NAPHTHALEN-2-YL]-IH-INDAZOLE-5-CARBOXYLIC ACID AMIDE

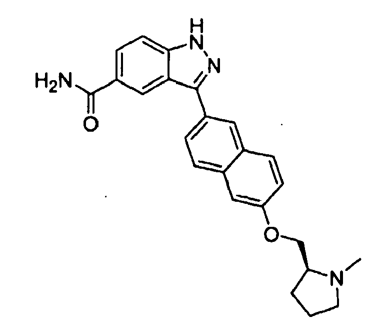

### A. (S) 3-[6-(1-Methyl-pyrrolidin-2-ylmethoxy)-naphtbalen-2-yl]-1-(tetrahydro-pyran-2-yl)-1H-indazole-5-carboxylic acid amide.

(S) 3-[6-(1-Methyl-pyrrolidin-2-ylmethoxy)-naphthalen-2-yl)-1-(tetrahydro-pyran-2-yl)-1H-indazole-5-carbonitrile (80 mg, 0.172 mmol) was dissolved in tert-butyl alcohol (3 mL) and potassium hydroxide (96 mg, 1.72 mmol) was added. The reaction mixture was heated at 75°C for 3 h. Solvent was removed in vacuo and crude was purified by column chromatography (SiO₂, 20% methanol/methylene chloride → 50% methanol/methylene chloride) to yield 62 mg of the title compound (75% yield). ES-MS (m/z) 485 [M+1]⁺.

### B. (S) 3-[6-(1-Methyl-pyrrolidin-2-ylmethoxypnaphthalen-2-yl]-1H-indazole-5-carboxylic acid amide, hydrochloride

(S) 3-[6-(1-Methyl-pyrrolidin-2-ylmethoxy)-naphthalen-2-yl]-1-(tetrahydro-pyran-2-yl)-1H-indazole-5-carboxylic acid amide (62 mg, 0.103 mmol) was dissolved in methanol (3 mL) and cooled to 0°C. Hydrogen chloride gas was bubbled through the solution for five minutes. The reaction was allowed to reach room temperature. The yellow precipitate formed was filtered off, washed with ethyl ether (3 x 2 mL) and dried under high vacuum. 56.2 mg (99.7% purity) of the title compound were obtained (100% yield). ¹H NMR (methanol-d₄, 400 MHz) δ 8.73 (s, 1H), 8.43 (s, 1H), 8.09 (d, 1H), 7.95 (m, 3H), 7.61 (d, 1H), 7.40 (s, 1H), 7.29 (dd, 1H), 4.55 (dd, 2H), 4.38 (m, 1H), 3.94 (m, 1H), 3.73 (m, 1H), 3.09 (s, 3H), 2.43 (m, 1H), 2.21 (m, 1H), 2.11 (m, 2H). ES-MS (m/z) 401 [M+1]⁺.

### EXAMPLE 114

### SYNTHESIS OF (S) 3-[6-(1-METHYL-PYRROLIDIN-2- YLMETHOXY)-NAPHTHALEN-2-YL]-1H-INDAZOLE-5-CARBOXYLIC ACID (3,3-DIMETHYLBUTYL)-AMIDE

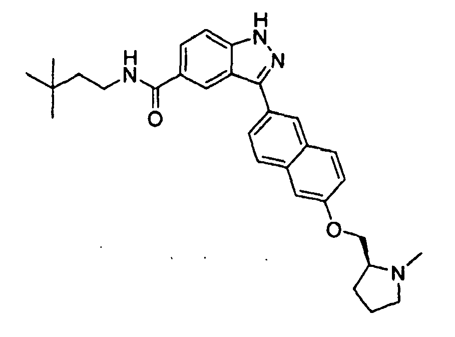

### A. (S) 3-[6-(1-Methyl-pyrrolidin-2-ylmethoxy)-naphthalen-2-yl]-1-(tetrahydro-pyran-2-yl)-1H-indazole-5-carboxylic acid (3,3-dimethyl-butyl)-amide.

(S) 3-[6-(1-Methyl-pyrrolidin-2-ylmethoxy)-naphthaleri-2-yl]-1-(tetrahydro-pyran-2-yl)-1H-indazole-5-carbonitrile (100 mg, 0.215 mmol) was dissolved in a 4:1 mixture of ethanol and water (4 mL) and potassium hydroxide (120.4 mg, 2.15 mmol) was added. The reaction was heated at 130°C for 48 h, after which hydrolysis to the acid was complete. Solvent was removed *in vacuo* and the residue was dried under high vacuum overnight and used without further purification. The residue was dissolved in methylene chloride (5 mL) and 1-(3-dimethylaminopropyl)-3-ethylcarbodiimide hydrochloride (100 mg, 0.523 mmol), 1-hydroxybenzotriazole hydrate (59 mg, 0.436 mmol), triethylamine (0.12 mL, 0.872 mmol) and 3,3-dimethylbutylamine (0.07 mL, 0.523 mmol) were added. The reaction mixture was stirred at room temperature for 16 h after which the solvent was removed *in vacuo* and the crude purified by HPLC (20 → 80% acetonitrile/ water) to yield the title compound (40 mg, 33% yield). ES-MS (m/z) 569 [M+1]⁺.

### B. (S) 3-[6-(1-Methyl-pyrrolidin-2-ylmethoxy)-naphthalen-2-yl]-1H-indazole-5-carboxylic acid (3,3-dimethyl-butyl)-amide, hydrochloride

The title compound was prepared according to the procedure described in Example 113, step B using (S) 3-[6-(1-methyl-pyrrolidin-2-ylmethoxy)-naphthalen-2-yl]-1-(tetrahydro-pyran-2-yl)-1H-indazole-5-carboxylic acid (3,3-dimethyl-butyl)-amide (40 mg, 0.07 mmol). 30.9 mg of the title compound were obtained (85% yield, 100% pure). ¹H NMR (methanol-d₄, 400 MHz) δ 8.65 (s, 1H), 8.43 (s, 1H), 8.10 (dd, 1H), 7.93 (m, 3H), 7.61 (d, 1H), 7.41 (d, 1H), 7.30 (dd, 1H), 4.57 (dd, 2H), 4.36 (m, 1H), 3.95 (m, 1H), 3.74 (m, 1H), 3.44 (m, 2H), 3.10 (s, 3H), 2.43 (m, 1H), 2.24 (m, 1H), 2.11 (m, 2H), 1.56 (m, 2H), 0.98 (s, 9H). ES-MS (m/z) 485 [M+1]⁺.

### EXAMPLE 115

### SYNTHESIS OF 1-[2-(6-{5-[5-(2,2-DIMETHYL-PROPYL)-1H-[1,2,4]TRLAZOL-3-YL]-1H-INDAZOL-3-YL}-NAPHTHALEN-2-YLOXY)-ETHYL]-3-METHYL-IMIDAZOLIDIN-2-ONE

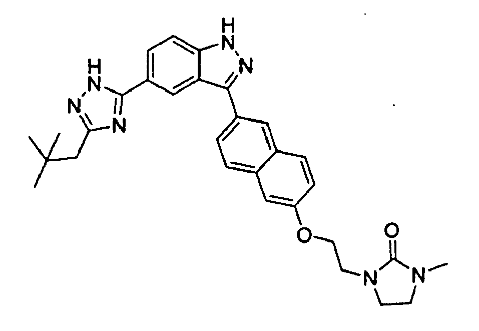

### A. 1-[2-(6-Bromo-naphthalen-2-yloxy)-ethyl]-imidazolidin-2-one

The title compound was prepared following the procedure described in Example 112, step A using 6-bromo-2-naphthol (2 g, 8.97 mmol), 1-(2-hydroxyethyl)-2-imidazolidinone (1.52 g, 11.66 mmol), triphenylphosphine (3.52 g, 13.45 mmol) and diisopropyl azodicarboxylate (2.65 mL, 13.45 mmol). Crude was purified by column chromatography (SiO₂, 1:1 n Hexanes: ethyl acetate → ethyl acetate → 20% methanol/ethyl acetate) to give 1.82 g of a white powder (61% yield). ES-MS (m/z) 335 [M+1]⁺.

### B. 1-[2-(6-Bromo-naphthalen-2-yloxy)-ethyl]-3-methyl-imidazolidin-2-one

A solution of 1-[2-(6-bromo-naphthalen-2-yloxy)-ethyl]-imidazolidin-2-one (0.5 g, 1.49 mmol) in THF: DMF (5:1, 6 mL) was cooled to 0°C. Sodium hydride (60% in mineral oil, 90 mg, 2.24 mmol) was added, followed by iodomethane (0.139 mL, 2.24 mmol). The reaction mixture was allowed to reach room temperature and was stirred for an hour. The reaction was quenched by the addition of water and extracted with ethyl acetate (3 x 7 mL). The combined organic layers were dried over magnesium sulfate and filtered. Solvent was removed *in vacuo* and residue was purified by column chromatography (SiO₂, ethyl acetate) to yield the title compound (488.5 mg, 94% yield). ES-MS (m/z) 349 [M+1]⁺.

### C. 3-{6-[2-(3-Methyl-2-oxo-imidazolidin-1-yl)-ethoxy]-naphthalen-2-yl}-1-(tetrabydro-pyran-2-yl)-1H-indazole-5-carbonitrile

To a stirred solution of 1-[2-(6-bromo-naphthalen-2-yloxy)-ethyl]-3-methyl-imidazolidin-2-one (488.5 mg, 1.40 mmol) in DMF (8 mL) were added sequentially bis(pinacolato) diboron (355 mg, 1.40 mmol), potassium acetate (412.2 mg, 4.2 mmol) and [1,1'-bis(diphenylphosphino)ferrocene]dichloropalladium (II), complex with dichloromethane (1:1) catalyst (114 mg, 0.14 mmol). Reaction mixture was heated in an oil bath at 95°C for 18 h. LCMS showed formation of the corresponding boronate ester. 3-Bromo-1-(tetrahydro-pyran-2-yl)-1H-indazole-5-carbonitrile (see WO 02/10137, example 149C) (471.0 mg, 1.54 mmol) and potassium phosphate (882 mg, 4.2 mmol) were added and the reaction mixture was heated again at 95°C for 18 h. Reaction mixture was filtered through Celite and solvent was removed *in vacuo.* Crude was purified by column chromatography (1:1 n-hexanes/ ethyl acetate→ 2:3 n-hexanes/ ethyl acetate → ethyl acetate) to yield 115 mg of the title compound (16% yield). ES-MS (m/z) 496 [M+1]⁺.

### D. 3-{6-[2-(3-Methyl-2-oxo-imidazolidin-1-yl)-ethoxy]-naphthalen-2-yl}-1H-indazole-5-carboximidic acid ethyl ester, hydrochloride

The title compound was prepared according to the procedure described in example [KIRAN'S example 11B] using 3-{6-[2-(3-methyl-2-oxo-imidazolidin-1-yl)-ethoxy]-naphthalen-2-yl}-1-(tetrahydro-pyran-2-yl)-1H-indazole-5-carbonitrile (115 mg, 0.23 mmol). 113 mg of a yellow solid were obtained (100% yield). ES-MS (m/z) 458 [M+1]⁺.

### E. 1-[2-(6-{5-[5-(2,2-Dimethyl-propyl)-1H-[1,2,4]triazol-3-yl]-1H-indazol-3-yl}-naphthalen-2-yloxy)-ethyl]-3-methyl-imidazolidin-2-one.

The title compound was prepared according to the procedure described in example Example 12, step E using 3-{6-[2-(3-Methyl-2-oxo-imidazolidin-1-yl)-ethoxy]-naphthalen-2-yl}-1H-indazole-5-carboximidic acid ethyl ester, hydrochloride (113 mg, 0.23 mmol), 3,3-dimethyl-butyric acid hydrazide (119.3 mg, 0.92 mmol), triethylamine (0.96 mL, 6.9 mmol) in ethanol (9 mL) to yield 18.8 mg (15% yield) of pure material (98.1 %) after purification by HPLC (20 → 80% acetonitrile/ water) and column chromatography (SiO₂ 5% methanol/ ethyl acetate). ¹H NMR (methanol-d₄, 400 MHz) δ 8.81 (s, 1H), 8.44 (s, 1H), 8.09 (dd, 2H), 7.92 (m, 2H), 7.64 (d, 1H), 7.30 (d, 1H), 7.19 (dd, 1H), 4.24 (t, 2H), 3.62 (t, 2H), 3.54 (dd, 2H), 3.33 (dd, 2H), 2.75 (s, 3H), 2.71 (m, 2H), 1.03 (s, 9H). ES-MS (m/z) 524 [M+1]⁺.

### EXAMPLE 116

### SYNTHESIS OF 5-(5-MORPHOLIN-4-YLMETHYL-1H-[1,2,4]TRIAZOL-3-YL)-3-[6-(PYRIDIN-2-YLMETHOXY)-NAPHTHALEN-2-YL]-1H-INDAZOLE

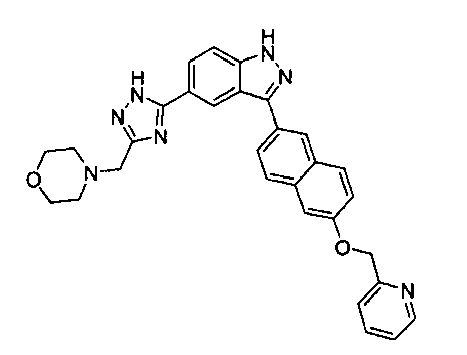

The title compound was prepared according to the procedure described in example Example 12, step E using 3-[6-(pyridine-2-ylmethoxy)-naphthalen-2-yl]-1H-indazole-5-carboximidic acid ethyl ester, hydrochloride (150 mg, 0.30 mmol), morpholin-4-yl-acetic acid hydrazide (144.5 mg, 0.91 mmol), triethylamine (1.26 mL, 9.09 mmol) in ethanol (5 mL) to yield 25.4 mg (16% yield) of pure material (99.2%) after purification by HPLC (10 → 40% acetonitrile/ water) and column chromatography (SiO₂, 20% methanol/ methylene chloride + 1% triethylamine). ¹H NMR (methanol-d₄, 400 MHz) δ 8.85 (s, 1H), 8.58 (m, 1H), 8.48 (s, 1H), 8.11 (m, 2H), 8.00 (d, 1H), 7.90 (m, 2H), 7.68 (m, 2H), 7.40 (m, 2H), 7.33 (dd, 1H), 5.33 (s, 2H), 3.72 (m, 6H), 2.58 (m, 4H). ES-MS (m/z) 518 [M+1]⁺.

### EXAMPLE 117

### SYNTHESIS OF 3-[6-(PYRIDIN-2-YLMETHOXY)-NAPHTHALEN-2-YL)-5-(5-PYRROLIDIN-1-YLMETHYL-1H-[1,2,4]TRIAZOL-3-YL)-1H-INDAZOLE

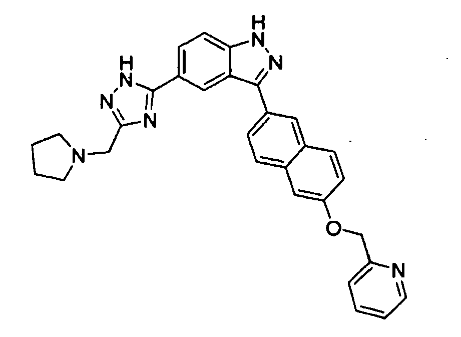

The title compound was prepared according to the procedure described in example Example 12, step E using 3-[6-(pyridine-2-ylmethoxy)-naphthalen-2-yl]-1H-indazole-5-carboximidic acid ethyl ester, hydrochloride (150 mg, 0.30 mmol), pyrrolidin-1-yl-acetic acid hydrazide (130.05 mg, 0.91 mmol), triethylamine (1.26 mL, 9.09 mmol)in ethanol (5 mL) to yield 30.9 mg (20% yield) of pure material (99.3%) after purification by HPLC (10 → 40% acetonitrile/ water) and column chromatography (SiO₂ 20% methanol/ methylene chloride + 1% triethylamine). ¹H NMR (methanol-d₄, 400 MHz) δ 8.82 (s, 1H), 8.55 (d, 1H), 8.45 (s, 1H), 8.08 (m, 2H), 7.98 (d, 1H), 7.87 (m, 2H), 7.66 (m, 2H), 7.38 (m, 2H), 7.31 (dd, 1H), 5.36 (s, 2H), 3.93 (s, 2H), 2.77 (m, 4H), 1.87 (m, 4H). ES-MS (m/z) 502 [M+1]⁺.

### EXAMPLE 118

### SYNTHESIS OF (S) 3-[6-(1-ETHYL-PYRROLIDIN-2-YLMETHOXY)-NAPHTHALEN-2-YL]-1H-INDAZOLE-5-CARBOXYLIC ACID ISOBUTYL-AMIDE

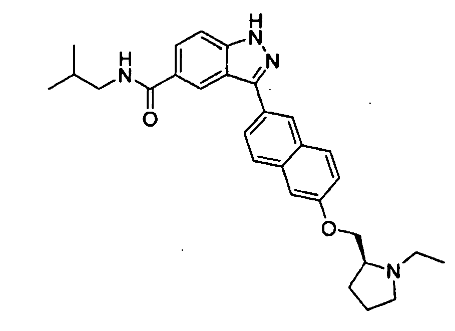

### A. (S) 3-[6-(1-Ethyl-pyrrolidin-2-ylmethoxy)-naphthalen-2-yl]-1-(tetrahydro-pyran-2-yl)-1H-indazole-5-carboxylic acid isobutyl-amide.

(S) 3-[6-(1-Ethyl-pyrrolidin-2-ylmethoxy)-naphthalen-2-yl]-1-(tetrahydro-pyran-2-yl)-1H-indazole-5-carbonitrile (96 mg, 0.21 mmol) was dissolved in a 4:1 mixture of ethanol and water (3 mL) and potassium hydroxide (116.9 mg, 2.1 mmol) was added. The reaction was heated at 130°C for18 h, after which hydrolysis to the acid was complete. Solvent was removed *in vacuo* and the residue was dried under high vacuum overnight and used without further purification. The residue was dissolved in methylene chloride (2 mL) and O-benzotriazol-1yl-N,N,N',N'-tetramethyluronium hexafluorophosphate (HBTU) (95.6 mg, 0.25 mmol), 1-hydroxybenzotriazole hydrate (HOBt) (28.4 mg, 0.21 mmol), triethylamine (0.09 mL, 0.63 mmol) and isobutylamine (41 µL, 0.42 mmol) were added. The reaction mixture was stirred at room temperature for 10 min after which the solvent was removed *in vacuo* and the crude purified by HPLC (20 → 80% acetonitrile/ water) to yield the title compound (80 mg, 69% yield). ES-MS (m/z) 555 [M+1]⁺.

### B. (S) 3-[6-(1-Ethyl-pyrrolidin-2-ylmethoxy)-naphthalen-2-yl)-1H-indazole-5-carboxylic acid isobutyl-amide, hydrochloride

The title compound was prepared according to the procedure described in Example 113, step B using (S) 3-[6-(1-ethyl-pyrrolidin-2-ylmethoxy)-naphthalen-2-yl]-1-(tetrahydro-pyran-2-yl)-1H-indazole-5-carboxylic acid isobutyl-amide (80 mg, 0.14 mmol). 50.2 mg of the title compound were obtained (69% yield, 98.3% purity). 1H NMR (methanol-d4, 400 MHz) δ 8.66 (s, 1H), 8.44 (s, 1H), 8.10 (dd, 1H), 7.94 (m, 3H), 7.61 (dd, 1H), 7.40 (d, 1H), 7.29 (dd, 1H), 4.52 (dd, 2H), 4.34 (m, 1H), 4.04 (m, 1H), 3.71 (m, 1H), 3.64 (m, 1H), 3.44 (m, 2H), 3.22 (m, 2H), 2.39 (m, 1H), 2.21 (m, 1H), 2.11 (m, 2H), 1.95 (m, 1H), 1.41 (t, 3H), 0.96 (s, 6H). ES-MS (m/z) 471 [M+1]⁺.

### EXAMPLE 119

### SYNTHESIS OF (S) 3-[6-(1-ETHYL-PYRROLIDIN-2-YLMETHOXY)-NAPHTHALEN-2-YL]-1H-INDAZOLE-5-CARBOXYLIC ACID (2-PYRROLIDINE-1-ETHYL)-AMIDE

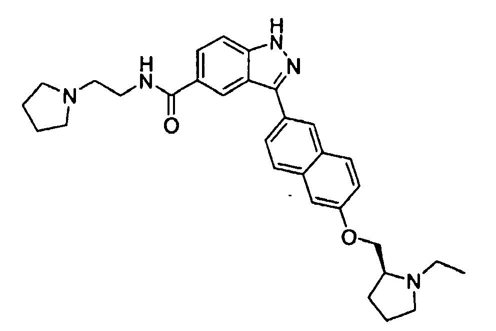

### A. (S) 3-[6-(1-Ethyl-pyrrolidin-2-ylmethoxy)-naphthalen-2-yl]-1-(tetrahydro-pyran-2-yl)-1H-indazole-5-carboxylic acid (2-pyrrolidine-1-yl-ethyl)-amide.

(S) 3-[6-(1-Ethyl-pyrrolidin-2-ylmethoxy)-naphthalen-2-yl]-1-(tetrahydro-pyran-2-yl)-1H-indazole-5-carbonitrile (96 mg, 0.21 mmol) was dissolved in a 4:1 mixture of ethanol and water (3 mL) and potassium hydroxide (116.9 mg, 2.1 mmol) was added. The reaction was heated at 130°C for18 h, after which hydrolysis to the acid was complete. Solvent was removed *in vacuo* and the residue was dried under high vacuum overnight and used without further purification. The residue was dissolved in methylene chloride (2 mL) and O-benzotriazol-1yl-N,N,N',N'-tetramethyluronium hexafluorophosphate (HBTU) (95.6 mg, 0.25 mmol), 1-hydroxybenzotriazole hydrate (HOBt) (28.4 mg, 0.21 mmol), triethylamine (0.09 mL, 0.63 mmol) and 1-(2-aminoethyl)pyrrolidine (53 µL, 0.42 mmol) were added. The reaction mixture was stirred at room temperature for 10 min after which the solvent was removed *in vacuo* and the crude purified by HPLC (20 → 80% acetonitrile/ water) to yield the title compound (57 mg, 46% yield). ES-MS (m/z) 596 [M+1]⁺.

### B. (S) 3-[6-(1-Ethyl-pyrrolidin-2-ylmethoxy)-naphthalen-2-yl]-1H-indazole-5-carboxylic acid (2-pyrrolidine-1-yl-ethyl)-amide, hydrochloride

The title compound was prepared according to the procedure described in Example 113, step B using (S) 3-[6-(1-ethyl-pyrrolidin-2-ylmethoxy)-naphthalen-2-yl]-1-(tetrahydro-pyran-2-yl)-1H-indazole-5-carboxylic acid (2-pyrrolidine-1-yl-ethyl)-amide (57 mg, 0.096 mmol). 22.8 mg of the title compound were obtained (46% yield, 98.1% purity). ¹H NMR (methanol-d₄, 400 MHz) δ 8.76 (s, 1H), 8.48 (s, I H), 8.11 (dd, 1H), 8.02 (d, 1H), 7.95 (dd, 2H), 7.64 (d, 1H), 7.40 (d, 1H), 7.29 (dd, 1H), 4.50 (dd, 1H), 4.37 (m, 1H), 4.04 (m, 1H), 3.79 (m, 4H), 3.73 (m, 1H), 3.64 (m, 1H), 3.46 (t, 2H), 3.18 (m, 2H), 2.40 (m, 1H), 2.10 (m, 7H), 1.41 (t, 3H). ES-MS (m/z) 512 [M+1]⁺.

### EXAMPLE 120

### SYNTHESIS OF (S) 3-[6-(1-ETHYL-PYRROLIDIN-2-YLMETHOXY)-NAPHTHALEN-2-YL]-1H-INDAZOLE-5-CARBOXYLIC ACID TERT-BUTYLAMIDE

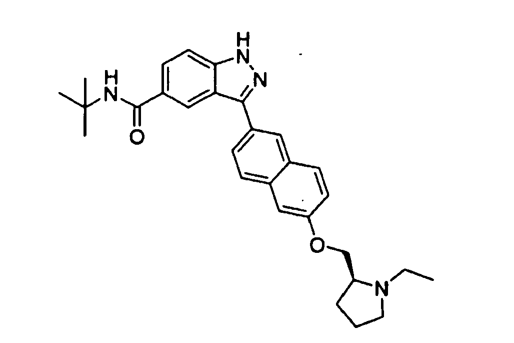

### A. (S) 3-[6-(1-Ethyl-pyrrolidin-2-ylmethoxy)-naphtbalen-2-yl]-1-(tetrahydro-pyran-2-yl)-1H-indazole-5-carboxylic acid tert-butylamide

(S) 3-[6-(1-Ethyl-pyrrolidin-2-ylmethoxy)-naphthalen-2-yl]-1-(tetrahydro-pyran-2-yl)-1H-indazole-5-carbonitrile (96 mg, 0.21 mmol) was dissolved in a 4:1 mixture of ethanol and water (3 mL) and potassium hydroxide (116.9 mg,2.1 mmol) was added. The reaction was heated at 130°C for18 h, after which hydrolysis to the acid was complete. Solvent was removed *in vacuo* and the residue was dried under high vacuum overnight and used without further purification. The residue was dissolved in methylene chloride (2 mL) and O-benzotriazol-1yl-N,N,N',N'-tetramethyluronium hexafluorophosphate (HBTU) (95.6 mg, 0.25 mmol), 1-hydroxybenzotriazole hydrate (HOBt) (28.4 mg, 0.21 mmol), triethylamine (0.09 mL, 0.63 mmol) and tert-butylamine (44 µL, 0.42 mmol) were added. The reaction mixture was stirred at room temperature for 10 min after which the solvent was removed *in vacuo* and the crude purified by HPLC (20 → 80% acetonitrile/ water) to yield the title compound (75 mg, 64% yield). ES-MS (m/z) 555 [M+1]⁺.

### B. (S) 3-[6-(1-Etbyl-pyrrolidin-2-ylmetboxy)-napbtbalen-2-yl)-1H-indazole-5-carboxylic acid tert-butylamide, hydrochloride

The title compound was prepared according to the procedure described in Example 113, step B using (S) 3-[6-(1-ethyl-pyrrolidin-2-ylmethoxy)-naphthalen-2-yl]-1-(tetrahydro-pyran-2-yl)-1H-indazole-5-carboxylic acid tert-butylamide (75 mg, 0.135 mmol). 60.3 mg of the title compound were obtained (88% yield, 98.7% purity). ¹H NMR (methanol-d₄, 400 MHz) δ 8.56 (s, 1H), 8.43 (s, 1H), 8.09 (dd, 1H), 7.95 (m, 2H), 7.83 (dd, 1H), 7.58 (dd, 1H), 7.40 (d, 1H), 7.28 (dd, 1H), 4.52 (dd, 1H), 4.34 (m, 1H), 4.05 (m, 1H), 3.71 (m, 1H), 3.60 (m, 1H), 3.24 (m, 2H), 2.40 (m, 1H), 2.14 (m, 3H), 1.47 (s, 9H), 1.41 (t, 3H). ES-MS (m/z) 471 [M+1]⁺.

### EXAMPLE 121

### SYNTHESIS OF (S) 3-[6-(1-ETHYL-PYRROLIDIN-2-YLMETHOXY)-NAPHTHALEN-2-YL]-1H-INDAZOLE-5-CARBOXYLIC ACID (3-METHYLBUTYL)-AMIDE

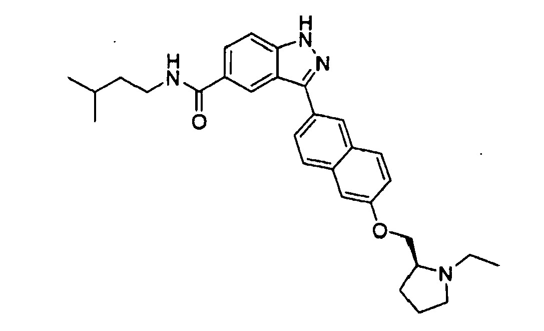

### A. (S) 3-[6-(1-Ethyl-pyrrolidin-2-ylmethoxy)-naphthalen-2-yl]-1-(tetrabydro-pyran-2-yl)-1H-indazole-5-carboxylic acid (3-metbylbutyl)-amide.

(S) 3-[6-(1-Ethyl-pyrrolidin-2-ylmethoxy)-naphthalen-2-yl]--(tetrahyclro-pyran-2-yl)-1H-indazole-5-carbonitrile (96 mg, 0.21 mmol) was dissolved in a 4:1 mixture of ethanol and water (3 mL) and potassium hydroxide (116.9 mg, 2.1 mmol) was added. The reaction was heated at 130°C for 18 h, after which hydrolysis to the acid was complete. Solvent was removed *in vacuo* and the residue was dried under high vacuum overnight and used without further purification. The residue was dissolved in methylene chloride (2 mL) and O-benzotriazol-1yl-N,N,N',N'-tetramethyluronium hexafluorophosphate (HBTU) (95.6 mg, 0.25 mmol), 1-hydroxybenzotriazole hydrate (HOBt) (28.4 mg, 0.21 mmol), triethylamine (0.09 mL, 0.63 mmol) and isoamylamine (49 µL, 0.42 mmol) were added. The reaction mixture was stirred at room temperature for 10 min after which the solvent was removed *in vacuo* and the crude purified by HPLC (20 → 80% acetonitrile/ water) and column chromatography (SiO₂, 10% methanol/methylene chloride) to yield the title compound (60 mg, 50% yield). ES-MS (m/z) 569 [M+1]⁺.

### B. (S) 3-[6-(1-Etbyl-pyrrolidin-2-ylmetboxy)-napbtbalen-2-yl)-1H-indazole-5-carboxylic acid (3-metbylbutyl)-amide, hydrochloride

The title compound was prepared according to the procedure described in Example 113, step B using (S) 3-[6-(1-ethyl-pyrrolidin-2-ylmethoxy)-naphthalen-2-yl]-1-(tetrahydro-pyran-2-yl)-1H-indazole-5-carboxylic acid (3-methylbutyl)-amide (60 mg, 0.106 mmol). 34.9 mg of the title compound were obtained (64% yield, 98.1% purity). ¹H NMR (methanol-d₄, 400 MHz) δ 8.64 (s, 1H), 8.43 (s, 1H), 8.09 (dd, 1H), 7.95 (m, 2H), 7.89 (dd, 1H), 7.61 (d, 1H), 7.39 (s, 1H), 7.28 (dd, 1H), 4.51 (dd, 1H), 4.35 (m, 1H), 4.04 (m, 1H), 3.72 (m, 1H), 3.64 (m, 1H), 3.43 (t, 2H), 3.25 (m, 2H), 2.38 (m, 1H), 2.13 (m, 3H), 1.66 (m, 1H), 1.53 (m, 2H), 1.41 (t, 3H), 0.95 (d, 6H). ES-MS (m/z) 485 (M+1]⁺.

### EXAMPLE 122

### SYNTHESIS OF (S) 3-[6-(1-ETHYL-PYRROL)DIN-2-YLMETHOXY)-NAPHTHALEN-2-YL]-1H-INDAZOLE-5-CARBOXYLIC ACID (1,1-DIMETHYLPROPYL)-AMIDE

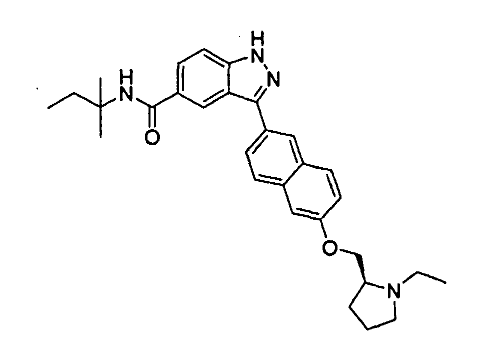

### A. (S) 3-[6-(1-Ethyl-pyrrolidin-2-ylmethoxy)-naphthalen-2-yl]-1-(tetrahydro-pyran-2-yl)-1H-indazole-5-carboxylic acid (1,1-dimethylpropyl)-amide

(S) 3-[6-(1-Ethyl-pyrrolidin-2-ylmethoxy)-naphthalen-2-yl]-1-(tetrahydr-pyran-2-yl)-1H-indazole-5-carbonitrile (96 mg, 0.21 mmol) was dissolved in a 4:1 mixture of ethanol and water (3 mL) and potassium hydroxide (116.9 mg, 2.1 mmol) was added. The reaction was heated at 130°C for18 h, after which hydrolysis to the acid was complete. Solvent was removed in *vacuo* and the residue was dried under high vacuum overnight and used without further purification. The residue was dissolved in methylene chloride (2 mL) and O-benzotriazol-1yl-N,N,N',N'-tetramethyluronium hexafluorophosphate (HBTU) (95.6 mg, 0.25 mmol), 1-hydroxybenzotriazole hydrate (HOBt) (28.4 mg, 0.21 mmol), triethylamine (0.09 mL, 0.63 mmol) and *tert*-amylamine (49 µL, 0.42 mmol) were added. The reaction mixture was stirred at room temperature for 10 min after which the solvent was removed in *vacuo* and the crude purified by HPLC (20 → 80% acetonitrile/ water) to yield the title compound (40 mg, 33.5% yield). ES-MS (m/z) 569 [M+1]⁺.

### B. (S) 3-[6-(1-Ethyl-pyrrolidin-2-ylmethoxy)-naphthalen-2-yl]-1H-indazole-5-carboxylic acid (1,1-dimethylpropyl)-amide, hydrochloride

The title compound was prepared according to the procedure described in Example 113, step B using (S) 3-[6-(1-ethyl-pyrrolidin-2-ylmethoxy)-naphthalen-2-yl]-1-(tetrahydro-pyran-2-yl)-1H-indazole-5-carboxylic acid (1,1-dimethylpropyl)-amide (40 mg, 0.07 mmol). 19.1 mg of the title compound were obtained (52% yield, 99.4% purity). ¹H NMR (methanol-d₄, 400 MHz) δ 8.56 (s, 1H), 8.43 (s, 1H), 8.09 (d, 1H), 7.95 (m, 2H), 7.83 (dd, 1H), 7.59 (d, 1H), 7.40 (s, 1H), 7.28 (dd, 1H), 4.51 (dd, 1H), 4.35 (m, 1H), 4.04 (m, 1H), 3.67 (m, 4H), 2.39 (m, 1H), 2.13 (m, 3H), 1.88 (q, 2H), 1.41 (m, 9H), 0.91 (t, 3H). ES-MS (m/z) 485 [M+I]⁺.

### EXAMPLE 123

### SYNTHESIS OF (S) [3-[6-(1-ETHYL-PYRROLIDIN-2-YLMETHOXY)-NAPHTHALEN-2-YL]-1H-INDAZOLE-5-YL]-PYRROLIDIN-1-YL-METHANONE

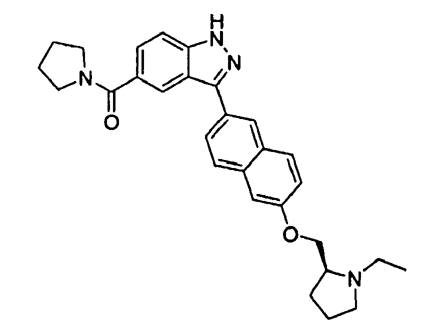

### A. (S) [3-[6-(1-Ethyl-pyrrolidin-2-ylmethoxy)-naphthalen-2-yl]-1-(tetrahydro-pyran-2-yl)-1H-indazole-5-yl]-pyrrolidin-1-yl-methanone.

(S) 3-[6-(1-Ethyl-pyrrolidin-2-ylmethoxy)-naphthalen-2-yl]-1-(tetrahydro-pyran-2-yl)-1H-indazole-5-carbonitrile (96 mg, 0.21 mmol) was dissolved in a 4:1 mixture of ethanol and water (3 mL) and potassium hydroxide (116.9 mg, 2.1 mmol) was added. The reaction was heated at 130°C for18 h, after which hydrolysis to the acid was complete. Solvent was removed in *vacuo* and the residue was dried under high vacuum overnight and used without further purification. The residue was dissolved in methylene chloride (2 mL) and O-benzotriazol-1yl-N,N,N',N'-tetramethyluronium hexafluorophosphate (HBTU) (95.6 mg, 0.25 mmol), 1-hydroxybenzotriazolehydrate (HOBt) (28.4 mg, 0.21 mmol), triethylamine (0.09 mL, 0.63 mmol) and pyrrolidine (35 µL, 0.42 mmol) were added. The reaction mixture was stirred at room temperature for 10 min after which the solvent was removed in *vacuo* and the crude purified by HPLC (20 → 80% acetonitrile/ water) and column chromatography (SiO₂ 10% methanol/methylene chloride) to yield the title compound (55 mg, 47% yield). ES-MS (m/z) 553 [M+1]⁺.

### B. (S) [3-[6-(1-Ethyl-pyrrolidin-2-ylmethoxy)-naphthalen-2-yl]-1H-indazole-5-yl]-pyrrolidin-1-yl methanone, hydrochloride

The title compound was prepared according to the procedure described in Example 113, step B using (S) [3-(6-(1-ethyl-pyrrolidin-2-ylmethoxy)-naphthalen-2-yl]-1-(tetrahydro-pyran-2-yl)-1H-indazole-5-yl]-pyrrolidin-1-yl-methanone (55 mg, 0.10 mmol). 35.5 mg of the title compound were obtained (70% yield, 99.4% purity). ¹H NMR (methanol-d₄, 400 MHz) δ 8.34 (d, 2H), 8.04 (d, 1H), 7.93 (t, 2H), 7.63 (m, 2H), 7.38 (s, 1H), 7.26 (d, 1H), 4.49 (d, 1H), 4.35 (m, 1H), 4.02 (m, 1H), 3.71 (m, 1H), 3.63 (m, 3H), 3.54 (t, 2H), 3.23 (m, 2H), 2.38 (m, 1H), 2.07 (m, 5H), 1.88 (m, 2H), 1.40 (t, 3H). ES-MS (m/z) 469 [M+1]⁺.

### EXAMPLE 124

### SYNTHESIS OF (S) 3-[6-(1-ETHYL-PYRROLIDIN-2-YLMETHOXY)-NAPHTHALEN-2-YL]-1H-INDAZOLE-5-CARBOXYLIC ACID (2,2-DIMETHYLPROPYL)-AMIDE

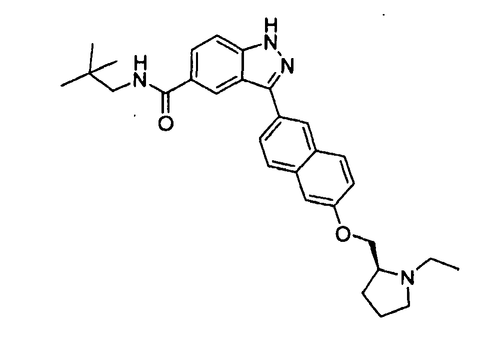

### A. (S) 3-[6-(1-Ethyl-pyrrolidin-2-ylmeihoxy)-naphthalen-2-yl]-1-(tetrahydro-pyran-2-yl)-1H-indazole-5-carboxylic acid (2,2-dimethyl-propyl)-amide

(S) 3-[6-(1-Ethyl-pyrrolidin-2-ylmethoxy)-naphthalen-2-yl)-1-(tetrahydro-pyran-2-yl)-1H-indazole-5-carbonitrile (96 mg, 0.21 mmol) was dissolved in a 4:1 mixture of ethanol and water (3 mL) and potassium hydroxide (116.9 mg, 2.1 mmol) was added. The reaction was heated at 130°C for18 h, after which hydrolysis to the acid was complete. Solvent was removed *in vacuo* and the residue was dried under high vacuum overnight and used without further purification. The residue was dissolved in methylene chloride (2 mL) and O-benzotriazol-1yl-N,N,N',N'-tetramethyluronium hexafluorophosphate (HBTU) (95.6 mg, 0.25 mmol), 1-hydroxybenzotriazole hydrate (HOBt) (28.4 mg, 0.21 mmol), triethylamine (0.09 mL, 0.63 mmol) and neopentylamine (TCI) (25 µL, 0.42 mmol) were added. The reaction mixture was stirred at room temperature for 10 min after which the solvent was removed *in vacuo* and the crude purified by HPLC (20 → 80% acetonitrile/ water) to yield the title compound (40 mg, 37% yield). ES-MS (m/z) 569 [M+1]⁺.

### B. (S) 3-[6-(1-Ethyl-pyrrolidin-2-ylmethoxy)-naphthalen-2-yl]-1H-indazole-5-carboxylic acid (2,2-dimethyl-propyl)-amide, hydrochloride

The title compound was prepared according to the procedure described in Example 113, step B using (S) 3-[6-(1-ethyl-pyrrolidin-2-ylmethoxy)-naphthalen-2-yl]-1-(tetrahydro-pyran-2-yl)-1H-indazole-5-carboxylic acid (2,2-dimethyl-propyl)-amide (40 mg, 0.07 mmol). 23 mg of the title compound were obtained (63% yield, 99.5% purity). 1H NMR (methanol-d₄, 400 MHz) δ 8.66 (s, 1H), 8.43 (s, 1H), 8.09 (m, 1H), 7.93 (m, 3H), 7.62 (d, 1H), 7.39 (d, 1H), 7.28 (m, 1H), 4.49 (m, 1H), 4.35 (m, 1H), 4.03 (m, 1H), 3.72 (m, 1H), 3.64 (m, 1H), 3.24 (m, 4H), 2.39 (m, 1H), 2.12 (m, 3H), 1.40 (t, 3H), 0.97 (s, 9H). ES-MS (m/z) 485 [M+1]⁺.

### EXAMPLE 125

### SYNTHESIS OF (S) 3-[6-(1-ETHYL-PYRROLIDIN-2-YLMETHOXY)-NAPHTHALEN-2-YL]-1H-INDAZOLE-5-CARBOXYLIC ACID (2-METHOXYETHYL)-AMIDE

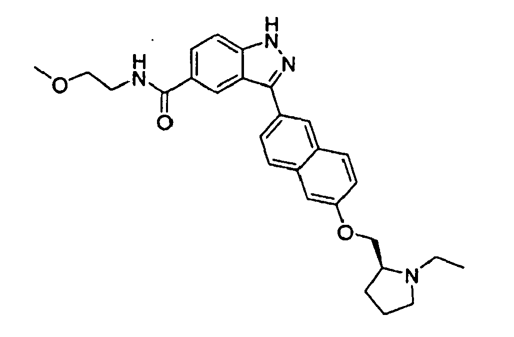

### A. (S) 3-[6-(1-Ethyl-pyrrolidin-2-ylmethoxy)-naphthalen-2-yl]-1-(tetrahydro-pyran-2-yl)-1H-indazole-5-carboxylic acid (2-methoxy-ethyl)-amide

(S) 3-[6-(1-Ethyl-pyrrolidin-2-ylmetboxy)-naphthalen-2-yl]-1-(tetrahydro-pyran-2-yl)-1H-indazole-5-carbonitrile (96 mg, 0.21 mmol) was dissolved in a 4:1 mixture of ethanol and water (3 mL) and potassium hydroxide (116.9 mg, 2.1 mmol) was added. The reaction was heated at 130°C for18 h, after which hydrolysis to the acid was complete. Solvent was removed *in vacuo* and the residue was dried under high vacuum overnight and used without further purification. The residue was dissolved in methylene chloride (2 mL) and O-benzotriazol-1yl-N,N,N',N'-tetramethyluronium hexafluorophosphate (HBTU) (95.6 mg, 0.25 mmol), 1-hydroxybenzotriazole hydrate (HOBt) (28.4 mg, 0.21 mmol), triethylamine (0.09 mL, 0.63 mmol) and 2-methoxyethylamine (37 µL, 0.42 mmol) were added. The reaction mixture was stirred at room temperature for 10 min after which the solvent was removed in *vacuo* and the crude purified by column chromatography (SiO₂, 2% methanol/ methylene chloride→ 10% methanol/ methylene chloride) to yield the title compound (80.9 mg, 69% yield). ES-MS (m/z) 557 [M+1]⁺.

### B. (S) 3-[6-(1-Ethyl-pyrrolidin-2-ylmethoxyhnaphthalen-2-yl]-1H-indazole-5-carboxylic acid (2-methoxy-ethyl)-amide, hydrochloride

The title compound was prepared according to the procedure described in Example 113, step B using (S) 3-[6-(1-ethyl-pyrrolidin-2-ylmethoxy)-naphthalen-2-yl]-1-(tetrahydro-pyran-2-yl)-1H-indazole-5-carboxylic acid (2-methoxy-ethyl)-amide (80.9 mg, 0.145 mmol). 63.6 mg of the title compound were obtained (86% yield, 94% purity). ¹H NMR (methanol-d₄, 400 MHz) δ 8.66 (s, 1H), 8.42 (s, 1H), 8.08 (d, 1H), 7.93 (m, 3H), 7.60 (d, 1H), 7.38 (s, 1H), 7.27 (d, 1H), 4.50 (m, 1H), 4.35 (m, 1H), 4.03 (m, 1H), 3.64 (m, 6H), 3.36 (s, 3H), 3.24 (m, 2H), 2.39 (m, 1H), 2.13 (m, 3H), 1.41 (t, 3H). ES-MS (m/z) 473 [M+1]⁺.

### EXAMPLE 126

### SYNTHESIS OF (S) 3-[6-(1-ETHYL-PYRROLIDIN-2-YLMETHOXY)-NAPHTHALEN-2-YL]-1H-INDAZOLE-5-CARBOXYLIC ACID SEC-BUTYLAMIDE

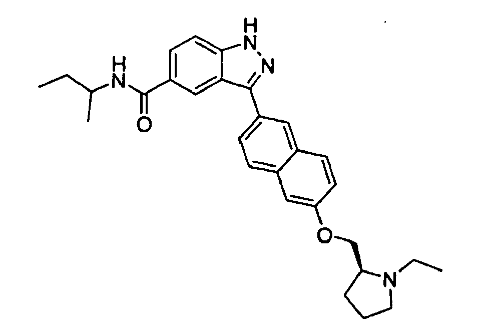

### A. (S) 3-[6-(1-Ethyl-pyrrolidin-2-ylmethoxyhnaphthalen-2-yl]-1-(tetrahydro-pyran-2-yl)-1H-indazole-5-carboxylic acid sec-butylamide

(S) 3-[6-(1-Ethyl-pyrrolidin-2-ylmethoxy)-naphthalen-2-yl]-1-(tetrahydro-pyran-2-yl)-1H-indazole-5-carbonitrile (96 mg, 0.21 mmol) was dissolved in a 4:1 mixture of ethanol and water (3 mL) and potassium hydroxide (116.9 mg, 2.1 mmol) was added. The reaction was heated at 130°C for18 h, after which hydrolysis to the acid was complete. Solvent was removed *in vacuo* and the residue was dried under high vacuum overnight and used without further purification. The residue was dissolved in methylene chloride (2 mL) and O-benzotriazol-1yl-N,N,N',N'-tetramethyluronium hexafluorophosphate (HBTU) (95.6 mg, 0.25 mmol), 1-hydroxybenzotriazole hydrate (HOBt) (28.4 mg, 0.21 mmol), triethylamine (0.09 mL, 0.63 mmol) and *sec*-butylamine (43 µL, 0.42 mmol) were added. The reaction mixture was stirred at room temperature for 10 min after which the solvent was removed *in vacuo* and the crude purified by column chromatography (SiO₂ 5% methanol/ methylene chloride→ 10% methanol/methylene chloride) to yield the title compound (75.1 mg, 65% yield). ES-MS (m/z) 555 [M+1]⁺.

### B. (S) 3-[6-(1-Ethyl-pyrrolidin-2-ylmethoxy)-naphthalen-2-yl]-1H-indazole-5-carboxylic acid sec-butylamide, hydrochloride

The title compound was prepared according to the procedure described in Example 113, step B using (S) 3-[6-(1-ethyl-pyrrolidin-2-ylmethoxy)-naphthalen-2-yl]-1-(tetrahydro-pyran-2-yl)-1H-indazole-5-carboxylic acid sec-butylamide (75.1 mg, 0.135 mmol). 63.6 mg of the title compound were obtained (93% yield, 98.3% purity). ¹H NMR (methanol-d₄, 400 MHz) δ 8.64 (s, 1H), 8.42 (s, 1H), 8.09 (dd, 1H), 7.93 (m, 3H), 7.60 (d, 1H), 7.39 (d, 1H), 7.27 (dd, 1H), 4.50 (m, 1H), 4.35 (m, 1H), 4.05 (m, 2H), 3.72 (m, 1H), 3.62 (m, 1H), 3.23 (m, 2H), 2.39 (m, 1H), 2.13 (m, 3H), 1.61 (m, 2H), 1.41 (t, 3H), 1.23 (d, 3H), 0.96 (t, 3H). ES-MS (m/z) 471 [M+1]⁺.

### EXAMPLE 127

### SYNTHESIS OF (S) 3-[6-(1-ETHYL-PYRROLIDIN-2-YLMETHOXY)-NAPHTHALEN-2-YL]-1H-INDAZOLE-5-CARBOXYLIC ACID BUTYLAMIDE

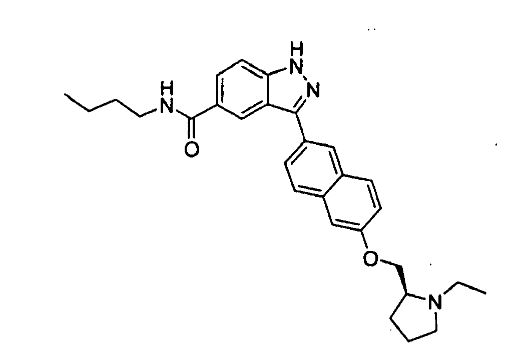

### A. (S) 3-[6-(1-Ethyl-pyrrolidin-2-ylmethoxy)-naphthalen-2-yl]-1-(tetrahydro-pyran-2-yl)-1H-indazole-5-carboxylic acid butylamide

(S) 3-[6-(1-Ethyl-pyrrolidin-2-ylmethoxy)-naphthalen-2-yl]-1-(tetrahydro-pyran-2-yl)-1H-indazole-5-carbonitrile (96 mg, 0.21 mmol) was dissolved in a 4:1 mixture of ethanol and water (3 mL) and potassium hydroxide (116.9 mg, 2.1 mmol) was added. The reaction was heated at 130°C for18 h, after which hydrolysis to the acid was complete. Solvent was removed *in vacuo* and the residue was dried under high vacuum overnight and used without further purification. The residue was dissolved in methylene chloride (2 mL) and O-benzotriazol-1yl-N,N,N',N'-tetramethyluronium hexafluorophosphate (HBTU) (95.6 mg, 0.25 mmol), 1-hydroxybenzotriazole hydrate (HOBt) (28.4 mg, 0.21 mmol), triethylamine (0.09 mL, 0.63 mmol) and butylamine (42 µL, 0.42 mmol) were added. The reaction mixture was stirred at room temperature for 10 min after which the solvent was removed *in vacuo* and the crude purified by column chromatography (SiO₂ 5% methanol/ methylene chloride) to yield the title compound (71.6 mg, 62% yield). ES-MS (m/z) 555 [M+1]⁺.

### B. (S) 3-[6-(1-Ethyl-pyrrolidin-2-ylmetboxy)-naphthalen-2-yl]-1H-indazole-5-carboxylic acid butylamide, hydrochloride

The title compound was prepared according to the procedure described in Example 113, step B using (S) 3-[6-(1-ethyl-pyrrolidin-2-ylmethoxy)-naphthalen-2-yl]-1-(tetrahydro-pyran-2-yl)-1H-indazole-5-carboxylic acid butylamide (71.6 mg, 0.129 mmol). 58.3 mg of the title compound were obtained (89% yield, 99% purity). ¹H NMR (methanol-d₄, 400 MHz) δ 8.63 (s, 1H), 8.41 (s, 1H), 8.07 (d, 1H), 7.92 (m, 3H), 7.60 (d, 1H), 7.37 (d, 1H), 7.26 (d, 1H), 4.47 (m, 1H), 4.34 (m, 1H), 4.01 (m, 1H), 3.67 (m, 3H), 3.40 (t, 2H), 2.94 (s, 1H), 2.38 (m, 1H), 2.11 (m, 3H), 1.61 (m, 2H), 1.41 (m, 5H), 0.95 (t, 3H). ES-MS (m/z) 471 [M+1]⁺.

### EXAMPLE 128

### SYNTHESIS OF (S) 3-[6-(1-ETHXL-PYRROLIDIN-2-YLMETHOXY)-NAPHTHALEN-2-YL]-1H-INDAZOLE-5-CARBOXYLIC ACID CYCLOPROPYLMETHYL-AMIDE

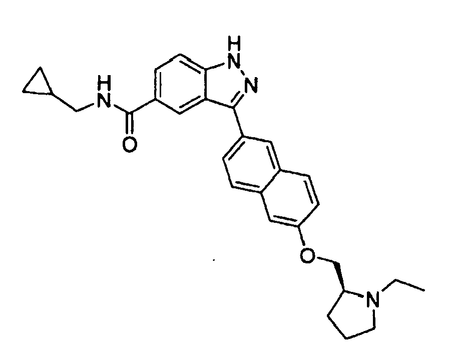

### A. (S) 3-[6-(1-Ethyl-pyrrolidin-2-ylmethoxy)-naphthalen-2-yl]-1-(tetrahydro-pyran-2-yl)-1H-indazole-5-carboxylic acid cyclopropylmethyl-amide

(S) 3-[6-(1-Ethyl-pyrrolidin-2-ylmethoxy)-naphthalen-2-yl]-1-(tetrahydro-pyran-2-yl)-1H-indazole-5-carbonitrile (96 mg, 0.21 mmol) was dissolved in a 4:1 mixture of ethanol and water (3 mL) and potassium hydroxide (116.9 mg, 2.1 mmol) was added. The reaction was heated at 130°C for18 h, after which hydrolysis to the acid was complete. Solvent was removed *in vacuo* and the residue was dried under high vacuum overnight and used without further purification. The residue was dissolved in methylene chloride (2 mL) and O-benzotriazol-1yl-N,N,N',N'-tetramethyluronium hexafluorophosphate (HBTU) (95.6 mg, 0.25 mmol), 1-hydroxybenzotriazole hydrate (HOBt) (28.4 mg, 0.21 mmol), triethylamine (0.09 mL, 0.63 mmol) and (aminomethyl)cyclopropane (36 µL, 0.42 mmol) were added. The reaction mixture was stirred at room temperature for 10 min after which the solvent was removed *in vacuo* and the crude purified by column chromatography (SiO₂, 5% methanol/ methylene chloride) to yield the title compound (72 mg, 62% yield). ES-MS (m/z) 553 [M+1]⁺.

### B. (S) 3-[6-(1-Ethyl-pyrrolidio-2-ylmetboxy)-naphthalen-2-yl]-1H-indazole-5-carboxylic acid cyclopropylmethyl-amide, hydrochloride

The title compound was prepared according to the procedure described in Example 113, step B using (S) 3-[6-(1-ethyl-pyrrolidin-2-ylmethoxy)-naphthalen-2-yl]-1-(tetrahydro-pyran-2-yl)-1H-indazole-5-carboxylic acid cyclopropylmethyl-amide (72 mg, 0.130 mmol). 58.5 mg of the title compound were obtained (89% yield, 99% purity). ¹H NMR (methanol-d₄, 400 MHz) δ 8.67 (s, 1H), 8.44 (s, 1H), 8.10 (dd, 1H), 7.96 (m, 3H), 7.63 (d, 1H), 7.41 (d, 1H), 7.29 (dd, 1H), 4.52 (m, 1H), 4.37 (m, 1H), 4.04 (m, **1**H), 3.68 (m, 3H), 2.96 (m, 3H), 2.40 (m, 1H), 2.14 (m, 3H), 1.42 (t, 3H), 1.13 (m, 1H), 0.52 (m, 2H), 0.29 (m, 2H). ES-MS (m/z) 469 [M+1]⁺.

### EXAMPLE 129

### SYNTHESIS OF (S) 3-[6-(1-ETHYL-PYRROLIDIN-2-YLMETHOXY)-NAPHTHALEN-2-YL]-1H-INDAZOLE-5-CARBOXYLIC ACID CYCLOPROPYLMETHYL-AMIDE

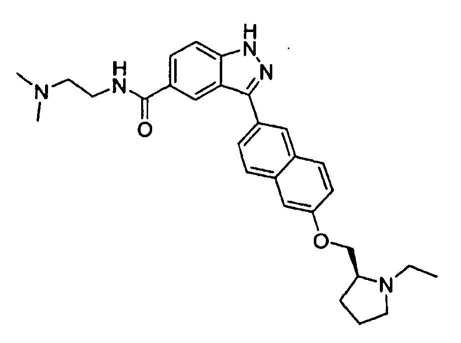

### A. (S) 3-[6-(1-Ethyl-pyrrolidin-2-ylmethoxy)-naphthalen-2-yl]-1-(tetrahydro-pyran-2-yl)-1H-indazole-5-carboxylic acid (2-dimethylamino-ethyl)-amide.

(S) 3-[6-(1-Ethyl-pyrrolidin-2-ylmethoxy)-naphthalen-2-yl]-1-(tetrahydro-pyran-2-yl)-1H-indazole-5-carbonitrile (96 mg, 0.21 mmol) was dissolved in a 4:1 mixture of ethanol and water (3 mL) and potassium hydroxide (116.9 mg, 2.1 mmol) was added. The reaction was heated at 130°C for18 h, after which hydrolysis to the acid was complete. Solvent was removed in *vacuo* and the residue was dried under high vacuum overnight and used without further purification. The residue was dissolved in methylene chloride (2 mL) and O-benzotriazol-1yl-N,N,N',N'-tetramethyluronium hexafluorophosphate (HBTU) (95.6 mg, 0.25 mmol), 1-hydroxybenzotriazole hydrate (HOBt) (28.4 mg, 0.21 mmol), triethylamine (0.09 mL, 0.63 mmol) and N,N-dimethylethylenediamine (45 µL, 0.42 mmol) were added. The reaction mixture was stirred at room temperature for 10 min after which the solvent was removed *in vacuo* and the crude purified by column chromatography (SiO₂, 10% methanol/ methylene chloride) to yield the title compound (23 mg, 19% yield). ES-MS (m/z) 570 [M+1]⁺.

### B. (S) 3-[6-(1-Ethyl-pyrrolidin-2-ylmethoxy)-naphthalen-2-yl]-1H-indazole-5-carboxylic acid (2-dimethylamino-ethyl)-amide, hydrochloride

The title compound was prepared according to the procedure described in Example 113, step B using (S) 3-[6-(1-ethyl-pyrrolidin-2-ylmethoxy)-naphthalen-2-yl]-1-(tetrahydro-pyran-2-yl)-1H-indazole-5-carboxylic acid (2-dimethylamino-ethyl)-amide (23 mg, 0.04 mmol). 23 mg of the title compound were obtained (100% yield, 95% purity). ¹H NMR (methanol-d₄, 400 MHz) δ 8.77 (s, 1H), 8.49 (s, 1H), 8.10 (dd, 1H), 8.04 (d, 1H), 8.00 (dd, 1H), 7.94 (d, 1H), 7.64 (dd, 1H), 7.40 (d, 1H), 7.28 (dd, 1H), 4.51 (m, 1H), 4.37 (m, 1 H), 4.04 (m, 1 H), 3.80 (t, 2H), 3.73 (m, 1H), 3.64 (m, 1H), 3.41 (m, 2H), 3.24 (m, 2H), 2.97 (s, 6H), 2.39 (m, 1H), 2.12 (m, 3H), 1.41 (t, 3H). ES-MS (m/z) 486 [M+1]⁺.

### EXAMPLE 130

### SYNTHESIS OF 5-(5-ISOBUTYL-5H-[1,2,4]TRIAZOL-3-YL)-3-{6-[2-(2-METHYL-PIPERIDIN-1-YL)-ETHOXY]-NAPHTHALEN-2-YL}-1H-INDAZOLE

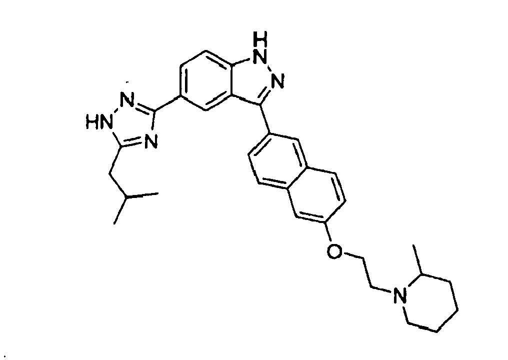

### A. (2-Methyl-piperidin-1-yl)-acetic acid ethyl ester

To a mixture of 2-methylpiperidine (5.0 g, 50 mmol) and ethyl bromoacetate (6.7 mL, 61 mmol) in THF (400 mL) at 0 °C was added triethylamine (14 mL, 0.10 mol) dropwise. After 18 hours, solvent was removed. The residue was resuspended in water and neutralized with 1.0 N HCl_{(aq)}. Extraction of the aqueous phase with dichloromethane followed by drying (MgSO₄) gave crude product which was used without further purification (2.7 g, 29 %): R_{f} = 0.58 (30% ethyl acetate, 70% hexanes).

### B. 2-(2-Methyl-piperidin-1-yl)-ethanol

To a solution of (2-methyl-piperidin-1-yl)-acetic acid ethyl ester (2.7 g, 14 mmol) in THF (150 mL) was added lithium aluminum hydride (0.66 g, 17 mmol) in small portions. After 10 minutes, Na₂SO₄-10H₂O was added until fizzing ceased. After 1.0 hour, the mixtire was filtered over Celite, dried (MgSO₄) and concentrated to give the title compound (1.9 g, 90 %): R_{f}= 0.00 (30 % ethyl acetate, 70 % hexanes).

### C. 1-[2-(6-Bromo-naphthalen-2-yloxy)-ethyl]-2-methyl-piperidine

A mixture of 6-bromo-2-naphthol (1.5 g, 6.6 mmol), racemic 2-(2-methyl-piperidin-1-yl)-ethanol (1.9 g, 13 mmol), triphenylphosphine (3 mmol P / gram polymer supported, 3.5 g, 13 mmol) and diisopropyl azodicarboxylate (2.7 g, 13 mmol) in THF (75 mL) was stirred at ambient temperature for 1 hour. The entire mixture was filtered over Celite and concentrated to give the crude product. Purification was carried out by flash chromatography using hexanes as the eluent to give the title compound (2.4 g, 11 %): ES-MS (m/z) 348 [M+1]⁺.

### D. 6-[2-(2-Methyl-piperidin-1-yl)-ethoxy]-naphthalen-2-boronic acid

To a solution of 1-[2-(6-bromo-naphthalen-2-yloxy)-ethyl]-2-methyl-piperidine (2.4 g, 7.0 mmol) in THF (130 mL) at -78 °C was added n-butyllithium (8.4 mmol) and stirred for 45 minutes. Trimethylborate (2.4 mL, 21 mmol) was added and stirred for an additional 45 minutes. Aqueous saturated ammonium chloride (20 mL) was added and the mixture was allowed to warm to ambient temperature. Water (40 mL) was added and the layers were partitioned. The aqueous layer was extracted with ethyl acetate and the combined organic extracts were dried (MgSO₄) and concentrated to give the title compund (1.8 g, 82 %).

### E. 3-{6-[2-(2-Methyl-cyclohexyl)-ethoxy]-naphthalen-2-yl}-1-(tetrahydro-pyran-2-yl)-1H-indazole-5-carbonitrile

The title compound (0.47 g, 25 %) was prepared as described in Example 149D (WO 02/10137) from 3-bromo-1-perhydro-2*H*-pyran-2-yl-1*H*-indazole-5-carbonitrile (Example 149C in WO 02/10137, 1.2 g, 3.9 mmol) and 6-[2-(2-methyl-piperidin-1-yl)-ethoxy]-naphthalen-2-boronic acid (1.8 g, 5.8 mmol): ES-MS (m/z) 495 [M+1]⁺.

### F. 3-{6-[2-(2-Methyl-cyclobexyl)-etboxy]-naphthalen-2-yl}-1H-indazole-5-carboximidic acid ethyl ester

To a flask was charged 3-{6-[2-(2-methyl-cyclohexyl)-ethoxy]-naphthalen-2-yl}-1-(tetrahydro-pyran-2-yl)-1*H-*indazole-5-carbonitrile (0.47 g, 0.94 mmol) and ethanol (100 mL). HCl (g) was bubbled through the stirring solution until saturated. After 18 hours, mixture was concentrated and slurried with diethyl ether. Solid product was filtered and washed with excess diethyl ether. Product was dried to afford the title compound as the HCl salt (0.38 g, 82 %): ES-MS (m/z) 457 [M+1]⁺.

### G. 5-(5-Isobutyl-5H-[1,2,4]triazol-3-yl)-3-{6-[2-(2-methyl-piperidin-1-yl)-ethoxy]-naphthalen-2-yl}-1H-indazole

To a pressure vessel containing a solution of 3-{6-[2-(2-methyl-cyclohexyl)-ethoxy]-naphthalen-2-yl}-1*H-*indazole-5-carboximidic acid ethyl ester (0.38 g, 0.77 mmol) in methanol (8 mL) was added triethylamine (0.78 g, 7.7 mmol). After 10 minutes, 3-methyl-butyric acid hydrazide (0.18 g, 1.5 mmol) was added and the mixture was heated to 90°C for 18h. The mixture was concentrated and purified by preparatory HPLC to give title compound as a racemic mixture (0.18 g, 46 %): ¹H NMR (DMSO) 8.72 (br s, 1H) 8.44 (br s, 1H) 8.16 - 7.95 (cm, 4H) 7.68 (d, 1H) 7.44 (br s, 1H) 7.24 (br s, 1H) 4.21 (br s, 1H) 4.08 (q, 1H) 3.30 (d, 1H) 3.15 (br s, 1H) 2.93 (br s, 1H) 2.75 (br s, 1H) 2.62 (br s, 2H) 2.35 (cm, 1H) 2.10 (cm, 1H) 1.62 (br s, 2H) 1.40 (br s, 1H) 1.18 (t, 1H) 1.10 (br s, 2H) 0.95 (d, 6H); ES-MS (m/z) 509 [M+1]⁺; 98.6 % purity.

### EXAMPLE 131

### SYNTHESIS OF TERT-BUTYL-{5-[3-(6-METHOXY-NAPHTHALEN-2-YL)-1H-IFlDAZOL-5-YL]-2H-[1,2,4]TRIAZOL-3-YLMETHYL)-AMINE

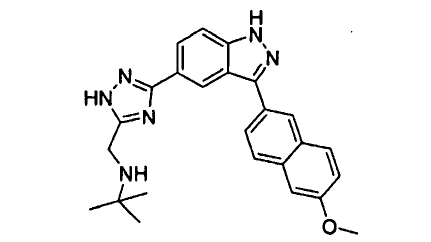

### A. tert-Butylamino-acetic acid ethyl ester

A mixture of tert-butylamine (5.0 g, 68 mmol), triethylamine (6.9 g, 68 mmol) and ethyl bromoacetate (11 g, 68 mmol) in dichloromethane (300 mL) was allowed to stir for 18 hours at ambient temperature. Solvent was removed and the residue was partitioned between water and ethyl acetate. The aqueous phase was extracted with ethyl acetate; combined organic layers were washed with brine and water, dried (MgSO4) and concentrated to give crude product. Purification was carried out by flash chromatography, using 1:1 ethyl acetate - hexanes as the eluent, to give the title compound (3.0 g, 27 %): ¹H NMR (DMSO) 4.09 (q, 2H) 3.27 (s, 2H) 1.18 (t, 3H) 0.98 (s, 9H).

### B. tert-Butylamino-acetic acid hydrazide

A solution of *tert*-Butylamino-acetic acid ethyl ester (1.5 g, 9.6 mmol), ethanol (20 mL) and hydrazine (0.33 mL, 11 mmol) was stirred at 90°C for 18 hours. The mixture was concentrated and dried to give the title compound (1.2 g, 80 %): ¹H NMR (DMSO) 3.11 (s, 2H) 1.06 (s, 9H).

### C. 4-Fluoro-3-[hydroxy-(6-methoxy-naphthalen-2-yl)-methyl-benzonitrile

To a flask containing THF (250 mL) was added lithium diisopropylamide, which was subsequently cooled to -78°C. A solution of p-fluorobenaonitrile (15.5 g, 128 mmol) in THF (55 mL) was added to the cold mixture dropwise. After 20 minutes, a solution of 6-methoxy-2-naphthaldehyde in THF (100 mL) was added dropwise. After 10 minutes, the reaction mixture was allowed to slowly warm to ambient temperature. Water (20 mL) was added and the THF was evaporated. Ether (200 mL) was added and the layers were partitioned. The ether layer was washed with 5% aqueous HCl (2 x 100 mL), brine (100 mL) and was dried (MgSO₄) and concentrated. The crude product was purified by flash chromatography using a gradient of 5% to 20% ethyl acetate in hexanes to give pure title compound (11.2 g, 57 %): ES-MS (m/z) 290 [M-17]⁺.

### D. 4-Ftuoro-3-(6-metboxy-naphthalen-2-carbonyl)-benzonitrile

To a flask was charged 4-fluoro-3-[hydroxy-(6-methoxy-naphthalen-2-yl)-methyl-benzonitrile (1.75 g, 5.71 mmol), dichloromethane (90 mL), and pyridinium chlorochromate (1.29 g, 5.99 mmol). The mixture was heated at 40°C for 3 hours, after which time the solvent was evaporated. Crude product was purified by flash chromatography using dichloromethane as the eluent to give the title compound (1.59 g, 91 %): ES-MS (m/z) 306 [M+I]⁺.

### E. 3-(6-Methoxynaphthalen-2-yl)-1H-indazole-5-carbonitrile

To a flask was charged 4-fluoro-3-(4-fluorobenzoyl)-benzonitrile (7.00g, 22.9 mmol), THF (250 mL) and hydrazine monohydrate (5.74g, 115 mmol). The mixture was allowed to stir at ambient temperature for 4 hours, after which time water (300 mL) was added and the resulting precipitate was filtered, washed with water and dried to afford the title compound (6.6 g, 96 %): ES-MS (m/z) 300 [M+1]⁺.

### F. 3-(6-Methoxynapbthatea-2-yl)-1H-indazote-5-carboximidic acid ethyl ester

To a flask was charged 3-(6-methoxynaphthalen-2-yl)-1H-indazole-5-carbonitrile (6.4 g, 21 mmol) and ethanol (650 mL). HCl gas was bubbled through the stirring solution until saturated. After 18 hours, mixture was concentrated and slurried with diethyl ether. Solid product was filtered and washed with excess diethyl ether. Product was dried under vacuum to afford the title compound as the HCl salt (6.2g, 76%): ES-MS (m/z) 346 [M+1]⁺.

### G. tert-Butyl-{5-[3-(6-methoxy-naphthalen-2-yl]-1H-indazol-5-yl]-2H-[1,2,4]triazol-3-ylmethyl}-amine

To a flask was charged 3-(6-methoxynaphthalen-2-yl)-1H-indazole-5-carboximidic acid ethyl ester (0.78 g, 2.0 mmol), methanol (25 mL), and triethylamine (5.7 mL, 41 mmol). After 10 minutes, *tert*-butylamino-acetic acid hydrazide (1.2 g, 8.2 mmol) was added and the mixture was heated at 90 °C for 18 hours. The mixture was concentrated and purified by preparatory HPLC to give to title compound (95 mg, 11 %): ¹H NMR (DMSO) 8.74 (s, 1H) 8.44 (s, 1H) 8.10 (t, 2H) 8.01 (t, 2H) 7.69 (d, 1H) 7.41 (d, 1H) 7.24 (dd, 1H) 3.92 (s, 3H) 3.83 (s, 2H) 1.10 (s, 9H); ES-MS (m/z) 427 [M+1]⁺; 98.4 % purity.

### EXAMPLE 132

### SYNTHESIS OF 5-(5-TERT-BUTOXYMETHYL-5H-[1,2,4]TRIAZOL-3-YL)-3{6-[2-(2R,6S-DIMETHYL-PEPERIDIN-1-YL)-ETHOXY]-NAPHTHALEN-2-YL)-1H-INDAZOLE

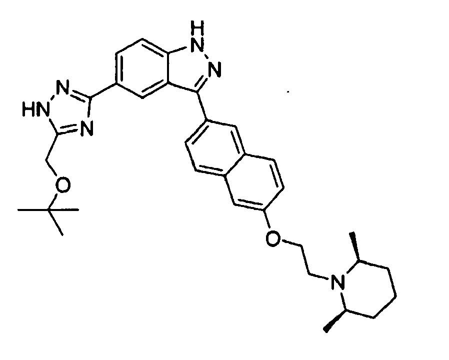

### A. (2S,6R-Dimethyl-piperidin-1-yl)-acetic acid ethyl ester

The title compound (22 g, 25 %) was prepared as described in Example 130, step A using cis-2,6-dimethylpiperidine (51 g, 0.45 mol): ¹H NMR (CDCl₃) 4.16 (q, 2H) 3.58 (s, 2H) 2.90 - 2.78 (cm, 2H) 1.72 -1.62 (cm, 1H) 1.62 - 1.53 (cm, 2H) 1.48 - 1.18 (cm, 3H) 1.27 (t, 3H) 1.11 (d, 6H).

### B. 2-(2S,6R-Dimethyl-piperidin-1-yl)-ethanol

The title compound (13 g, 75 %) was prepared as described in Example 130, step B using (2*S*,6*R*-Dimethyl-piperidin-1-yl)-acetic acid ethyl ester (22 g, 0.11 mol): R_{f}= 0.00 (30 % ethyl acetate, 70 % hexanes).

### C. 1-[2-(6-Bromo-naphthalen-2-yloxy)-ethyl]-2S,6R-dimethyl-piperidine

The title compound (20 g, 92 %) was prepared as described in Example 130, step C using 2-(2*S*,6*R*-dimethyl-piperidin-1-yl)-ethanol (14 g, 90 mmol): ES-MS (m/z) 362 [M+1]⁺.

### D. 3-{6-[2-(2S,6R-Dimethyl-piperidin-1-yl)-ethoxy]-naphthalen-2-yl}-1-(tetrahydro-pyran-2-yl)-1H-indazole-5-carbonitrile

A mixture of 1-[2-(6-bromo-naphthalen-2-yloxy)-ethyl]-2*S*,6*R*-dimethylpiperidine (11 g, 30 mmol), bis(pinacolato)diboron (7.6 g, 30 mmol), 1,1'-bis(diphenylphosphino)- ferrocene]dichloropalladium(II) complex with dichloromethane (1:1) (2.5 g, 3.0 mmol) and potassium acetate (8.9 g, 90 mmol) in dimethylformamide (350 mL) was heated to 85 °C under nitrogen. After 2.5 hours, 3-bromo-1-perhydro-2*H-*pyran-2-yl-1*H*-indazole-5-carbonitrile (see WO 02/10137, Example 149 C) 10 g, 33 mmol) was added followed by potassium phosphate (tribasic, 19 g, 90 mmol). After 18 hours, solvent was evaporated and residue was resuspended in 1:1 water - ethyl acetate. The layers were partitioned and the aqueous phase was extracted with ethyl acetate. Combined organic layers were dried (MgSO₄) and concentrated to give crude product which was subsequently purified by flash chromatography using 1:19 methanol - dichloromethane to give the title compound (4.6 g, 30 %): ES-MS (m/z) 509 [M+1]⁺.

### E. 3-{6-[2-(2S,6R-Dimethyl-piperidin-1-yl)-ethoxy]-naphthalen-2-yl}-1H-indazole-5-carboximidic acid ethyl ester

The title compound (4.2 g, 91 %) was prepared as described in Example 130, step F from 3-{6-[2-(2*S*,6*R*-dimethyl-piperidin-1-yl)-ethoxy]-naphthalen-2-yl}-1-(tetrahydro-pyran-2-yl)-1*H*-indazole-5-carbonitrile (4.6 g, 9.0 mmol): ES-MS (m/z) 471 [M+1]⁺.

### F. 5-(5-tert-Butoxymethyl-5H-[1,2,4]triazol-3-yl)-3{6-[2-(2S,6R-dimethyl-piperidin-1-yl)-ethoxy]-naphthalen-2-yl}-1H-indazole

The title compound (27 mg, 2 %) was prepared as described in Example 130, step G using 3-{6-[2-(2*R*,6*S*-dimethyl-piperidin-1-yl)-ethoxy]-naphthalen-2-yl}-1*H*-indazole-5-carboximidic acid ethyl ester (1.5 g, 3.0 mmol) and *tert*-butoxy-acetic acid hydrazide (0.65 g, 4.4 mmol): ¹H NMR (DMSO) 8.72 (br s, 1H) 8.43 (br s, 1H) 8.09 (d, 2H) 7.99 (t, 2H) 7.69 (br s, 1H) 7.43 (d 1H) 7.21 (d, 1H) 4.51 (cm, 2H) 4.13 (t, 2H) 3.09 (t, 2H) 2.54 (cm, 2H) 1.66 - 1.48 (cm, 4H) 1.40 - 1.10 (cm, 2H) 1.25 (s, 9H) 1.12 (d, 6H); ES-MS (m/z) 553 [M+1]⁺; 98.3 % purity.

### EXAMPLE 133

### SYNTHESIS OF 1-[2-(6-{5-[5-(2,2-DIMETHYL-PROPYL)-1H-[1,2,4]TRIAZOL-3-YL]-1H-INDAZOL-3-YL}-NAPHTHALEN-2-YLOXY)-ETHYL]-PYRROLIDIN-2-ONE

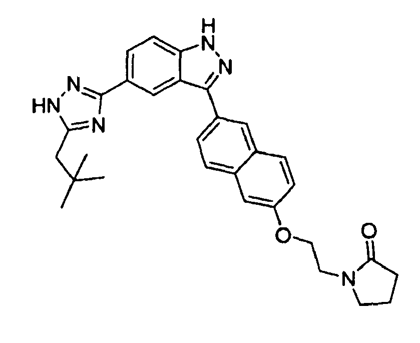

### A. 1-[2-(6-Bromo-naphthalen-2-yloxy)-ethyl]-pyrrolidin-2-one

The title compound (2.7 g, 21 %) was prepared as described in Example 130, step C using 6-bromo-2-naphthol (9.0 g, 39 mmol) and 1-(2-hydroxyethyl)-2-pyrrolidinone (10 g, 77 mmol). Pure product crystallized from ethanol over 18 hours: ¹H NMR (CDCl₃) 7.86 (s, 1H) 7.70 - 7.42 (cm, 3H) 7.20 - 6.98 (cm, 2H) 4.22 (t, 2H) 3.72 (t, 2H) 3.58 (t, 2H) 2.38 (t, 2H) 2.01 (m, 2H).

### B. 3-[6-[2-(2-Oxo-pyrrolidin-1-yl)-ethoxy]-naphthalen-2-yl}-1-(tetrahydro-pyran-2-yl)-1H-indazole-5-carbonitrile

The title compound (1.3 g, 61 %) was prepared as described in Example 132, step D D using 1-[2-(6-bromo-naphthalen-2-yloxy)-ethyl)-pyrrolidin-2-one: ES-MS (m/z) 481 [M+1]⁺.

### C. 3-{6-[2-(2-Oxo-pyrrolidin-1-yl)-ethoxy]-naphthalen-2-yl}-1H-indazole-5-carboximidic acid ethyl ester

The title compound (1.0 g, 76 %) was prepared as described in Example 130, step F using 3-{6-[2-(2-oxo-pyrrolidin-1-yl)-ethoxy]-naphthalen-2-yl}-1-(tetrahydro-pyran-2-yl)-1*H*-indazole-5-carbonitrile (1.3 g, 2.7 mmol): ES-MS (m/z) 443 [M+1]⁺.

### D. 1-[2-(6-{5-[5-(2,2-Dimethyl-propyl)-1H-indazol-3-yl)-naphthalen-2-yloxy)-ethyl]-pyrrolidin-2-one

The title compound (190 mg, 36 %) was prepared as described in Example 130, step G using 3-{6-[2-(2-oxo-pyrolidin-1-yl)-ethoxy]-naphthalen-2-yl}-1*H*-indazole-5-carboximidic acid ethyl ester (0.50 g, 1.0 mmol) and 3,3-dimethyl-butyric acid hydrazide (0.27 g, 2.1 mmol): ¹H NMR (DMSO) 8.71 (br s, 1H) 8.42 (br s, 1H) 8.09 (d, 2H) 8.07 - 7.94 (m, 2H) 7.68 (br d, 1H) 7.44 (d, 1H) 7.24 (dd, 1H) 4.30 (t, 2H) 3.65 (t, 2H) 3.52 (t, 2H) 2.65 (br s, 2H) 2.24 (t, 2H) 1.94 (m, 2H) 1.00 (s, 9H); ES-MS (m/z) 509 [M+1]⁺; 99.4 % purity.

### EXAMPLE 134

### SYNTHESIS OF 1-(2-{6-[5-(5-PYRROLIDIN-1-YLMETHYL-1H-[1,2,4]TRIAZOL-3-4L)-1 H-INDAZOL-3-YL]-NAPHTHALEN-2-YLOXY} -ETHYL)-PYRROLIDIN-2-ONE

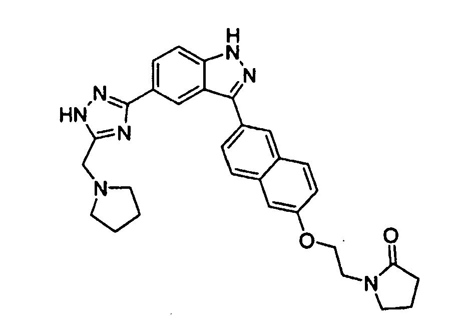

The title compound (30 mg, 6 %) was prepared as described in Example 130, step G using 3-{6-[2-(2-oxo-pyrrolidin-1-yl)-ethoxy]-naphthalen-2-yl)-1*H*-indazole-5-carboximidic acid ethyl ester (Example 133, step C, 0.50 g, 1.0 mmol) and pyrrolidin-1-yl-acetic acid hydrazide (0.27 g, 2.1 mmol): ¹H NMR (DMSO) 8.74 (br s, 1H) 8.44 (br s, 1H) 8.11 (br t, 2H) 8.00 (dd, 2H) 7.69 (d, 1H) 7.43 (d, 1H) 7.24 (dd, 1H) 4.26 (t, 2H) 3.65 (t, 2H) 3.51 (t, 2H) 3.27 (s, 2H) 2.25 (t, 2H) 1.95 (m, 2H) 1.78 - 1.63 (cm, 6H); ES-MS (m/z) 523 [M+2]⁺; 99.5 % purity.

### EXAMPLE 135

### SYNTHESIS OF 5-(5-ISOBUTYL-5H-[1,2,4]TRLAZOL-3-YL)-3-{6-[2-(2S-METHYL-PIPERIDIN-1-YL)-ETHOXY]-NAPHTHALEN-2-YL}-1H-INDAZOLE

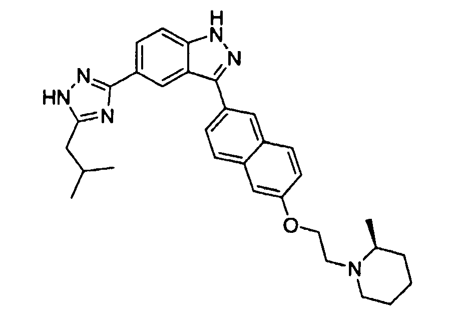

### A. (2S-Methyl-piperidin-1-yl)-acetic acid ethyl ester

The title compound (7.4 g, 92 %) was prepared as described in Example 130, step A using (*S*)-(+)-2-methylpiperidine (4.3 g, 43 mmol): R_{f}= 0.56 (30 % ethyl acetate, 70 % hexanes).

### B. 2-(2S-Methyl-piperidin-1-yl)-ethanol

The title compound (5.4 g, 95 %) was prepared as described in Example 130, step B using (2*S*-methyl-piperidin-1-yl)-acetic acid ethyl ester (7.4 g, 40 mmol).

### C. 1-[2-(6-Bromo-naphthalen-2-yloxy)-ethyl]-2-methyl-piperidine

The title compound (1.7 g, 19 %) was prepared as described in Example 130, step C using 2-(2*S*-methyl-piperidin-1-yl)-ethanol (7.5 g, 52 mmol): ES-MS (m/z) 348 [M+1]⁺.

### D. 3-(6-[2-(2S-Methyl-piperidin-1-yl)-ethoxy]-naphthalen-2-yl}-1-(tetrahydro-pyran-2-yl)-1H-indazole-5-carbonitrile

The title compound (1.2 g, 51 %) was prepared as described in Example 132, step D using 1-[2-(6-bromo-naphthalen-2-yloxy)-ethyl]-2-methyl-piperidine (1.7 g, 4.9 mmol): ES-MS (m/z) 495 [M+1]⁺.

### E. 3-{6-[2-(2S-Methyl-piperidin-1-yl)-ethoxy]-naphthalen-2-yl}-1H-indazole-5-carboximidic acid ethyl ester

The title compound (1.1 g, 87 %) was prepared as described in Example 130, step F using 3-{6-[2-(2*S*-methyl-piperidin-1-yl)-ethoxy]-naphthalen-2-yl}-1-(tetrahydro-pyran-2-yl)-1*H*-indazole-5-carbonitrile (1.2 g, 2.7 mmol): ES-MS (m/z) 457 [M+1]⁺.

### F. 5-(5-Isobutyl-5H-{1,2,4]triazol-3-yl)-3-{6-[2-(2S-methyl-piperidin-1-yl)-ethoxy]-naphthalen-2-yl}-1H-indazole

The title compound (0.20 g, 38 %) was prepared as described in Example 130, step G using 3-{6-[2-(2*S*-methyl-piperidin-1-yl)-ethoxy]-naphthalen-2-yl}-1*H*-indazole-5-carboximidic acid ethyl ester (0.50 g, 1.0 mmol) and 3-methyl-butyric acid hydrazide (0.24 g, 2.0 mmol): ¹H NMR (DMSO) 8.72 (br s, 1H) 8.43 (br s, 1H) 8.14 - 8.05 (cm, 2H) 7.99 (dd, 2H) 7.68 (d, 1H) 7.43 (d, 1H) 7.22 (dd, 1H) 4.20 (t, 2H) 3.07 (dt, 1H) 2.93 (dt, 1H) 2.74 (dt, 1H) 2.62 (br d, 2H) 2.47 - 2.52 (cm, 1H) 2.32 (dt, 1H) 2.11 (m, 1H) 1.68 - 1.10 (cm, 8H) 1.08 (d, 3H) 0.95 (d, 6H); ES-MS (m/z) 509 [M+1]⁺; 99.6 % purity.

### EXAMPLE 136

### SYNTHESIS OF 3-{2-[2-(2S,6R-DIMETHYL-PIPERIDIN-1-YL)-ETHOXY]-NAPHTHALEN-2-YL}-5-(5-PYRROLIDDU-1-YLMETHYL-5H-[ 1,2,4]TRIAZOL-3-YL)-1H-INDAZOLE

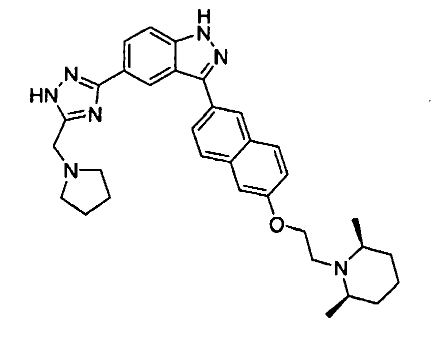

The title compound (51 mg, 3 %) was prepared as described in Example 130, step G using 3-{6-[2-(2S,6R-dimethyl-piperidin-1-yl)-ethoxy]-naphthalen-2-yl}-1H-indazole-5-carboximidic acid ethyl ester (Example 132, step E, 1.5 g, 3.0 mmol) and pyrrolidin-1-yl-acetic acid hydrazide (LN-1034-90, 0.77 g, 5.9 mmol): 1H NMR (DMSO) 8.75 (s, 1H) 8.44 (s, 1H) 8.10 (br t, 2H) 8.00 (t, 2H) 7.69 (d, 1H) 7.43 (d, 1H) 7.21 (dd, 1H) 4.12 (t, 2H) 3.76 (s, 2H) 3.03 (t, 2H) 2.56 (m, 4H) 1.72 (m, 4H) 1.67 - 1.10 (cm, 8H) 1.13 (d, 6H); ES-MS (m/z) 551 [M+2]+; 99.2 % purity.

### EXAMPLE 137

### SYNTHESIS OF 3-{6-[2-(2S-METHYL-PIPERIDIN-1-YL)-ETHOXY]-NAPHTHALEN-2-YL}-5-(5-PYRROLIDIN-1-YLMETHYL-5H-[1,2,4]TRIZOL-3-YL)-1H-INDAZOLE

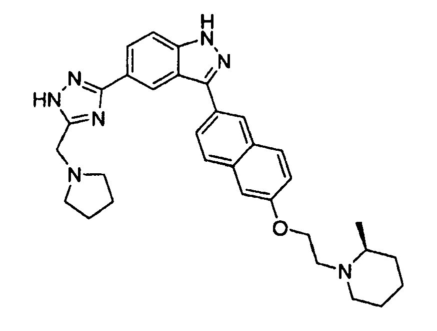

The title compound (35 mg, 12 %) was prepared as described in Example 130, step G using 3-{6-[2-(2*S*-methyl-piperidin-1-yl)-ethoxy]-naphthalen-2-yl}-1*H*-indazole-5-carboximidic acid ethyl ester (0.27 g, 0.54 mmol) and pyrrolidin-1-yl-acetic acid hydrazide (0.14 g, 1.1 mmol): ¹H NMR (DMSO) 8.74 (s, 1H) 8.44 (s, 1H) 8.16 - 8.05 (cm, 2H) 8.00 (t, 2H) 7.70 (d, 1H) 7.43 (d, 1H) 7.22 (dd, 1H) 1.29 (t, 2H) 3.75 (t, 2H) 3.13 - 3.00 (cm, 1H) 3.01 - 2.86 (cm, 1H) 2.82 - 2.67 (cm, 1H) 2.62 - 2.45 (cm) 2.45 - 2.23 (cm) 1.80 - 1.11 (cm) 1.08 (d, 3H); ES-MS (m/z) 537 [M+2]⁺; 99.9 % purity.

### EXAMPLE 138

### SYNTHESIS OF 5-(5-CYCLOPROPYLMETHYL-5H-[1,2,4]TRIAZOL-3-YL)-3-{6-[2-(2S,6R-DIMETHYL-PIPERIDIN-1-YL)-ETHOXY]-NAPHTHALEN-2-YL}-1H-INDAZOLE

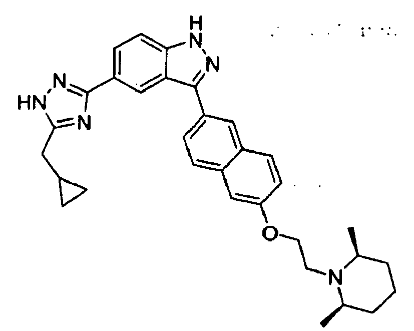

### A. 5-(5-Cyclopropylmethyl-5H-[1,2,4]triazol-3-yl)-3-{6-[2-(2S,6R-dimethyl-piperidin-1-yl)-ethoxy]-naphthalen-2-yl}-1 H-indazole

The title compound (125 mg, 5 %) was prepared as described in Example 130, step G using 3-{6-[2-(2*S*,6*R*-dimethyl-piperidin-1-yl)-ethoxy]-naphthalen-2-yl}-1*H-*indazole-5-carboximidic acid ethyl ester (Example 132, step E, 2.4 g, 4.8 mmol) and cyclopropyl-acetic acid hydrazide (1.1 g, 9.6 mmol): ¹H NMR (DMSO) 8.72 (s, 1H) 8.42 (s, 1H) 8.09 (cm, 2H) 7.99 (t, 2H) 7.68 (d, 1H) 7.42 (d, 1H) 7.21 (dd, 1H) 4.12 (t, 2H) 3.03 (t, 2H) 2.68 (s, 2H) 2.60 - 2.45 (cm) 1.66 - 1.48 (cm) 1.44 - 0.93 (cm) 1.12 (d, 3H) 0.51 (d, 2H) 0.25 (d, 2H); ES-MS (m/z) 521 [M+1]⁺; 99.6 % purity.

### EXAMPLE 139

### SYNTHESIS OF 5-[5-(2,2-DIMETHYL-PROPYL)-5H-[1,2,4]TRIAZOL-3-YL]-{6-[2-(2S-METHYL-PIPERIDIN-1-YL)-ETHOXY]-NAPHTHALEN-2-YL} -1H-INDAZOLE

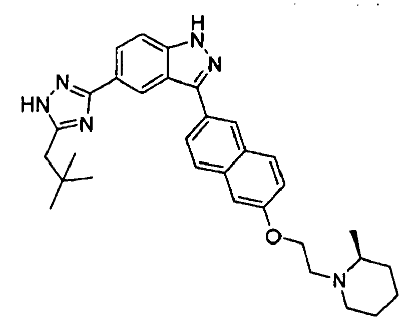

### A. 5-[5-(2,2-Dimethyl-propyl)-5H-[1,2,4]triazol-3-yl]-{6-[2-(2S-methyl-piperidin-1-yl)-ethoxy]-naphthalen-2-yl}-1H-indazole

The title compound (54 mg, 16 %) was prepared as described in Example 130, step G using 3-{6-[2-(2*S*-methyl-piperidin-1-yl)-ethoxy]-naphthalen-2-yl}-1*H-*indazole-5-carboximidic acid ethyl ester (Example 6-E, 0.31 g, 0.63 mmol) and t-butylacetic acid hydrazide (0.16 g, 1.2 mmol): ¹H NMR (DMSO) 8.72 (s, 1H) 8.42 (s, 1H) 8.16 - 8.02 (cm, 2H) 7.98 (t, 2H) 7.68 (d, 1H) 7.43 (d, 1H) 7.22 (dd, 1H) 4.20 (t, 2H) 3.13 - 3.02 (m, 1H) 2.98 - 2.87 (cm, 1H) 2.80 - 2.69 (m, 1H) 2.63 (br s, 2H) 2.48 - 2.37 (br s, 1H) 2.37 - 2.26 (br t, 1H) 1.70 - 1.10 (cm, 6H) 1.09 (d, 3H) 1.00 (s, 9H); ES-MS (m/z) 523 [M+1]⁺; 99.5 % purity.

### EXAMPLE 140

### SYNTHESIS OF 3-{6-[2-(2S,6R-DIMETHYL-PIPERIDIN-1-YL)-ETHOXY]-NAPHTHALEN-2-YL}-5-(5-ISOPROPYL-5H-[1,2,4]TRIAZOL-3-YL)-1H-INDAZOLE

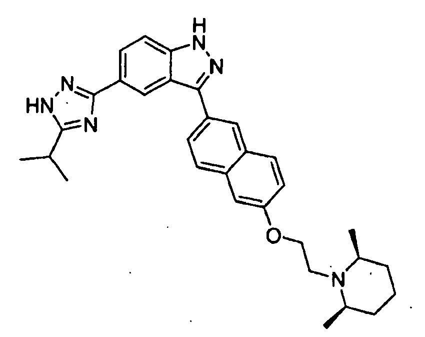

### A. 3-{6-[2-(2S,6R-Dimethyl-piperidin-1-yl)-ethoxy]-naphthalen-2-yl}-5-(5-isopropyl-5H-[1,2,4]triazol-3-yl)-1H-indazole

The title compound (155 mg, 6 %) was prepared as described in Example 130, step G using 3-{6-[2-(2*S*,6*R*-dimethyl-piperidin-1-yl)-ethoxy]-naphthalen-2-yl}-1*H-*indazole-5-carboximidic acid ethyl ester (Example 132, step E, 2.4 g, 4.8 mmol) and isobutyric acid hydrazide (0.58 g, 5.7 mmol): ¹H NMR (DMSO) 8.72 (s, 1H) 8.43 (s, 1H) 8.14 - 8.03 (cm, 2H) 7.99 (t, 2H) 7.68 (d, 1H) 7.42 (d, 1H) 7.21 (dd, 1H) 4.11 (t, 2H) 3.03 (t, 2H) 2.53 (m, 2H) 1.67 - 0.93 (cm, 7H) 1.33 (d, 6H) 1.12 (d, 6H); ES-MS (m/z) 509 [M+1]⁺; 99.8 % purity.

### EXAMPLE 141

### SYNTHESIS OF 3-(6-DIFLUOROMETHOXY-NAPHTHALEN-2-YL)-5-(5-MORPHOLIN-4-YLMETHYL-5H-[1,2,4]TRIAZOL-3-YL)-1H-INDAZOLE

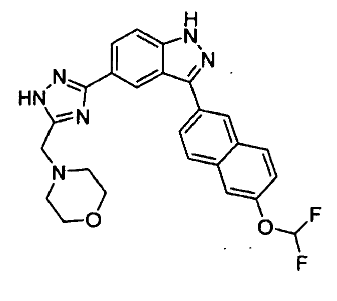

### A. 6-Methoxynaphthalen-2-yl-boronic acid

The title compound (7.55 g, 87 %) was prepared as described in Example 130, step D using 6-bromo-2-naphthol (10.0 g, 42.9 mmol), n-butyllithium (94.4 mmol) and trimethyl borate (14.4 mL, 129 mmol).

### B. 3-(6-Hydroxynaphthalen-2-yl-1-(tetrahydropyran-2-yl)-1H-indazole-5-carbonitrile

The title compound was prepared (see WO 02/10137, Example 149D) from 3-bromo-1-perhydro-2H-pyran-2-yl-1H-indazole-5-carbonitrile (see WO 02/10137, Example 149C, 2.00 g, 6.53 mmol) and 6-methoxynaphthalen-2-yl-boronic acid (1.84 g, 9.80 mmol): ES-MS (m/z) 370 [M+1]⁺.

### C. 3-(6-Difluoromethoxynaphthalen-2-yl)-1-(tetrahydropyran-2-yl)-1H-indazole-5-carbonitrile

To a flask was charged 3-(6-hydroxynaphthalen-2-yl-1-(tetrahydropyran-2-yl)-1H-indazole-5-carbornitrile (0.91 g, 2.46 mmol), 20% aqueous NaOH (10 mL), and 3 mL of THF. The stirred mixture was cooled to 0°C and a cold solution of THF (15 mL) saturated with chlorodifluoromethane was added dropwise. The reaction mixture was allowed to warm to ambient temperature and stirred for 24 hours. Water (25 mL) was added and the THF was removed under reduced pressure. The aqueous layer was extracted with ethyl acetate, dried (MgSO₄) and concentrated to give the title compound (282 mg, 27 %); ES-MS (m/z) 420 [M+1]⁺.

### D. 3-(6-Difluoromethoxynaphthalen-2-yl)-1H-indazole-5-carboximidic acid ethyl ester

The title compound (245 mg, 96 %) was prepared as described in Example 130, step F from 3-(6-Difluoromethoxynaphthalen-2-yl)-1-(tetrahydropyran-2-yl)-1H-indazole-5-carbonitrile (280 mg, 0.67 mmol): ES-MS (m/z) 382 [M+1]+.

### E. 3-(6-Difluoromethoxy-naphthalen-2-yl)-5-(5-morpholin-4-ylmethyl-5H-[1,2,4]triazol-3-yl)-1H-indazole

The title compound (46 mg, 17 %) was prepared as described in Example 130, step G using 3-(6-difluoromethoxynaphthalen-2-yl)-1H-indazole-5-carboximidic acid ethyl ester (0.24 g, 0.57 mmol), triethylamine (0.58 g, 5.7 mmol), and morpholin-4-yl-acetic acid hydrazide (0.14 g, 0.86 mmole): ¹H NMR (CH₃OD) 8.78 (s, 1H) 8.58 (s, 1H) 8.27 - 8.18 (cm, 2H) 8.15 - 8.07 (cm, 2H) 7.77 (d, 1H) 7.73 (s, 1H) 7.45 (dd, 1H) 7.43 (t, 1H) 3.66 (br s, 1H) 3.59 (t, 4H) 3.33 (s, 1H) 2.49 (m, 4H); ES-MS (m/z) 477 [M+1]⁺; 99.3 % purity.

### EXAMPLE 142

### SYNTHESIS OF 5-[5-(2,2-DIMETHYL-PROPYL)-5H-[1,2,4]TRLAZOL-3-YL]-3-[6-(PYRIDIN-3-YLMETHOXY)-NAPHTHALEN-2-YL]-1H-INDAZOLE

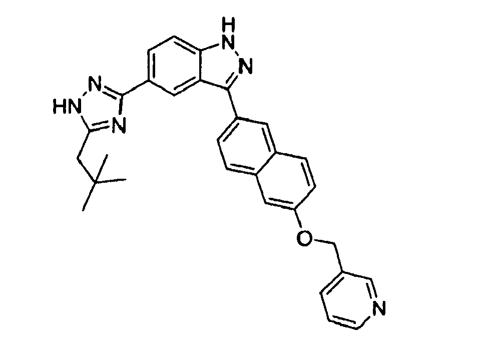

### A. 3-(6-Hydroxy-naphthalen-2-yl)-1-(tetrahydro-pyran-2-yl)-3a,7a-dihydro-1 H-indene-5-carbonitrile

The title compound was prepared (see WO 02/10137, Example 149D) from 3-bromo-1-perhydro-2H-pyran-2-yl-1H-indene-5-carbonitrile (see WO 02/10137, Example 149C) and 6-methoxynaphthalen-2-yl-boronic acid.

### B. 3-[6-(Pyridin-3-ylmethoxy)-naphthalen-2-yl]-1-(tetrahydro-pyran-2-yl)-1 H-indazole-5-carbonitrile

To a pressure vessel containing 3-(6-hydroxy-naphthalen-2-yl)-1-(tetrahydro-pyran-2-yl)-3a,7a-dihydro-1*H*-indene-5-carbonitrile (0.50 g, 1.3 mmol) in 1:1 THF-H₂O (11 mL) was added aqueous NaOH (0.9 mL, 5.4 mmol). After 10 minutes, 3-(bromomethyl) pyridine-HBr salt (0.41 g, 1.6 mmol) was added. The vessel was sealed and heated to 37 °C for 18 hours. Solvent was evaporated and the crude product was partitioned between water and ethyl acetate. The aqueous layer was extracted with ethyl acetate and combined organic extracts were dried (MgSO₄) and concentrated. Purification was carried out by flash chromtography using 1:1 hexanes - ethyl acetate as the eluent to give the title compound (163 mg, 26 %): ES-MS (m/z) 461 [M+1]⁺.

### C. 3-[6-(Pyridin-3-ylmethoxy)-naphthalen-2-yl]-1H-indazole-5-carboximidic acid ethyl ester

The title compound (133 mg, 82 %) was prepared as described in Example 130, step F using 3-[6-(pyridin-3-ylmethoxy)-naphthalen-2-yl]-1-(tetrahydro-pyran-2-yl)-1*H-*indazole-5-carbonitrile (163 mg, 0.35 mmol): ES-MS (m/z) 423 [M+1]⁺.

### D. 5-[5-(2,2-dimethyl-propyl)-5H-[1,2,4]triazol-3-yl]-3-[6-(pyridin-3-ylmethoxy)-naphthalen-2-yl]-1H-indazole

The title compound (31 mg, 22 %) was prepared as described in Example 130, step F using 3-[6-(pyridin-3-ylmethoxy)-naphthalen-2-yl]-1*H*-indazole-5-carboximidic acid ethyl ester (133 mg, 0.29 mmol), triethylamine (0.40 mL, 2.9 mmol) and neopentyl-acetic acid hydrazide (75 mg, 0.58 mmol): ¹H NMR (DMSO) 13.30 (m, 2H) 8.82 - 8.73 (cm, 3 H) 8.21 - 7.90 (cm, 4H) 7.70 (dd, 1H) 7.55 (m, 1H) 7.47 (dd, 1H) 7.32 (m, 1H) 5.32 (s, 2H) 2.61 (d, 2H) 1.00 (s, 9H); ES-MS (m/z) 489 [M+1]⁺; 99.7 % purity.

### EXAMPLE 143

### SYNTHESIS OF 3-[5-(5-MORPHOLIN-4-YLMETHYL-5H-[1,2,4]TRIAZOL-3-YL)-1H-INDAZOL-3-YL]-ISOQUINOLINE

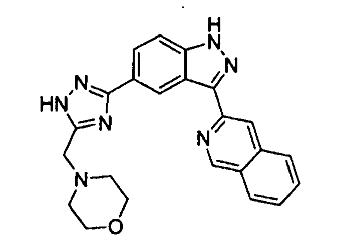

### A. Isoquinolin-3-yl-methanol

The title compound (2.8 g, 64 %) was prepared as described in Example 130, step B using methyl isoquinoline-3-carboxylate (5.1 g, 27 mmol). Purification was carried out by flash chromatography using 1:19 dichloromethane - methanol as the eluent: ES-MS (m/z) 160 [M+1]⁺.

### B. Isoquinoline-3-carbaldehyde

To a flask containing DMSO (2.7 mL, 38 mmol) in dichloromethane (150 mL) at -78 °C was added oxalyl chloride (1.7 mL, 19 mmol) dropwise, followed by a solution of isoquinolin-3-yl-methanol (2.8 g, 17 mmol) in dichloromethane (10 mL). After 10 minutes, triethylamine (12 mL, 87 mmol) was added dropwise. After 5 minutes, the cold reaction mixture was allowed to warm slowly to ambient temperature. After 1.0 hour, the crude mixture was washed with aqueous HCl which was subsequently extracted with dichloromethane. Combined organic extracts were dried (MgSO₄) and concentrated to give the title compound (2.1 g, 78 %): ES-MS (m/z) 158 [M+1]⁺.

### C. 4-Fluoro-3-(hydroxy-isoquinolin-3-yl-methyl)-benzonitrile

The title compound (0.57 g, 17 %) was prepared as described in Example 131, step C using isoquinoline-3-carbaldehyde (2.1 g, 13 mmol): ES-MS (m/z) 279 [M+1]⁺.

### D. 4-Fluoro-3-(isoquinoline-3-carbonyl)-benzonitrile

The title compound (0.53 g, 93 %) was prepared as described in Example 143, step B using 4-fluoro-3-(hydroxy-isoquinolin-3-yl-methyl)-benzonitrile: ES-MS (m/z) 277 [M+1]⁺.

### E. 3-Isoquinolin-3-yl-1H-indazole-5-carbonitrile

The title compound (0.53 g, 90 %) was prepared as described in Example 130, step E using 4-Fluoro-3-(isoquinoline-3-carbonyl)-benzonitrile (0.60 g, 2.2 mmol): ES-MS (m/z) 271 [M+1]⁺.

### F. 3-Isoquinolin-3-yl-1H-indazole-5-carboximidic acid ethyl ester

The title compound (0.55 g, 80 %) was prepared as described in Example 130, step F using 3-isoquinolin-3-yl-1*H*-indazole-5-carbonitrile (0.53 g, 2.0 mmol): ES-MS (m/z) 317 [M+1]⁺.

### G. 3-[5-(5-Morpholin-4-ylmethyl-5H-[1,2,4]triazol-3-yl)-1H-indazol-3-yl]-isoquinoline

The title compound (64 mg, 10 %) was prepared as described in Example 130, step G using 3-isoquinolin-3-yl-1*H*-indazole-5-carboximidic acid ethyl ester (0.55 g, 1.6 mmol), triethylamine (2.2 mL, 16 mmol) and morpholin-4-yl-acetic acid hydrazide (0.50 g, 3.1 mmol): ¹H NMR (CH₃OD) 9.46 (s, 1H) 9.29 (s, 1H) 8.57 (s, 1H), 8.20 - 8.00 (cm, 3H) 7.86 - 7.65 (cm, 3H) 3.80 (s, 2H) 3.76 (t, 4H) 2.64 (s, 4H); ES-MS (m/z) 412 [M+1]⁺; 99.3 % purity.

### Biochemical and Biological Evaluation

### Procedures Useful For The Biological Evaluation Of The Indazole Compounds.

In addition to the extensive literature disclosing the role of HSDs in various diseases and disorders, Examples 51-123 below describe assays useful for testing the Indazole Compounds of the present invention.

### Example 144

Kinase Assays for JNK1, JNK2, JNK3, IKK1, IKK2EE, p38α, p38β, MKK3, MKK4, MKK6, MKK7, CDK2/E, CDK2/A, PKCα, ERK, PKA, AKT1, AKT2, SGK, IRTK, ABL, and SRC

Inhibition of JNK1, JNK2, JNK3, IKK1, IKIC2EE, p38α, p38β, MKK3, MKK4, MKK6, MKK7, CDK2/E, CDK2/A, PKCα, ERK and PKA was determined by monitoring the transfer of radio-labled phosphate from ATP(γ33P) to a protein substrate, and precipitation of the product using trichloroacetic acid, as described in Bennett et al., Proc. Natl. Acad. Sci., 98:13681-13686 (2001). ATP was at 3 times the Kₘ for the relevant kinase.

Inhibition of the following kinases was monitored by the transfer of radiolabeled phosphate from ATP to a specific substrate peptide and capture of the peptide on P81 charged filter paper: AKT1, AKT2, and SGK. ATP was at the Kₘ for the relevant kinase. Activities for IRTK, ABL, and SRC were monitored by transfer of phosphate from ATP to a biotinylated peptide substrate and detection of the phosphorylated peptide using the LANCE technology (Perkin Elmer). ATP was at 3 times the Kₘ for the relevant kinase.

### Example 145 Serine/Threonine Kinase TCA Precipitation and SPA Assays

IKK1(his₆), IKK2(his₆. S177E,S181E), INK1(his₆), JNK2(his₆), JNK3(his₆), p38-2(gst), MEK6(gst), and MKK3(gst) were produced in house by expression in bacteria and purification by affinity tag chromatography. PKA α-catalytic subunit (BIOMOL SE-122), PKC-α (BIOMOL SE-143), MAP Kinase 1/ERK1 (Upstate Biotechnology 14-188) were purchased, and PKC-θ (his₆) was from Byk-Gulden. All kinase assays were carried out using ATP at a final concentration of three fold the apparent Km. Kinases were diluted in DB (20 mM HEPES pH 7.6, 0.1 mM EDTA, 2.5 mM MgCl₂, 0.004%(w/v) Triton X100, 2 µg/ml Leupeptin, 20 mM β-glycerol phosphate, 0.1 mM Na₃VO₄, 2 mM dithiothreitol) and mixed with the appropriate substrate to give the following final concentrations: 50 µg/ml IκBα(gst,1-54), (IKK1(his₆), IKK2(his₆, S177E,S181E)); 100 µg/ml c-Jun(gst,1-79), (JNK1 (his₆), JNK2(his₆); JNK3(his₆)), 100 µg/ml ATF2(gst), (p38-2(gst)), 50 µg/ml p38(gst), (MKK6); 100 µg/ml p38(gst,K108M), (MKK3); 100 µg/ml myelin basic protein (Upstate Biotechnology 13-104; PKA, ERK1, PKC-α) in HBB (20 mM HEPES pH 7.6, 50 mM NaCl, 0.1 mM EDTA, 2.5 mM MgCl₂, 0.05%(w/v) Triton X100). The enzyme/substrate mix was added to test compound dissolved in DB containing and DMSO to give a final DMSO concentration of 2%(v/v). Enzyme, substrate and compound were allowed to equilibrate at room temperature for 15 minutes. IKK1(his₆) and IKK2EE(his₆), reactions were started by addition of 1/10^{th} volume ATP in kinase buffer A (20 mM HEPES pH 7.6, 20 mM MgCl₂, 20 mM MnCl₂, 0.06%(w/v) Triton X100, 60 mM β-glycerol phosphate, 60 mM NaF, 6 mM dithiothreitol, 6 mM benzamidine, 48 mM para-nitrophenyl phosphate, 50 µCi/ml ³³P-ATP). JNK1(his₆), JNK2(his₆), JNK3(his_{b}), p38-2(gst), MEK(gst), and MKK3(gst) reactions were started by addition of 1/10^{th} volume ATP in kinase buffer B (130 mM MgCl₂, 6 mM dithiothreitol, 150 mM para-nitrophenyl phosphate, 50 µCi/ml ³³P-ATP). For all enzymes except PKC-θ(his₆) reactions were allowed to proceed for 60 minutes before quenching via precipitation with trichloroacetic acid at a final concentration of 7.2% for 30 minutes. Reaction products were collected onto glass microfilter (Millipore MAHF CIH60) 96-well plates using a Packard Filtermate, washed with Phosphate Buffered Saline and quantified by scintillation counting using a Packard Topcount. PKC-θ(his₆) reactions were allowed to progress for 60 minuted before being terminated by addition of an equal volume of 1.33mg/ml SPA beads suspended in 22.5 mM ATP, 0.12% Triton X100 and 6 mM EGTA. The SPA beads were allowed to equilibrate for one hour and the reaction product read using a Packard Topcount.

ATP competition studies for JNK2 were carried out using the same procedure except that ATP was used at 3 and 30 fold the Km.

Reversibility assays for JNK2 were carried out under essentially the same conditions, however kinase was prebound to Ni²⁺ chelate plates (Pierce 15242). Bound kinase was preincubated with compound in DB:HBB (2:3) plus 2% DMSO, for 30 minutes at room temperature. The Ni²⁺ plates were washed and compound eluted with three volumes HBB. Kinase reactions were started by addition of substrate and ATP and allowed to progress (with harvest and quantification) under conditions identical to the TCA precipitation assay described above. Controls contained compound during the reaction step.

### Example 146

### Tyrosine Kinase TRF assays

Zap70, Lck, and Lyn were provided by Pliva and EGFR was from BIOMOL (SE-116). Reactions were carried out in substrate coated 96 well plates (poly-glutamine-tyrosine at 50□g/well, Sigma P0275 Lck, Lyn, and EGFR; myelin basic protein 5 □g/well Upstate Biotechnology 13-1-4, Zap70.) Lck, Lyn, and EGFR kinase assays were carried out with ATP at a final concentration of three fold the apparent Km, in 25 mM HEPES pH 7.4, 10 mM MgCl2, 10 mM MnCl2, 0.2 mM DTT, 0.2% Tween 20, 2% DMSO, 1% BSA and 300 ng/ml Europium labeled anti-phosphotyrosine antibody (Wallac AD0038). Kinases were preincubated with compound for 15 minutes prior to starting the reaction by addition of ATP. Reactions were allowed to progress for 60 minutes. Plates were washed with 6 times with Phosphate Buffered Saline, 0.1 % Triton X100 followed by addition of 100 □l/well Enhancement Solution (Wallac 1244-105). Europium TRF was quantified on a Wallac Victor fluorimeter. Zap70 kinase reactions were carried out under the same conditions except that antibody and BSA were omitted. Plates were washed 3 times with Tris Buffered Saline, 0.02% Tween 20, blocked with Tris Buffered Saline, 3% BSA and incubated with Europium labeled anti-phosphotyrosine antibody (Wallac AD0038) in Tris Buffered Saline, 1% BSA, 0.02% Tween 20 for 30 min. Final washes and quantification were as described for Lck, Lyn and EGFR.

### Examples 147-235 Other Kinase Assays

The assays briefly described below in Examples 54-123 were carried out as described in Davies et al., Biochem. J., 351:95-105 (2000). All assays were carried out at 10 µM ATP unless otherwise noted.

### Kinase Dilution

All kinases are pre-diluted to a 10x working concentration prior to addition into the assay. The composition of the dilution buffer for each kinase is described below in Table 3.

In addition, the following abbreviations are used: h is human; r is rat; m is mouse; b is bovine; and y is yeast.

**Table 3**

| Buffer Compositions Used In Various Kinase Assays | |
|---|---|
| Buffer composition | Kinase(s) |
| 50 mM Tris pH 7.5, 0.1 mM EGTA, 0.1 mM NaVanadate, 0.1% β-mercaptoethanol, 1 mg/ml BSA | Blk, c-RAF, CSK, FGFR3, IGF-1R, IR, Lyn, MAPK1, MAPK2, MKK4, MKK6, MKK7β, SAPK2a, SAPK2b, SAPK3, SAPK4, Syk, and ZAP-70 |
| 50 mM Tris pH 7.5, 0.1 mM EGTA, 0.1% β-mercaptoethanol, 1 mg/ml BSA | JNK1α1, JNK2α2, JNK3, PRK2, and ROCK-II |
| 50 mM Tris pH 7.5, 0.05% β-mercaptoethanol, 1 mg/ml BSA | PDK1 |
| 25 mM Tris pH 7.5, 0.1 mM EGTA, 0.1 % β-mercaptoethanol, 1 mg/ml BSA | MEK1 |
| 20 mM MOPS pH 7.0, 1 mM EDTA, 0.1% β-mercaptoethanol, 0.01% Brij-35, 5% glycerol, 1 mg/ml BSA | ABL, CDK1/cyclinB, CDK2/cyclinA,CDK2/cyclinE, CDK3/cyclinE, CDK5/p35, CDK6/cyclinD3, CDK7/cyclinH/ MAT1, CHK1, CHK2, CK1, cSRC, Fes, Fyn, GSK3β, IKKα, IKKβ, Lck, MAPKAP-K2, MSK1, p70S6K, PAK2, PDGFRα, PDGFRβ, PKA, PKBα, PKBβ, PKCθ, Rsk1, Rsk2, Rsk3, SGK, and Yes |
| 20 mM Hepes pH 7.4, 0.15 M NaCl, 0.1 mM EGTA, 5 mM DTT, 0.1% Triton X-100, 50% glycerol | CK2 |
| 180 mM Hepes pH 7.4, 3.6 mM DTT, | AMPK |
| 0.07% Brij-35 | |
| 40 mM Hepes pH 7.4, 1 mg/ml BSA | CaMKII, CaMKIV |
| 20 mM Hepes pH 7.4, 0.03% Triton X-100 | PKCα, PKCβII, PKCγ, PKCε |
| 20 mM Na-β-glycerophosphate pH 7.5, 0.1% β-mercaptoethanol, 0.1 mM EGTA, 1 mg/ml BSA | PRAK |

### Substrates

All substrates are dissolved and diluted to working stocks in de-ionised water, apart from histone H1, which is diluted to a 10x working stock in 20 mM MOPS pH 7.4 prior to addition into the assay, and ATF2 which is typically stored at a 20x working stock in 50 mM Tris pH 7.5, 150 mM NaCl, 0.1 mM EGTA, 0.03% Brij-35, 50% glycerol, 1 mM benzamidine, 0.2 mM PMSF and 0.1 % β-mercaptoethanol.

### Example 147 SGK(h) Assay

In a final reaction volume of 25 µl, SGK(h) (5-10 mU) is incubated with 8 mM MOPS pH 7.0, 0.2 mM EDTA, 30 µM GRPRTSSFAEGKK, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 148 GSK3β (h) assay

In a final reaction volume of 25 µl, GSK3β (h) (5-10 mU) is incubated with 8 mM MOPS pH 7.0, 0.2 mM EDTA, 20 µM YRRAAVPPSPSLSRHSSPHQS(p)EDEEE (phospho GS2 peptide), 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 50 mM phosphoric acid and once in methanol prior to drying and scintillation counting.

### Example 149 AMPK(r) Assay

In a final reaction volume of 25 µl, AMPK(r) (5-10 mU) is incubated with 50 mM Hepes pH 7.4, 1 mM DTT, 0.02% Brij-35, 200 µM AMP, 200 µM AMARAASAAALARRR, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 150 CHK1(h) Assay

In a final reaction volume of 25 µl, CHK1(h) (5-10 mU) is incubated with 8 mM MOPS pH 7.0, 0.2 mM EDTA, 200 µM KKKVSRSGLYRSPSMPENLNRPR, 10 mM MgAcetate and [y-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 151 CK2(h) Assay

In a final reaction volume of 25 µl, CK2(h) (5-10 mU) is incubated with 20 mM Hepes pH 7.6, 0.15 M NaCl, 0.1 mM EDTA, 5 mM DTT, 0.1% Triton X-100, 165 µM RRRDDDSDDD, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 152 Lck(h) Assay

In a final reaction volume of 25 µl, Lck(h) (5-10 mU) is incubated with 50 mM Tris pH 7.5, 0.1 mM EGTA, 0.1 mM NaVanadate, 250 µM KVEKIGEGTYGVVYK (Cdc2 peptide), 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 153 CDK2/cyclinA (h) Assay

In a final reaction volume of 25 µl, CDK2/cyclinA (h) (5-10 mU) is incubated with 8 mM MOPS pH 7.0, 0.2 mM EDTA, 0.1 mg/ml histone H1, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 154 MAPK2 (m) Assay

In a final reaction volume of 25 µl, MAPK2 (m) (5-10 mU) is incubated with 25 mM Tris pH 7.5, 0.02 mM EGTA, 0.33 mg/ml myelin basic protein, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 155 SAPK2a (h) Assay

In a final reaction volume of 25 µl, SAPK2a (h) (5-10 mU) is incubated with 25 mM Tris pH 7.5, 0.02 mM EGTA, 0.33 mg/ml myelin basic protein, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 156 SAPK2b (h) Assay

In a final reaction volume of 25 µl, SAPK2b (h) (5-10 mU) is incubated with 25 mM Tris pH 7.5, 0.02 mM EGTA, 0.33 mg/ml myelin basic protein, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 157 SAPK3 (h) Assay

In a final reaction volume of 25 µl, SAPK3 (h) (5-10 mU) is incubated with 25 mM Tris pH 7.5, 0.02 mM EGTA, 0.33 mg/ml myelin basic protein, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 158 SAPK4 (h) Assay

In a final reaction volume of 25 µl, SAPK4 (h) (5-10 mU) is incubated with 25 mM Tris pH 7.5, 0.02 mM EGTA, 0.33 mg/ml myelin basic protein, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 159 MSK1 (h) Assay

In a final reaction volume of 25 µl, MSK1 (h) (5-10 mU) is incubated with 8 mM MOPS pH 7.0, 0.2 mM EDTA, 30 µM GRPRTSSFAEGKK, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 160 PKBα (h) Assay

In a final reaction volume of 25 µl, PKBα (h) (5-10 mU) is incubated with 8 mM MOPS pH 7.0,0.2 mM EDTA, 30 µM GRPRTSSFAEGKK, 10 mM MgAcetate and [γ-³³P-ATP] (Specific activity approx. 500 cpm/pmol, concentration as required). The reaction is initiated by the addition of Mg²⁺ [γ³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 161 ROCK-II (r) Assay

In a final reaction volume of 25 µl, ROCK-II (r) (5-10 mU) is incubated with 50 mM Tris pH 7.5, 0.1 mM EGTA, 30 µM KEAKEKRQEQIAKRRRLSSLRASTSKSGGSQK, 10 mM MgAcetate and [γ-³³P-ATP] (Specific activity approx. 500 cpm/pmol, concentration as required). The reaction is initiated by the addition of Mg²⁺ [γ³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 162 p70S6K (h) Assay

In a final reaction volume of 25 µl, p70S6K (h) (5-10 mU) is incubated with 8 mM MOPS pH 7.0, 0.2 mM EDTA, 100 µM KKRNRTLTV, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 163 PKA (b) Assay

In a final reaction volume of 25 µl, PKA (b) (5-10 mU) is incubated with 8 mM MOPS pH 7.0, 0.2 mM EDTA, 30 µM LRRASLG (Kemptide), 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 50 mM phosphoric acid and once in methanol prior to drying and scintillation counting.

### Example 164 MAPKAP-K2 (h) Assay

In a final reaction volume of 25 µl, MAPKAP-K2 (h) (5-10 mU) is incubated with 50 mM Na-β-glycerophosphate pH 7.5, 0.1 mM EGTA, 30 µM KKLNRTLSVA, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 165 JNK1α1 (h) Assay

In a final reaction volume of 25 µl, JNKL1α1 (h) (5-10 mU) is incubated with 50 mM Tris pH 7.5, 0.1 mM EGTA, 0.1% β-mercaptoethanol, 3 µM ATF2, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 166 JNK2α2 (h) Assay

In a final reaction volume of 25 µl, JNK2α2 (h) (5-10 mU) is incubated with 50 mM Tris pH 7.5, 0.1 mM EGTA, 0.1% β-mercaptoethanol, 3 µM ATF2, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 167 JNK3 (r) Assay

In a final reaction volume of 25 µl, JNK3 (r) (5-10 mU) is incubated with 50 mM Tris pH 7.5, 0.1 mM EGTA, 0.1% β-mercaptoethanol, 250 µM peptide, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 168 PRAK (h) Assay

In a final reaction volume of 25 µl, PRAK (h) (5-10 mU) is incubated with 50 mM Na-β-glycerophosphate pH 7.5, 0.1 mM EGTA, 30 µM KKLRRTLSVA, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 50 mM phosphoric acid and once in methanol prior to drying and scintillation counting.

### Example 169 CHK2 (h) Assay

In a final reaction volume of 25 µl, CHK2 (h) (5-10 mU) is incubated with 8 mM MOPS pH 7.0, 0.2 mM EDTA, 200 µM KKKVSRSGLYRSPSMPENLNRPR, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 170 MAPK1 (h) Assay

In a final reaction volume of 25 µl, MAPK1 (h) (5-10mU) is incubated with 25 mM Tris pH 7.5, 0.02 mM EGTA, 250 µM peptide, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 171 c-RAF (h) Assay

In a final reaction volume of 25 µl, c-RAF (h) (5-10 mU) is incubated with 25 mM Tris pH 7.5, 0.02 mM EGTA, 0.66 mg/ml myelin basic protein, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 172 CDK1/cyclinB (h) Assay

In a final reaction volume of 25 µl, CDK1/cyclinB (h) (5-10 mU) is incubated with 8 mM MOPS pH 7.0, 0.2 mM EDTA, 0.1 mg/ml histone H1, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 173 cSRC (h) Assay

In a final reaction volume of 25 µl, cSRC (h) (5-10 mU) is incubated with 8 mM MOPS pH 7.0, 0.2 mM EDTA, 250 µM KVEKIGEGTYGVVYK (Cdc2 peptide), 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 174 CaMKII (r) Assay

In a final reaction volume of 25 µl, CaMKII (r) (5-10 mU) is incubated with 40 mM Hepes pH 7.4, 5 mM CaCl₂, 30 µg/ml calmodulin, 30 µM KKLNRTLSVA, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 175 PRK2 (h) Assay

In a final reaction volume of 25 µl, PRK2 (h) (5-10 mU) is incubated with 50 mM Tris pH 7.5, 0.1 mM EGTA, 0.1% β-mercaptoethanol, 30 µM AKRRRLSSLRA, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 176 PDK1 (h) Assay

In a final reaction volume of 25 µl, PDK1 (h) (5-10 mU) is incubated with 50 mM Tris pH 7.5, 100 µM KTFCGTPEYLAPEVRREPRILSEEEQEMFRDFDYIADWC (PDKtide), 0.1% β-mercaptoethanol, 10 mM MgAcetate and [γ-³³P-ATP] (Specific activity approx. 500 cpm/pmol, concentration as required). The reaction is initiated by the addition of Mg²⁺ [γ-³³p-ATp]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 177 Fyn (h) Assay

In a final reaction volume of 25 µl, Fyn (h) (5-10 mU) is incubated with 50 mM Tris pH 7.5, 0.1 mM EGTA, 0.1 mM NaVanadate, 250 µM KVEKIGEGTYGWYK (Cdc2 peptide), 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 178 PKCα (h) Assay

In a final reaction volume of 25 µl, PKCα (h) (5-10 mU) is incubated with 20 mM Hepes pH 7.4, 0.03% Triton X-100, 0.1 mM CaCl₂, 0.1 mg/ml phosphatidylserine, 10 µg/ml diacylglycerol, 0.1 mg/ml histone H1, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 179 PKCβII (h) Assay

In a final reaction volume of 25 µl, PKCβII (h) (5-10 mU) is incubated with 20 mM Hepes pH 7.4, 0.03% Triton X-100, 0.1 mM CaCl₂, 0.1 mg/ml phosphatidylserine, 10 µg/ml diacylglycerol, 0.1 mg/ml histone H1, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 180 PKCγ (h) Assay

In a final reaction volume of 25 µl, PKCγ (h) (5-10 mU) is incubated with 20 mM Hepes pH 7.4, 0.03% Triton X-100, 0.1 mM CaCl₂, 0.1 mg/ml phosphatidylserine, 10 µg/ml diacylglycerol, 0.1 mg/ml histone H1, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 181 CK1 (y) Assay

In a final reaction volume of 25 µl, CK1 (y) (5-10 mU) is incubated with 8 mM MOPS pH 7.0, 0.2 mM EDTA, 200 µM KRRRALS(p)VASLPGL, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 182 ZAP-70 (h) Assay

In a final reaction volume of 25 µl, ZAP-70 (h) (5-10 mU) is incubated with 50 mM Tris pH 7.5, 0.1 mM EGTA, 0.1 mM NaVanadate, 0.1% β-mercaptoethanol, 0.1 mg/ml poly(Glu, Tyr) 4:1, 10 mM MnCl₂, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a Filtermat A and washed three times for 5 minutes in 75 mM phosphoric acid and once in methanol prior to drying and scintillation counting.

### Example 183 MEK1 (h) Assay

In a final reaction volume of 25 µl, MEK1 (h) (1-5 mU) is incubated with 50 mM Tris pH 7.5, 0.2 mM EGTA, 0.1% β-mercaptoethanol, 0.01% Brij-35, 1 µM inactive MAPK2 (m), 10 mM MgAcetate and cold ATP (concentration as required). The reaction is initiated by the addition of the MgATP. After incubation for 40 minutes at room temperature, 5 µl of this incubation mix is used to initiate a MAPK2 (m) assay, which is described on page 7 of this book.

### Example 184 MKK4 (m) Assay

In a final reaction volume of 25 µl, MKK4 (m) (1-5 mU) is incubated with 50 mM Tris pH 7.5, 0.1 mM EGTA, 0.1% β-mercaptoethanol, 0.1 mM NaVanadate, 2 µM inactive JNK1α1 (h), 10 mM MgAcetate and cold ATP (concentration as required). The reaction is initiated by the addition of the MgATP. After incubation for 40 minutes at room temperature, 5 µl of this incubation mix is used to initiate a JNK1α1 (h) assay, which is exactly as described on page 10 of this book except that ATF2 is replaced with 250 µM peptide.

### Example 185 MKK7β (h) Assay

In a final reaction volume of 25 µl, MKK7β (h) (1-5 mU) is incubated with 50 mM Tris pH 7.5, 0.1 mM EGTA, 0.1% β-mercaptoethanol, 0.1 mM NaVanadate, 2 µM inactive JNK1α1 (h), 10 mM MgAcetate and cold ATP (concentration as required). The reaction is initiated by the addition of the MgATP. After incubation for 40 minutes at room temperature, 5 µl of this incubation mix is used to initiate a JNK1α1 (h) assay, which is exactly as described on page 10 of this book except that ATF2 is replaced with 250 µM peptide.

### Example 186 MKK6 (h) Assay

In a final reaction volume of 25 µl, MKK6 (h) (1-5 mU) is incubated with 50 mM Tris pH 7.5, 0.1 mM EGTA, 0.1% β-mercaptoethanol, 0.1 mM NaVanadate, 1 mg/ml BSA, 1 µM inactive SAPK2a (h), 10 mM MgAcetate and cold ATP (concentration as required). The reaction is initiated by the addition of the MgATP. After incubation for 40 minutes at room temperature, 5 µl of this incubation mix is used to initiate a SAPK2a (h) assay, which is described on page 8 of this book.

### Example 187 IKKα (h) Assay

In a final reaction volume of 25 µl, IKKα (h) (5-10 mU) is incubated with 8 mM MOPS pH 7.0, 0.2 mM EDTA, 200 µM peptide, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 188 IKKβ (h) Assay

In a final reaction volume of 25 µl, IKKβ (h) (5-10 mU) is incubated with 8 mM MOPS pH 7.0, 0.2 mM EDTA, 100 µM peptide, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 189 PKCθ (h) Assay

In a final reaction volume of 5 µl, PKCθ (5-10 mU) is incubated with 8 mM MOPS pH 7.0, 0.2 mM EDTA, 0.1 mg/ml histone H1, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 190 CaMKIV (h) Assay

In a final reaction volume of 25 µl, CaMKIV (h) (5-10 mU) is incubated with 40 mM Hepes pH 7.4, 5 mM CaCl₂, 30 µg/ml calmodulin, 30 µM KKLNRTLSVA, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 191 Blk (m) Assay

In a final reaction volume of 25 µl, Blk (m) (5-10 mU) is incubated with 50 mM Tris pH 7.5, 0.1 mM EGTA, 0.1 mM NaVanadate, 0.1% β-mercaptoethanol, 0.1 mg/ml poly(Glu, Tyr) 4:1, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a Filtermat A and washed three times for 5 minutes in 75 mM phosphoric acid and once in methanol prior to drying and scintillation counting.

### Example 192 Syk (h) Assay

In a final reaction volume of 25 µl, Syk (h) (5-10 mU) is incubated with 50 mM Tris pH 7.5, 0.1 mM EGTA, 0.1 mM NaVanadate, 0.1% β-mercaptoethanol, 0.1 mg/ml poly(Glu, Tyr) 4:1, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a Filtermat A and washed three times for 5 minutes in 75 mM phosphoric acid and once in methanol prior to drying and scintillation counting.

### Example 193 CSK (h) Assay

In a final reaction volume of 25 µl, CSK (h) (5-10 mU) is incubated with 50 mM Tris pH 7.5, 0.1 mM EGTA, 0.1 mM NaVanadate, 0.1% β-mercaptoethanol, 0.1 mg/ml poly(Glu, Tyr) 4:1, 10 mM MnCl₂, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a Filtermat A and washed three times for 5 minutes in 75 mM phosphoric acid and once in methanol prior to drying and scintillation counting.

### Example 194 Lyn (m) Assay

In a final reaction volume of 25 µl, Lyn (m) (5-10 mU) is incubated with 50 mM Tris pH 7.5, 0.1 mM EGTA, 0.1 mM NaVanadate, 0.1% β-mercaptoethanol, 0.1 mg/ml poly(Glu, Tyr) 4:1, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a Filtermat A and washed three times for 5 minutes in 75 mM phosphoric acid and once in methanol prior to drying and scintillation counting.

### Example 195 CDK3/cyclinE (h) Assay

In a final reaction volume of 25 µl, CDK3/cyclinE (h) (5-10 mU) is incubated with 8 mM MOPS pH 7.0, 0.2 mM EDTA, 0.1 mg/ml histone H1, 10 mM MgAcetate and [γ-³³P-ATP] (Specific activity approx. 500 cpm/pmol, concentration as required). The reaction is initiated by the addition of Mg²⁺ [γ-³³P-ATP]. After incubation.for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 196 CDK5/p35 (h) Assay

In a final reaction volume of 25 µl, CDK5/p35 (h) (5-10 mU) is incubated with 8 mM MOPS pH 7.0, 0.2 mM EDTA, 0.1 mg/ml histone H1, 10 mM MgAcetate and [y-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 197 CDK2/cyclinE (h) Assay

In a final reaction volume of 25 µl, CDK2/cyclinE (h) (5-10 mU) is incubated with 8 mM MOPS pH 7.0, 0.2 mM EDTA, 0.1 mg/ml histone H1, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 198 CDK6/cyclinD3 (h) Assay

In a final reaction volume of 25 µl, CDK6/cyclinD3 (h) (5-10 mU) is incubated with 8 mM MOPS pH 7.0, 0.2 mM EDTA, 0.1 mg/ml histone H1, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 199 CDK7/cyclinH/MAT1 (h) Assay

In a final reaction volume of 25 µl, CDK7/cyclinH/MAT1 (h) (5-10 mU) is incubated with 8 mM MOPS pH 7.0, 0.2 mM EDTA, 500 µM peptide, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 200 Rsk3 (h) Assay

In a final reaction volume of 25 µl, Rsk3 (h) (5-10 mU) is incubated with 8 mM MOPS pH 7.0, 0.2 mM EDTA, 30 µM KKKNRTLSVA, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 201 IR (h) Assay

In a final reaction volume of 25 µl, IR (h) (5-10 mU) is incubated with 50 mM Tris pH 7.5, 0.1 mM EGTA, 0.1 mM NaVanadate, 0.1% β-mercaptoethanol, 250 µM KKSRGDYMTMQIG, 10 mM MnCl₂, 10 mM MgAcetate and [γ³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 202 IGF-1R (h) Assay

In a final reaction volume of 25 µl, IGF-1R (h) (5-10 mU) is incubated with 50 mM Tris pH 7.5, 0.1 mM EGTA, 0.1 mM NaVanadate, 0.1% β-mercaptoethanol, 250 µM KKKSPGEYVNIEFG, 10 mM MnCl₂, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 203 PKBβ (h) Assay

In a final reaction volume of 25 µl, PKBβ (h) (5-10 mU) is incubated with 8 mM MOPS pH 7.0, 0.2 mM EDTA, 30 µM GRPRTSSFAEGKK, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 204 FGFR3 (h) Assay

In a final reaction volume of 25 µl, FGFR3 (h) (5-10 mU) is incubated with 50 mM Tris pH 7.5, 0.1 mM EGTA, 0.1 mM NaVanadate, 0.1% β-mercaptoethanol, 0.1 mg/ml poly(Glu, Tyr) 4:1, 10 mM MnCl₂, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a Filtermat A and washed three times for 5 minutes in 75 mM phosphoric acid and once in methanol prior to drying and scintillation counting.

### Example 205 PDGFRα(h) Assay

In a final reaction volume of 25 µl, PDGFRα (h) (5-10 mU) is incubated with 8 mM MOPS pH 7.0, 0.2 mM EDTA, 0.1 mg/ml poly(Glu, Tyr) 4:1, 10 mM MnCl₂, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a Filtermat A and washed three times for 5 minutes in 75 mM phosphoric acid and once in methanol prior to drying and scintillation counting.

### Example 206 PDGFRβ(h) Assay

In a final reaction volume of 25 µl, PDGFRβ (h) (5-10 mU) is incubated with 8 mM MOPS pH 7.0, 0.2 mM EDTA, 0.1 mg/ml poly(Glu, Tyr) 4:1, 10 mM MnCl₂, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a Filtermat A and washed three times for 5 minutes in 75 mM phosphoric acid and once in methanol prior to drying and scintillation counting.

### Example 207 MAPK2(h) Assay

In a final reaction volume of 25 µl, MAPK2(h) (5-10 mU) is incubated with 25 mM Tris pH 7.5, 0.02 mM EGTA, 0.33 mg/ml myelin basic protein, 10 mM MgAcetate and [γ-³³P-ATP] (Specific activity approx. 500 cpm/pmol, concentration as required). The reaction is initiated by the addition of Mg²⁺ [y-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 208 ROCK-II(h) Assay

In a final reaction volume of 25 µl, ROCK-11 (h) (5-10 mU) is incubated with 50 mM Tris pH 7.5, 0.1 mM EGTA, 30 µM KEAKEKRQEQIAKRRRLSSLRASTSKSGGSQK, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 209 PKA(h) Assay

In a final reaction volume of 25 µl, PKA(h) (5-10 mU) is incubated with 8 mM MOPS pH 7.0, 0.2 mM EDTA, 30 µM LRRASLG (Kemptide), 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 50 mM phosphoric acid and once in methanol prior to drying and scintillation counting.

### Example 210 Rsk1(r) Assay

In a final reaction volume of 25 µl, Rsk1(r) (5-10 mU) is incubated with 8 mM MOPS pH 7.0, 0.2 mM EDTA, 30 µM KKKNRTLSVA, 10 mM MgAcetate and [γ-³³P-ATP] (Specific activity approx. 500 cpm/pmol, concentration as required). The reaction is initiated by the addition of Mg²⁺ [y-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 211 Rsk2(h) Assay

In a final reaction volume of 25 µl, Rsk2(h) (5-10 mU) is incubated with 8 mM MOPS pH 7.0, 0.2 mM EDTA, 30 µM KKKNRTLSVA, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 212 PAK2(h) Assay

In a final reaction volume of 25 µl, PAK2(h) (5-10 mU) is incubated with 8 mM MOPS pH 7.0, 0.2 mM EDTA, 30 µM KEAKEKRQEQIAKRRRLSSLRASTSKSGGSQK, 10 mM MgAcetate and [µ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 213 Fes(h) Assay

In a final reaction volume of 25 µl, Fes(h) (5-10 mU) is incubated with 8 mM MOPS pH 7.0, 0.2 mM EDTA, 0.1 mg/ml poly(Glu, Tyr) 4:1, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a Filtermat A and washed three times for 5 minutes in 75 mM phosphoric acid and once in methanol prior to drying and scintillation counting.

### Example 214 Yes(h) Assay

In a final reaction volume of 25 µl, Yes(h) (5-10 mU) is incubated with 8 mM MOPS pH 7.0, 0.2 mM EDTA, 0.1 mg/ml poly(Glu, Tyr) 4:1, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a Filtermat A and washed three times for 5 minutes in 75 mM phosphoric acid and once in methanol prior to drying and scintillation counting.

### Example 215 ABL(m) Assay

In a final reaction volume of 25 µl, ABL(m) (5-10 mU) is incubated with 8 mM MOPS pH 7.0, 0.2 mM EDTA, 50 µM EAIYAAPFAKKK, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 216 PKCε(h) Assay

In a final reaction volume of 25 µl, PKCε(h) (5-10 mU) is incubated with 20 mM Hepes pH 7.4, 0.03% Triton X-100, 0.1 mg/ml phosphatidylserine, 10 µg/ml diacylglycerol, 50 µM ERMRPRKRQGSVRRRV, 10 mM MgAcetate and [γ-³³P-ATP] (Specific activity approx. 500 cpm/pmol, concentration as required). The reaction is initiated by the addition of Mg²⁺ [γ-³³P-ATP]. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Example 217 Kinase Binding Affinities For Illustrative Indazole Compounds

Using the appropriate assay protocols as listed above, kinase inhibition data for Compounds 21 and 71 was determined for the following kinases: CDK1/cyclin B, CDK2/cyclin A, cSRC, Yes, MEK1, and Rskl. The Results are presented below in Table 4.

**Table 4**

| Compound (3µM) | CDK1/cyclin B (% control) | CDK2/cyclin A (% control) | cSRC (% control) | Yes (% control) | MEK1 (% control) | Rsk1 (% control) |
|---|---|---|---|---|---|---|
| **20** | 2 | 4 | 2 | 2 | 4 | 4 |
| **92** | 1 | 2 | 67 | 24 | 11 | 29 |

All experiments were performed at [ATP] = 10 µM

The examples above illustrate assays that may readily be performed to determine the ability of the Indazole Compounds to modulate the activity of various kinases. It will be apparent that such assays or other suitable assays known in the art may be used to select an Indazole Compound having a desired level of activity against a selected target kinase.

The invention can be summarized by the following items:
1. A compound having the formula: 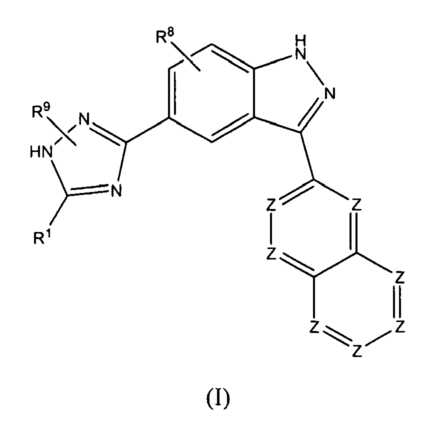 and isomers, prodrugs and pharmaceutically acceptable salts, solvates and hydrates thereof,
wherein:
R¹ is -H, -halo, -CN, -C₁-C₆ alkyl, -C₁-C₆ alkenyl, -C₁-C₆ alkynyl, -OR³, -N(R⁴)₂, -CN, -NO₂, -C(O)R⁵, -OC(O)R⁵, -NHC(O)R⁵, -SO₂R⁶, -aryl, -heterocycle, -heteroaryl, -cycloalkyl, -(C₁-C₆ alkylene)-R² or -O-(C₁-C₆ alkylene)-R²;
R² is -H, -halo, -C₁-C₆ alkyl, -C₁-C₆ alkenyl, -C₁-C₆ alkynyl, -OR³, -N(R⁴)₂, -CN, -NO₂, -C(O)R⁵, -OC(O)R⁵, -NHC(O)R⁵, -SO₂R⁶, -aryl, -heterocycle, -heteroaryl, or -cycloalkyl;
R³ is independently -H, -C₁-C₆ alkyl, -C₁-C₆ alkenyl, -C₁-C₆ alkynyl, -C₁-C₆ haloalkyl, -cycloalkyl, -aryl, or -heterocycle;
each occurrence of R⁴ is independently -H, -C₁-C₆ alkyl, -C₁-C₆ alkenyl, -C₁-C₆ alkynyl, -cycloalkyl, -aryl, -heterocycle, or -(C₁-C₆ alkylene)-OR³;
R⁵ is -H, -C₁-C₆ alkyl, -C₁-C₆ alkenyl, -C₁-C₆ alkynyl, -cycloalkyl, -aryl, -heterocycle, -OR³, -N(R⁴)₂,
R⁶ is -H, -C₁-C₆ alkyl, -C₁-C₆ alkenyl, -C₁-C₆ alkynyl, -N(R⁴)₂, -cycloalkyl, -aryl, or -heterocycle;
each occurrence of Z is -C(R⁷)- or -N-, wherein up to 3 occurences of Z can be -N-;
R⁷ is -H, -halo, -C₁-C₆ alkyl, -C₁-C₆ haloalkyl, -O-(C₁-C₆ haloalkyl), -C₁-C₆ alkenyl, -C₁-C₆ alkynyl, -OR³, -N(R⁴)₂, -CN, -NO₂, -C(O)R⁵, -OC(O)R⁵, -NHC(O)R⁵, -SO₂R⁶, -aryl, -heterocycle, -cycloalkyl, -C(O)NH-(C₁-C₆ alkylene)ₙ-cycloalkyl, -C(O)NH-(C₁-C₆ alkylene)ₙ-aryl, -C(O)NH-(C₁-C₆ alkylene )ₙ-heterocycle, -(C₁-C₆ alkylene)-cycloalkyl, -(C₁-C₆ alkylene)-aryl, -(C₁-C₆ alkylene)-heterocycle, -O-(C₁-C₆ alkylene)-C(O)R⁵, -O-(C₁-C₆ alkylene)-N(R⁴)₂, -O-(C₁-C₆ alkylene)-cycloalkyl, -O-(C₁-C₆ alkylene)-aryl, or
   -O-(C₁-C₆ alkylene)-heterocycle, wherein R⁷ is attached to the bicyclic ring system via a carbon atom and n is 0 or 1;
R⁸ is -H, -halo, -C₁-C₆ alkyl, -C₁-C₆ haloalkyl, -O-(C₁-C₆ haloalkyl), -C₁-C₆ alkenyl, -C₁-C₆ alkynyl, -OR³, -N(R⁴)₂, -CN, -NO₂, -C(O)R⁵, -OC(O)R⁵, -NHC(O)R⁵, -SO₂R⁶, -aryl, -heterocycle, or -cycloalkyl; and
R⁹ is -H, -C₁-C₆ alkyl, or cycloalkyl.
2. A compound having the formula: 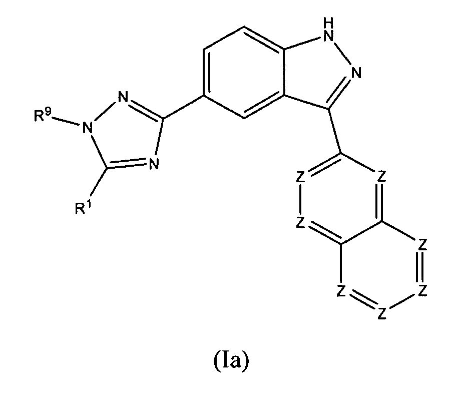 and isomers, prodrugs and pharmaceutically acceptable salts, solvates and hydrates thereof, wherein:
R¹ is -H, -halo, -C₁-C₆ alkyl, -C₁-C₆ alkenyl, -C₁-C₆ alkynyl, -OR³, -N(R⁴)₂, -CN, -NO₂, -C(O)R⁵, -OC(O)R⁵, -NHC(O)R⁵, -SO₂R⁶, -aryl, -heterocycle, -heteroaryl, -cycloalkyl, -(C₁-C₆ alkylene)-R² or -O-(C₁-C₆ alkylene)-R²;
R² is -H, -halo, -C₁-C₆ alkyl, -C₁-C₆ alkenyl, -C₁-C₆ alkynyl, -OR³, -N(R⁴)₂, -CN, -NO₂, -C(O)R⁵, -OC(O)R⁵, -NHC(O)R⁵, -SO₂R⁶, -aryl, - heterocycle, -heteroaryl, or -cycloalkyl;
R³ is independently -H, -C₁-C₆alkyl, -C₁-C₆ alkenyl, -C₁-C₆ alkynyl, -C₁-C₆ haloalkyl, -cycloalkyl, -aryl, or -heterocycle;
each occurrence of R⁴ is independently -H, -C₁-C₆ alkyl, -C₁-C₆ alkenyl, -C₁-C₆ alkynyl, -cycloalkyl, -aryl, -heterocycle, or -(C₁-C₆ alkylene)-OR³;
R⁵ is -H, -C₁-C₆ alkyl, -C₁-C₆ alkenyl, -C₁-C₆ alkynyl, -cycloalkyl, -aryl, -heterocycle, -OR³, -N(R⁴)₂,
R⁶ is -H, -C₁-C₆ alkyl, -C₁-C₆ alkenyl, -C₁-C₆ alkynyl, -N(R⁴)₂, -cycloalkyl, -aryl, or -heterocycle;
each occurrence of Z is -C(R⁷)- or -N-, wherein up to 3 occurences of Z can be -N-;
R⁷ is -H, -halo, -C₁-C₆ alkyl, -C₁-C₆ haloalkyl, -O-(C₁-C₆ haloalkyl), -C₁-C₆ alkenyl, -C₁-C₆ alkynyl, -OR³, -N(R⁴)₂, -CN, -NO₂, -C(O)R⁵, -OC(O)R⁵, -NHC(O)R⁵, -SO₂R⁶, -aryl, -heterocycle, -cycloalkyl, -C(O)NH-(C₁-C₆ alkylene)ₙ-cycloalkyl, -C(O)NH-(C₁-C₆ alkylene)ₙ-aryl, -C(O)NH-(C₁-C₆ alkylene)ₙ-heterocycle, -(C₁-C₆ alkylene)-cycloalkyl, -(C₁-C₆ alkylene)-aryl, -(C₁-C₆ alkylene)-heterocycle, -O-(C₁-C₆ alkylene)-C(O)R⁵, -O-(C₁-C₆ alkylene)-N(R⁴)₂, -O-(C₁-C₆ alkylene)-cycloalkyl, -O-(C₁-C₆ alkylene)-aryl, or -O-(C₁-C₆ alkylene)-heterocycle, wherein R⁷ is attached to the bicyclic ring system via a carbon atom and n is 0 or 1; and
R⁹ is -H, -C₁-C₆ alkyl, or cycloalkyl.
3. A compound having the formula: 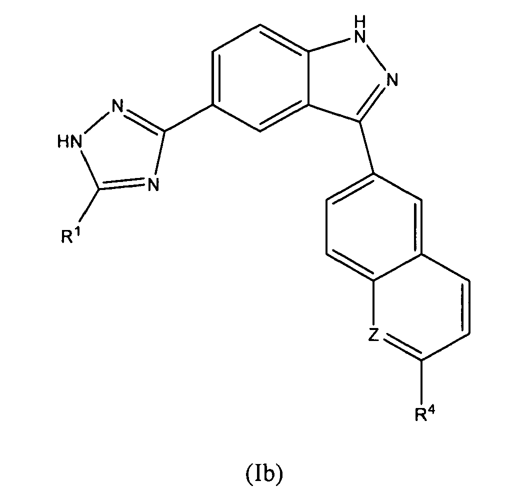 and isomers, prodrugs and pharmaceutically acceptable salts, solvates and hydrates thereof, wherein:
R¹ is -H, -C₁-C₆ alkyl, -(C₁-C₆ alkylene)-R² or -O-(C₁-C₆ alkylene)-R²;
R² is -C₁-C₆ alkyl, -C₁-C₆ alkoxy, -OH, -N(R³)₂, -aryl, -heteroaryl, -heterocycle, or -cycloalkyl;
each occurrence of R³ is independently -H, -C₁-C₆ alkyl, or -C₁-C₆ alkylene-(C₁-C₆ alkoxy);
R⁴ is -N(R⁵)₂, -O-C₁-C₆ alkyl, -C(O)NH-(C₁-C₆ alkylene)ₘ-heterocycle, -C(O)-heterocycle, -C(O)NH-(C₁-C₆ alkylene)ₘ-heteroaryl, -C(O)-heteroaryl, -(C₁-C₆ alkylene)-cycloalkyl, -O-(C₁-C₆ alkylene)-N(R⁵)₂, -O-(C₁-C₆ alkylene)ₘ-heterocycle, -O-(C₁-C₆ alkylene)ₘ-heteroaryl, -O-(C₁-C₆ alkylene)ₘ-cycloalkyl or -O-(C₁-C₆ alkylene)ₘ-C(O)R⁵;
Z is -CH- or -N-;
each occurrence of R⁵ is independently -H or -C₁-C₆ alkyl; and
m is 0 or 1.
4. A compound having the formula: 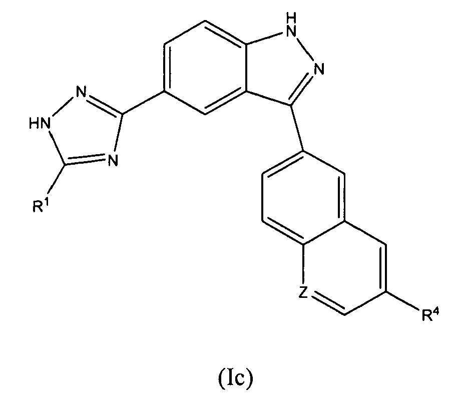 and isomers, prodrugs and pharmaceutically acceptable salts, solvates and hydrates thereof, wherein:
R¹ is -H, -(C₁-C₆ alkylene)-R² or -O-(C₁-C₆ alkylene)-R²;
R² is -C₁-C₆ alkyl, -C₁-C₆ alkoxy, -OH, -N(R³)₂, -aryl, -heteroaryl, -heterocycle, or -cycloalkyl;
each occurrence of R³ is independently -H, -C₁-C₆ alkyl, or -C₁-C₆ alkylene-(C₁-C₆ alkoxy);
R⁴ is -C(O)NH-(C₁-C₆ alkylene)ₘ-heterocycle, -C(O)-heterocycle, -C(O)NH-(C₁-C₆ alkylene)ₘ-heteroaryl, -C(O)-heteroaryl, -(C₁-C₆ alkylene)-cycloalkyl, -O-(C₁-C₆ alkylene)-N(R⁵)₂, -O-(C₁-C₆ alkylene)ₘ-heterocycle, -O-(C₁-C₆ alkylene)ₘ-heteroaryl,
   -O-(C₁-C₆ alkylene)ₘ-cycloalkyl or -O-(C₁-C₆ alkylene)ₘ-C(O)R⁵;
Z is -CH- or -N-;
each occurrence of R⁵ is independently -H or -C₁-C₆ alkyl; and
m is 0 or 1.
5. A compound having the formula: 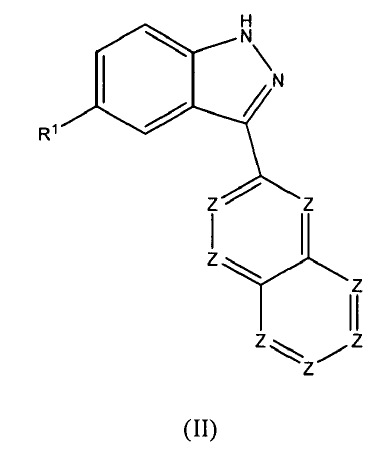 and isomers, prodrugs and pharmaceutically acceptable salts, solvates and hydrates thereof,
wherein:
R¹ is -COOR², -CN, -NO₂, -C(O)-heterocycle, -CH=CH-heterocycle, -C(O)N(R²)₂, or -1*H-*tetrazol-5-yl;
each occurrence of R² is independently -H, -C₁-C₆ alkyl, -hydroxyalkyl, -C₁-C₆ alkyl-N(R²)₂, -C₁-C₆ alkyl-O-C₁-C₆ alkyl, -(CH₂)ₙ-cycloalkyl, -(CH₂)ₙ-heteroaryl, -(CH₂)ₙ-heterocycle, or -(CH₂)ₙ-aryl;
each occurrence of Z is -C(R³)- or -N-, wherein up to 3 occurrences of Z can be -N-;
R³ is -COOR², -CN, -NO₂, -C(O)N(R²)₂, N(R²)₂, -C(O)O-(C₁-C₆ alkyl), -C(O)NH-(CH₂)ₙ-heterocycle, -C(O)NH-(CH₂)ₙ-heteroaryl, -C(O)-heterocycle, -C(O)-heteroaryl, -(CH₂)ₙ-heterocycle, -(CH₂)ₙ-heteroaryl, -(CH₂)ₚ-cycloalkyl, -O-(CH₂)ₙ- N(R²)₂, -O-(CH₂)ₙ-heterocycle), -O-(CH₂)ₙ-heteroaryl, or -O-(CH₂)ₙ-cycloalkyl);
m is 0 or 1;
n is an integer ranging from 0 to 3; and
p is an integer ranging from 1 to 3.
6. The compound of item 1 wherein -Z is -CH-.
7. The compound of item 1 wherein R¹ is C₁-C₆ alkyl.
8. The compound of item 1 wherein R¹ is -(C₁-C₆ alkylene)-heterocycle.
9. The compound of item 1 wherein R¹ is -CH₂-heterocycle.
10. The compound of item 1 wherein R¹ is -(C₁-C₆ alkylene)-cycloalkyl.
11. The compound of item 1 wherein R⁷ is -H.
12. The compound of item 1 wherein R⁷ is -halo.
13. The compound of item 1 wherein R⁷ is -O-C₁-C₆ alkyl.
14. The compound of item 1 wherein R⁷ is -O-(C₁-C₆ alkylene)-heterocycle.
15. The compound of item 1 wherein R⁷ is -O-(C₁-C₆ alkylene)-heterocycle.
16. The compound of item 1 wherein R⁸ and R⁹ each -H.
17. The compound of item 2 wherein Z is -CH-.
18. The compound of item 2 wherein R¹ is -C₁-C₆ alkyl.
19. The compound of item 18 wherein R¹ is methyl, isopropyl, isobutyl, *t*-butyl or isopentyl.
20. The compound of item 2 wherein R¹ is -(C₁-C₆ alkylene)-heterocycle.
21. The compound of item 20 wherein R¹ is -CH(CH₃)-(pyrrolidin-1-yl).
22. The compound of item 2 wherein R¹ is -CH₂-heterocycle.
23. The compound of item 22 wherein R¹ is -CH₂-(pyrrolidin-1-yl), -CH₂-(piperidin-1-yl), -CH₂-(morpholin-1-yl), -CH₂-(2-methyl-pyrrolidin-1-yl), -CH₂-(2-methyl-piperidin-1-yl), -CH₂-(2,6-dimethyl-piperidin-1-yl), -CH₂-(4-methyl-piperidin-1-yl), or -CH₂-(azepan-1-yl).
24. The compound of item 2 wherein R¹ is -O-(C₁-C₆ alkylene)-heterocycle.
25. The compound of item 2 wherein R¹ is -O-CH₂-heterocycle.
26. The compound of item 2 wherein R¹ is -(C₁-C₆ alkylene)-N(R⁴)₂, where each R⁴ is independently -H or -C₁-C₆ alkyl.
27. The compound of item 2 wherein R¹ is -(C₁-C₆ alkylene)-N(R⁴)₂, where each R⁴ is -(C₁-C₆ alkylene)-O-C₁-C₆ alkyl.
28. The compound of item 2 wherein R⁷ is -H.
29. The compound of item 2 wherein R⁷ is -halo.
30. The compound of item 2 wherein R⁷ is -O-C₁-C₆ alkyl.
31. The compound of item 2 wherein R⁷ is -O-(C₁-C₆ alkylene)-heterocycle.
32. The compound of item 2 wherein R⁸ and R⁹ each -H.
33. The compound of item 2 wherein R₁ is -(C₁-C₆ alkylene)-heterocycle, R⁷ is -O-(C₁-C₆ alkyl), and R⁸ and R⁹ are each -H.
34. The compound of item 2 wherein R¹ is -(C₁-C₆ alkylene)-N(R⁴)₂, R⁷ is -O-(C₁-C₆ alkyl), and R⁸ and R⁹ are each -H.
35. The compound of item 3 wherein R¹ is -H.
36. The compound of item 3 wherein R¹ is -C₁-C₆ alkyl.
37. The compound of item 3 wherein R¹ is -(C₁-C₆ alkylene)-cycloalkyl.
38. The compound of item 3 wherein R¹ is -CH₂-cycloalkyl.
39. The compound of item 38 wherein R¹ is -CH₂-cyclopropyl.
40. The compound of item 38 wherein R¹ is -CH₂-cyclopentyl.
41. The compound of item 3 wherein R¹ is -(C₁-C₆ alkylene)-heterocycle.
42. The compound of item 3 wherein R¹ is -CH₂-heterocycle.
43. The compound of item 3 wherein R⁴ is -O-(C₁-C₆ alkylene)-heterocycle.
44. The compound of item 43 wherein R⁴ is -O-(CH₂)₂-(pyrrolidin-1-yl), -O-(CH₂)₂-(piperidin-1-yl), -O-(CH₂)₃-(piperidin-1-yl), -O-(CH₂)₂-(1-methylpyrrolidin-2(R)-yl), -O-(CH₂)₂-(1-methyl-pyrrolidin-2(S)-yl), -O-(CH₂)₂-(1-ethyl-pyrrolidin-2(R)-yl), -O-(CH₂)₂-(2(S)-methyl-pyrrolidin-1-yl), -O-(CH₂)₂-(2(R)-methyl-pyrrolidin-1-yl), -O-(CH₂)₂-(2-methyl-piperidin-1-yl), -O-(CH₂)₂-(2,6-dimethyl-piperidin-1-yl), -O-(CH₂)₂-(2(S),6(R)-dimethyl-piperidin-1-yl), -O-(CH₂)₃-(2(S),6(R)-dimethyl-piperidin-1-yl), -O-(CH₂)₂-(1,4-dimethyl-piperazin-2-yl), -O-(CH₂)₂-(pyrrolidin-1-yl-2-one), -O-(CH₂)₂-(imidazole-1-yl), -O-CH₂CH(isopropyl)(pyrrolidin-1-yl), -O-(CH₂)₂-(2(S)-methyl-piperidin-1-yl), -O-(CH₂)₃-(2(S)-methyl-piperidin-1-yl), or -O-(CH₂)₂-(azepan-1-yl).
45. The compound of item 3 wherein R⁴ is -O-CH₂-heterocycle.
46. The compound of item 45 wherein R⁴ is -O-CH₂-(1-methylpyrrolidin-2(S)-yl), -O-CH₂-(1-ethyl-pyrrolidin-2(S)-yl), -O-CH₂-(1-methylpyrrolidin-2(R)-yl), -O-CH₂-(3-methyl-3*H-*imidazol-4-yl), -O-CH₂-(pyridin-2-yl), -O-CH₂-(morpholin-1-yl), -O-CH₂-(2-methyl-pyrrolidin-1-yl), -O-CH₂-(1,4-dimethyl-piperazin-2-yl), -O-CH₂-(pyrrolidin-1-yl-2-one), -O-CH₂-(2,2,6,6-tetramethyl-piperidin-1-yl), or -O-CH₂-(1-methyl-piperazin-2-yl).
47. The compound of item 3 wherein R¹ is -H and R⁴ is -O-(C₁-C₆ alkylene)-heterocycle.
48. The compound of item 3 wherein R¹ is -CH₂-heterocycle and R⁴ is -O-(C₁-C₆ alkylene)-heterocycle.
49. The compound of item 3 wherein R¹ is -C₁-C₆ alkyl and R⁴ is -O-(C₁-C₆ alkylene)-heterocycle.
50. The compound of item 3 wherein Z is -CH-, R¹ is -(C₁-C₆ alkylene)-R², R² is -N(R³)₂ and R⁴ is -OCH₃.
51. The compound of item 3 wherein Z is -CH-, R¹ is -C₁-C₆ alkyl and R⁴ is -O-(C₂ alkylene)-N(R⁵)_{2.}
52. The compound of item 4 wherein R₁ is -C₁-C₆ alkyl.
53. The compound of item 4 wherein R₁ is -CH₂-heterocycle.
54. The compound of item 53 wherein R₁ is -CH₂-piperidinyl.
55. The compound of item 4 wherein R₄ is -OCH₃.
56. The compound of item 4 wherein R₄ is -O-(C₁-C₆ alkylene)-heterocycle.
57. The compound of item 56 wherein R₄ is -O-(C₁-C₆ alkylene)-piperidinyl or -O-(C₁-C₆ alkylene)-pyrrolidinyl.
58. The compound of item 4 wherein R₁ is -C₁-C₆ alkyl and R₄ is -O-(C₁-C₆ alkylene)-heterocycle.
59. The compound of item 5 wherein Z is -CH-.
60. The compound of item 5 wherein R¹ is -CN.
61. The compound of item 5 wherein R¹ is -NO₂.
62. The compound of item 5 wherein R¹ is -C(O)NH₂.
63. The compound of item 5 wherein R¹ is -COOH.
64. The compound of item 5 wherein R¹ is -C(O)O-(C₁-C₆ alkyl).
65. The compound of item 5 wherein R³ is -O-(C₁-C₆ alkylene)-heterocycle.
66. The compound of item 2, wherein the compound has the formula as indicated below:

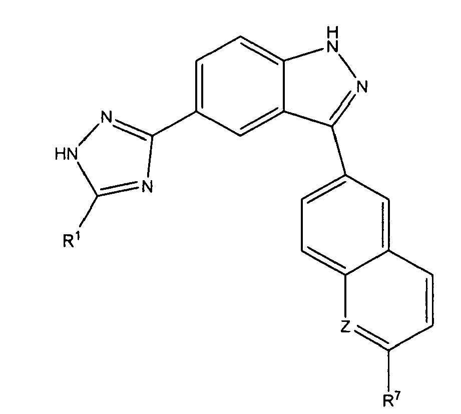

| R¹ | R⁷ | Z |
|---|---|---|
| -H | -OCH₃ | -CH- |
| -H | -OCH₂CH₃ | -CH- |
| -H | -O-n-butyl | -CH- |
| -H | -H | -CH- |
| -Me | -OCH₃ | -CH- |
| -isobutyl | -OCH₃ | -CH- |
| -isobutyl | -H | -CH- |
| -CH₂-pyrrolidin-1-yl | -H | -CH- |
| -CH₂-pyrrolidin-1-yl | -OCH₂CH₃ | -CH- |
| -CH₂-pyrrolidin-1-yl | -OH | -CH- |
| -CH₂-pyrrolidin-1-yl | -C(O)OCH₂CH₃ | -CH- |
| -CH₂-pyrrolidin-1-yl | -OCHF₂ | -CH- |
| -CH₂-pyrrolidin-1-yl | -F | -CH- |
| -CH₂-pyrrolidin-1-yl | -C(O)NHCH₂CH₃ | -CH- |
| -CH₂-pyrrolidin-1-yl | -O(CH₂)₂-CH(CH₃)₂ | -CH- |
| -CH₂-pyrrolidin-1-yl | -OH | -CH- |
| -CH₂-pyrrolidin-1-yl | -OCH₃ | -CH- |
| -CH₂-pyrrolidin-1-yl | -C(O)NH₂ | -CH- |
| -CH₂-piperidin-1-yl | -OCH₃ | -CH- |
| -CH₂-morpholin-1-yl | -OCH₃ | -CH- |
| -OCH₂-(2-methyl-pyrrolidin-1-yl) | -OCH₃ | -CH- |
| -cyclopentyl | -OCH₃ | -CH- |
| -(CH₂)₂-piperidin-1-yl | -OCH₃ | -CH- |
| -CH₂-(1-methyl-piperidin-4-yl) | -OCH₃ | -CH- |
| -CH₂OH | -OCH₃ | -CH- |
| -CH(CH₃)-(pyrrolidin-1-yl) | -OCH₃ | -CH- |
| -CH₂-(2-methyl-piperidin-1-yl | -OCH₃ | -CH- |
| -CH₂-(2,6-dimethyl-piperidin-1-yl | -OCH₃ | -CH- |
| -CH₂-(azepan-1-yl) | -OCH₃ | -CH- |
| -CH₂-(piperazin-1-yl) | -OCH₃ | -CH- |
| -CH2-(1-acetyl-piperazin-4-yl) | -OCH₃ | -CH- |
| -CH₂NH₂ | -OCH₃ | -CH- |
| -CH₂N(CH₃)₂ | -OCH₃ | -CH- |
| -(CH₂)₂NH₂ | -OCH₃ | -CH- |
| -CH₂NH-(t-butyl) | -OCH₃ | -CH- |
| CH₂N(CH₂CH₂OCH₃)₂ | -OCH₃ | -CH- |
| -CH₂-piperidin-1-yl | -OCH₃ | -N- |
| -CH₂-morpholin-1-yl | -H | -N- |
| -CH₂-morpholin-1-yl | -OCH₃ | -N- |
| -CH₂-morpholin-1-yl | -N(CH₃)₂ | -N- |
| -CH₂-morpholin-1-yl | -N(H)(CH₃) | -N- |
| -CH₂-pyrrolidin-1-yl | -OCH₃ | -N- |

or a pharmaceutically acceptable salt or solvate thereof.
67. The compound of item 3, wherein the compound has the formula as indicated below:

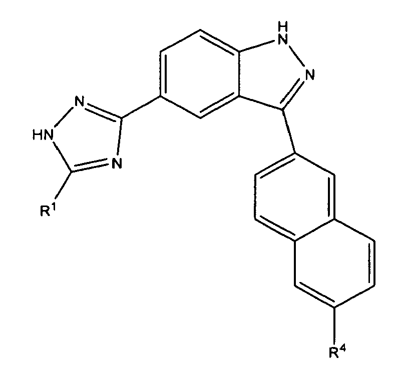

| R¹ | R⁴ |
|---|---|
| -H | -O(CH₂)₂-(pyrrolidin-1-yl) |
| -H | -O(CH₂)₂-(piperidin-1-yl) |
| -Me | -O(CH₂)₂-(azepan-1-yl) |
| -Me | -O(CH₂)₂-(pyrrolidin-1-yl) |
| -Me | -O(CH₂)₂-(2,6-dimethyl-piperidin-1-yl) |
| -CN | -OH |
| -isopropyl | -O(CH₂)₂-(2(S),6(R)-dimethyl-piperidin-1-yl) |
| -isobutyl | -O(CH₂)₂-(piperidin-1-yl) |
| -isobutyl | -O(CH₂)₃-(piperidin-1-yl) |
| -isobutyl | -O(CH₂)₂-(pyrrolidin-1-yl) |
| -isobutyl | -O(CH₂)₂-(azepan-1-yl) |
| -isobutyl | -O(CH₂)₂-(2,6-dimethyl-piperidin-1-yl) |
| -isobutyl | -O(CH₂)₂-(1-methyl-pyrrolidin-2(R)-yl) |
| -isobutyl | -O(CH₂)₂-(2-methyl-piperidin-1-yl) |
| -isobutyl | -O-CH₂-(pyridin-2-yl) |
| -isobutyl | -O(CH₂)₂-(2(S),6(R)-dimethyl-piperidin-1-yl) |
| -isobutyl | -O(CH₂)₃-(2(S),6(R)-dimethyl-piperidin-1-yl) |
| -isobutyl | -OCH₂-(1,4-dimethyl-piperazin-2-yl) |
| -isobutyl | -O(CH₂)₂-(2,2,6,6-tetramethyl-piperidin-1-yl) |
| -isobutyl | -O(CH₂)₂-(2-methyl-1-piperidyl) |
| -isobutyl | -O(CH₂)₂-(2(S)-methyl-1-piperidyl) |
| -isobutyl | -O(CH₂)₂-(2(R)-methyl-1-piperidyl) |
| -isobutyl | -O(CH₂)₂-(2(S)-methyl-pyrrolidin-1-yl) |
| -isobutyl | -O(CH₂)₂-(2(R)-methyl-pyrrolidin-1-yl) |
| -isobutyl | -OCH₂-(1-methyl-pyrrolidin-2(S)-yl) |
| -isobutyl | -OCH₂-(1-methyl-pyrrolidin-2(R)-yl) |
| -isobutyl | -OCH₂-(1-ethyl-pyrrolidin-2(R)-yl) |
| -isobutyl | -OCH₂-(1-ethyl-pyrrolidin-2(S)-yl) |
| -isobutyl | -OCH₂-(2-methyl-pyrrolidin-1-yl-2-one) |
| -isobutyl | -OCH₂-(3-methyl-3*H-*imidazol-4-yl) |
| -isobutyl | -O(CH₂)₂N(CH₃)₂ |
| -isobutyl | -O(CH₂)₂N(isopropyl)₂ |
| -isobutyl | -O(CH₂)₂-(2(R),6(S)-dimethyl-piperidin-1-yl) |
| -isobutyl | -O(CH₂)₃-(2(R),6(S)-dimethyl-piperidin-1-yl) |
| -isobutyl | -O(CH₂)₂-(2,5-dimethyl-pyrrolidin-1-yl) |
| -isobutyl | -O(CH₂)₂-(2(R),5(S)-dimethyl-pyrrolidin-1-yl) |
| -isobutyl | -O(CH₂)₂-(2(S),5(R)-dimethyl-pyrrolidin-1-yl) |
| -isobutyl | -O(CH₂)-C(O)-(2(S),6(R)-dimethyl-piperidin-1-yl) |
| -isobutyl | -O(CH₂)-C(O)-(2(R),6(S)-dimethyl-piperidin-1-yl) |
| -t-butyl | -O(CH₂)₂-(pyrrolidin-1-yl) |
| -t-butyl | -O(CH₂)₂-(azepan-1-yl) |
| -t-butyl | -O(CH₂)₂-(piperidin-1-yl) |
| -t-butyl | -O(CH₂)₂-(2(S),6(R)-dimethyl-piperidin-1 yl) |
| -t-butyl | -OCH₂-(1-methyl-pyrrolidin-2(S)-yl) |
| -t-butyl | -OCH₂-(1-methyl-pyrrolidin-2(R)-yl) |
| -t-butyl | -OCH₂-(1,4-dimethyl-piperazin-2-yl) |
| -t-butyl | -O(CH₂)₂-imidazol-1-yl |
| -neopentyl | -F |
| -neopentyl | -O-(pyridin-2-yl) |
| -neopentyl | -OCH₂-(pyridin-2-yl) |
| -neopentyl | -O(CH₂)₂-(pyridin-3-yl) |
| -neopentyl | -OCH₂-(1,4-dimethyl-piperazin-2-yl) |
| -neopentyl | -O(CH₂)₂-(pyrrolidin-1-yl) |
| -neopentyl | -C(O)NH(CH₂)₂-(pyrrolidin-1-yl) |
| -neopentyl | -OCH₂-(1-methyl-piperidin-2-yl) |
| -neopentyl | -O(CH₂)₂-(2(S),6(R)-dimethyl-piperidin-1-yl) |
| -neopentyl | -O(CH₂)₂-(2(R),6(S)-dimethyl-piperidin-1-yl) |
| -neopentyl | -O(CH₂)-C(O)-(2(R),6(S)-dimethyl-piperidin-1-yl) |
| -neopentyl | -O(CH₂)-C(O)-(2(S),6(R)-dimethyl-piperidin-1-yl) |
| -neopentyl | -OCH₂-(1-methyl-pyrrolidin-2(S)-yl) |
| -neopentyl | -OCH₂-(1-methyl-pyrrolidin-2(R)-yl) |
| -neopentyl | -O(CH₂)₂-(pyrrolidin-1-yl) |
| -neopentyl | -O(CH₂)₂-(2,5-dimethyl-pyrrolidin-1-yl) |
| -neopentyl | -O(CH₂)₂-(2(R),5(S)-dimethyl-pyrrolidin-1-yl) |
| -neopentyl | -O(CH₂)₂-(2(S),5(R)-dimethyl-pyrrolidin-1-yl) |
| -neopentyl | -O-(CH₂)₂-N(ethyl)(isopropyl) |
| -neopentyl | -O(CH₂)₃-(2(S),6(R)-dimethyl-piperidin-1-yl) |
| -neopentyl | -O(CH₂)₃-(2(S),6(R)-dimethyl-piperidin-1-yl) |
| -neopentyl | -OCH₂-(1,4-dimethyl-piperazin-2-yl) |
| -neopentyl | -O(CH₂)₂-(pyrrolidin-1-yl-2-one) |
| -neopentyl | -OCH₂CH(isopropyl)(pyrrolidin-1-yl) |
| -neopentyl | -OCH₂-(1-methyl-piperazin-2-yl) |
| -neopentyl | -O(CH₂)₂-(imidazol-1-yl) |
| -neopentyl | -O(CH₂)₂-(2(S)-methyl-piperidin-1-yl) |
| -neopentyl | -OCH₂-(1-ethyl-pyrrolidin-2(S)-yl) |
| -neopentyl | -OCH₂-(1-ethyl-pyrrolidin-2(R)-yl) |
| -neopentyl | -O(CH₂)₂-(2,2,6,6-tetramethyl-piperidin-1-yl) |
| -neopentyl | -OCH₂-(1-methyl-pyrrolidin-2(R)-yl) |
| -neopentyl | -O(CH₂)₂N(isopropyl)₂ |
| -neopentyl | -O-(CH₂)₂-3-methyl-imidazolidin-2-one |
| -neopentyl | -O(CH₂)₂-(2(S),6(R)-dimethyl-piperidin-1-yl |
| -CH₂-pyrrolidin-1-yl | -Cl |
| -CH₂-pyrrolidin-1-yl | -O(CH₂)₂-(piperidin-1-yl) |
| -CH₂-pyrrolidin-1-yl | -O(CH₂)₂-(pyrrolidin-1-yl) |
| -CH₂-pyrrolidin-1-yl | -O(CH₂)₂-(azepan-1-yl) |
| -CH₂-pyrrolidin-1-yl | -O(CH₂)₂-cyclopentyl |
| -CH₂-pyrrolidin-1-yl | -O(CH₂)₂-(2(S),6(R)-dimethyl-piperidin-1-yl |
| -CH₂-pyrrolidin-1-yl | -O(CH₂)₃-(2(S)-methyl-piperidin-1-yl) |
| -CH₂-pyrrolidin-1-yl | -OCH₂-(1-methyl-pyrrolidin-2(S)-yl) |
| -CH₂-pyrrolidin-1-yl | -OCH₂-(1-methyl-pyrrolidin-2(R)-yl) |
| -CH₂-pyrrolidin-1-yl | -OCH₂-(pyrrolidin-1-yl-2-one) |
| -CH₂-pyrrolidin-1-yl | -O(CH₂)₂-pyrrolidin-1-yl-2-one |
| -CH₂-pyrrolidin-1-yl | -OCH₂-(2-methyl-pyrrolidin-1-yl-2-one) |
| -CH₂-pyrrolidin-1-yl | -C(O)-(pyrrolidin-1-yl) |
| -CH₂-pyrrolidin-1-yl | -OCH₂-(pyridin-2-yl) |
| -CH₂-pyrrolidin-1-yl | -O(CH₂)₂-(pyridin-2-yl) |
| -CH₂-pyrrolidin-1-yl | -O(CH₂)₂-(pyridin-3-yl) |
| -CH₂-pyrrolidin-1-yl | -C(O)-NH₂ |
| -CH₂-pyrrolidin-1-yl | -O(CH₂)₂N(CH₃)₂ |
| -CH₂-pyrrolidin-1-yl | -O(CH₂)₂-(2(S)-methyl-1-piperidyl) |
| -CH₂-pyrrolidin-1-yl | -O(CH₂)₂-(2(R)-methyl-1-piperidyl) |
| -CH₂-morpholin-1-yl | -OCHF₂ |
| -CH₂-morpholin-1-yl | -O(CH₂)₂-(pyrrolidin-1-yl) |
| -CH₂-morpholin-1-yl | -O(CH₂)₂-(piperidin-1-yl) |
| -CH₂-morpholin-1-yl | -OCH₂-(pyridin-2-yl) |
| -CH₂-morpholin-1-yl | -O(CH₂)₂-(pyridin-2-yl) |
| -CH₂-cyclopentyl | -O(CH₂)₂-(2(S),6(R)-dimethyl-piperidin-1-yl |
| -CH₂-cyclopropyl | -O(CH₂)₂-(piperidin-1-yl) |
| -CH₂-cyclopropyl | -O(CH₂)₂-(pyrrolidin-1-yl) |
| -CH₂-cyclopropyl | -O(CH₂)₂-(azepan-1-yl) |
| -CH₂-cyclopropyl | -O(CH₂)₂-(2(S),6(R)-dimethyl-piperidin-1-yl) |
| -CH₂-O-*t*-butyl | -O(CH₂)₂-(2(S),6(R)-dimethyl-piperidin-1-yl) |
| -CH₂-O-*t*-butyl | -O(CH₂)₂-pyrrolidin-1-yl-2-one |
| -CH₂-NH(*t*-butyl) | -OCH₃ |
| -CH₂-N((CH₂)₂-O-CH₃)₂ | -OCH₃ |
| 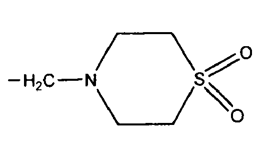 | -OCH₃ |

or a pharmaceutically acceptable salt or solvate thereof.
68. The compound of item 4, wherein the compounds has the formula as indicated below:

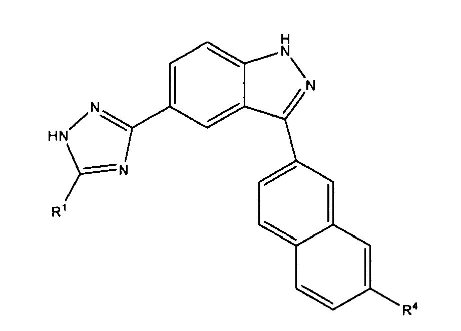

| R¹ | R⁴ |
|---|---|
| -isobutyl | -O(CH₂)₂-(2(S),6(R)-dimethyl-piperidin-1-yl) |
| -isobutyl | -O(CH₂)₂-(2(R),6(S)-dimethyl-piperidin-1-yl) |
| -isobutyl | -O(CH₂)₃-(2(S),6(R)-dimethyl-piperidin-1-yl) |
| -isobutyl | -O(CH₂)₃-(2(R),6(S)-dimethyl-piperidin-1-yl) |
| -isopentyl | -O(CH₂)₂-(2(S),6(R)-dimethyl-piperidin-1-yl) |
| -isopentyl | -O(CH₂)₂-(2(R),6(S)-dimethyl-piperidin-1-yl) |
| -isopentyl | -O(CH₂)₃-(2(S),6(R)-dimethyl-piperidin-1-yl) |
| -isopentyl | -O(CH₂)₃-(2(R),6(S)-dimethyl-piperidin-1-yl) |
| -isopentyl | -O(CH₂)₂-(pyrrolidin-1-yl) |
| -CH₂-piperidin-1-yl | -OCH₃ |
| -CH₂-morpholin-1-yl | -OCH₃ |
| -CH₂-morpholin-1-yl | -H |
| -CH₂-pyrrolidin-1-yl | -OCH₃ |

or a pharmaceutically acceptable salt thereof.
69. The compound of item 5, wherein the compound has the formula as indicated below:

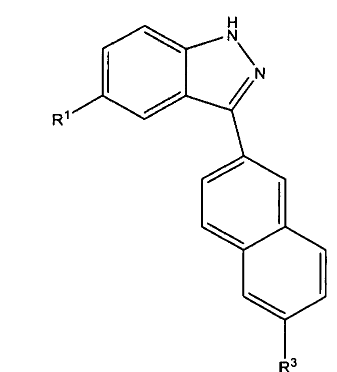

| R¹ | R³ |
|---|---|
| -CN | -O(CH₂)₂-(1-pyrrolidinyl) |
| -C(O)NH₂ | -OCH₂-(1-methyl-2-(R)-pyrrolidinyl) |
| -C(O)NH₂ | -OCH₂-(1-methyl-2-(S)-pyrrolidinyl) |
| -CH=CH-(1,2,4-triazol-3-yl-methylmorpholin-1-yl | -OCH₃ |
| -C(O)NH(3,3-dimethylbutane) | -O-(CH₂)₂-N(isopropyl)₂ |
| -C(O)NH-CH₂-cyclopropyl | -OCH₂-(1-ethyl-pyrrolidin-2(S)-yl) |
| -C(O)NH-CH₂-cyclopropyl | -OCH₂-(1-ethyl-pyrrolidin-2(R)-yl) |
| -C(O)NH(butyl) | -OCH₂-(1-ethyl-pyrrolidin-2(S)-yl) |
| -C(O)NH(butyl) | -OCH₂-(1-ethyl-pyrrolidin-2(R)-yl) |
| -C(O)NH(isobutyl) | -OCH₂-(1-ethyl-pyrrolidin-2(S)-yl) |
| -C(O)NH(isobutyl) | -OCH₂-(1-ethyl-pyrrolidin-2(R)-yl) |
| -C(O)NH(t-butyl) | -OCH₂-(1-ethyl-pyrrolidin-2(S)-yl) |
| -C(O)NH(t-butyl) | -OCH₂-(1-ethyl-pyrrolidin-2(R)-yl) |
| -C(O)NH(sec-butyl) | -OCH₂-(1-ethyl-pyrrolidin-2(S)-yl) |
| -C(O)NH(sec-butyl) | -OCH₂-(1-ethyl-pyrrolidin-2(R)-yl) |
| -C(O)NH(isopentyl) | -O-(CH₂)₂-N(isopropyl)₂ |
| -C(O)NH(isopentyl) | -OCH₂-(-ethyl-pyrrolidin-2(S)-yl) |
| -C(O)NH(isopentyl) | -OCH₂-(-ethyl-pyrrolidin-2(R)-yl) |
| -C(O)NH(ethyl) | -OCH₃ |
| -C(O)NH((CH₂)₂-morpholin-1-yl) | -OCH₃ |
| -C(O)NH((CH₂)₂-pyrrolidin-1-yl) | -OCH₃ |
| -C(O)NH((CH₂)₂-pyrrolidin-1-yl) | -OCH₂-(1-methyl-pyrrolidin-2(S)-yl) |
| -C(O)NH((CH₂)₂-pyrrolidin-1-yl) | -OCH₂-(1-methyl-pyrrolidin-2(R)-yl) |
| -C(O)NH((CH₂)₂OCH₃) | -OCH₃ |
| -C(O)NH((CH₂)₂OCH₃) | -OCH₂-(1-methyl-pyrrolidin-2(S)-yl) |
| -C(O)NH((CH₂)₂OCH₃) | -OCH₂-(1-methyl-pyrrolidin-2(R)-yl) |
| -C(O)-pyrrolidin-1-yl | -OCH₂-(1-methyl-pyrrolidin-2(S)-yl) |
| -C(O)-pyrrolidin-1-yl | -OCH₂-(1-methyl-pyrrolidin-2(R)-yl) |
| -C(O)NH((CH₂)₂N(CH₃)₂) | -OCH₂-(1-methyl-pyrrolidin-2(S)-yl) |
| -C(O)NH((CH₂)₂OCH₃) | -OCH₂-(1-methyl-pyrrolidin-2(R)-yl) |

or a pharmaceutically acceptable salt thereof.
70. A composition comprising the compound or pharmaceutically acceptable salt of the compound of item 1 and a pharmaceutically acceptable carrier or diluent.
71. A composition comprising the compound or pharmaceutically acceptable salt of the compound of item 2 and a pharmaceutically acceptable carrier or diluent.
72. A composition comprising the compound or pharmaceutically acceptable salt of the compound of item 3 and a pharmaceutically acceptable carrier or diluent.
73. A composition comprising the compound or pharmaceutically acceptable salt of the compound of item 5 and a pharmaceutically acceptable carrier or diluent.
74. A composition comprising the compound or pharmaceutically acceptable salt of the compound of item 66 and a pharmaceutically acceptable carrier or diluent.
75. A composition comprising the compound or pharmaceutically acceptable salt of the compound of item 67 and a pharmaceutically acceptable carrier or diluent.
76. A composition comprising the compound or pharmaceutically acceptable salt of the compound of item 69 and a pharmaceutically acceptable carrier or diluent.
77. A method for modulating protein kinase signal transduction in or between cells, comprising administering to a patient in need thereof a therapeutically effective amount of the compound of any one of items 1, 2, 3 or 5, or a pharmaceutically acceptable salt thereof.
78. The method of item 77 wherein the type of modulation is inhibition.
79. The method of item 77 wherein the protein kinase is ABL, AKT1, AKT2, AMPK, Aurora A, Aurora-B, Blk, CaMKII, CaMKIV, CDK1/B, CDK2/A, CDK2/E, CDK3/E, CDK5/p35, CDK6/D3, CDK7/H/MAT1, CHK1, CHK2, CK2, CSK, ERK, EGFR, Fes, FGFR3, Fyn, GSK3β, IGF-1R, IKKα, IKKβ, IKK1, IKK2EE, IR, IRTK, JNK1α1, JNK2α2, JNK3, Lck, Lyn, MAPK1, MAPK2, MAPKAP-K2, MEK1, MKK3, MKK4, MKK6, MKK7, MKK7β, MSK1, p38α, p38β, p70S6K, PAK2, PDGGRα, PDK1, PKA, PKBα, PKBβ, PKCα, PKCε, PKCγ, PKCθ, PKCβII, PKA, PRAK, PRK2, c-RAF, ROCK-II, Rsk1, Rsk2, Rsk3, SAPK2a, SAPK2b, SAPK3, SAPK4, SGK, c-SRC, Syk, Yes, or ZAP-70.
80. A method for modulating the activity of one or more protein kinases, comprising administering to a patient in need thereof a therapeutically effective amount of the compound of any one of items 1, 2, 3 or 5, or a pharmaceutically acceptable salt thereof.
81. The method of item 80 wherein the type of modulation is inhibition.
82. The method of item 80 wherein the protein kinase is ABL, AKT1, AKT2, AMPK, Aurora-A, Aurora-B, Blk, CaMKII, CaMKIV, CDK1/B, CDK2/A, CDK2/E, CDK3/E, CDK5/p35, CDK6/D3, CDK7/H/MAT1, CHK1, CHK2, CK2, CSK, ERK, EGFR, Fes, FGFR3, Fyn, GSK3β, IGF-1R, IKKα, IKKβ, IKK1, IKK2EE, IR, IRTK, JNK1α1, JNK2α2, JNK3, Lck, Lyn, MAPK1, MAPK2, MAPKAP-K2, MEK1, MKK3, MKK4, MKK6, MKK7, MKK7β, MSK1, p38α, p38β, p70S6K, PAK2, PDGGRα, PDK1, PKA, PKBα, PKBβ, PKCα, PKCε, PKCγ, PKCθ, PKCβII, PKA, PRAK, PRK2, c-RAF, ROCK-II, Rsk1, Rsk2, Rsk3, SAPK2a, SAPK2b, SAPK3, SAPK4, SGK, c-SRC, Syk, Yes, or ZAP-70.
83. A method for treating or preventing a proliferative disorder, comprising administering to a patient in need thereof a therapeutically effective amount of the compound of item 1 or a pharmaceutically acceptable salt thereof.
84. A method for treating or preventing a proliferative disorder, comprising administering to a patient in need thereof a therapeutically effective amount of the compound of item 2 or a pharmaceutically acceptable salt thereof.
85. A method for treating or preventing a proliferative disorder, comprising administering to a patient in need thereof a therapeutically effective amount of the compound of item 3 or a pharmaceutically acceptable salt thereof.
86. A method for treating or preventing a proliferative disorder, comprising administering to a patient in need thereof a therapeutically effective amount of the compound of item 5 or a pharmaceutically acceptable salt thereof.
87. The method of any one of items 83-86 wherein the treating or preventing comprises the *in vivo* inhibition of one or more protein kinases.
88. The method of any one of items 83-86, wherein the proliferative disorder is benign prostatic hyperplasia, familial adenomatosis polyposis, neuro-fibromatosis, atherosclerosis, pulmonary fibrosis, arthritis, psoriasis, glomerulonephritis, restenosis following angioplasty or vascular surgery, hypertrophic scar formation, inflammatory bowel disease, transplantation rejection, endotoxic shock, fungal infections, or a defective apoptosis-associated condition.
89. A method for treating or preventing cancer, comprising administering to a patient in need thereof a therapeutically effective amount of the compound of item 1 or a pharmaceutically acceptable salt thereof.
90. A method for treating or preventing cancer, comprising administering to a patient in need thereof a therapeutically effective amount of the compound of item 2 or a pharmaceutically acceptable salt thereof.
91. A method for treating or preventing cancer, comprising administering to a patient in need thereof a therapeutically effective amount of the compound of item 3 or a pharmaceutically acceptable salt thereof.
92. A method for treating or preventing cancer, comprising administering to a patient in need thereof a therapeutically effective amount of the compound of item 5 or a pharmaceutically acceptable salt thereof.
93. The method of any one of items 89-92 wherein the treating or preventing comprises the *in* vivo inhibition of one or more protein kinases.
94. The method of any one of items 89-92, wherein the cancer is lung cancer, breast cancer, colorectal cancer, prostate cancer, brain cancer, esophageal cancer, pancreatic cancer, stomach cancer, liver cancer, kidney cancer, adrenal cancer, testicular cancer, ovarian cancer, cervical cancer, leukemia, Hodgkin's disease, non-Hodgkin's lymphoma, skin cancer, bone cancer, a cancer of the central nervous system, or a cancer of the blood or lymphatic system.
95. The method of any one of items 89-92 comprising the administration of an additional anticancer agent.
96. A method for treating or preventing a neurological disorder, comprising administering to a patient in need thereof a therapeutically effective amount of the compound of item 1 or a pharmaceutically acceptable salt thereof.
97. A method for treating or preventing a neurological disorder, comprising administering to a patient in need thereof a therapeutically effective amount of the compound of item 2 or a pharmaceutically acceptable salt thereof.
98. A method for treating or preventing a neurological disorder, comprising administering to a patient in need thereof a therapeutically effective amount of the compound of item 3 or a pharmaceutically acceptable salt thereof.
99. A method for treating or preventing a neurological disorder, comprising administering to a patient in need thereof a therapeutically effective amount of the compound of item 5 or a pharmaceutically acceptable salt thereof.
100. The method of any one of items 96-99 wherein the treating or preventing comprises the *in vivo* inhibition of one or more protein kinases.
101. The method of any one of items 96-99 wherein the neurological disorder is stroke, ischemia, trauma- induced cerebral edema, hypoxia-induced cerebral edema, ocular edema, macular edema, or brain-tumor associated cerebral edema.
102. A prodrug of the compound of item 1 where the prodrug is a biohydrolyzable amide, a biohydrolyzable ester, a biohydrolyzable carbamate, a biohydrolyzable carbonate, or a biohydrolyzable ureide.
103. A prodrug of the compound of item 2 where the prodrug is a biohydrolyzable amide, a biohydrolyzable ester, a biohydrolyzable carbamate, a biohydrolyzable carbonate, or a biohydrolyzable ureide.
104. A prodrug of the compound of item 5 where the prodrug is a biohydrolyzable amide or a biohydrolyzable ester.
105. The method of any one of items 83-86, 89-92, or 96-99 wherein the patient is a human.
106. The compound of any one of items 1, 2, 3, or 5 that is in isolated and purified form.

## Claims

1. A compound having the formula: 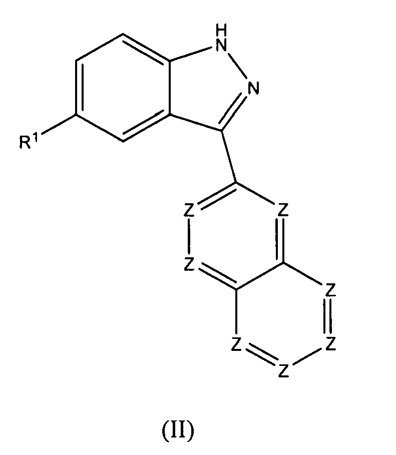 and tautomers and pharmaceutically acceptable salts, solvates and hydrates thereof,
wherein:
R¹ is -COOR², -CN, -C(O)-heterocycle, -C(O)N(R²)₂, or -C(O)NHR²;
each occurrence of R² is independently -C₁-C₆ alkyl, -hydroxyalkyl, -C₁-C₆ alkyl-N(R²)₂, -C₁-C₆ alkyl-O-C₁-C₆ alkyl, -(CH₂)ₙ-cycloalkyl, -(CH₂)ₙ-heteroaryl, -(CH₂)ₙ-heterocycle, or -(CH₂)ₙ-aryl;
each occurrence of Z is -CH-, -C(R³)- or -N-, wherein up to 3 occurrences of Z can be -N-;
R³ is -COOR^{2'}, -CN, -NO₂, -C(O)N(R^{2'})₂, N(R^{2'})₂, -C(O)O-(C₁-C₆ alkyl), -C(O)NH-(CH₂)ₙ-heterocycle, -C(O)NH-(CH₂)ₙ-heteroaryl, -C(O)-heterocycle, -C(O)-heteroaryl, -(CH₂)ₙ-heterocycle, -(CH₂)ₙ-heteroaryl, -(CH₂)ₚ-cycloalkyl, -O-(CH₂)ₙ- N(R^{2'})₂, -O-(CH₂)ₙ-heterocycle, -O-(CH₂)ₙ-heteroaryl, or -O-(CH₂)ₙ-cycloalkyl;
each occurrence of R^{2'} is independently -H, -C₁-C₆ alkyl, -hydroxyalkyl, -C₁-C₆ alkyl-N(R²)₂, -C₁-C₆ alkyl-O-C₁-C₆ alkyl, -(CH₂)ₙ-cycloalkyl, -(CH₂)ₙ-heteroaryl, -(CH₂)ₙ-heterocycle, or -(CH₂)ₙ-aryl;
m is 0 or 1;
n is an integer ranging from 0 to 3; and
p is an integer ranging from 1 to 3.

2. The compound of claim 1 wherein Z is -CH-.

3. The compound of claim 1 wherein R¹ is -CN.

4. The compound of claim 1 wherein R¹ is -C(O)O-(C₁-C₆ alkyl).

5. The compound of claim 1 wherein R³ is -O-(C₁-C₆ alkylene)-heterocycle.

6. The compound of claim 1, wherein the compound has the formula as indicated below: 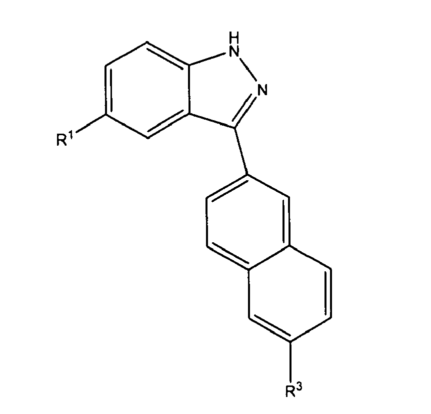
| R¹ | R³ |
|---|---|
| -CN | -O(CH₂)₂-(1-pyrrolidinyl) |
| -C(O)NH(3,3-dimethylbutane) | -O-(CH₂)₂-N(isopropyl)₂ |
| -C(O)NH-CH₂-cyclopropyl | -OCH₂-(1-ethyl-pyrrolidin-2(S)-yl) |
| -C(O)NH-CH₂-cyclopropyl | -OCH₂-(1-ethyl-pyrrolidin-2(R)-yl) |
| -C(O)NH(butyl) | -OCH₂-(1-ethyl-pyrrolidin-2(S)-yl) |
| -C(O)NH(butyl) | -OCH₂-(1-ethyl-pyrrolidin-2(R)-yl) |
| -C(O)NH(isobutyl) | -OCH₂-(-ethyl-pyrrolidin-2(S)-yl) |
| -C(O)NH(isobutyl) | -OCH₂-(-ethyl-pyrrolidin-2(R)-yl) |
| -C(O)NH(t-butyl) | -OCH₂-(1-ethyl-pyrrolidin-2(S)-yl) |
| -C(O)NH(t-butyl) | -OCH₂-(1-ethyl-pyrrolidin-2(R)-yl) |
| -C(O)NH(sec-butyl) | -OCH₂-(1-ethyl-pyrrolidin-2(S)-yl) |
| -C(O)NH(sec-butyl) | -OCH₂-(1-ethyl-pyrrolidin-2(R)-yl) |
| -C(O)NH(isopentyl) | -O-(CH₂)₂-N(isopropyl)₂ |
| -C(O)NH(isopentyl) | -OCH₂-(1-ethyl-pyrrolidin-2(S)-yl) |
| -C(O)NH(isopentyl) | -OCH₂-(1-ethyl-pyrrolidin-2(R)-yl) |
| -C(O)NH(ethyl) | -OCH₃ |
| -C(O)NH((CH₂)₂-morpholin-1-yl) | -OCH₃ |
| -C(O)NH((CH₂)₂-pyrrolidin-1-yl) | -OCH₃ |
| -C(O)NH((CH₂)₂-pyrrolidin-1-yl) | -OCH₂-(1-methyl-pyrrolidin-2(S)-yl) |
| -C(O)NH((CH₂)₂-pyrrolidin-1-yl) | -OCH₂-(1-methyl-pyrrolidin-2(R)-yl) |
| -C(O)NH((CH₂)₂OCH₃) | -OCH₃ |
| -C(O)NH((CH₂)₂OCH₃) | -OCH₂-(1-methyl-pyrrolidin-2(S)-yl) |
| -C(O)NH((CH₂)₂OCH₃) | -OCH₂-(1-methyl-pyrrolidin-2(R)-yl) |
| -C(O)-pyrrolidin-1-yl | -OCH₂-(1-methyl-pyrrolidin-2(S)-yl) |
| -C(O)-pyrrolidin-1-yl | -OCH₂-(1-methyl-pyrrolidin-2(R)-yl) |
| -C(O)NH((CH₂)₂N(CH₃)₂) | -OCH₂-(1-methyl-pyrrolidin-2(S)-yl) |
| -C(O)NH((CH₂)₂OCH₃) | -OCH₂-(1-methyl-pyrrolidin-2(R)-yl) |
or a pharmaceutically acceptable salt thereof.

7. A composition comprising the compound or pharmaceutically acceptable salt of the compound of any one of claims 1 to 6 and a pharmaceutically acceptable carrier or diluent.

8. The compound of any one of claims 1 to 6, or a pharmaceutically acceptable salt thereof for modulating protein kinase signal transduction in or between cells.

9. The compound of any one of claims 1 to 6, or a pharmaceutically acceptable salt thereof for modulating the activity of one or more protein kinases.

10. The compound of claim 8 or 9 wherein the type of modulation is inhibition.

11. The compound of claim 8 or 9 wherein the protein kinase is ABL, AKT1, AKT2, AMPK, Aurora-A, Aurora-B, Blk, CaMKII, CaMKIV, CDK1/B, CDK2/A, CDK2/E, CDK3/E, CDK5/p35, CDK6/D3, CDK7/H/MAT1, CHK1, CHK2, CK2, CSK, ERK, EGFR, Fes, FGFR3, Fyn, GSK3β, IGF-1R, IKKα, IKKβ, IKKI, IKK2EE, IR, IRTK, JNK1α1, JNK2α2, JNK3, Lck, Lyn, MAPK1, MAPK2, MAPKAP-K2, MEK1, MKK3, MKK4, MKK6, MKK7, MKK7β, MSKI, p38α, p38β, p70S6K, PAK2, PDGGRα, PDK1, PKA, PKBα, PKBβ, PKCα, PUCE, PKCγ, PKCθ, PKCβII, PKA, PRAK, PRK2, c-RAF, ROCK-II, Rsk1, Rsk2, Rsk3, SAPK2a, SAPK2b, SAPK3, SAPK4, SGK, c-SRC, Syk, Yes, or ZAP-70.

12. The compound of any one of claims 1 to 6 or a pharmaceutically acceptable salt thereof for treating or preventing a proliferative disorder.

13. The compound of claim 12, wherein the proliferative disorder is benign prostatic hyperplasia, familial adenomatosis polyposis, neuro-fibromatosis, atherosclerosis, pulmonary fibrosis, arthritis, psoriasis, glomerulonephritis, restenosis following angioplasty or vascular surgery, hypertrophic scar formation, inflammatory bowel disease, transplantation rejection, endotoxic shock, fungal infections, or a defective apoptosis-associated condition.

14. The compound of any one of claims 1 to 6 or a pharmaceutically acceptable salt thereof for treating or preventing cancer.

15. The compound of claim 14, wherein the cancer is lung cancer, breast cancer, colorectal cancer, prostate cancer, brain cancer, esophageal cancer, pancreatic cancer, stomach cancer, liver cancer, kidney cancer, adrenal cancer, testicular cancer, ovarian cancer, cervical cancer, leukemia, Hodgkin's disease, non-Hodgkin's lymphoma, skin cancer, bone cancer, a cancer of the central nervous system, or a cancer of the blood or lymphatic system.

16. The compound of claim 14 which is prepared to be administered with an additional anticancer agent.

17. The compound of any one of claims 1 to 6 or a pharmaceutically acceptable salt thereof for treating or preventing a neurological disorder.

18. The compound of claim 17 wherein the neurological disorder is stroke, ischemia, trauma- induced cerebral edema, hypoxia-induced cerebral edema, ocular edema, macular edema, or brain-tumor associated cerebral edema.

19. The compound of any one of claims 12, 14 and 17 wherein the treating or preventing comprises *the in vivo* inhibition of one or more protein kinases.

20. The compound of any one of claims 8 to 19 wherein the patient is a human.

21. The compound of any one of claims 1 to 6 that is in isolated and purified form.
